# EUROPEAN PATENT APPLICATION

(11) **EP 4 834 893 A1**
(43) Date of publication of application: **04.11.2026**
(21) Application number: 24912951.1
(22) Date of filing: 25.12.2024
(51) Int. Cl.: C07D 333/24, C07D 409/06, C07D 409/14, C07D 413/06, C07D 413/14, C07D 495/04, G02F 1/061

(54) **COMPOUND, PRECURSOR, USE OF PRECURSOR, PRODUCTION METHOD, ELECTRO-OPTIC MATERIAL, ELECTRO-OPTIC FILM, AND ELECTRO-OPTIC ELEMENT**

(30) Priority: 26.12.2023 JP 2023219521; 26.12.2023 JP 2023219474; 26.12.2023 JP 2023219370
(71) Applicant: Idemitsu Kosan Co.,Ltd., Tokyo 100-8321 (JP)
(72) Inventor: ONODERA Shingo, Tokyo 100-8321 (JP); HASHIMOTO Shingo, Tokyo 100-8321 (JP); HARADA Yosuke, Tokyo 100-8321 (JP); YASUKAWA Keiichi, Tokyo 100-8321 (JP); IIDA Maiko, Tokyo 100-8321 (JP); FUKAMI Takuto, Tokyo 100-8321 (JP); SHIOMI Takushi, Tokyo 100-8321 (JP); ISHII Hirotoshi, Tokyo 100-8321 (JP); FURUKAWA Masaki, Tokyo 100-8321 (JP); OUCHI Seiya, Tokyo 100-8321 (JP)
(74) Representative: Gille Hrabal Partnerschaftsgesellschaft mbB Patentanwälte
(86) International application number: PCT/JP2024/046027
(87) International publication number: WO 2025/143065

(57) **Abstract**

A compound has a donor moiety, a π-conjugated moiety, and an acceptor moiety, and satisfies at least one selected from the group consisting of conditions (I), (II), and (III) below, Condition (I): the donor moiety has a specific structure represented by a formula (D1) or a formula (D2), Condition (II): the acceptor moiety has a specific structure represented by a formula (A1) or a formula (A2), and Condition (III): the π-conjugated moiety has a specific structure represented by a formula (P1), a formula (P2), or a formula (P3).

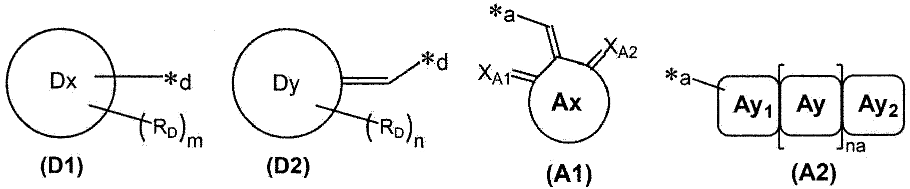

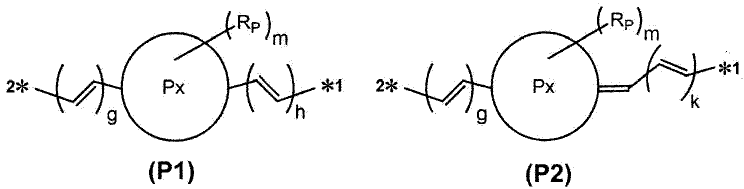

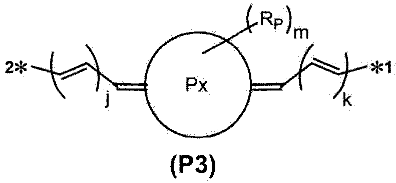

## Description

### TECHNICAL FIELD

The present invention relates to a compound, a precursor, use of the precursor, a production method, an electro-optic material, an electro-optic film, and an electro-optic device.

### BACKGROUND ART

Electro-optic materials are key materials for optical modulators that convert electrical signals into optical signals, for example, within optical transceivers. In recent years, research has progressed on organic materials that can function as electro-optic materials. Among organic compounds applicable to electro-optic materials, electro-optic compounds have attracted attention. Organic materials used as electro-optic materials enable electrical-to-optical conversion (conversion of electrical signals into optical signals) at high speed and with low power consumption, and are therefore expected to contribute, for example, to high-speed, large-capacity communications and to power saving in data centers.

Electro-optic materials include chromophore moieties that respond to an electric field (referred to as EO molecules). EO is an abbreviation for Electro-Optic. An EO molecule is an electro-optic compound having a donor moiety, a π-conjugated moiety, and an acceptor moiety. An electro-optic material can be obtained, for example, by dispersing EO molecules as electro-optic compounds in a polymer serving as a host material, or by bonding EO molecules as electro-optic compounds to a main chain of a polymer serving as a host material.

For example, Patent Literature 1 and Patent Literature 2 disclose compounds applicable to electro-optic materials, the compounds including a donor moiety having an amino group, a conjugated moiety having carbon-carbon conjugated π bonds, and an acceptor moiety not containing -SO₂-.

### CITATION LIST

### PATENT LITERATURE(S)

Patent Literature 1: JP 2016-006079 A
Patent Literature 2: JP 2022-097407 A

### SUMMARY OF THE INVENTION

### PROBLEM(S) TO BE SOLVED BY THE INVENTION

Electrical-to-optical conversion is achieved, for example, by applying an electro-optic effect. The electro-optic effect is a phenomenon in which the refractive index of a material changes when a voltage is applied to the material. Due to high speed and low power consumption of the electro-optic effect, applying the electro-optic effect enables electro-optic conversion to be achieved at high speed and with low power consumption. The electro-optic effect is attributed to electronic polarization of EO molecules, and in order to achieve higher speed and lower power consumption, it is desirable that the EO molecules exhibit a larger electro-optic effect. Accordingly, EO molecules as electro-optic compounds are required to have a large hyperpolarizability.

The invention provides a novel compound applicable to an electro-optic material, a precursor, a production method using the precursor, an electro-optic material containing the compound, an electro-optic film including the compound or the electro-optic material, and an electro-optic device including the compound or the electro-optic material.

### MEANS FOR SOLVING THE PROBLEM(S)

As a result of intensive studies in view of the above issues, the inventors have found that, in a compound having a donor moiety, a π-conjugated moiety, and an acceptor moiety, introduction of a group having a predetermined novel structure into the donor moiety allows the compound to be applied to an electro-optic material, and have thus completed the invention.

Further, as a result of intensive studies in view of the above issues, the inventors have found that a compound having a donor moiety, a π-conjugated moiety, and an acceptor moiety can be applied to an electro-optic material by introducing a group having a predetermined novel structure into the acceptor moiety of the compound, thereby completing the invention.

Further, as a result of intensive studies in view of the above issues, the inventors have found that a compound having a donor moiety, a π-conjugated moiety, and an acceptor moiety can be applied to an electro-optic material by introducing a group having a predetermined novel structure into the π-conjugated moiety of the compound, thereby completing the invention.

That is, the gist of the invention is as follows.
[1] A compound having a donor moiety, a π-conjugated moiety, and an acceptor moiety, in which the compound satisfies at least one selected from the group consisting of conditions (I), (II), and (III) below,
   Condition (I): the donor moiety is represented by a formula (D1) or a formula (D2) below,
   Condition (II): the acceptor moiety is represented by a formula (A1) or a formula (A2) below, and
   Condition (III): the π-conjugated moiety is represented by a formula (P1), a formula (P2), or a formula (P3) below. 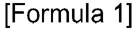 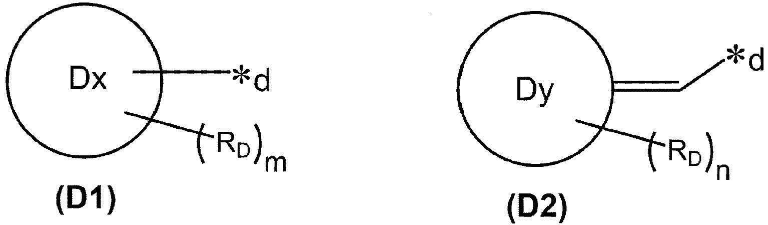
   In the formulae (D1) and (D2):
      a ring Dx and a ring Dy are each independently a five-membered or six-membered aromatic hydrocarbon ring, a five-membered or six-membered aliphatic hydrocarbon ring, or a five-membered or six-membered heterocycle;
      the ring Dx has one or more substituents R_{D};
      the ring Dy has one or more substituents R_{D} or has no substituent R_{D};
      m is the number of the substituents R_{D} on the ring Dx and is an integer of 1 or more;
      n is the number of the substituents R_{D} on the ring Dy and is 0 or an integer of 1 or more;
      the substituents R_{D} on the ring Dx are bonded with the ring Dx to form a substituted or unsubstituted monocyclic ring, bonded with the ring Dx to form a substituted or unsubstituted fused ring, or not bonded with the ring Dx;
      the substituents R_{D} on the ring Dy are bonded with the ring Dy to form a substituted or unsubstituted monocyclic ring, bonded with the ring Dy to form a substituted or unsubstituted fused ring, or not bonded with the ring Dy;
      when the ring Dx has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
      when the ring Dy has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
      the substituents R_{D} on the ring Dx and the ring Dy are each independently a group represented by -N(R₁₀₁)(R₁₀₂), a group represented by -Si(R₁₀₃)(R₁₀₄)(R₁₀₅), a group represented by -C(=O)R₁₀₆, a group represented by -C(R₁₀₇)(R₁₀₈)(R₁₀₉), a group represented by -O(R₁₁₃), a substituted or unsubstituted alkyl group, a substituted or unsubstituted cycloalkyl group, a substituted or unsubstituted aryl group, or a substituted or unsubstituted heterocyclic group;
      R₁₀₁, R₁₀₂, R₁₀₃, R₁₀₄, R₁₀₅, R₁₀₆, R₁₀₇, R₁₀₈, R₁₀₉, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
      when the number of substituents R_{D} on the ring Dx and the ring Dy is 1 and the one substituent R_{D} is a group represented by -N(R₁₀₁)(R₁₀₂), R₁₀₁ and R₁₀₂ are neither hydrogen atoms nor substituted or unsubstituted alkyl groups;
      when the number of the substituents R_{D} on the ring Dx is 2 and one of the two substituents R_{D} is a group represented by -N(R₁₀₁)(R₁₀₂), the other thereof is not a group represented by -O(R₁₁₃);
      when a plurality of R₁₀₁ are present, the plurality of R₁₀₁ are mutually the same or different;
      when a plurality of R₁₀₂ are present, the plurality of R₁₀₂ are mutually the same or different;
      when a plurality of R₁₀₃ are present, the plurality of R₁₀₃ are mutually the same or different;
      when a plurality of R₁₀₄ are present, the plurality of R₁₀₄ are mutually the same or different;
      when a plurality of R₁₀₅ are present, the plurality of R₁₀₅ are mutually the same or different;
      when a plurality of R₁₀₆ are present, the plurality of R₁₀₆ are mutually the same or different;
      when a plurality of R₁₀₇ are present, the plurality of R₁₀₇ are mutually the same or different;
      when a plurality of R₁₀₈ are present, the plurality of R₁₀₈ are mutually the same or different;
      when a plurality of R₁₀₉ are present, the plurality of R₁₀₉ are mutually the same or different;
      when a plurality of R₁₁₃ are present, the plurality of R₁₁₃ are mutually the same or different; and
      *d is a bonding position. 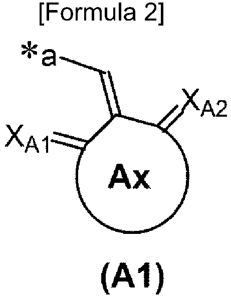
      In the formula (A1):
         X_{A1} and X_{A2} are each independently an oxygen atom (O) or C(CN)₂;
         when one of X_{A1} and X_{A2} is O and the other of X_{A1} and X_{A2} is C(CN)₂, the ring to which X_{A1} and X_{A2} are directly bonded is not a five-membered ring;
         the ring Ax is a substituted or unsubstituted monocyclic aromatic hydrocarbon group, a substituted or unsubstituted polycyclic aromatic hydrocarbon ring, a substituted or unsubstituted monocyclic aliphatic hydrocarbon ring, a substituted or unsubstituted polycyclic aliphatic hydrocarbon ring, a substituted or unsubstituted monocyclic heterocycle, or a substituted or unsubstituted polycyclic heterocycle; and
         *a represents a bonding position. 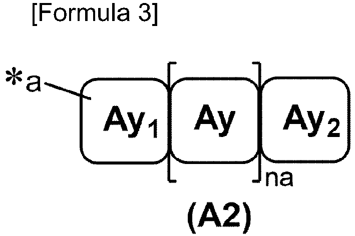
         In the formula (A2):
            na represents the number of rings Ay, and na is 0 or an integer of 1 or more;
            the rings Ay, a ring Ay₁ and a ring Ay₂ are each independently a substituted or unsubstituted monocyclic aromatic hydrocarbon group, a substituted or unsubstituted monocyclic aliphatic hydrocarbon ring, or a substituted or unsubstituted monocyclic heterocycle;
            when na is 0, the ring Ay₁ and the ring Ay₂ are fused to each other;
            when na is an integer of 1 or more, the ring Ay, the ring Ay₁, and the ring Ay₂ are each independently fused to an adjacent ring;
            when na is an integer of 2 or more, the ring Ay₁, the ring Ay₂, and two or more rings Ay include a fused ring structure in which the rings are fused in series to adjacent rings or a fused ring structure in which the rings are fused in a branched manner to adjacent rings; and
            *a represents a bonding position. 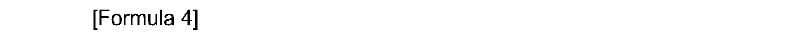 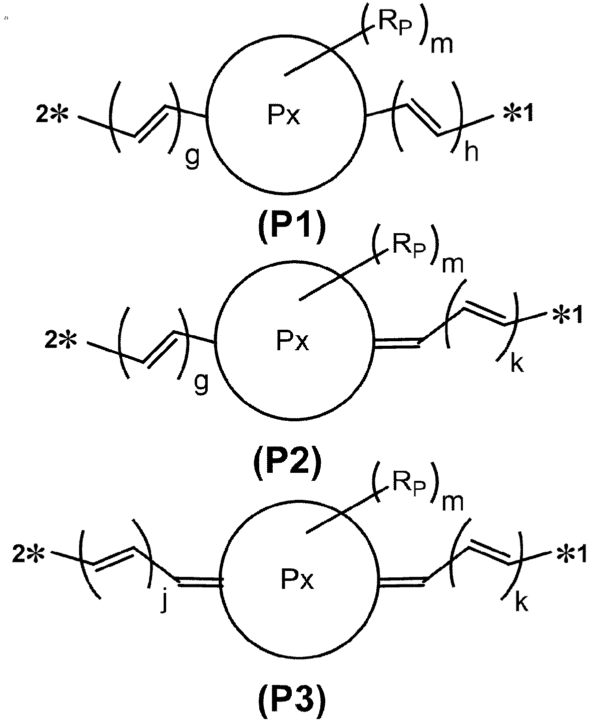
            In the formulae (P1), (P2), and (P3):
               the ring Px is a monocyclic aromatic hydrocarbon ring having one or more substituents R_{P}, a substituted or unsubstituted polycyclic aromatic hydrocarbon ring, a monocyclic aliphatic hydrocarbon ring having one or more substituents R_{P}, a substituted or unsubstituted polycyclic aliphatic hydrocarbon ring, a substituted or unsubstituted monocyclic heterocycle, or a substituted or unsubstituted polycyclic heterocycle;
               m is the number of substituents R_{P} and is 0 or an integer of 1 or more;
               a substituent R_{P} is a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
               R₁₀₁, R₁₀₂, R₁₀₃, R₁₀₄, R₁₀₅, R₁₀₆, R₁₀₇, R₁₀₈, and R₁₀₉ are each independently a hydrogen atom, a substituted or unsubstituted alkyl group, a substituted or unsubstituted cycloalkyl group, a substituted or unsubstituted aryl group, or a substituted or unsubstituted heterocyclic group;
               when a plurality of R₁₀₁ are present, the plurality of R₁₀₁ are mutually the same or different;
               when a plurality of R₁₀₂ are present, the plurality of R₁₀₂ are mutually the same or different;
               when a plurality of R₁₀₃ are present, the plurality of R₁₀₃ are mutually the same or different;
               when a plurality of R₁₀₄ are present, the plurality of R₁₀₄ are mutually the same or different;
               when a plurality of R₁₀₅ are present, the plurality of R₁₀₅ are mutually the same or different;
               when a plurality of R₁₀₆ are present, the plurality of R₁₀₆ are mutually the same or different;
               when a plurality of R₁₀₇ are present, the plurality of R₁₀₇ are mutually the same or different;
               when a plurality of R₁₀₈ are present, the plurality of R₁₀₈ are mutually the same or different;
               when a plurality of R₁₀₉ are present, the plurality of R₁₀₉ are mutually the same or different;
               when the ring Px is a monocyclic aromatic hydrocarbon ring, a monocyclic aliphatic hydrocarbon ring, or a monocyclic heterocycle having one or more substituents R_{P}, at least one of the substituents R_{P} is a group represented by -O-R_{P1}, and R_{P1} is a substituted or unsubstituted alkyl group or a substituted or unsubstituted aryl group;
               when the ring Px is a naphthalene ring, the naphthalene ring as the ring Px has at least one group represented by -O-R_{P1}, and R_{P1} is a substituted or unsubstituted alkyl group or a substituted or unsubstituted aryl group;
               when the ring Px is a monocyclic heterocycle having no substituent R_{P}, the monocyclic heterocycle contains two or more heteroatoms as ring atoms;
               when the ring Px is a polycyclic heterocycle, at least one ring contains one or more heteroatoms as ring atoms;
               g, h, j, and k are each independently 0, 1, 2, or 3; and
               *1 and *2 each represent a bonding position.
[2] The compound according to [1], in which the donor moiety is represented by the formula (D1) or the formula (D2).
[3] The compound according to [1] or [2], in which the rings Dx and Dy in the formulae (D1) and (D2) are each independently a five-membered or six-membered aromatic hydrocarbon ring, or a five-membered or six-membered heterocycle.
[4] The compound according to any one of [1] to [3], in which the donor moiety is represented by a formula (D11), (D12), (D13), (D21), or (D22) below. 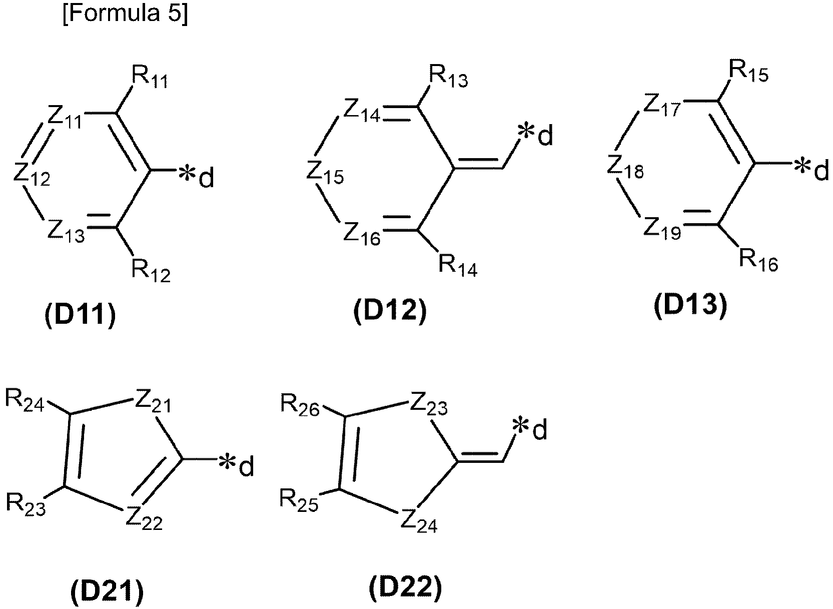 In the groups represented by the formulae (D11), (D12), (D13), (D21), and (D22):
   Z₁₁, Z₁₂, Z₁₃, Z₁₄, Z₁₆, and Z₁₉ are each independently C(R₁₀₀);
   Z₁₅, Z₁₇, and Z₁₈ are each independently an oxygen atom, a sulfur atom, C=R₁₀₀, N(R₁₁₀), or C(R₁₁₁)(R₁₁₂);
   Z₂₁, Z₂₃, and Z₂₄ are each independently an oxygen atom, a sulfur atom, N(R₁₁₀), or C(R₁₁₁)(R₁₁₂);
   Z₂₂ is C(R₁₀₀);
   at least one combination of adjacent two or more of R₁₁, R₁₂, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
   at least one combination of adjacent two or more of R₁₃, R₁₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
   at least one combination of adjacent two or more of R₁₅, R₁₆, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
   at least one combination of adjacent two or more of R₂₃, R₂₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
   at least one combination of adjacent two or more of R₂₅, R₂₆, R₁₁₀, R₁₁₁, and R₁₁₂ are mutually bonded to form a substituted or unsubstituted monocyclic ring, mutually bonded to form a substituted or unsubstituted fused ring, or not mutually bonded;
   R₁₁ to R₁₆, R₂₃ to R₂₆, R₁₀₀, and R₁₁₀ to R₁₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₇)(R₁₀₈)(R₁₀₉), or a group represented by -O(R₁₁₃); and
   *d is a bonding position.
[5] The compound according to any one of [1] to [4], in which the donor moiety is a compound represented by a formula (D111), (D112), (D113), (D121), (D122), (D123), or (D124) below. 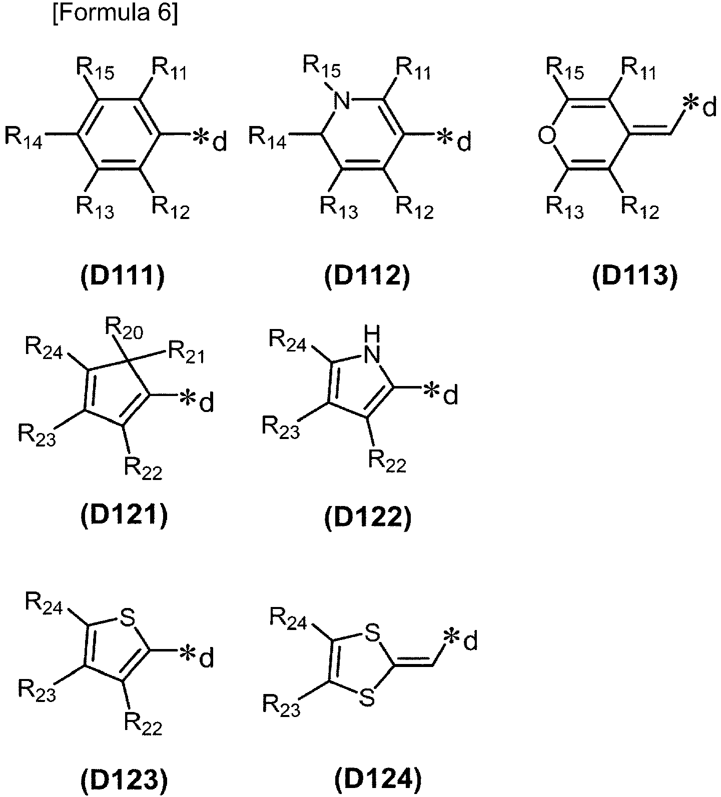 In the groups represented by the formulae (D111), (D112), (D113), (D121), (D122), (D123), and (D124):
   at least one combination of adjacent two or more of R₁₁ to R₁₅ are mutually bonded to form a substituted or unsubstituted monocyclic ring, mutually bonded to form a substituted or unsubstituted fused ring, or not mutually bonded;
   at least one combination of adjacent two or more of R₂₀ to R₂₄ are mutually bonded to form a substituted or unsubstituted monocyclic ring, mutually bonded to form a substituted or unsubstituted fused ring, or not mutually bonded;
   R₁₁ to R₁₅ and R₂₀ to R₂₄ forming neither the substituted or unsubstituted monocyclic ring nor the substituted or unsubstituted fused ring are each independently a hydrogen atom, a group represented by -N(R₁₀₁)(R₁₀₂), a group represented by -Si(R₁₀₃)(R₁₀₄)(R₁₀₅), a group represented by -C(=O)R₁₀₆, a group represented by -O(R₁₁₃), a substituted or unsubstituted alkyl group, a substituted or unsubstituted cycloalkyl group, a substituted or unsubstituted aryl group, or a substituted or unsubstituted heterocyclic group; and
   *d represents a bonding position.
[6] The compound according to any one of [1] to [5], in which the donor moiety is represented by a formula (D111-1A), (D111-1B), (D111-1C), (D111-1D), (D111-1E), (D111-1F), (D111-1G), or (D111-1H) below. 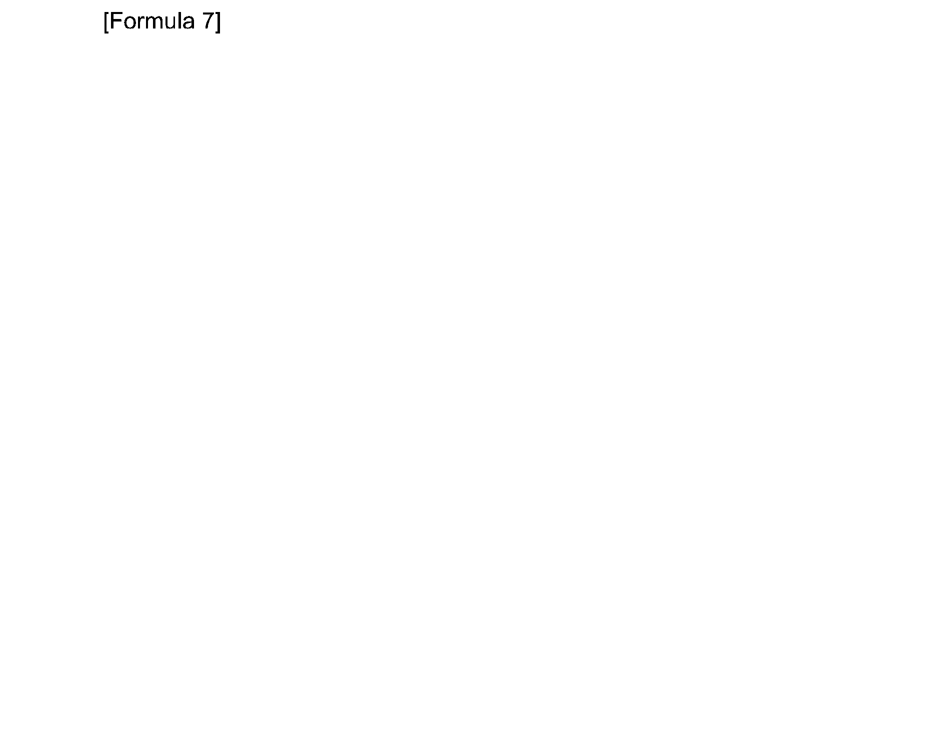 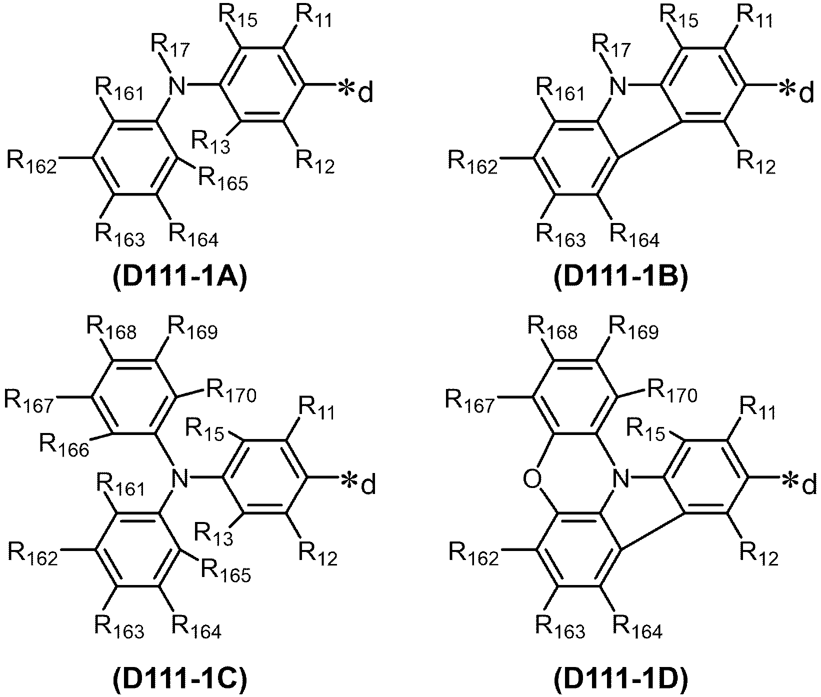 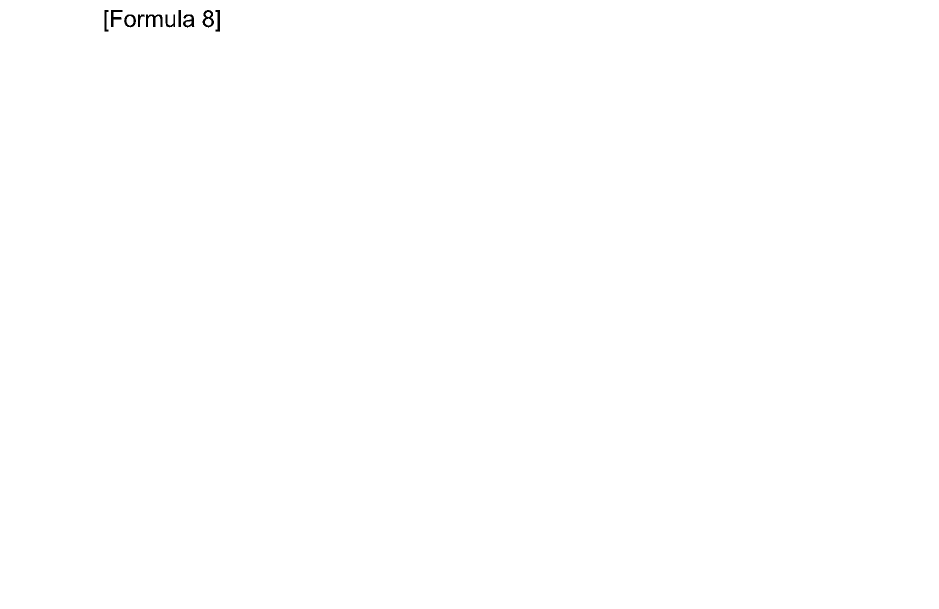 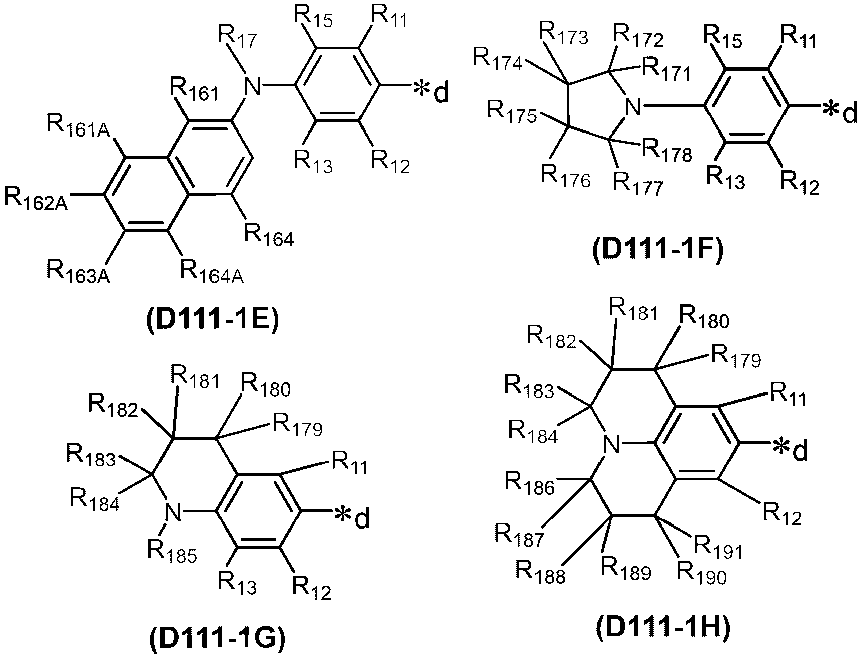 In the formulae (D111-1A), (D111-1B), (D111-1C), (D111-1D), (D111-1E), (D111-1F), (D111-1G), and (D111-1H):
   R₁₁ to R₁₃ and R₁₅ are each independently a hydrogen atom, a substituted or unsubstituted alkyl group, a substituted or unsubstituted cycloalkyl group, a substituted or unsubstituted aryl group, or a substituted or unsubstituted heterocyclic group;
      R₁₇, R₁₆₁ to R₁₉₁, and R_{161A} to R_{164A} are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
   *d represents a bonding position.
[7] The compound according to any one of [1] to [6], in which the donor moiety is represented by a formula (D122-1), (D123-1A), or (D123-1B) below. 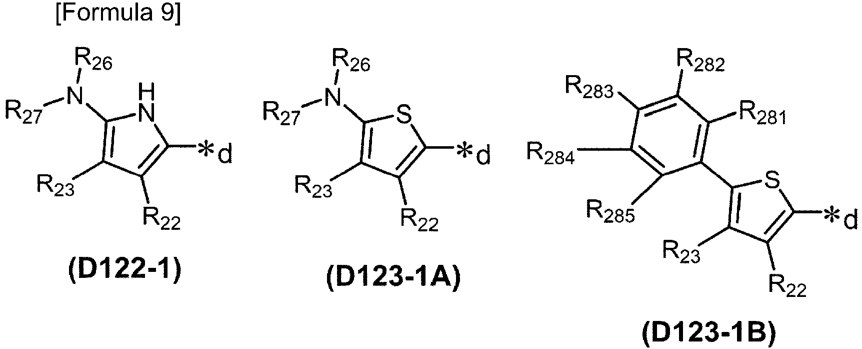 In the formulae (D122-1), (D123-1A), and (D123-1B):
   R₂₂, R₂₃, R₂₆, and R₂₇ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃);
   at least one combination of adjacent two or more of R₂₈₁, R₂₈₂, R₂₈₃, R₂₈₄, and R₂₈₅ are mutually bonded to form a substituted or unsubstituted monocyclic ring, mutually bonded to form a substituted or unsubstituted fused ring, or not mutually bonded;
   R₂₈₁ to R₂₈₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by -N(R₁₀₁)(R₁₀₂), or a group represented by -O(R₁₁₃); and
   *d represents a bonding position.
[8] The compound according to any one of [1] to [7], in which the donor moiety is represented by a formula (D123-2A), (D123-3A), or (D123-4A) below. 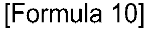 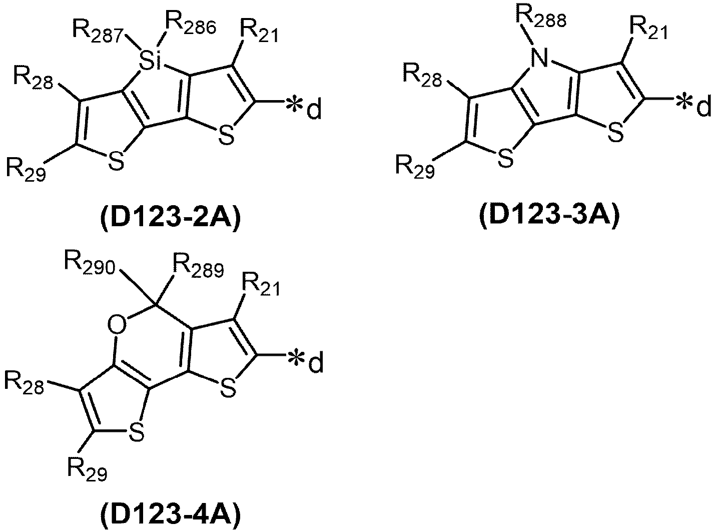 In the formulae (D123-2A), (D123-3A), and (D123-4A):
   R₂₁, R₂₈, R₂₉, R₂₈₆, R₂₈₇, R₂₈₈, R₂₈₉, and R₂₉₀ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
   *d represents a bonding position.
[9] The compound according to any one of [1] to [8], in which the donor moiety is represented by a formula (D112-1) or (D121-1) below. 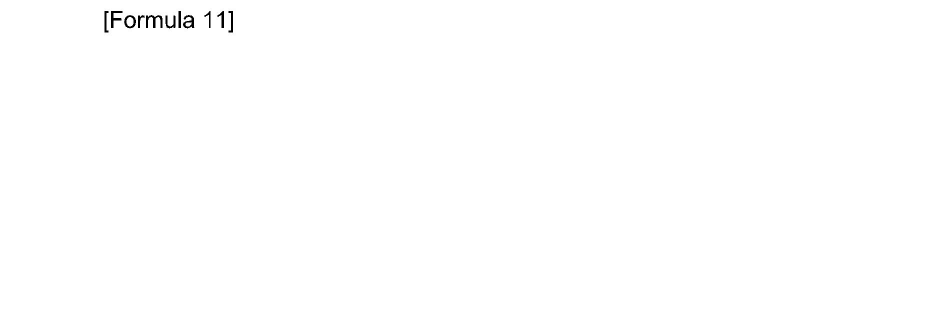 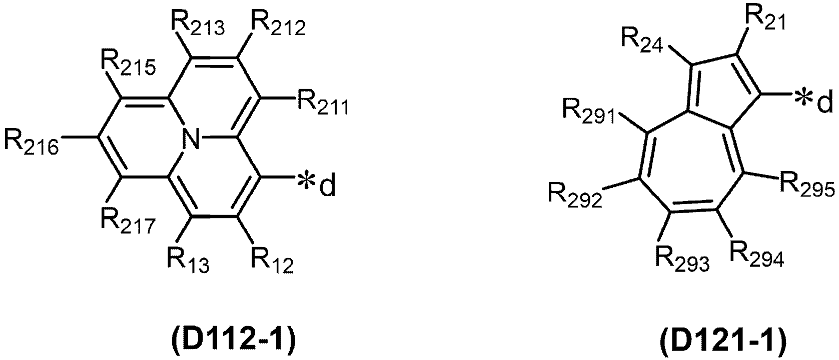 In the formulae (D112-1) and (D121-1):
   R₁₂, R₁₃, R₂₁, R₂₄, R₂₁₁ to R_{213,}, R₂₁₅ to R₂₁₇, and R₂₉₁ to R₂₉₅ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by - O(R₁₁₃); and
   *d represents a bonding position.
[10] The compound according to [5], in which the donor moiety is represented by the formula (D111).
[11] The compound according to [10], in which R₁₁ to R₁₅ in the formula (D111) are each independently a hydrogen atom or a group represented by -O(R₁₁₃), provided that at least one of R₁₁ to R₁₅ is a group represented by -O(R₁₁₃).
[12] The compound according to [7], in which the donor moiety is represented by the formula (D123-1B).
[13] The compound according to [12], in which R₂₈₁ to R₂₈₅ in the formula (D123-1B) are each independently a hydrogen atom, a group represented by -N(R₁₀₁)(R₁₀₂), or a group represented by -O(R₁₁₃).
[14] The compound according to [12] or [13], in which at least one of R₂₈₁ to R₂₈₅ in the formula (D123-1B) is a group represented by -O(R₁₁₃).
[15] The compound according to any one of [12] to [14], in which three of R₂₈₁ to R₂₈₅ in the formula (D123-1B) are groups represented by -O(R₁₁₃), and the remaining two are hydrogen atoms.
[16] The compound according to any one of [12] to [15], in which R₂₈₃ in the formula (D123-1B) is a group represented by -O(R₁₁₃), one of R₂₈₁ and R₂₈₂ is a group represented by -O(R₁₁₃) and the other thereof is a hydrogen atom, and one of R₂₈₄ and R₂₈₅ is a group represented by -O(R₁₁₃) and the other thereof is a hydrogen atom.
[17] The compound according to any one of [12] to [16], in which, when at least one selected from the group consisting of R₂₈₁ to R₂₈₅ in the formula (D123-1B) is a group represented by -O(R₁₁₃), R₁₁₃ in R₂₈₁ to R₂₈₅ is a substituted or unsubstituted alkyl group having 1 to 5 carbon atoms.
[18] The compound according to [12], in which one of R₂₈₁ to R₂₈₅ in the formula (D123-1B) is a group represented by -N(R₁₀₁)(R₁₀₂), and the remaining four are hydrogen atoms.
[19] The compound according to any one of [1] to [18], in which the acceptor moiety is represented by the formula (A1) or (A2).
[20] The compound according to any one of [1] to [19], in which the acceptor moiety is represented by a formula (A11) or (A12) below. 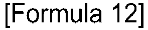 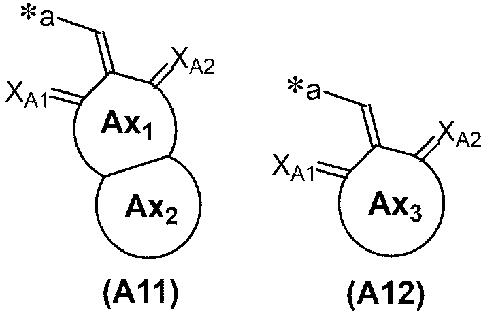 In the formulae (A11) and (A12):
   X_{A1} and X_{A2} respectively represent the same as X_{A1} and X_{A2} in the formula (A1);
   the rings Ax₁, Ax₂, and Ax₃ are each independently a substituted or unsubstituted monocyclic aromatic hydrocarbon ring, a substituted or unsubstituted monocyclic aliphatic hydrocarbon ring, or a substituted or unsubstituted monocyclic heterocycle; and
   *a represents a bonding position.
[21] The compound according to any one of [1] to [20], in which the acceptor moiety is represented by a formula (A11-1), (A11-2), or (A12-1) below. 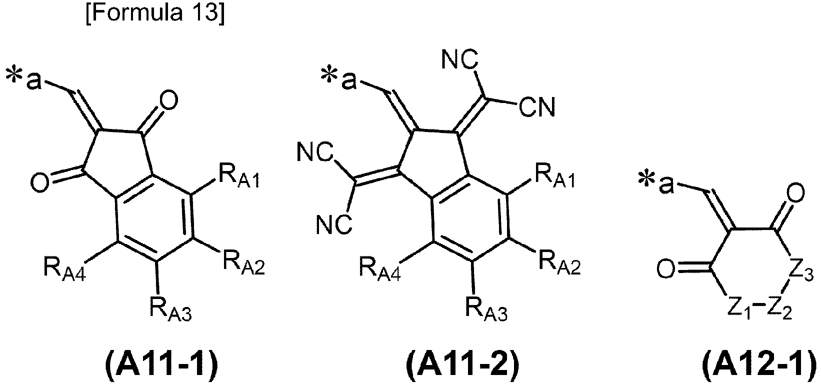 In the formulae (A11-1) and (A11-2):
   R_{A1}, R_{A2}, R_{A3}, and R_{A4} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx;
   in the formula (A12-1):
      Z₁, Z₂, and Z₃ are each independently N(R_{A5}), C=O, or C(R_{A6})(R_{A7});
      R_{A5}, R_{A6}, and R_{A7} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx;
      Rx is a substituted or unsubstituted alkyl group, and when a plurality of Rx are present, the plurality of Rx are mutually the same or different; and
      *a represents a bonding position.
[22] The compound according to any one of [1] to [21], in which the acceptor moiety is represented by a formula (A111-1), (A111-2), (A112-1), or (A121-1) below. 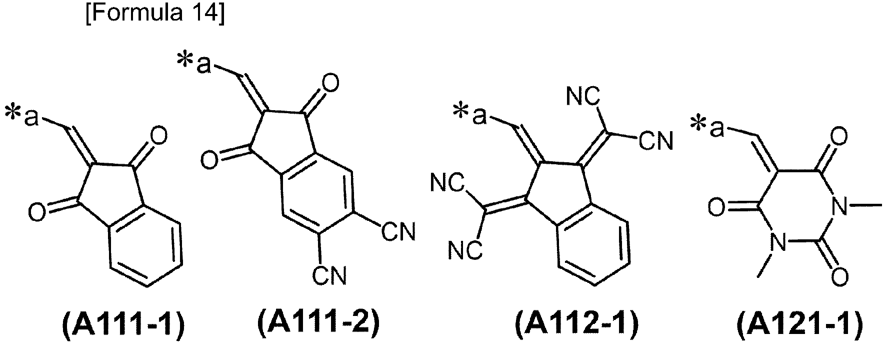 In the formulae (A111-1), (A111-2), (A112-1), and (A121-1), *a represents a bonding position.
[23] The compound according to any one of [1] to [19], in which na in the formula (A2) is 1 or 2.
[24] The compound according to any one of [1] to [19] and [23], in which the acceptor moiety is represented by a formula (A21) or (A22) below. 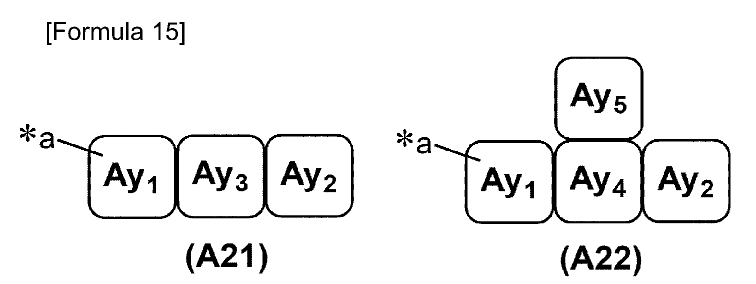 In the formula (A21):
   the ring Ay₁ and the ring Ay₂ represent the same as the ring Ay₁ and the ring Ay₂ in the formula (A2), respectively; and
   the ring Ay₃ represents the same as the ring Ay in the formula (A2),
   in the formula (A22):
      the ring Ay₁ and the ring Ay₂ represent the same as the ring Ay₁ and the ring Ay₂ in the formula (A2), respectively; and
      the ring Ay₄ and the ring Ay₅ each independently represent the same as the ring Ay in the formula (A2), and
      *a represents a bonding position.
[25] The compound according to any one of [1] to [19], [23], and [24], in which the ring Ay₁ and the ring Ay₂ are each independently a nitrogen-containing ring.
[26] The compound according to any one of [1] to [19] and [23] to [25], in which the ring Ay₁ and the ring Ay₂ are each independently a six-membered nitrogen-containing ring.
[27] The compound according to any one of [1] to [19] and [23] to [26], in which the acceptor moiety is represented by a formula (A21-1) or (A22-1) below. 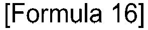 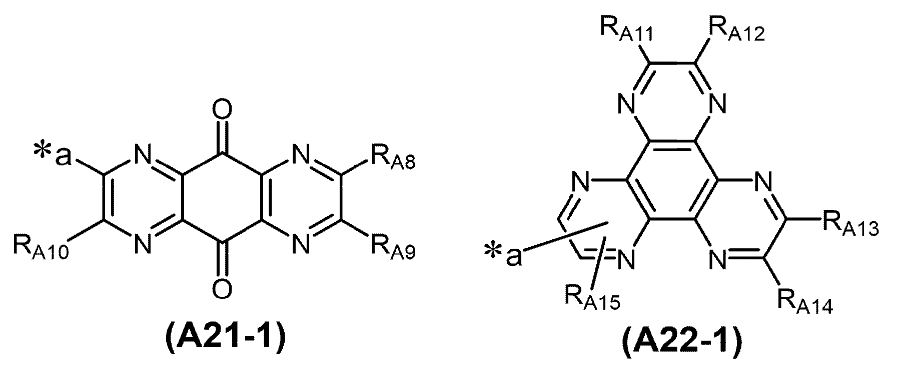 In the formulae (A21-1) and (A22-1):
   R_{A8}, R_{A9}, R_{A10}, R_{A11}, R_{A12}, R_{A13}, R_{A14}, and R_{A15} are each independently a hydrogen atom, a halogen atom, a cyano group, a substituted or unsubstituted alkyl group, a substituted or unsubstituted aryl group, -NO₂, -CHO, -CORx, -COORx, or - SO₂Rx;
   Rx is a substituted or unsubstituted alkyl group, and when a plurality of Rx are present, the plurality of Rx are mutually the same or different; and
   *a represents a bonding position.
[28] The compound according to any one of [1] to [19] and [23] to [27], in which the acceptor moiety is represented by a formula (A211-1) or (A221-1) below. 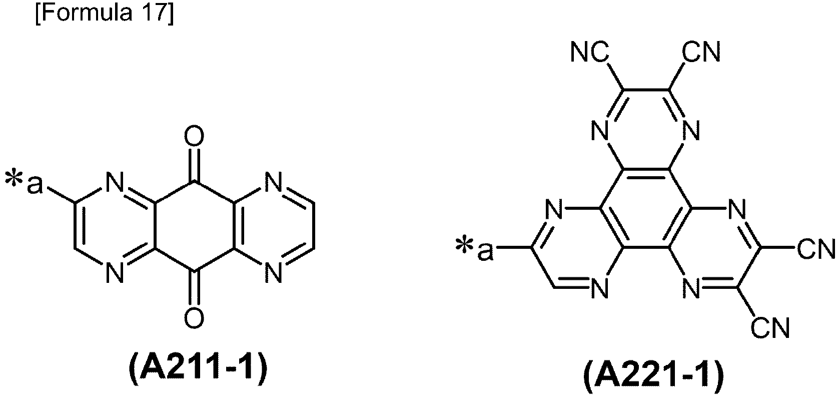 In the formulae (A211-1) and (A221-1), *a represents a bonding position.
[29] The compound according to any one of [1] to [19], [23], and [24], in which the acceptor moiety is represented by a formula (A21-2) below. 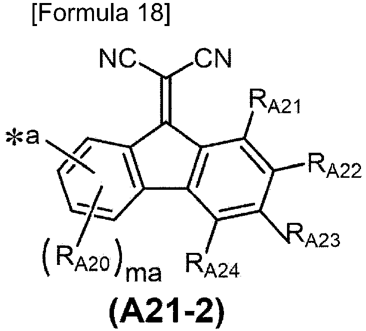 In the formula (A21-2):
   ma represents the number of R_{A20} bonded to the ring, ma is 3, and the three R_{A20} are mutually the same or different;
   R_{A20}, R_{A21}, R_{A22}, R_{A23}, and R_{A24} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx;
   Rx is a substituted or unsubstituted alkyl group, and when a plurality of Rx are present, the plurality of Rx are mutually the same or different; and
   *a represents a bonding position.
[30] The compound according to any one of [1] to [19], [23], [24], and [29], in which the acceptor moiety is represented by a formula (A212-1) below. 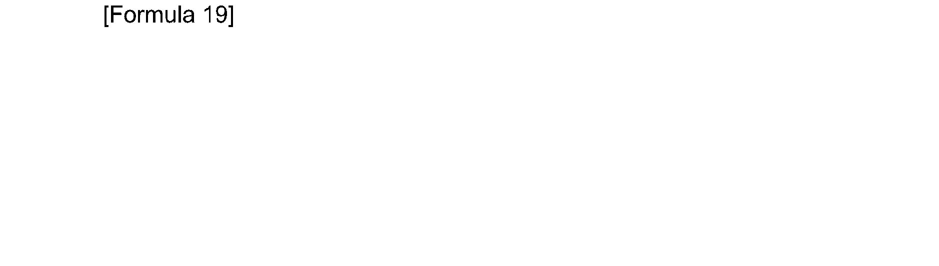 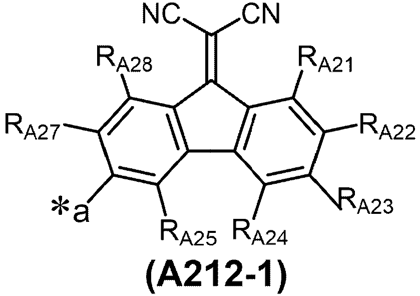 In the formula (A212-1):
   R_{A21}, R_{A22}, R_{A23}, R_{A24}, R_{A25}, R_{A27}, and R_{A28} are each independently a hydrogen atom, a halogen atom, a cyano group, a substituted or unsubstituted alkyl group, a substituted or unsubstituted aryl group, -NO₂, -CHO, -CORx, -COORx, or - SO₂Rx;
   Rx is a substituted or unsubstituted alkyl group, and when a plurality of Rx are present, the plurality of Rx are mutually the same or different; and
   *a represents a bonding position.
[31] The compound according to any one of [1] to [30], in which the acceptor moiety has at least one structure selected from the group consisting of a nitrogen-containing ring, CN, C=O, and C=C(CN)₂.
[32] The compound according to any one of [1] to [31], in which the π-conjugated moiety is represented by the formula (P1), (P2), or (P3).
[33] The compound according to any one of [1] to [32], in which the π-conjugated moiety is represented by the formula (P11-1), (P12-1), (P13-1), (P14-1), or (P15-1). 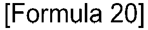 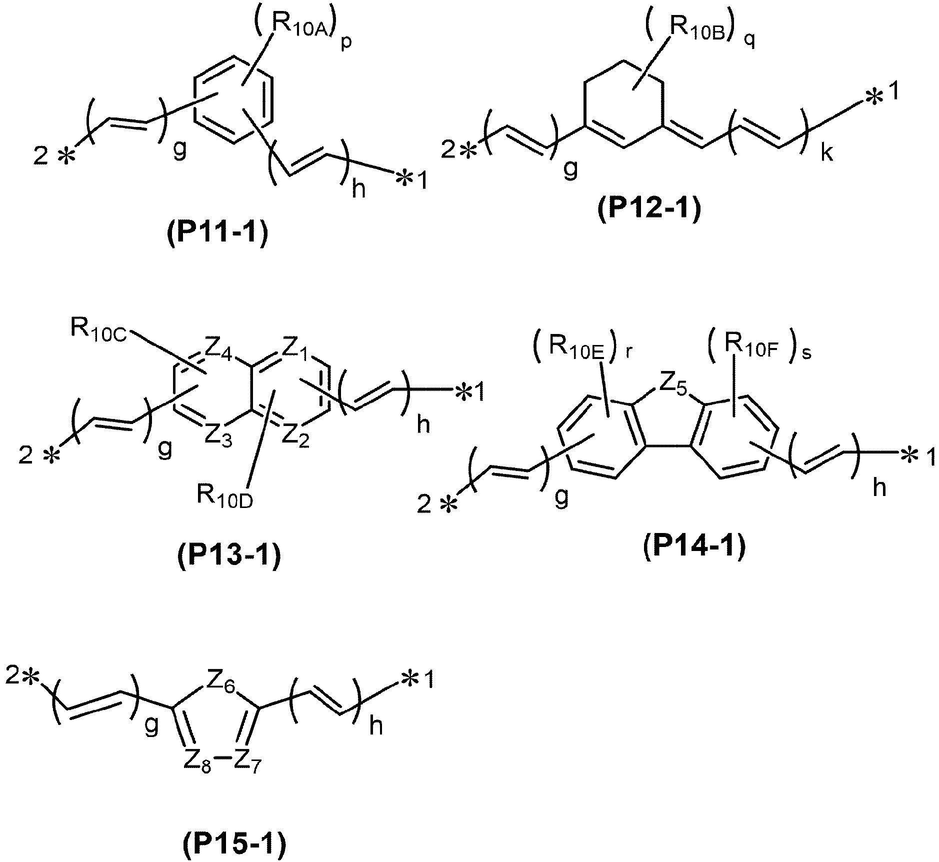 In the formulae (P11-1), (P12-1), (P13-1), (P14-1), and (P15-1):
   p, q, r, and s are each an integer of 1 or more;
   Z₁, Z₂, Z₃, Z₄, Z₇, and Z₈ are each independently C(R₁₀₀) or a nitrogen atom;
   a plurality of R₁₀₀ are mutually the same or different;
   Z₅ and Z₆ are each independently an oxygen atom, a sulfur atom, N(R₁₁₀), or C(R₁₁₁ )(R₁₁₂);
   at least one combination of adjacent two or more of R₁₀₀, R_{10A}, R_{10B}, R_{10C}, R_{10D}, R_{10E}, and R_{10F} are mutually bonded to form a substituted or unsubstituted monocyclic ring, mutually bonded to form a substituted or unsubstituted fused ring, or not mutually bonded;
   R₁₀₀, R_{10A}, R_{10B}, R_{10C}, R_{10D}, R_{10E}, and R_{10F} forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
   when a plurality of R_{10A} are present, the plurality of R_{10A} are mutually the same or different;
   when a plurality of R_{10B} are present, the plurality of R_{10B} are mutually the same or different;
   when a plurality of R_{10C} are present, the plurality of R_{10C} are mutually the same or different;
   when a plurality of R_{10D} are present, the plurality of R_{10D} are mutually the same or different;
   when a plurality of R_{10E} are present, the plurality of R_{10E} are mutually the same or different;
   when a plurality of R_{10F} are present, the plurality of R_{10F} are mutually the same or different;
   R₁₀₁, R₁₀₂, R₁₀₃, R₁₀₄, R₁₀₅, R₁₀₆, R₁₀₇, R₁₀₈, R₁₀₉, R₁₁₀, R₁₁₁, and R₁₁₂ are each independently a hydrogen atom, a substituted or unsubstituted alkyl group, a substituted or unsubstituted cycloalkyl group, a substituted or unsubstituted aryl group, or a substituted or unsubstituted heterocyclic group;
   when a plurality of R₁₀₁ are present, the plurality of R₁₀₁ are mutually the same or different;
   when a plurality of R₁₀₂ are present, the plurality of R₁₀₂ are mutually the same or different;
   when a plurality of R₁₀₃ are present, the plurality of R₁₀₃ are mutually the same or different;
   when a plurality of R₁₀₄ are present, the plurality of R₁₀₄ are mutually the same or different;
   when a plurality of R₁₀₅ are present, the plurality of R₁₀₅ are mutually the same or different;
   when a plurality of R₁₀₆ are present, the plurality of R₁₀₆ are mutually the same or different;
   when a plurality of R₁₀₇ are present, the plurality of R₁₀₇ are mutually the same or different;
   when a plurality of R₁₀₈ are present, the plurality of R₁₀₈ are mutually the same or different;
   when a plurality of R₁₀₉ are present, the plurality of R₁₀₉ are mutually the same or different;
   when a plurality of R₁₁₀ are present, the plurality of R₁₁₀ are mutually the same or different;
   when a plurality of R₁₁₁ are present, the plurality of R₁₁₁ are mutually the same or different;
   when a plurality of R₁₁₂ are present, the plurality of R₁₁₂ are mutually the same or different; and
   g, h, and k are each independently 0, 1, 2, or 3,
   in the formula (P11-1): when p is 1, R_{10A} is a group represented by -O(R₁₀₉); and when p is 2 or more, at least one of R_{10A} is a group represented by -O(R₁₀₉), and R₁₀₉ is a substituted or unsubstituted alkyl group or a substituted or unsubstituted aryl group,
   in the formula (P12-1): when q is 1, R_{10B} is a group represented by -O(R₁₀₉); when q is 2 or more, at least one of R_{10B} is a group represented by -O(R₁₀₉), and R₁₀₉ is a substituted or unsubstituted alkyl group or a substituted or unsubstituted aryl group,
   in the formula (P13-1): when Z₁, Z₂, Z₃, and Z₄ are C(R₁₀₀), at least one of R_{10C}, R_{10D}, or the four R₁₀₀ is a group represented by -O(R₁₀₉), and R₁₀₉ is a substituted or unsubstituted alkyl group or a substituted or unsubstituted aryl group,
   in the formula (P15-1): when Z₇ and Z₈ are C(R₁₀₀), at least one of the two R₁₀₀ is a group represented by -O(R₁₀₉), and R₁₀₉ is a substituted or unsubstituted alkyl group or a substituted or unsubstituted aryl group, and
   *1 and *2 each represent a bonding position.
[34] The compound according to any one of [1] to [33], in which the π-conjugated moiety is represented by a formula (P11-1A), (P12-1A), (P13-1A), (P13-1B), (P14-1A), (P14-1B), (P15-1A), or (P15-1B) below. 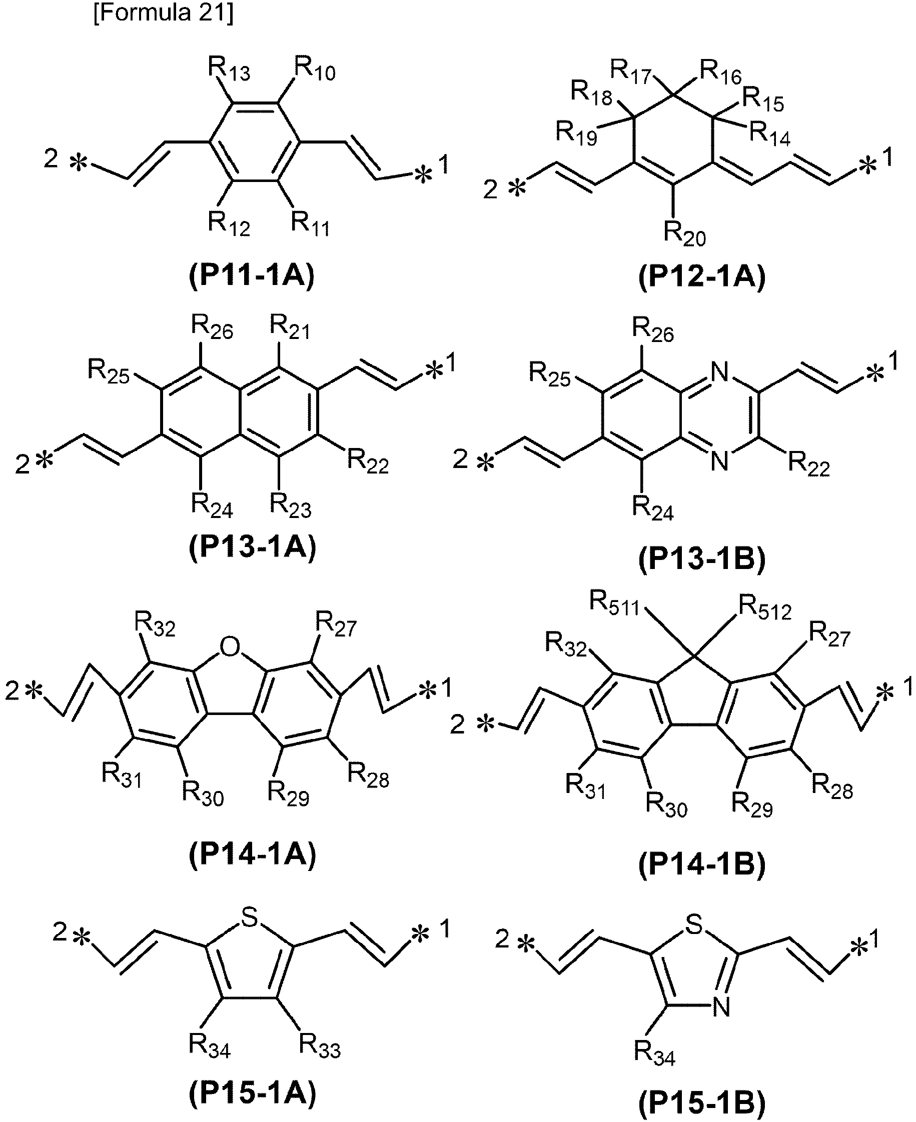 In the formulae (P11-1A), (P12-1A), (P13-1A), (P13-1B), (P14-1A), (P14-1B), (P15-1A), and (P15-1B):
   at least one combination of adjacent two or more of R₁₀ to R₃₄, R₅₁₁, and R₅₁₂ are mutually bonded to form a substituted or unsubstituted monocyclic ring, mutually bonded to form a substituted or unsubstituted fused ring, or not mutually bonded;
   R₁₀ to R₃₄, R₅₁₁, and R₅₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉),
   in the formula (P11-1A): when at least one of R₁₀ to R₁₃ is a group represented by -O(R₁₀₉), and R₁₀₉ is a substituted or unsubstituted alkyl group or a substituted or unsubstituted aryl group,
   in the formula (P12-1A): when at least one of R₁₄ to R₂₀ is a group represented by -O(R₁₀₉), and R₁₀₉ is a substituted or unsubstituted alkyl group or a substituted or unsubstituted aryl group,
      in the formula (P13-1A): when at least one of R₂₁ to R₂₆ is a group represented by - O(R₁₀₉), and R₁₀₉ is a substituted or unsubstituted alkyl group or a substituted or unsubstituted aryl group,
   in the formula (P15-1A): when at least one of R₃₃ to R₃₄ is a group represented by -O(R₁₀₉), and R₁₀₉ is a substituted or unsubstituted alkyl group or a substituted or unsubstituted aryl group, and
   *1 and *2 each represent a bonding position.
[35] The compound according to any one of [1] to [34], represented by a formula (1) below.
   [Formula 22]

   D-L-A (1)

   In the formula (1), D is the donor moiety, L is the π-conjugated moiety, and A is the acceptor moiety.
[36] The compound according to any one of [1] to [35], in which the donor moiety is a group having electron-donating properties, and the acceptor moiety is a group having electron-withdrawing properties.
[37] A precursor represented by a formula (DP-XM1), (DP-XM2), (DP-XM3), (DP-XM4), (DP-YM1), (DP-YM2), (DP-YM3), or (DP-YM4) below. 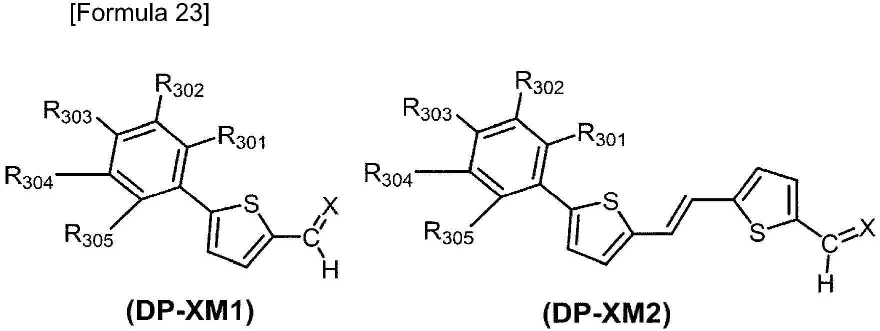 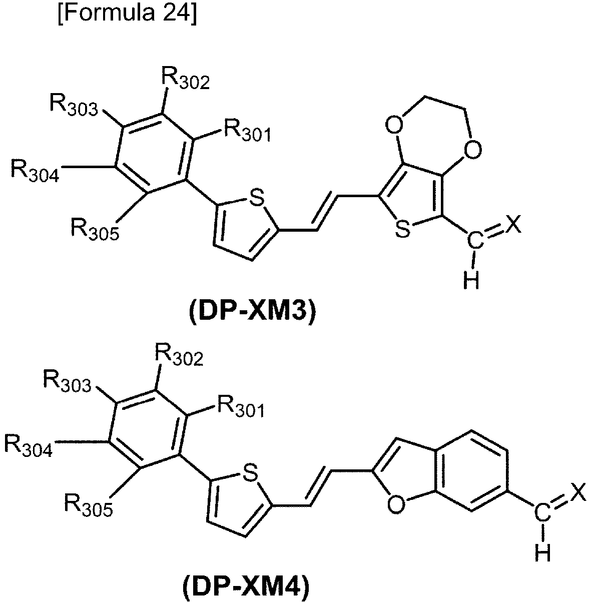 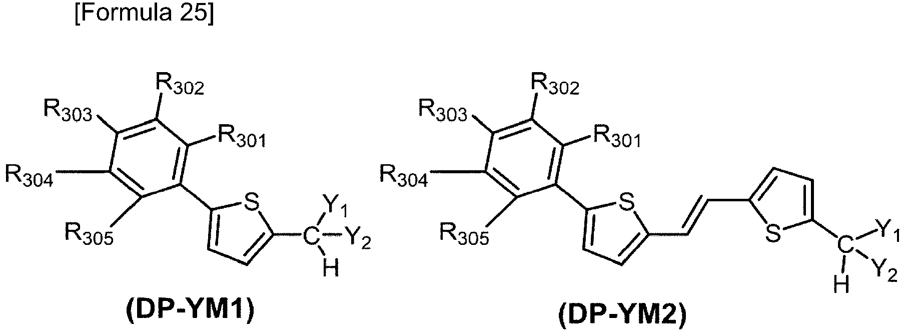 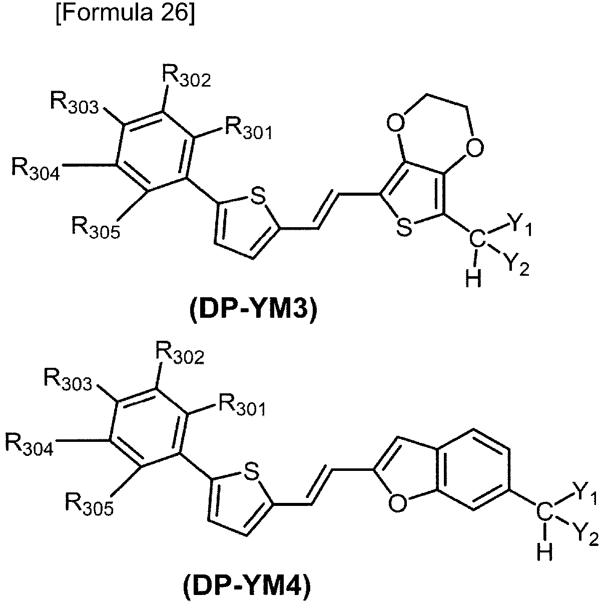 In the formulae (DP-XM1), (DP-XM2), (DP-XM3), (DP-XM4), (DP-YM1), (DP-YM2), (DP-YM3), and (DP-YM4):
   at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
   R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
   R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group,
   in the formulae (DP-XM1), (DP-XM2), (DP-XM3), and (DP-XM4): X is an oxygen atom or a sulfur atom,
   in the formulae (DP-YM1), (DP-YM2), (DP-YM3), and (DP-YM4):
      a combination of Y₁ and Y₂ are mutually bonded to form a substituted or unsubstituted monocyclic ring, mutually bonded to form a substituted or unsubstituted fused ring, or not mutually bonded;
      Y₁ and Y₂ forming neither the substituted or unsubstituted monocyclic ring nor the substituted or unsubstituted fused ring are each independently a substituted or unsubstituted alkoxy group or a group represented by -S(R₁₁₄);
      R₁₁₄ is a substituted or unsubstituted alkyl group;
      when a plurality of R₁₀₁ are present, the plurality of R₁₀₁ are mutually the same or different;
      when a plurality of R₁₀₂ are present, the plurality of R₁₀₂ are mutually the same or different;
      when a plurality of R₁₁₃ are present, the plurality of R₁₁₃ are mutually the same or different; and
      when a plurality of R₁₁₄ are present, the plurality of R₁₁₄ are mutually the same or different.
[38] Use of the precursor according to [37] in production of an electro-optic compound.
[39] A precursor used for producing the compound according to [1], in which the precursor is represented by a formula (DP-XM11), (DP-XM21), (DP-XM31), or (DP-XM41) below. 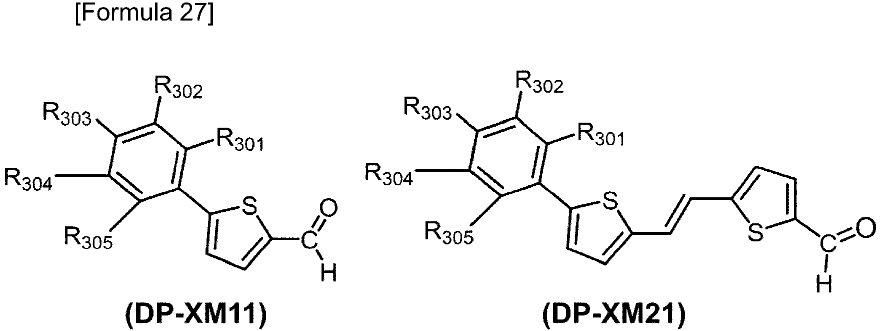 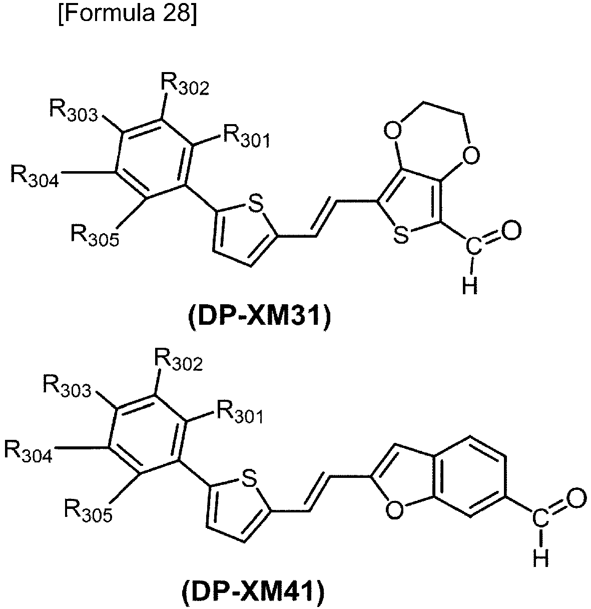 In the formulae (DP-XM11), (DP-XM21), (DP-XM31), and (DP-XM41):
   at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
   R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
   R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
   when a plurality of R₁₀₁ are present, the plurality of R₁₀₁ are mutually the same or different;
   when a plurality of R₁₀₂ are present, the plurality of R₁₀₂ are mutually the same or different; and
   when a plurality of R₁₁₃ are present, the plurality of R₁₁₃ are mutually the same or different.
[40] A precursor used for producing an electro-optic compound, in which the precursor is represented by a formula (DP-XM11), (DP-XM21), (DP-XM31), or (DP-XM41) below. 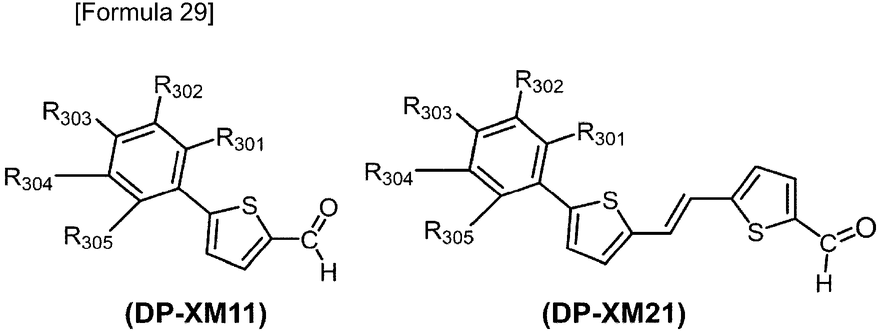 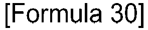 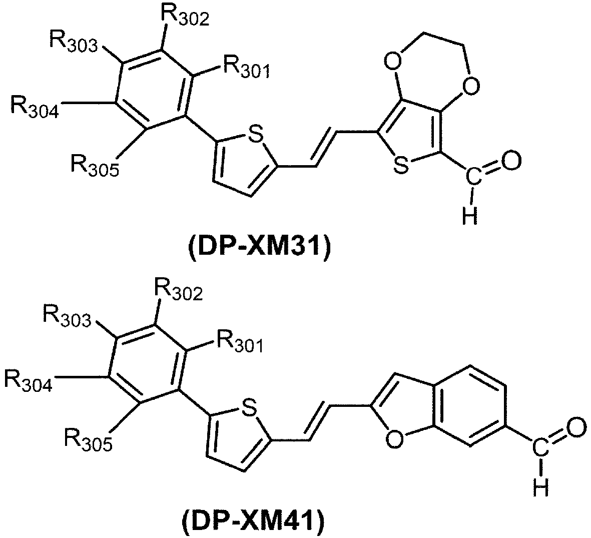 In the formulae (DP-XM11), (DP-XM21), (DP-XM31), and (DP-XM41):
   at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
   R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
   R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
   when a plurality of R₁₀₁ are present, the plurality of R₁₀₁ are mutually the same or different;
   when a plurality of R₁₀₂ are present, the plurality of R₁₀₂ are mutually the same or different; and
   when a plurality of R₁₁₃ are present, the plurality of R₁₁₃ are mutually the same or different.
[41] The precursor according to [39] or [40], in which R₃₀₁ to R₃₀₅ in the precursor represented by the formula (DP-XM11), (DP-XM21), (DP-XM31), or (DP-XM41) are each independently a hydrogen atom, a group represented by -N(R₁₀₁)(R₁₀₂), or a group represented by -O(R₁₁₃).
[42] The precursor according to any one of [39] to [41], in which the precursor represented by the formula (DP-XM11), (DP-XM21), (DP-XM31), or (DP-XM41) is represented by a formula (DP-M1), (DP-M2), (DP-M3), (DP-M4), (DP-M5), (DP-M6), (DP-M7), or (DP-M8) below. 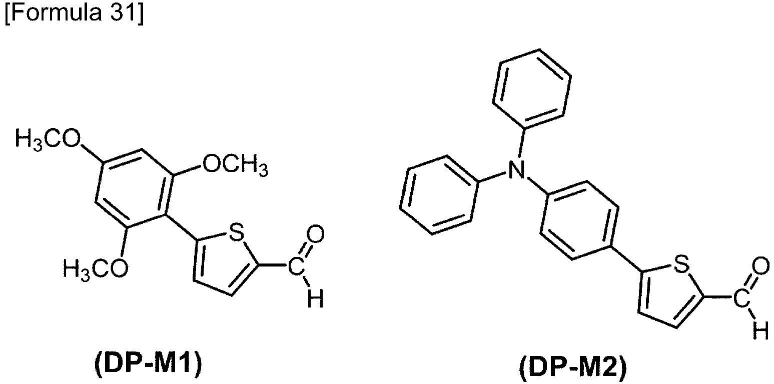 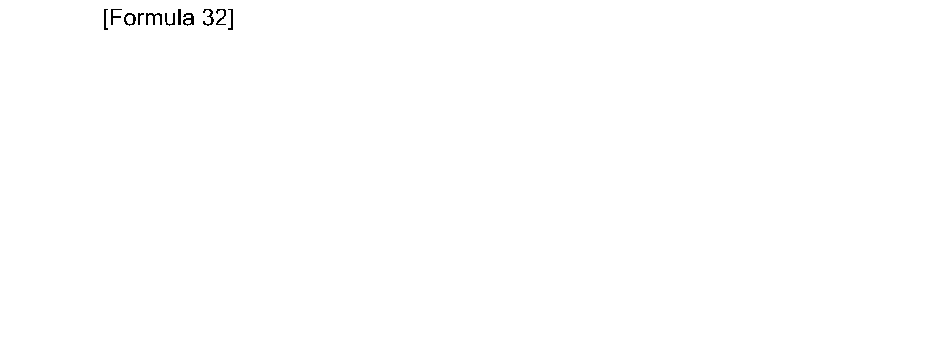 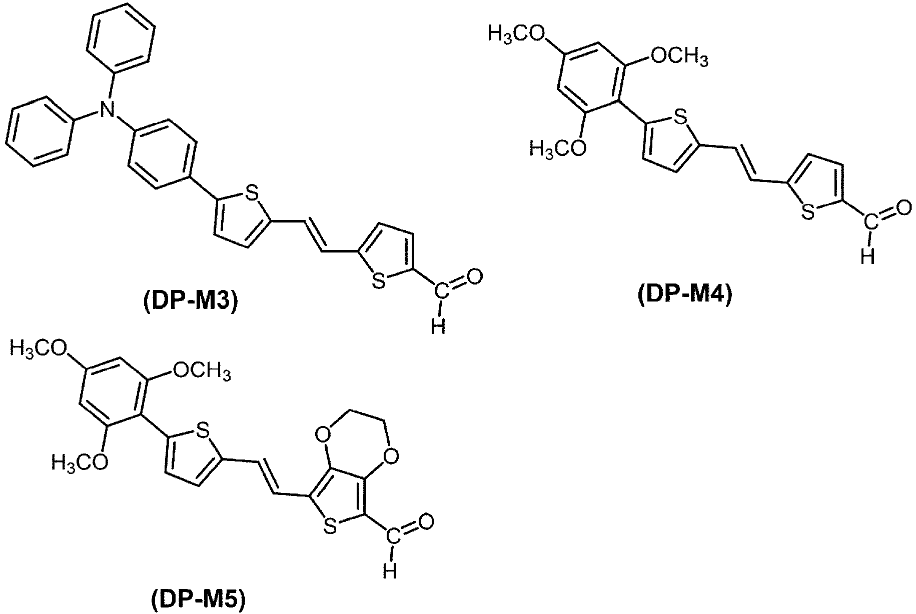 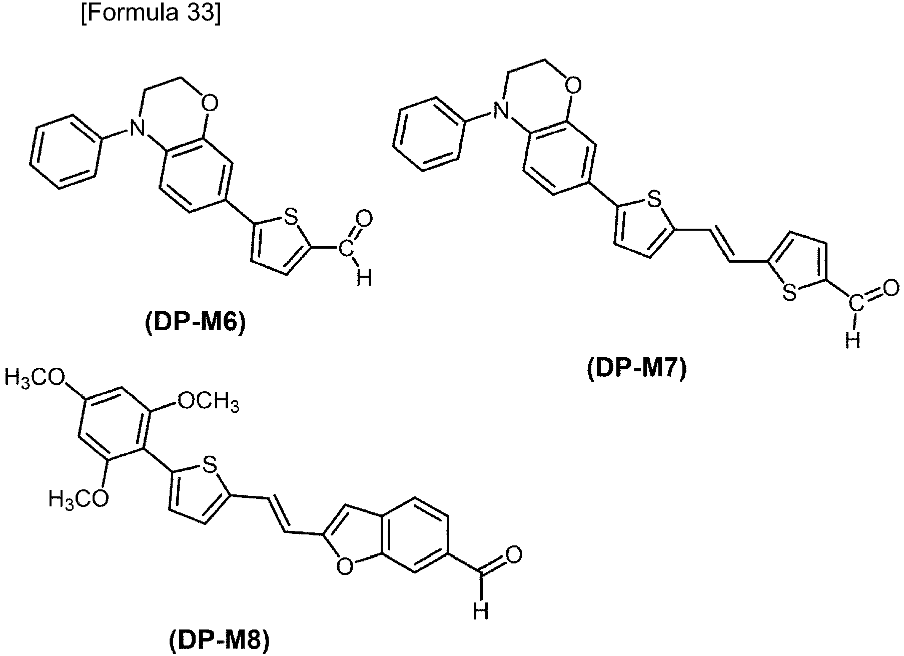
[43] Use of the precursor according to any one of [39] to [42].
[44] A method for producing the compound according to [1] using a precursor represented by a formula (DP-XM11), (DP-XM21), (DP-XM31), or (DP-XM41) below. 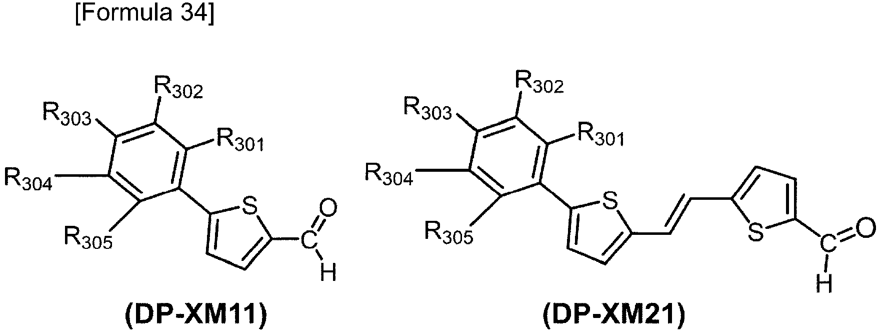 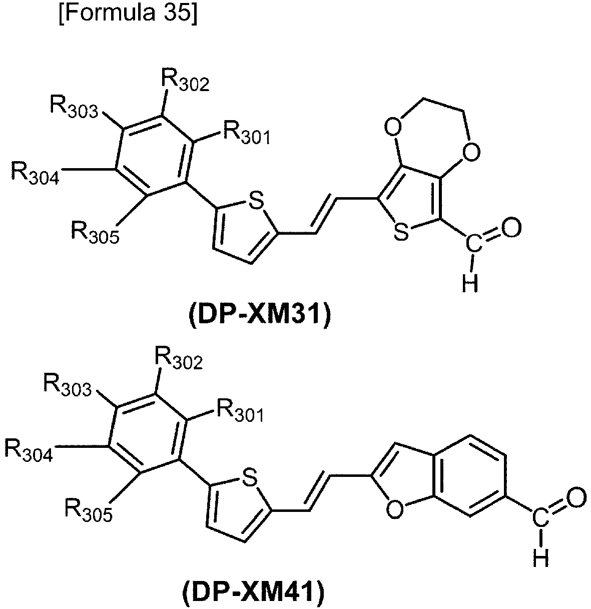 In the formulae (DP-XM11), (DP-XM21), (DP-XM31), and (DP-XM41):
   at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
   R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
   R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
   when a plurality of R₁₀₁ are present, the plurality of R₁₀₁ are mutually the same or different;
   when a plurality of R₁₀₂ are present, the plurality of R₁₀₂ are mutually the same or different; and
   when a plurality of R₁₁₃ are present, the plurality of R₁₁₃ are mutually the same or different.
[45] An electro-optic material including: an electro-optic compound and a host material, in which the electro-optic compound is the compound according to any one of [1] to [36].
[46] The electro-optic material according to [45], in which the host material is a polymer, and the electro-optic compound is bonded to the polymer as the host material.
[47] The electro-optic material according to [45], in which the host material is a polymer, and the electro-optic compound is not bonded to the polymer and is dispersed in the host material.
[48] An electro-optic film including the compound according to any one of [1] to [36].
[49] An electro-optic film comprising the electro-optic material according to any one of [45] to [47].
[50] An electro-optic device including the compound according to any one of [1] to [36].
[51] An electro-optic device including the electro-optic material according to any one of [45] to [47].
[52] An electro-optic device including the electro-optic film according to [48] or [49].

According to the above aspects of the invention, there can be provided a novel compound applicable to an electro-optic material, a precursor, a production method using the precursor, an electro-optic material containing the compound, an electro-optic film including the compound or the electro-optic material, and an electro-optic device including the compound or the electro-optic material.

### BRIEF DESCRIPTION OF DRAWINGS

Fig. 1 is a chart of a ¹H-NMR spectrum of a precursor S1 obtained in Example 1.
Fig. 2 is a chart of a ¹H-NMR spectrum of a compound C1 obtained in Example 1.
Fig. 3 is a chart of a ¹H-NMR spectrum of a precursor S3 obtained in Example 2.
Fig. 4 is a chart of a ¹H-NMR spectrum of a precursor S4 obtained in Example 2.
Fig. 5 is a chart of a ¹H-NMR spectrum of a compound C3 obtained in Example 2.
Fig. 6 is a chart of a ¹H-NMR spectrum of a compound C4 obtained in Example 3.
Fig. 7 is a chart of a ¹H-NMR spectrum of a compound C6 obtained in Example 4.
Fig. 8 is a chart of a ¹H-NMR spectrum of a precursor S5 obtained in Example 5.
Fig. 9 is a chart of a ¹H-NMR spectrum of a precursor S6 obtained in Example 5.
Fig. 10 is a chart of a ¹H-NMR spectrum of a precursor S7 obtained in Example 5.
Fig. 11 is a chart of a ¹H-NMR spectrum of a compound C7 obtained in Example 5.
Fig. 12 is a chart of a ¹H-NMR spectrum of a precursor S8 obtained in Example 6.
Fig. 13 is a chart of a ¹H-NMR spectrum of a precursor S9 obtained in Example 6.
Fig. 14 is a chart of a ¹H-NMR spectrum of a compound C8 obtained in Example 6.
Fig. 15 is a chart of a ¹H-NMR spectrum of a precursor S10 obtained in Example 7.
Fig. 16 is a chart of a ¹H-NMR spectrum of a precursor S11 obtained in Example 7.
Fig. 17 is a chart of a ¹H-NMR spectrum of a compound C9 obtained in Example 7.
Fig. 18 is a chart of a ¹H-NMR spectrum of a precursor S12 obtained in Example 8.
Fig. 19 is a chart of a ¹H-NMR spectrum of a precursor S13 obtained in Example 8.
Fig. 20 is a chart of a ¹H-NMR spectrum of a compound C10 obtained in Example 8.
Fig. 21 is a chart of a ¹H-NMR spectrum of a compound C11 obtained in Example 9.
Fig. 22 is a chart of a ¹H-NMR spectrum of a compound C12 obtained in Example 10.
Fig. 23 is a chart of a ¹H-NMR spectrum of a compound C13 obtained in Example 11.
Fig. 24 is a schematic diagram of an apparatus used in light resistance test.

### DESCRIPTION OF EMBODIMENT(S)

### First Exemplary Embodiment

### Compound

A compound according to a first exemplary embodiment has a donor moiety, a π-conjugated moiety, and an acceptor moiety.

The compound according to the first exemplary embodiment satisfies at least one selected from the group consisting of the following conditions (I), (II), and (III).

Condition (I): the donor moiety is represented by a formula (D1) or a formula (D2) below,

Condition (II): the acceptor moiety is represented by a formula (A1) or a formula (A2) below, and

Condition (III): the π-conjugated moiety is represented by a formula (P1), a formula (P2), or a formula (P3) below.

It is also preferable that the compound according to the first exemplary embodiment satisfies only one selected from the group consisting of the conditions (I), (II), and (III). It is also preferable that the compound according to the first exemplary embodiment satisfies only the conditions (I) and (II), only the conditions (II) and (III), or only the conditions (I) and (III). It is also preferable that the compound according to the first exemplary embodiment satisfies at least the condition (I), specifically, satisfies only the conditions (I) and (II) or only the conditions (I) and (III). It is also preferable that the compound according to the first exemplary embodiment satisfies all the conditions (I), (II), and (III).

Herein, when variable elements as variable groups and variable atoms in the compound according to the first exemplary embodiment are present in the number of nx, for example, as Rₙ₁, Rₙ₂, Rₙ₃, ..., Rₙₓ₋₁, and Rₙₓ, the description may be simplified as Rₙ₁ to Rₙₓ regardless of whether the numbers from n1 to nx are consecutive or non-consecutive. For example, when a compound represented by a formula having a specific structure includes variable elements "R₁₁, R₁₂, R₁₃, R₁₄, and R₁₅," "R₁₁, R₁₂, R₁₃, R₁₄, and R₁₅" may be described as "R₁₁ to R₁₅." Similar expressions may be described in the same manner. Variable elements represented by symbols other than R may also be described in the same manner.

### First Aspect

A compound according to a first aspect of the first exemplary embodiment is a compound that satisfies at least the condition (I). Hereinafter, the compound according to the first aspect of the first exemplary embodiment will be described.

### Donor Moiety in Compound according to First Aspect

In the compound according to the first aspect of the first exemplary embodiment, the donor moiety is represented by a formula (D1) or (D2) below.

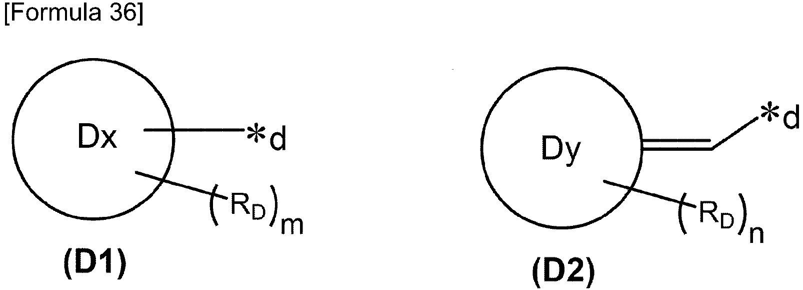

In the formulae (D1) and (D2):
a ring Dx and a ring Dy are each independently a five-membered or six-membered aromatic hydrocarbon ring, a five-membered or six-membered aliphatic hydrocarbon ring, or a five-membered or six-membered heterocycle;
the ring Dx has one or more substituents R_{D};
the ring Dy has one or more substituents R_{D} or has no substituent R_{D};
m is the number of the substituents R_{D} on the ring Dx and is an integer of 1 or more;
n is the number of the substituents R_{D} on the ring Dy and is 0 or an integer of 1 or more;
the substituents R_{D} on the ring Dx are bonded with the ring Dx to form a substituted or unsubstituted monocyclic ring, bonded with the ring Dx to form a substituted or unsubstituted fused ring, or not bonded with the ring Dx;
the substituents R_{D} on the ring Dy are bonded with the ring Dy to form a substituted or unsubstituted monocyclic ring, bonded with the ring Dy to form a substituted or unsubstituted fused ring, or not bonded with the ring Dy;
when the ring Dx has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
when the ring Dy has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
the substituents R_{D} on the ring Dx and the ring Dy are each independently a group represented by -N(R₁₀₁)(R₁₀₂), a group represented by -Si(R₁₀₃)(R₁₀₄)(R₁₀₅), a group represented by -C(=O)R₁₀₆, a group represented by -C(R₁₀₇)(R₁₀₈)(R₁₀₉), a group represented by -O(R₁₁₃), a substituted or unsubstituted alkyl group, a substituted or unsubstituted cycloalkyl group, a substituted or unsubstituted aryl group, or a substituted or unsubstituted heterocyclic group;
R₁₀₁, R₁₀₂, R₁₀₃, R₁₀₄, R₁₀₅, R₁₀₆, R₁₀₇, R₁₀₈, R₁₀₉, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when the number of substituents R_{D} on the ring Dx and the ring Dy is 1 and the one substituent R_{D} is a group represented by -N(R₁₀₁)(R₁₀₂), R₁₀₁ and R₁₀₂ are neither hydrogen atoms nor substituted or unsubstituted alkyl groups;
when the number of the substituents R_{D} on the ring Dx is 2 and one of the two substituents R_{D} is a group represented by -N(R₁₀₁)(R₁₀₂), the other thereof is not a group represented by -O(R₁₁₃);
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when a plurality of R₁₁₃ are present, the plurality of R₁₁₃ are mutually the same or different; and
*d is a bonding position.

In the compound according to the first aspect of the first exemplary embodiment, R₁₀₁ to R₁₀₉ and R₁₁₃ represent the same as R₁₀₁ to R₁₀₉ and R₁₁₃ in the formula (D1) and the formula (D2).

In the compound according to the first aspect of the first exemplary embodiment, it is preferable that a ring Dx and a ring Dy in the formula (D1) or (D2) are each independently a five- or six-membered aromatic hydrocarbon ring or a five- or six-membered heterocycle.

In the compound according to the first aspect of the first exemplary embodiment, it is also preferable that the donor moiety is represented by a formula (D11), (D12), (D13), (D21), or (D22) below.

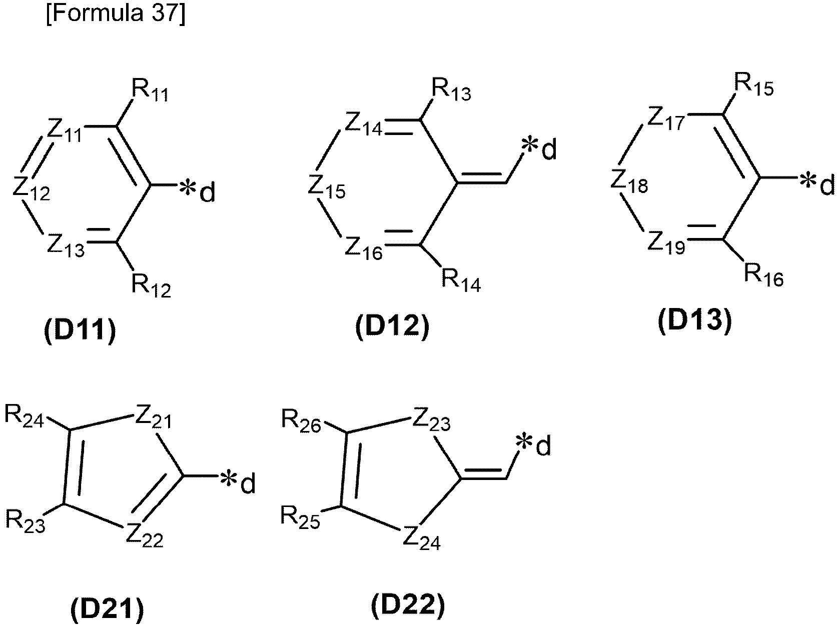

In the groups represented by the formulae (D11), (D12), (D13), (D21), and (D22):
Z₁₁, Z₁₂, Z₁₃, Z₁₄, Z₁₆, and Z₁₉ are each independently C(R₁₀₀);
Z₁₅, Z₁₇, and Z₁₈ are each independently an oxygen atom, a sulfur atom, C=R₁₀₀, N(R₁₁₀), or C(R₁₁₁)(R₁₁₂);
Z₂₁, Z₂₃, and Z₂₄ are each independently an oxygen atom, a sulfur atom, N(R₁₁₀), or C(R₁₁₁)(R₁₁₂);
Z₂₂ is C(R₁₀₀);
at least one combination of adjacent two or more of R₁₁, R₁₂, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₃, R₁₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₅, R₁₆, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₃, R₂₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₅, R₂₆, R₁₁₀, R₁₁₁, and R₁₁₂ are mutually bonded to form a substituted or unsubstituted monocyclic ring, mutually bonded to form a substituted or unsubstituted fused ring, or not mutually bonded;
R₁₁ to R₁₆, R₂₃ to R₂₆, R₁₀₀, and R₁₁₀ to R₁₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₇)(R₁₀₈)(R₁₀₉), or a group represented by -O(R₁₁₃); and
*d is a bonding position.

In the groups represented by the formulae (D11), (D12), (D13), (D21), and (D22), when one of R₁₁ to R₁₆, R₂₃ to R₂₆, R₁₀₀, and R₁₁₀ to R₁₁₂ forming neither the substituted or unsubstituted monocyclic ring nor the substituted or unsubstituted fused ring is a group represented by -N(R₁₀₁)(R₁₀₂), R₁₀₁ and R₁₀₂ are not both hydrogen atoms and are not both substituted or unsubstituted alkyl groups.

In the groups represented by the formulae (D11), (D12), (D13), (D21), and (D22), when one of two selected from the group consisting of R₁₁ to R₁₆, R₂₃ to R₂₆, and R₁₀₀ forming neither the substituted or unsubstituted monocyclic ring nor the substituted or unsubstituted fused ring is a group represented by -N(R₁₀₁)(R₁₀₂), the other is not a group represented by -O(R₁₁₃).

In the compound according to the first aspect of the first exemplary embodiment, it is preferable that the donor moiety is represented by a formula (D111), (D112), (D113), (D121), (D122), (D123), or (D124) below.

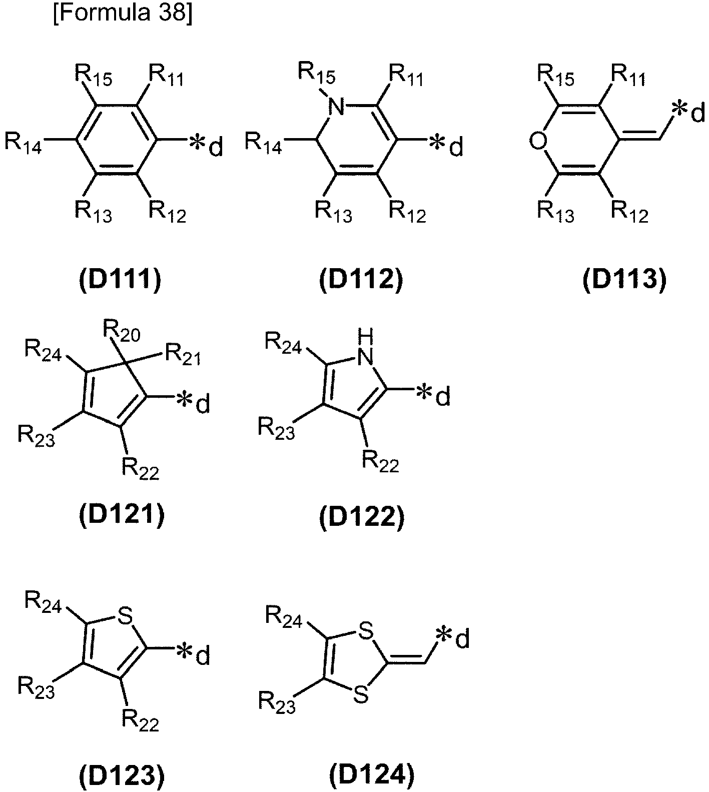

In the groups represented by the formulae (D111), (D112), (D113), (D121), (D122), (D123), and (D124):
at least one combination of adjacent two or more of R₁₁, R₁₂, R₁₃, R₁₄, and R₁₅ are mutually bonded to form a substituted or unsubstituted monocyclic ring, mutually bonded to form a substituted or unsubstituted fused ring, or not mutually bonded;
at least one combination of adjacent two or more of R₂₀, R₂₁, R₂₂, R₂₃, and R₂₄ are mutually bonded to form a substituted or unsubstituted monocyclic ring, mutually bonded to form a substituted or unsubstituted fused ring, or not mutually bonded;
R₁₁ to R₁₅ and R₂₀ to R₂₄ forming neither the substituted or unsubstituted monocyclic ring nor the substituted or unsubstituted fused ring are each independently a hydrogen atom, a group represented by -N(R₁₀₁)(R₁₀₂), a group represented by -Si(R₁₀₃)(R₁₀₄)(R₁₀₅), a group represented by -C(=O)R₁₀₆, a group represented by -O(R₁₁₃), a substituted or unsubstituted alkyl group, a substituted or unsubstituted cycloalkyl group, a substituted or unsubstituted aryl group, or a substituted or unsubstituted heterocyclic group; and
*d represents a bonding position.

In the groups represented by the formulae (D111), (D112), (D113), (D121), (D122), (D123), and (D124), when one of R₁₁ to R₁₅ and R₂₀ to R₂₄ forming neither the substituted or unsubstituted monocyclic ring nor the substituted or unsubstituted fused ring is a group represented by -N(R₁₀₁)(R₁₀₂), R₁₀₁ and R₁₀₂ are not both hydrogen atoms and are not both substituted or unsubstituted alkyl groups.

In the groups represented by the formulae (D111), (D112), (D113), (D121), (D122), (D123), and (D124), when one of two selected from the group consisting of R₁₁ to R₁₅ and R₂₀ to R₂₄ forming neither the substituted or unsubstituted monocyclic ring nor the substituted or unsubstituted fused ring is a group represented by - N(R₁₀₁)(R₁₀₂), the other is not a group represented by -O(R₁₁₃).

In the compound according to the first aspect of the first exemplary embodiment, it is also preferable that the donor moiety is represented by the formula (D111).

In the compound according to the first aspect of the first exemplary embodiment, it is also preferable that none of R₁₁ to R₁₅ in the formula (D111) are bonded to each other.

In the compound according to the first aspect of the first exemplary embodiment, it is also preferable that a combination of R₁₄ and R₁₅ in the formula (D111) are mutually bonded to form a substituted or unsubstituted monocyclic ring or a substituted or unsubstituted fused ring.

In one form of the compound according to the first aspect of the first exemplary embodiment, when one of R₁₁ to R₁₅ in the formula (D111) is a group represented by -N(R₁₀₁)(R₁₀₂), each of the remaining R₁₁ to R₁₅ is not a group represented by -O(R₁₁₃).

In the compound according to the first aspect of the first exemplary embodiment, it is also preferable that R₁₁ to R₁₅ in the formula (D111) are each independently a hydrogen atom or a group represented by -O(R₁₁₃).

In the compound according to the first aspect of the first exemplary embodiment, it is also preferable that at least one of R₁₁ to R₁₅ in the formula (D111) is a group represented by -O(R₁₁₃).

In the compound according to the first aspect of the first exemplary embodiment, it is also preferable that three of R₁₁ to R₁₅ in the formula (D111) are groups represented by -O(R₁₁₃).

In the compound according to the first aspect of the first exemplary embodiment, it is also preferable that three of R₁₁ to R₁₅ in the formula (D111) are groups represented by -O(R₁₁₃) and the remaining two thereof are hydrogen atoms.

In the compound according to the first aspect of the first exemplary embodiment, it is also preferable that, in the formula (D111), R₁₄ is a group represented by -O(R₁₁₃), one of R₁₁ and R₁₅ is a group represented by -O(R₁₁₃) and the other of R₁₁ and R₁₅ is a hydrogen atom, and one of R₁₂ and R₁₃ is a group represented by -O(R₁₁₃) and the other of R₁₂ and R₁₃ is a hydrogen atom.

In the compound according to the first aspect of the first exemplary embodiment, it is also preferable that, in the formula (D111), R₁₁, R₁₂, and R₁₄ are groups represented by -O(R₁₁₃), and R₁₃ and R₁₅ are each a hydrogen atom.

In the compound according to the first aspect of the first exemplary embodiment, it is also preferable that, when at least one selected from the group consisting of R₁₁ to R₁₅ in the formula (D111) is a group represented by -O(R₁₁₃), R₁₁₃ in R₁₁ to R₁₅ is an unsubstituted alkyl group.

In the compound according to the first aspect of the first exemplary embodiment, when at least one selected from the group consisting of R₁₁ to R₁₅ in the formula (D111) is a group represented by -O(R₁₁₃), R₁₁₃ in R₁₁ to R₁₅ is also preferably an unsubstituted alkyl group having 1 to 20 carbon atoms, also preferably an unsubstituted alkyl group having 1 to 10 carbon atoms, also preferably an unsubstituted alkyl group having 1 to 8 carbon atoms, also preferably an unsubstituted alkyl group having 1 to 5 carbon atoms, also preferably an unsubstituted alkyl group having 1 to 3 carbon atoms, and also preferably a methyl group.

In the compound according to the first aspect of the first exemplary embodiment, it is also preferable that the donor moiety is represented by a formula (D111-1A), (D111-1B), (D111-1C), (D111-1D), (D111-1E), (D111-1F), (D111-1G), or (D111-1H) below.

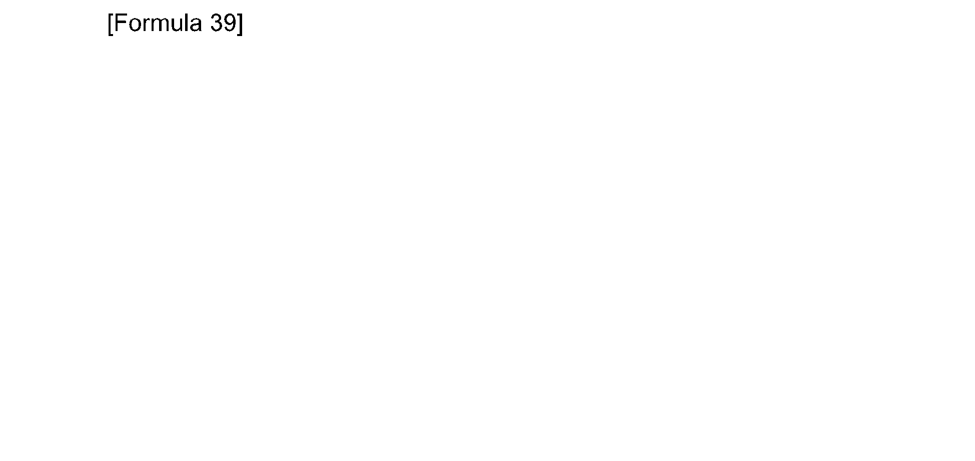

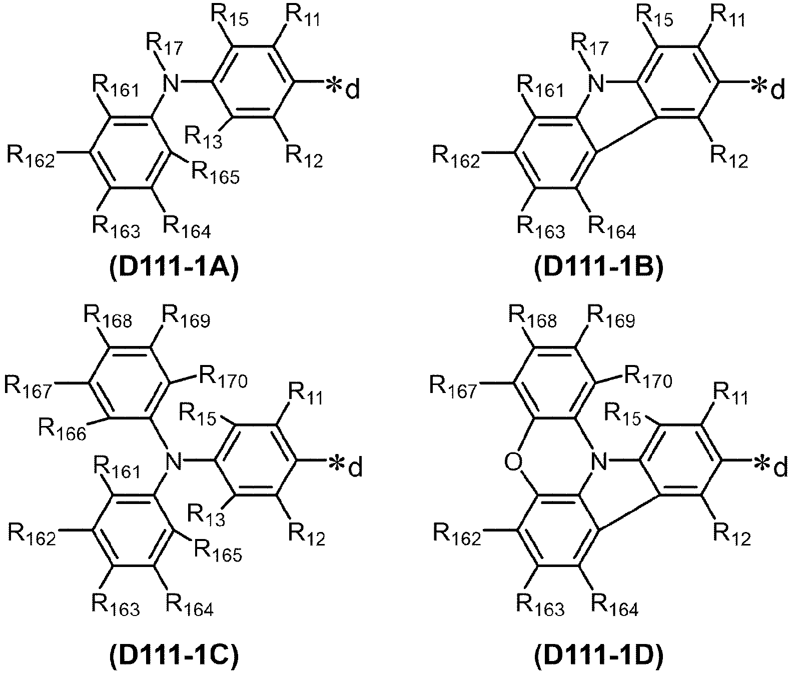

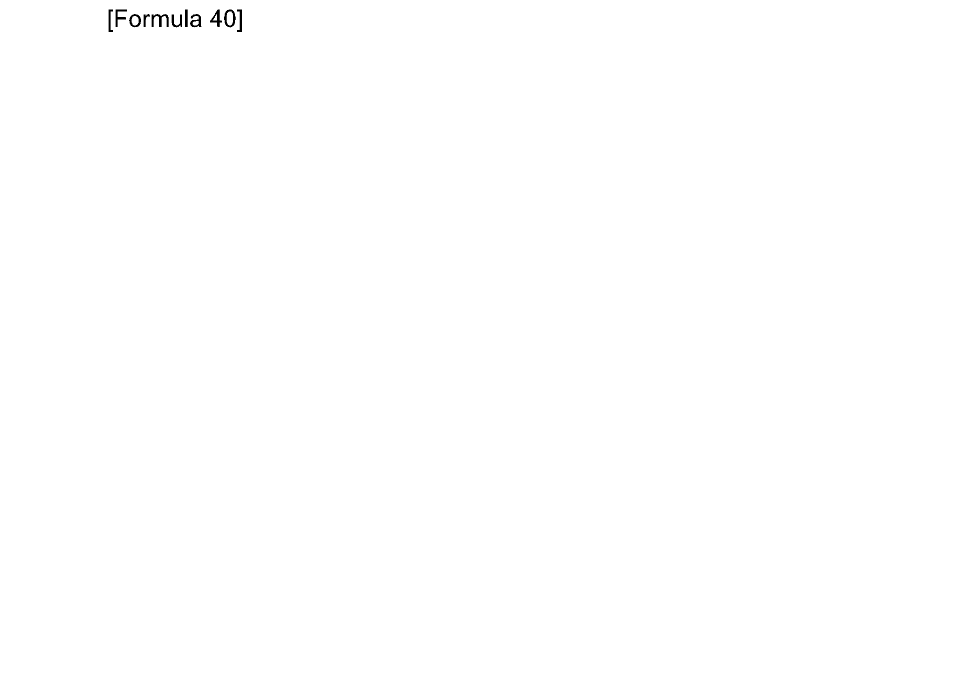

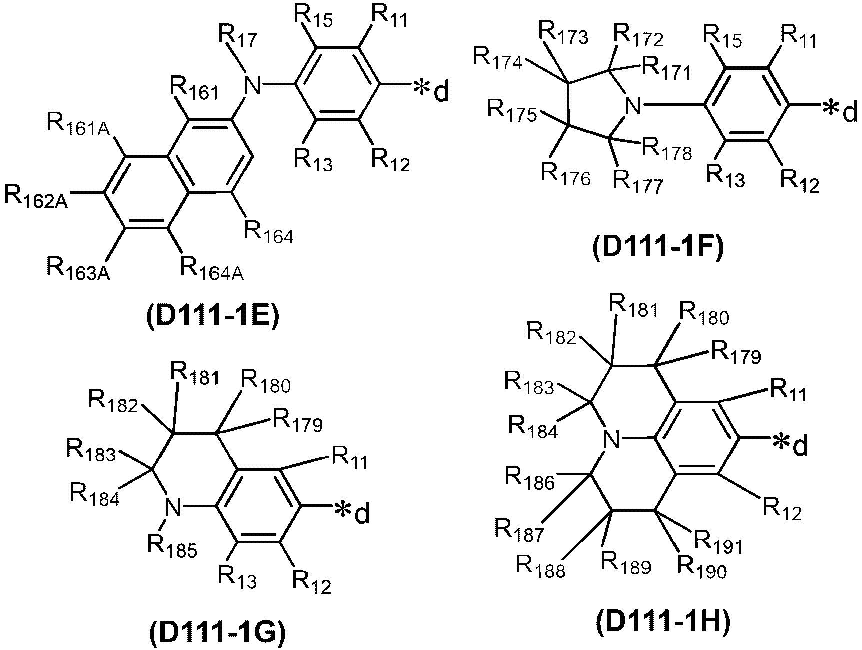

In the formulae (D111-1A), (D111-1B), (D111-1C), (D111-1D), (D111-1E), (D111-1F), (D111-1G), and (D111-1H):
R₁₁, R₁₂, R₁₃, and R₁₅ are each independently a hydrogen atom, a substituted or unsubstituted alkyl group, a substituted or unsubstituted cycloalkyl group, a substituted or unsubstituted aryl group, or a substituted or unsubstituted heterocyclic group;
R₁₇, R₁₆₁, R₁₆₂, R₁₆₃, R₁₆₄, R₁₆₅, R₁₆₆, R₁₆₇, R₁₆₈, R₁₆₉, R₁₇₀, R₁₇₁, R₁₇₂, R₁₇₃, R₁₇₄, R₁₇₅, R₁₇₆, R₁₇₇, R₁₇₈, R₁₇₉, R₁₈₀, R₁₈₁, R₁₈₂, R₁₈₃, R₁₈₄, R₁₈₅, R₁₈₆, R₁₈₇, R₁₈₈, R₁₈₉, R₁₉₀, R₁₉₁, R_{161A}, R_{162A}, R_{163A}, and R_{164A} are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

In the compound according to the first aspect of the first exemplary embodiment, it is also preferable that the donor moiety is represented by a formula (D122-1), (D123-1A), or (D123-1B) below.

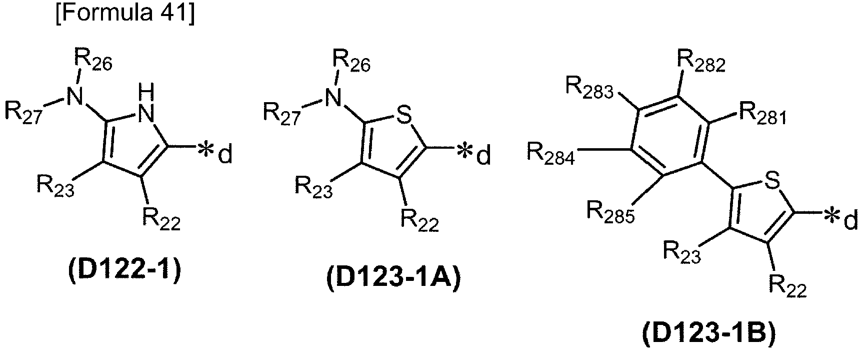

In the formulae (D122-1), (D123-1A), and (D123-1B):
R₂₂, R₂₃, R₂₆, and R₂₇ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃);
at least one combination of adjacent two or more of R₂₈₁, R₂₈₂, R₂₈₃, R₂₈₄, and R₂₈₅ are mutually bonded to form a substituted or unsubstituted monocyclic ring, mutually bonded to form a substituted or unsubstituted fused ring, or not mutually bonded;
R₂₈₁, R₂₈₂, R₂₈₃, R₂₈₄, and R₂₈₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃); and
*d represents a bonding position.

In the compound according to the first aspect of the first exemplary embodiment, it is also preferable that the donor moiety is represented by the formula (D123-1B).

In the compound according to the first aspect of the first exemplary embodiment, it is also preferable that none of R₂₈₁ to R₂₈₅ in the formula (D123-1B) are bonded to each other.

In the compound according to the first aspect of the first exemplary embodiment, it is also preferable that a combination of R₂₈₂ and R₂₈₃ in the formula (D123-1B) are mutually bonded to form a substituted or unsubstituted monocyclic ring or a substituted or unsubstituted fused ring.

In one form of the compound according to the first aspect of the first exemplary embodiment, it is also preferable that, when one of R₂₈₁ to R₂₈₅ in the formula (D123-1B) is a group represented by -N(R₁₀₁)(R₁₀₂), it is also preferable that each of the remaining R₂₈₁ to R₂₈₅ is not a group represented by -O(R₁₁₃).

In the compound according to the first aspect of the first exemplary embodiment, it is also preferable that R₂₈₁ to R₂₈₅ in the formula (D123-1B) are each independently a hydrogen atom, a group represented by -N(R₁₀₁)(R₁₀₂), or a group represented by -O(R₁₁₃).

In the compound according to the first aspect of the first exemplary embodiment, it is also preferable that R₂₈₁ to R₂₈₅ in the formula (D123-1B) are each independently a hydrogen atom or a group represented by -O(R₁₁₃).

In the compound according to the first aspect of the first exemplary embodiment, it is also preferable that R₂₈₁ to R₂₈₅ in the formula (D123-1B) are each independently a hydrogen atom or a group represented by -N(R₁₀₁)(R₁₀₂).

In the compound according to the first aspect of the first exemplary embodiment, it is also preferable that at least one of R₂₈₁ to R₂₈₅ in the formula (D123-1B) is a group represented by -O(R₁₁₃).

In the compound according to the first aspect of the first exemplary embodiment, it is also preferable that three of R₂₈₁ to R₂₈₅ in the formula (D123-1B) are groups represented by -O(R₁₁₃).

In the compound according to the first aspect of the first exemplary embodiment, it is also preferable that three of R₂₈₁ to R₂₈₅ in the formula (D123-1B) are groups represented by -O(R₁₁₃) and the remaining two thereof are hydrogen atoms.

In the compound according to the first aspect of the first exemplary embodiment, it is also preferable that, in the formula (D123-1B), R₂₈₃ is a group represented by -O(R₁₁₃), one of R₂₈₁ and R₂₈₂ is a group represented by -O(R₁₁₃) and the other of R₂₈₁ and R₂₈₂ is a hydrogen atom, and one of R₂₈₄ and R₂₈₅ is a group represented by -O(R₁₁₃) and the other of R₂₈₄ and R₂₈₅ is a hydrogen atom.

In the compound according to the first aspect of the first exemplary embodiment, it is also preferable that, in the formula (D123-1B), R₂₈₁, R₂₈₃, and R₂₈₅ are groups represented by -O(R₁₁₃), and R₂₈₂ and R₂₈₄ are each a hydrogen atom.

In the compound according to the first aspect of the first exemplary embodiment, it is also preferable that, when at least one selected from the group consisting of R₂₈₁ to R₂₈₅ in the formula (D123-1B) is a group represented by -O(R₁₁₃), R₁₁₃ in R₂₈₁ to R₂₈₅ is an unsubstituted alkyl group.

In the compound according to the first aspect of the first exemplary embodiment, when at least one selected from the group consisting of R₂₈₁ to R₂₈₅ in the formula (D123-1B) is a group represented by -O(R₁₁₃), R₁₁₃ in R₂₈₁ to R₂₈₅ is also preferably an unsubstituted alkyl group having 1 to 20 carbon atoms, also preferably an unsubstituted alkyl group having 1 to 10 carbon atoms, also preferably an unsubstituted alkyl group having 1 to 8 carbon atoms, also preferably an unsubstituted alkyl group having 1 to 5 carbon atoms, also preferably an unsubstituted alkyl group having 1 to 3 carbon atoms, and also preferably a methyl group.

In the compound according to the first aspect of the first exemplary embodiment, it is also preferable that one of R₂₈₁ to R₂₈₅ in the formula (D123-1B) is a group represented by -N(R₁₀₁)(R₁₀₂) and the remaining four thereof are hydrogen atoms.

In the compound according to the first aspect of the first exemplary embodiment, it is also preferable that, when at least one selected from the group consisting of R₂₈₁ to R₂₈₅ in the formula (D123-1B) is a group represented by - N(R₁₀₁)(R₁₀₂), R₁₀₁ and R₁₀₂ in R₂₈₁ to R₂₈₅ are each independently a substituted or unsubstituted aryl group.

In the compound according to the first aspect of the first exemplary embodiment, it is also preferable that, when at least one selected from the group consisting of R₂₈₁ to R₂₈₅ in the formula (D123-1B) is a group represented by - N(R₁₀₁)(R₁₀₂), R₁₀₁ and R₁₀₂ in R₂₈₁ to R₂₈₅ are unsubstituted phenyl groups.

In the compound according to the first aspect of the first exemplary embodiment, it is also preferable that the donor moiety is represented by a formula (D123-2A), (D123-3A), or (D123-4A) below.

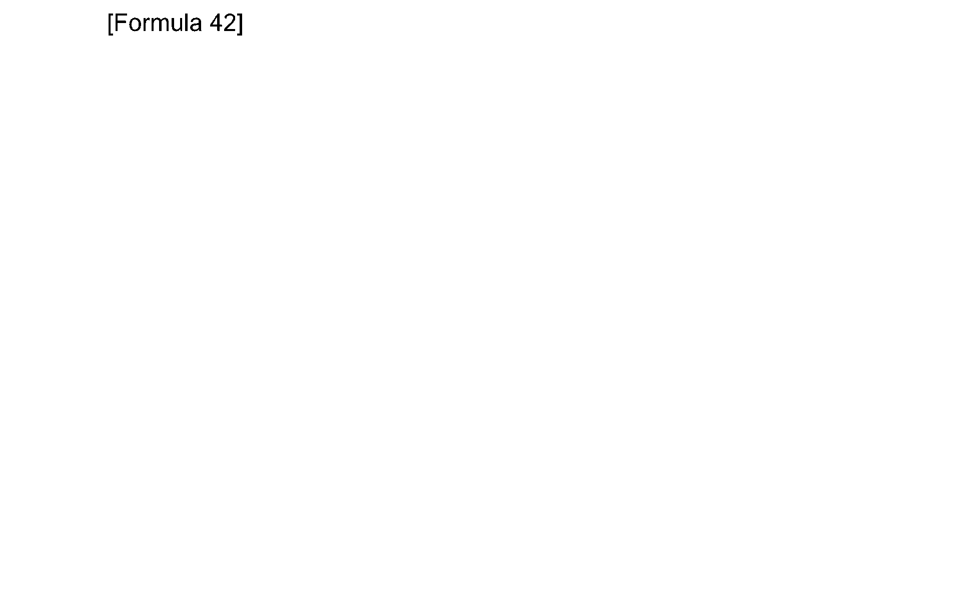

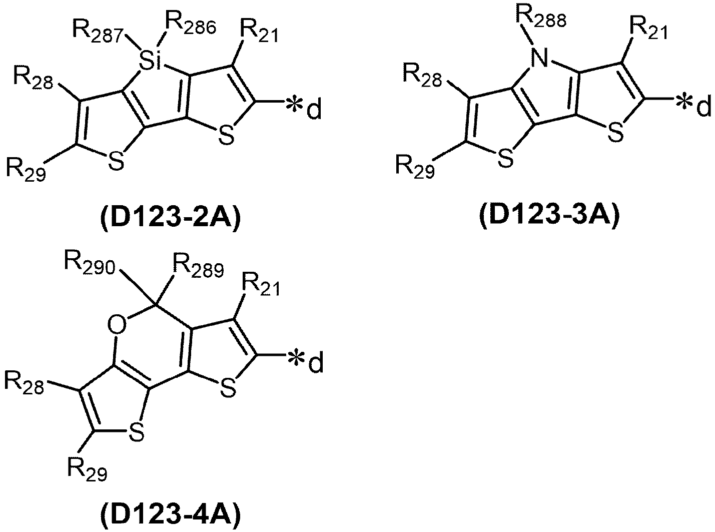

In the formulae (D123-2A), (D123-3A), and (D123-4A):
R₂₁, R₂₈, R₂₉, R₂₈₆, R₂₈₇, R₂₈₈, R₂₈₉, and R₂₉₀ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

In the compound according to the first aspect of the first exemplary embodiment, it is preferable that the donor moiety is represented by a formula (D112-1) or (D121-1) below.

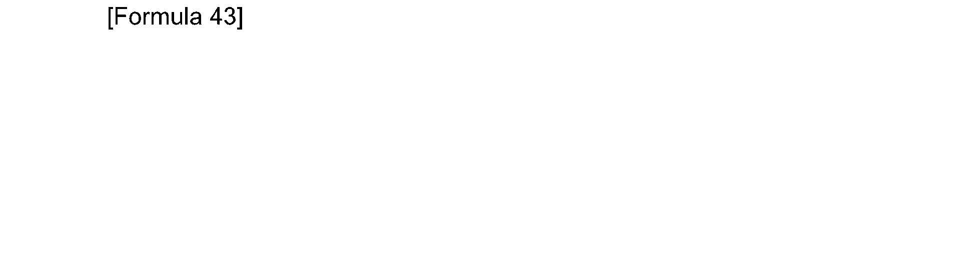

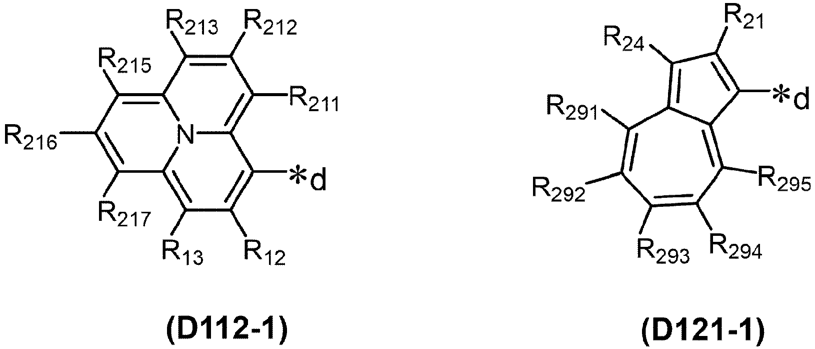

In the formulae (D112-1) and (D121-1):
R₁₂, R₁₃, R₂₁, R₂₄, R₂₁₁, R₂₁₂, R₂₁₃, R₂₁₅, R₂₁₆, R₂₁₇, R₂₉₁, R₂₉₂, R₂₉₃, R₂₉₄, and R₂₉₅ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

In the compound according to the first aspect of the first exemplary embodiment, it is also preferable that the donor moiety is represented by a formula (D111-1A-1) or (D111-1A-2) below.

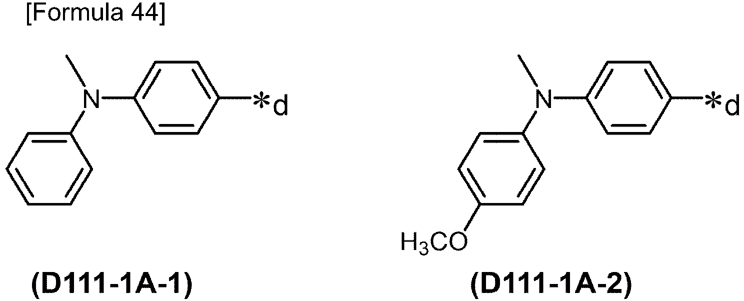

In the formulae (D111-1A-1) and (D111-1A-2), *d represents a bonding position.

In the compound according to the first aspect of the first exemplary embodiment, it is also preferable that the donor moiety is represented by a formula (D111-1B-1), (D111-1C-1), or (D111-1C-2) below.

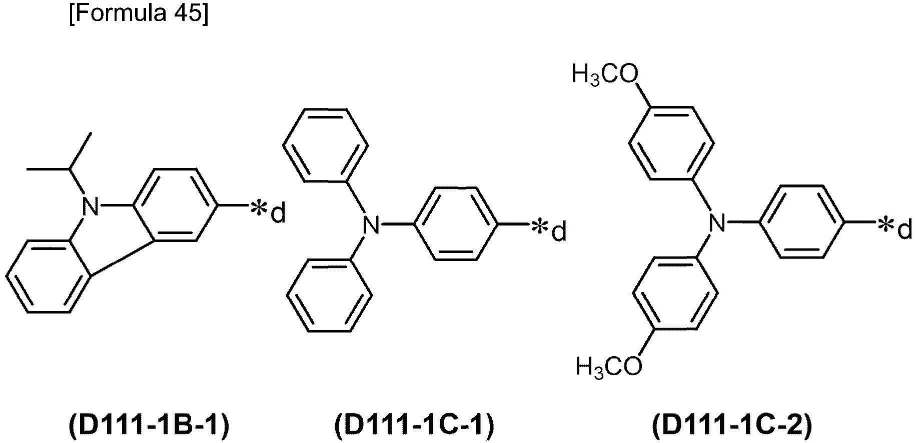

In the formulae (D111-1B-1), (D111-1C-1), and (D111-1C-2), *d represents a bonding position.

In the compound according to the first aspect of the first exemplary embodiment, it is also preferable that the donor moiety is represented by a formula (D111-1D-1), (D111-1E-1), or (D111-1F-1) below.

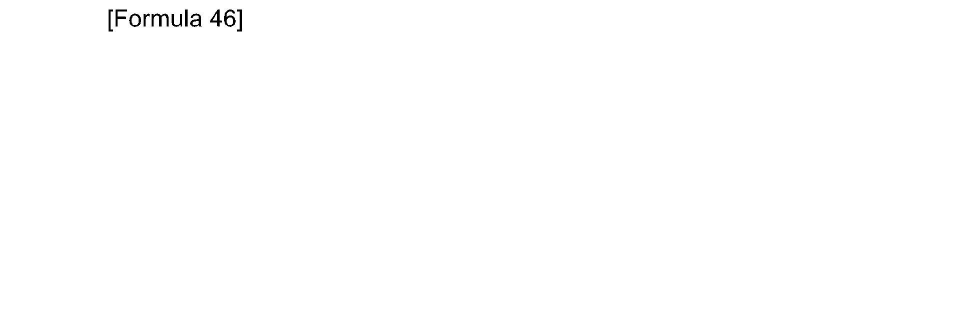

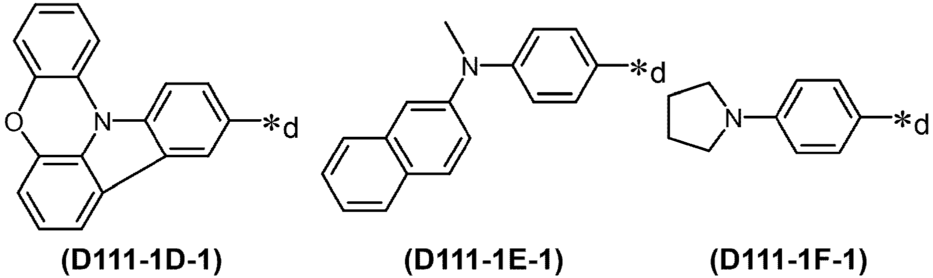

In the formulae (D111-1D-1), (D111-1E-1), and (D111-1F-1), *d represents a bonding position.

In the compound according to the first aspect of the first exemplary embodiment, it is also preferable that the donor moiety is represented by a formula (D111-1G-1) or (D111-1H-1) below.

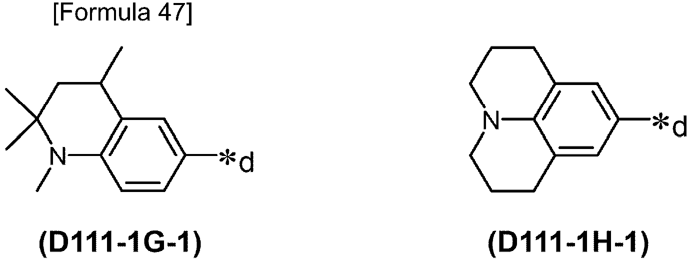

In the formulae (D111-1G-1) and (D111-1H-1), *d represents a bonding position.

In the compound according to the first aspect of the first exemplary embodiment, it is also preferable that the donor moiety is represented by a formula (D111-1J-1) below.

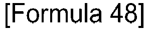

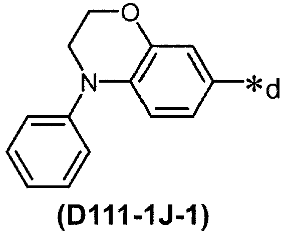

In the formula (D111-1J-1), *d represents a bonding position.

In the compound according to the first aspect of the first exemplary embodiment, it is also preferable that the donor moiety is represented by a formula (D111-2) below.

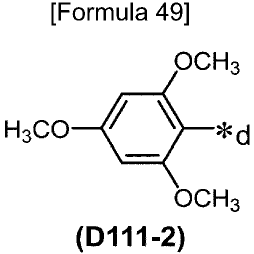

In the formula (D111-2), *d represents a bonding position.

In the compound according to the first aspect of the first exemplary embodiment, it is also preferable that the donor moiety is represented by a formula (D122-1-1), (D123-1A-1), (D123-1B-1), (D123-1B-2), (D123-1B-3), or (D123-1B-4) below.

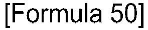

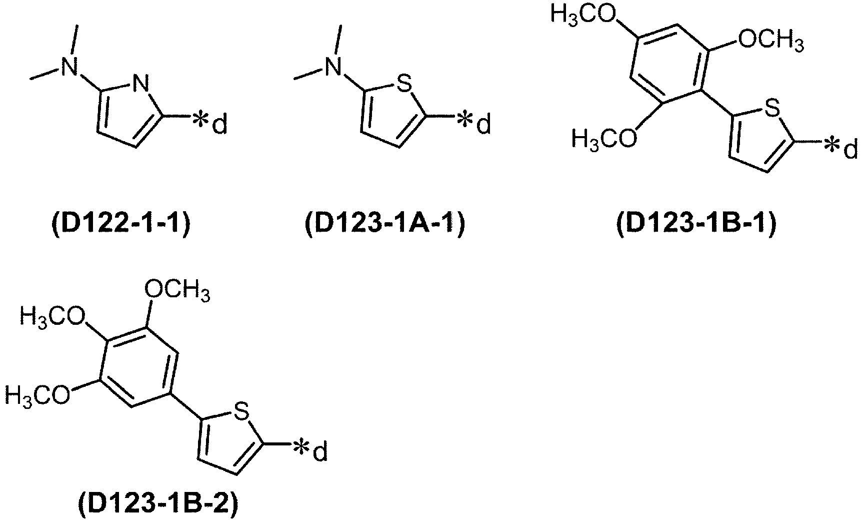

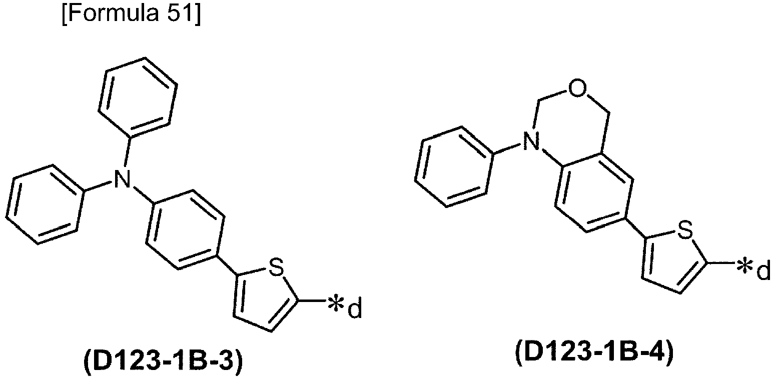

In the formulae (D122-1-1), (D123-1A-1), (D123-1B-1), (D123-1B-2), (D123-1B-3), and (D123-1B-4), *d represents a bonding position.

In the compound according to the first aspect of the first exemplary embodiment, it is also preferable that the donor moiety is represented by a formula (D124-1A-1), (D124-2A-1), or (D124-3A-1) below.

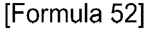

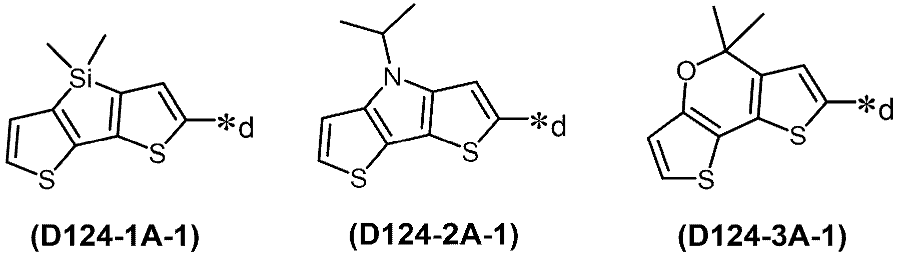

In the formulae (D124-1A-1), (D124-2A-1), and (D124-3A-1), *d represents a bonding position.

In the compound according to the first aspect of the first exemplary embodiment, it is also preferable that the donor moiety is represented by a formula (D112-1-1) or (D121-1-1) below.

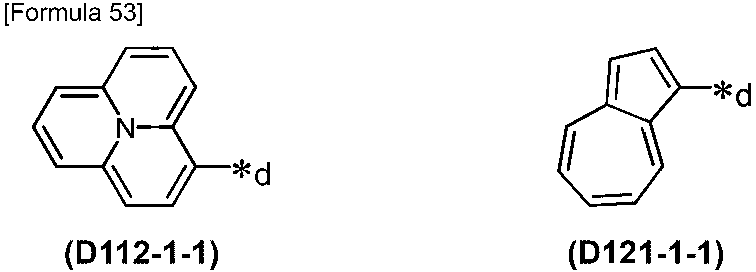

In the formulae (D112-1-1) and (D121-1-1), *d represents a bonding position.

In the compound according to the first aspect of the first exemplary embodiment, it is also preferable that the donor moiety is represented by a formula (D113-1) or (D125-1) below.

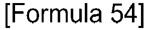

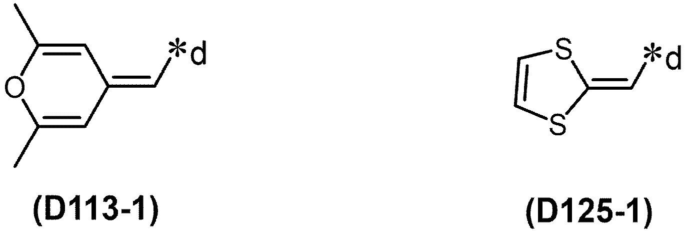

**In** the formulae (D113-1) and (D125-1), *d represents a bonding position.

**It** is preferable that the donor moiety is a group having electron-donating properties.

**In** the donor moiety of the compound according to the first aspect of the first exemplary embodiment, it is preferable that *d as a bonding position is a bonding position to the π-conjugated moiety.

### Acceptor Moiety in Compound according to First Aspect

As the acceptor moiety in the compound according to the first aspect of the first exemplary embodiment, a known acceptor moiety used in an electro-optic compound or an organic nonlinear optical material can be applied. **In** the compound according to the first aspect of the first exemplary embodiment, it is preferable that the acceptor moiety is a group having electron-withdrawing properties.

**In** the compound according to the first aspect of the first exemplary embodiment, it is also preferable that the acceptor moiety is represented by a formula (AX1) or (AX2) below.

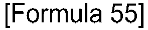

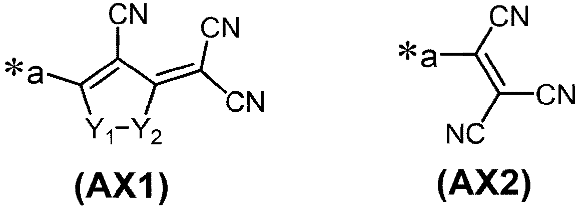

In the formulae (AX1) and (AX2): Y₁ and Y₂ are each independently - C(R_{A30})(R_{A31})-, -C(O)-, -O-, or -NR_{A32}-;
at least one of Y₁ or Y₂ is -O- or -NR_{A32}-;
R_{A30} and R_{A31} are each independently a hydrogen atom, a substituted or unsubstituted alkyl group, or a substituted or unsubstituted aryl group, R_{A32} is a hydrogen atom or a substituted or unsubstituted alkyl group; and
*a represents a bonding position.

In the first aspect, a second aspect, and a third aspect of the first exemplary embodiment, CN represents a cyano group.

In the compound according to the first aspect of the first exemplary embodiment, it is also preferable that the acceptor moiety is represented by a formula (AX11) or (AX21) below.

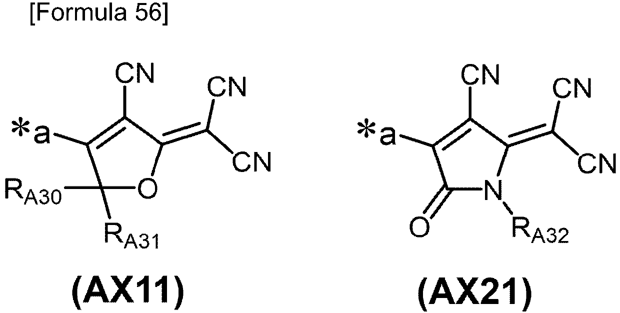

In the formulae (AX11) and (AX21):
R_{A30} and R_{A31} are each independently a hydrogen atom, a substituted or unsubstituted alkyl group, or a substituted or unsubstituted aryl group, R_{A32} is a hydrogen atom or a substituted or unsubstituted alkyl group; and
*a represents a bonding position.

In the acceptor moiety of the compound according to the first aspect of the first exemplary embodiment, at least one of R_{A30} or R_{A31} is preferably a substituted alkyl group, more preferably a haloalkyl group substituted with a halogen atom, and still more preferably a trifluoromethyl group.

In the compound according to the first aspect of the first exemplary embodiment, it is also preferable that the acceptor moiety is represented by a formula (AX11-1), (AX11-2), (AX21-1), or (AX21-2) below.

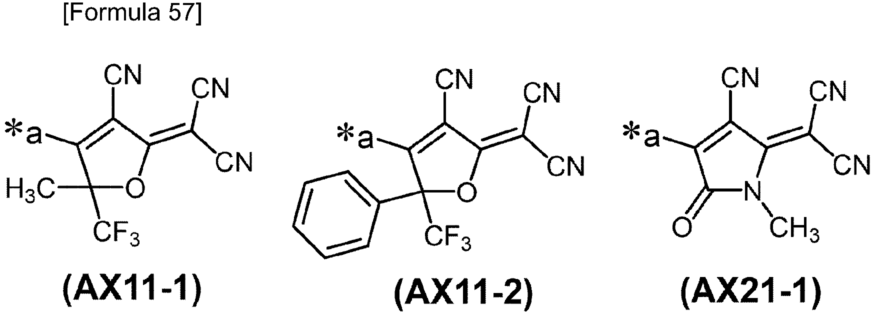

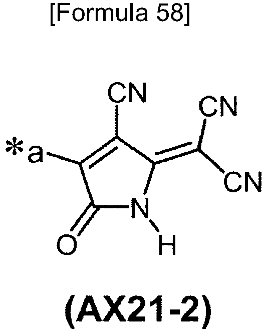

In the acceptor moiety of the compound according to the first aspect of the first exemplary embodiment, it is preferable that *a is a bonding position to the π-conjugated moiety.

In the compound according to the first aspect of the first exemplary embodiment, it is also preferable that the acceptor moiety is an acceptor moiety in a compound according to a second aspect described later.

In the compound according to the first aspect of the first exemplary embodiment, it is also preferable that the acceptor moiety is represented by a formula (A1) or (A2) in the compound according to the second aspect described later.

In the compound according to the first aspect of the first exemplary embodiment, when the acceptor moiety is the acceptor moiety in the compound according to the second aspect described later, it is preferable that the acceptor moiety is represented by the formula (A1) in the compound according to the second aspect described later.

In the compound according to the first aspect of the first exemplary embodiment, when the acceptor moiety is represented by the formula (A1) in the compound according to the second aspect described later, the acceptor moiety is also preferably represented by a formula (A11), more preferably represented by a formula (A11-b), still more preferably represented by a formula (A11-2), and still further more preferably represented by a formula (A111-2), in the compound according to the second aspect described below.

### π-conjugated Moiety in Compound according to First Aspect

As the π-conjugated moiety in the compound according to the first aspect of the first exemplary embodiment, a known π-conjugated moiety used in an organic nonlinear optical material or an electro-optic compound can be applied. The π-conjugated moiety may also be referred to as a π-conjugated bridge.

In the compound according to the first aspect of the first exemplary embodiment, it is preferable that the π-conjugated moiety has a conjugated system having conjugated multiple bonds. In the compound according to the first aspect of the first exemplary embodiment, for example, as long as π electrons are delocalized from the donor moiety to the acceptor moiety through molecular orbitals within the π-conjugated moiety, the π-conjugated moiety is not particularly limited. The conjugated multiple bonds of the π-conjugated moiety may be either double bonds or triple bonds, and are preferably double bonds. Examples of the π-conjugation include carbon-carbon conjugation and conjugation containing nitrogen.

In the compound according to the first aspect of the first exemplary embodiment, the π-conjugated moiety is also preferably represented by a formula (PX1), (PX2), (PX3), (PX4), (PX5), or (PX11) below.

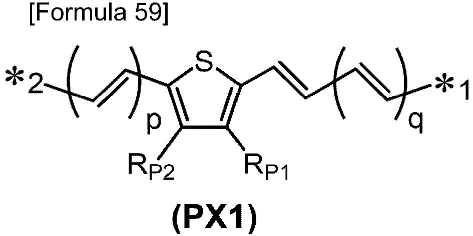

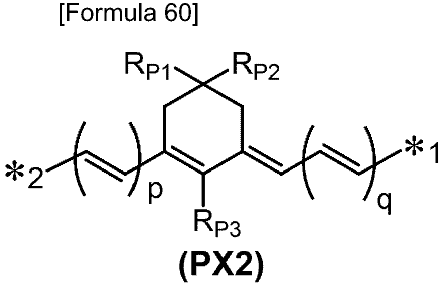

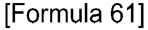

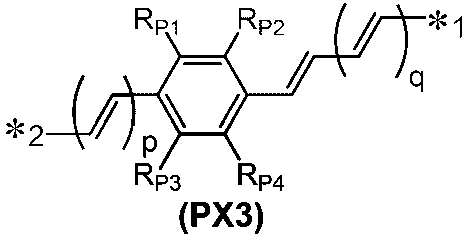

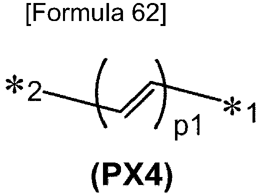

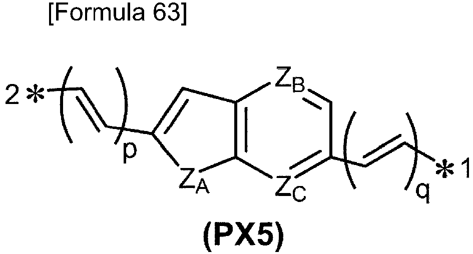

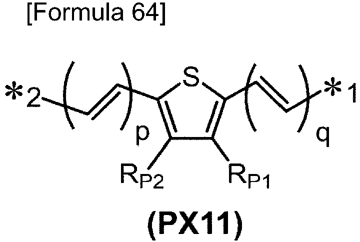

In the formulae (PX1), (PX2), (PX3), (PX5), and (PX11):
p and q are each independently 0, 1, 2, or 3;
in the formulae (PX1) and (PX11):
   at least one combination of adjacent two or more of R_{P1} and R_{P2} are mutually bonded to form a substituted or unsubstituted monocyclic ring, mutually bonded to form a substituted or unsubstituted fused ring, or not mutually bonded;
   R_{P1} and R_{P2} forming neither the substituted or unsubstituted monocyclic ring nor the substituted or unsubstituted fused ring, R_{P1}, R_{P2}, and R_{P3} in the formula (PX2), and R_{P1}, R_{P2}, R_{P3}, and R_{P4} in the formula (PX3) are each independently a hydrogen atom, a substituted or unsubstituted alkyl group, a substituted or unsubstituted alkoxy group, a substituted or unsubstituted aryl group, a substituted or unsubstituted alkenyl group, a substituted or unsubstituted cycloalkyl group, a substituted or unsubstituted cycloalkenyl group, a substituted or unsubstituted haloalkyl group, a substituted or unsubstituted aralkyl group, a substituted or unsubstituted aryloxy group, or a substituted or unsubstituted aralkyloxy group; and
   *1 and *2 are each a bonding position,
   in the formula (PX4):
      p1 is 1, 2, or 3;
      *1 and *2 are each a bonding position,
      in the formula (PX5):
         Z_{B} and Z_{C} are each independently C(R₁₀₀) or a nitrogen atom;
         a plurality of R₁₀₀ are mutually the same or different;
         Z_{A} is each independently an oxygen atom, a sulfur atom, N(R₁₁₀), or C(R₁₁₁)(R₁₁₂);
         when two or more R₁₀₀ are present, at least one combination of adjacent two or more of R₁₀₀ are mutually bonded to form a substituted or unsubstituted monocyclic ring, mutually bonded to form a substituted or unsubstituted fused ring, or not mutually bonded;
         R₁₀₀ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by -N(R₁₀₁)(R₁₀₂), a group represented by - Si(R₁₀₃)(R₁₀₄)(R₁₀₅), a group represented by -C(R₁₀₇)(R₁₀₈)(R₁₀₉), or a group represented by -O(R₁₁₃); and
         *1 and *2 each represent a bonding position.

In the π-conjugated moiety of the compound according to the first aspect of the first exemplary embodiment, it is also preferable that p is 1 and q is 0, and it is also preferable that p is 1 and q is 1. In the compound according to the first aspect of the first exemplary embodiment, it is also preferable that p1 is 1.

In the compound according to the first aspect of the first exemplary embodiment, the π-conjugated moiety is also preferably represented by a formula (PX1-1), (PX2-1), or (PX3-1) below. In the compound according to the first aspect of the first exemplary embodiment, the π-conjugated moiety is also preferably represented by a formula (PX1-1) below. The formula (PX1-1) represents the π-conjugated moiety represented by the formula (PX1) in which p is 1 and q is 0. The formula (PX2-1) represents the π-conjugated moiety represented by the formula (PX2) in which p is 1 and q is 0. The formula (PX3-1) represents the π-conjugated moiety represented by the formula (PX3) in which p is 1 and q is 0.

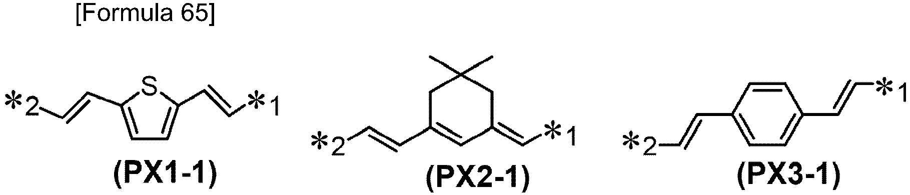

In the compound according to the first aspect of the first exemplary embodiment, the π-conjugated moiety is also preferably represented by a formula (PX11-1) or (PX11-2) below. In the compound according to the first aspect of the first exemplary embodiment, the π-conjugated moiety is also preferably represented by a formula (PX4-1) below. The formula (PX11-1) represents the π-conjugated moiety represented by the formula (PX11) in which p is 0 and q is 1. The formula (PX11-2) represents the π-conjugated moiety represented by the formula (PX11) in which p is 1 and q is 0. The formula (PX4-1) represents the π-conjugated moiety represented by the formula (PX4) in which p1 is 1.

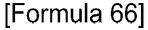

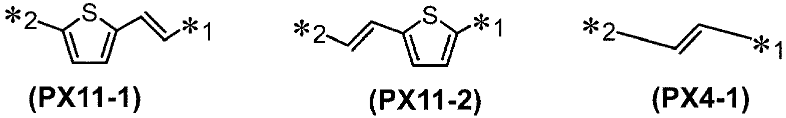

In the compound according to the first aspect of the first exemplary embodiment, the π-conjugated moiety is also preferably represented by a formula (PX5-1) below. The formula (PX5-1) represents the π-conjugated moiety represented by the formula (PX5) in which p is 1, q is 1, Z_{A} is an oxygen atom, Z_{B} and Z_{C} are each C(R₁₀₀), and R₁₀₀ is a hydrogen atom.

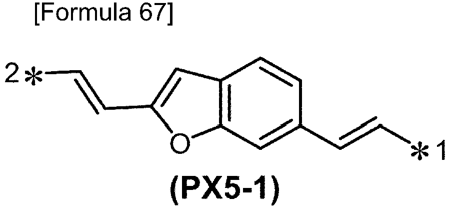

In the compound according to the first aspect of the first exemplary embodiment, when the π-conjugated moiety is represented by the formula (P1) in the compound according to the third aspect described later, it is also preferable that the π-conjugated moiety is represented by the formula (P15-1), more preferably represented by the formula (P15-1A), and still more preferably represented by the formula (P15-1A-3), in the compound according to the third aspect described later.

In the π-conjugated moiety of the compound according to the first aspect of the first exemplary embodiment, it is preferable that one of *1 and *2 is a bonding position to the acceptor moiety, and the other of *1 and *2 is a bonding position to the donor moiety.

In the compound according to the first aspect of the first exemplary embodiment, it is also preferable that the π-conjugated moiety is the π-conjugated moiety in the compound according to the third aspect described later.

In the compound according to the first aspect of the first exemplary embodiment, it is also preferable that the π-conjugated moiety is represented by the formula (P1), (P2), or (P3) in the compound according to the third aspect described later.

### Second Aspect

A compound according to the second aspect of the first exemplary embodiment is a compound that satisfies at least the condition (II). Hereinafter, the compound according to the second aspect of the first exemplary embodiment will be described.

### Acceptor Moiety in Compound according to Second Aspect

In the compound according to the second aspect of the first exemplary embodiment, an acceptor moiety is represented by a formula (A1) or (A2) below.

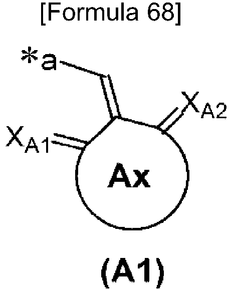

In the formula (A1):
X_{A1} and X_{A2} are each independently an oxygen atom (O) or C(CN)₂;
when one of X_{A1} and X_{A2} is O and the other of X_{A1} and X_{A2} is C(CN)₂, the ring to which X_{A1} and X_{A2} are directly bonded is not a five-membered ring;
the ring Ax is a substituted or unsubstituted monocyclic aromatic hydrocarbon group, a substituted or unsubstituted polycyclic aromatic hydrocarbon ring, a substituted or unsubstituted monocyclic aliphatic hydrocarbon ring, a substituted or unsubstituted polycyclic aliphatic hydrocarbon ring, a substituted or unsubstituted monocyclic heterocycle, or a substituted or unsubstituted polycyclic heterocycle; and
*a represents a bonding position.

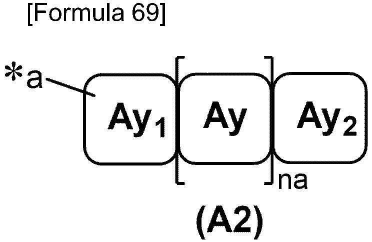

In the formula (A2):
na represents the number of rings Ay, and na is 0 or an integer of 1 or more;
the rings Ay, a ring Ay₁ and a ring Ay₂ are each independently a substituted or unsubstituted monocyclic aromatic hydrocarbon group, a substituted or unsubstituted monocyclic aliphatic hydrocarbon ring, or a substituted or unsubstituted monocyclic heterocycle;
when na is 0, the ring Ay₁ and the ring Ay₂ are fused to each other;
when na is an integer of 1 or more, the ring Ay, the ring Ay₁, and the ring Ay₂ are each independently fused to an adjacent ring;
when na is an integer of 2 or more, the ring Ay₁, the ring Ay₂, and two or more rings Ay include a fused ring structure in which the rings are fused in series to adjacent rings or a fused ring structure in which the rings are fused in a branched manner to adjacent rings; and
*a represents a bonding position.

In the compound according to the second aspect of the first exemplary embodiment, it is preferable that the acceptor moiety is represented by a formula (A11) or (A12) below.

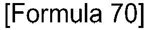

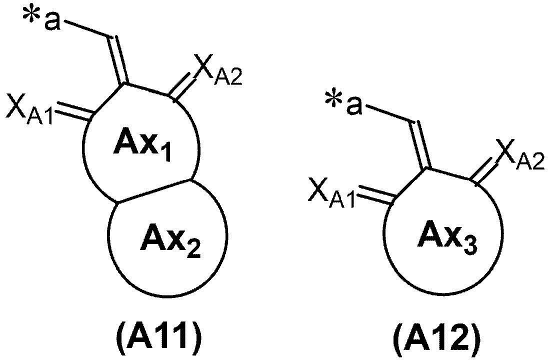

In the formulae (A11) and (A12):
X_{A1} and X_{A2} respectively represent the same as X_{A1} and X_{A2} in the formula (A1);
rings Ax₁, Ax₂, and Ax₃ are each independently a substituted or unsubstituted monocyclic aromatic hydrocarbon group, a substituted or unsubstituted monocyclic aliphatic hydrocarbon ring, or a substituted or unsubstituted monocyclic heterocycle; and
*a represents a bonding position.

In the compound according to the second aspect of the first exemplary embodiment, it is preferable that the acceptor moiety is represented by a formula (A11-a), (A11-b), or (A12-a) below.

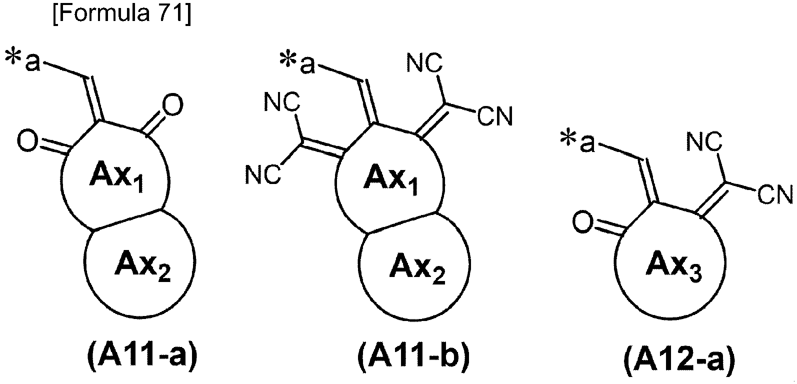

In the formulae (A11-a), (A11-b), and (A12-a):
rings Ax₁, Ax₂, and Ax₃ are each independently a substituted or unsubstituted monocyclic aromatic hydrocarbon group, a substituted or unsubstituted monocyclic aliphatic hydrocarbon ring, or a substituted or unsubstituted monocyclic heterocycle; and
*a represents a bonding position.

In the compound according to the second aspect of the first exemplary embodiment, it is preferable that the ring Ax₁ is a five-membered ring and the ring Ax₂ is a six-membered ring.

In the compound according to the second aspect of the first exemplary embodiment, it is preferable that the ring Ax₃ is a six-membered ring.

In the formula (A11), when one of X_{A1} and X_{A2} is O and the other of X_{A1} and X_{A2} is C(CN)₂, the ring Ax₁ to which X_{A1} and X_{A2} are directly bonded is not a five-membered ring.

In the formula (A12), when one of X_{A1} and X_{A2} is O and the other of X_{A1} and X_{A2} is C(CN)₂, the ring Ax₃ to which X_{A1} and X_{A2} are directly bonded is not a five-membered ring.

In the compound according to the second aspect of the first exemplary embodiment, it is also preferable that the acceptor moiety is represented by a formula (A11-c) or (A12-b) below.

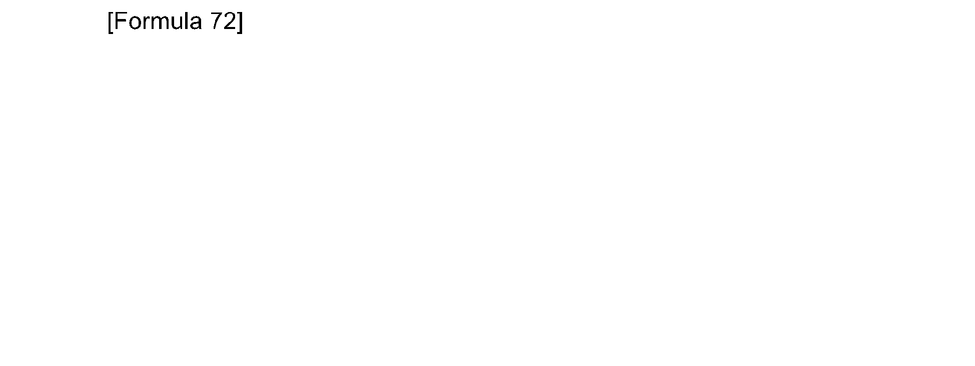

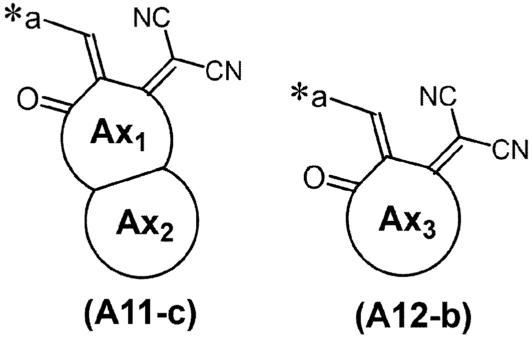

In the formula (A11-c) and the formula (A12-b): the ring Ax₁, the ring Ax₂, and the ring Ax₃ are each independently a substituted or unsubstituted monocyclic aromatic hydrocarbon ring, a substituted or unsubstituted monocyclic aliphatic hydrocarbon ring, or a substituted or unsubstituted monocyclic heterocycle; the ring Ax₁ and the ring Ax₃ are not five-membered rings; and *a represents a bonding position.

In the compound according to the second aspect of the first exemplary embodiment, it is preferable that the acceptor moiety is represented by a formula (A11-1), (A11-2) or (A12-1) below.

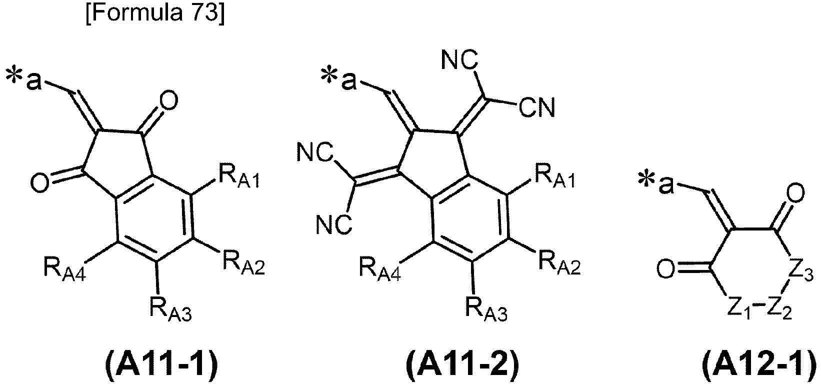

In the formulae (A11-1) and (A11-2):
R_{A1}, R_{A2}, R_{A3}, and R_{A4} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx,
in the formula (A12-1):
   Z₁, Z₂, and Z₃ are each independently N(R_{A5}), C=O, or C(R_{A6})(R_{A7});
   R_{A5}, R_{A6}, and R_{A7} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx;
   Rx is a substituted or unsubstituted alkyl group, and when a plurality of Rx are present, the plurality of Rx are mutually the same or different; and
   *a represents a bonding position.

In the formula (A12-1), Z₂ is preferably C=O.

In the formula (A12-1), it is preferable that Z₁ and Z₃ are each independently N(R_{A5}), two R_{A5} being mutually the same or different.

In the compound according to the second aspect of the first exemplary embodiment, it is also preferable that the acceptor moiety is represented by a formula (A12-1a) below.

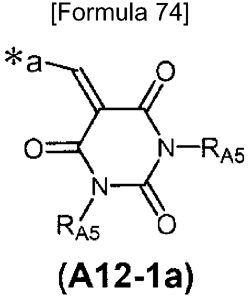

In the formula (A12-1a), R_{A5} represents the same as R_{A5} in the formula (A12-1), two R_{A5} are mutually the same or different, and *a represents a bonding position.

In the compound according to the second aspect of the first exemplary embodiment, it is preferable that the acceptor moiety is represented by a formula (A111-1), (A111-2), (A112-1), or (A121-1) below.

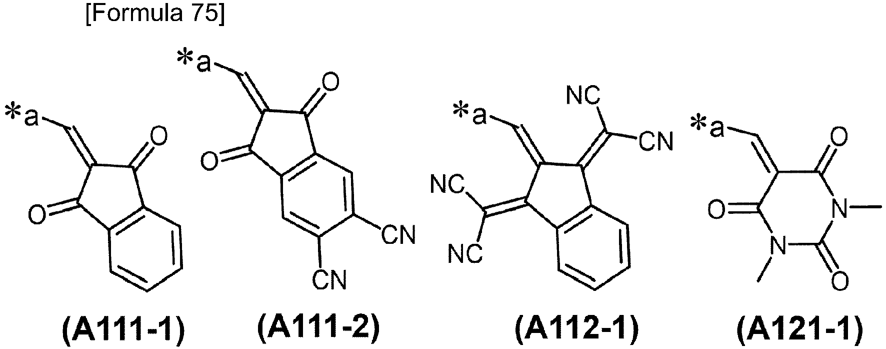

In the formulae (A111-1), (A111-2), (A112-1), and (A121-1), *a represents a bonding position.

In the compound according to the second aspect of the first exemplary embodiment, it is preferable that *a as a bonding position is a bonding position to the π-conjugated moiety.

In the compound according to the second aspect of the first exemplary embodiment, it is preferable that na in the formula (A2) is 1 or 2.

In the compound according to the second aspect of the first exemplary embodiment, it is preferable that the acceptor moiety is represented by a formula (A21) or (A22) below.

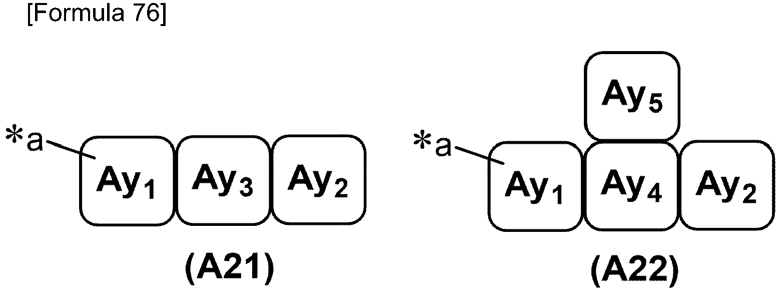

In the formula (A21):
the ring Ay₁ and the ring Ay₂ represent the same as the ring Ay₁ and the ring Ay₂ in the formula (A2), respectively; and
the ring Ay₃ represents the same as the ring Ay in the formula (A2),
in the formula (A22):
   the ring Ay₁ and the ring Ay₂ represent the same as the ring Ay₁ and the ring Ay₂ in the formula (A2), respectively; and
   the ring Ay₄ and the ring Ay₅ each independently represent the same as the ring Ay in the formula (A2), and
   *a represents a bonding position.

In the compound according to the second aspect of the first exemplary embodiment, it is preferable that at least one ring selected from the group consisting of the ring Ay, the ring Ay₁, the ring Ay₂, the ring Ay₃, the ring Ay₄, and the ring Ay₅ includes at least one structure selected from the group consisting of a structure represented by a formula (A200) below, a structure represented by a formula (A201) below, a structure represented by a formula (A202) below, and a structure represented by a formula (A203) below.

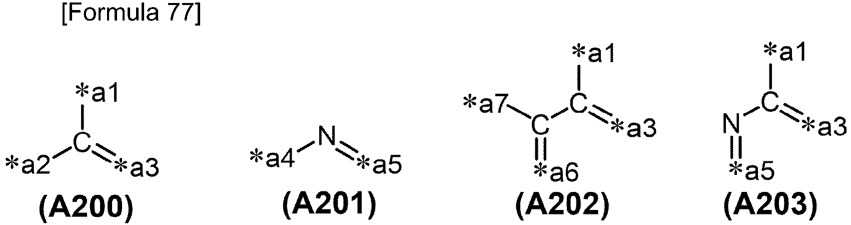

In the formulae (A200), (A201), (A202), and (A203), *a1, *a2, *a3, *a4, *a5, *a6, and *a7 each represent a bonding position.

In the compound according to the second aspect of the first exemplary embodiment, it is preferable that the ring Ay₁ includes at least one structure selected from the group consisting of a structure represented by the formula (A200), a structure represented by the formula (A201), a structure represented by the formula (A202), and a structure represented by the formula (A203).

In the compound according to the second aspect of the first exemplary embodiment, it is also preferable that the ring Ay₁ is a six-membered ring.

In the compound according to the second aspect of the first exemplary embodiment, it is preferable that the ring Ay₁ and the ring Ay₂ are each independently a nitrogen-containing ring.

In the compound according to the second aspect of the first exemplary embodiment, it is preferable that the ring Ay₁ is a six-membered nitrogen-containing ring, more preferably a substituted or unsubstituted azine ring, and still more preferably a substituted or unsubstituted pyrazine ring.

In the compound according to the second aspect of the first exemplary embodiment, it is preferable that the ring Ay₁ and the ring Ay₂ are each independently a six-membered nitrogen-containing ring, more preferably a substituted or unsubstituted azine ring, and still more preferably a substituted or unsubstituted pyrazine ring.

In the compound according to the second aspect of the first exemplary embodiment, it is also preferable that the ring Ay₁ is a benzene ring.

In the compound according to the second aspect of the first exemplary embodiment, it is preferable that the ring Ay₃ is a five-membered ring or a six-membered ring. In the compound according to the second aspect of the first exemplary embodiment, it is preferable that the ring Ay₃ is a five-membered ring or a six-membered ring and has one or more structures selected from the group consisting of CN, C=O, and C=C(CN)₂.

In the compound according to the second aspect of the first exemplary embodiment, it is preferable that the acceptor moiety is represented by a formula (A21-a) or (A21-b) below.

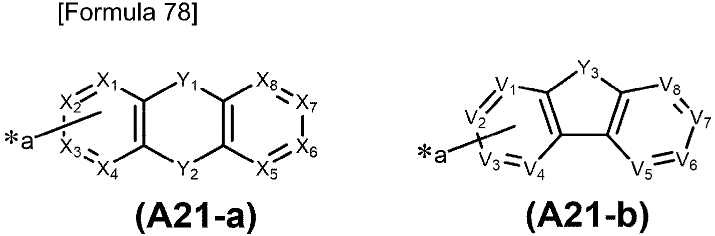

In the formula (A21-a):
Y₁ and Y₂ are each independently C=O, C=C(CN)₂, N(R_{A31}), or C(R_{A32})(R_{A33});
*a represents a bonding position, one selected from the group consisting of X₁, X₂, X₃, and X₄ is a carbon atom bonded to *a by a single bond, and
X₁, X₂, X₃, and X₄ not being the carbon atom bonded to *a by a single bond, and X₅, X₆, X₇, and X₈ are each independently a nitrogen atom or C(R_{A34}),
in the formula (A21-b):
   Y₃ is C=O, C=C(CN)₂, N(R_{A31}), or C(R_{A32})(R_{A33});
   *a represents a bonding position, one selected from the group consisting of V₁, V₂, V₃, and V₄ is a carbon atom bonded to *a by a single bond; and
   V₁, V₂, V₃, and V₄ not being the carbon atom bonded to *a by a single bond, and V₅, V₆, V₇, and V₈ are each independently a nitrogen atom or C(R_{A34}),
   in the formula (A21-a) and the formula (A21-b): R_{A31}, R_{A32}, R_{A33}, and R_{A34} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx; Rx is a substituted or unsubstituted alkyl group; when a plurality of Rx are present, the plurality of Rx are mutually the same or different; a plurality of R_{A31} are mutually the same or different; a plurality of R_{A32} are mutually the same or different; a plurality of R_{A33} are mutually the same or different; and a plurality of R_{A34} are mutually the same or different.

In the compound according to the second aspect of the first exemplary embodiment, it is preferable that at least one selected from the group consisting of X₁, X₂, X₃, and X₄ is a nitrogen atom, and at least one selected from the group consisting of X₅, X₆, X₇, and X₈ is a nitrogen atom.

In the compound according to the second aspect of the first exemplary embodiment, it is preferable that two selected from the group consisting of X₁, X₂, X₃, and X₄ are each a nitrogen atom, and two selected from the group consisting of X₅, X₆, X₇, and X₈ are each a nitrogen atom.

In the compound according to the second aspect of the first exemplary embodiment, it is preferable that one or both of Y₁ and Y₂ are C=O.

In the compound according to the second aspect of the first exemplary embodiment, V₁, V₂, V₃, and V₄ not being the carbon atom bonded to *a by a single bond, and V₅, V₆, V₇, and V₈ are preferably C(R_{A34}), and more preferably CH.

In the compound according to the second aspect of the first exemplary embodiment, Y₃ is preferably C=C(CN)₂.

In the compound according to the second aspect of the first exemplary embodiment, it is preferable that the ring Ay₁, the ring Ay₂, and the ring Ay₅ are each a nitrogen-containing ring. In the compound according to the second aspect of the first exemplary embodiment, it is preferable that the ring Ay₁, the ring Ay₂, and the ring Ay₅ are preferably a six-membered nitrogen-containing ring, more preferably a substituted or unsubstituted azine ring, and still more preferably a substituted or unsubstituted pyrazine ring.

In the compound according to the second aspect of the first exemplary embodiment, it is also preferable that the acceptor moiety is represented by a formula (A22-a) below.

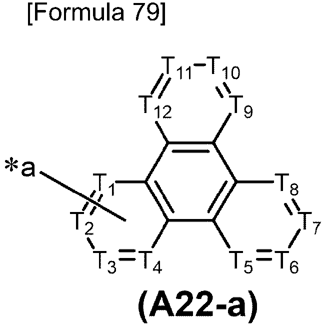

In the formula (A22-a):
*a represents a bonding position;
one selected from the group consisting of T₁, T₂, T₃, and T₄ is a carbon atom bonded to *a by a single bond;
T₁, T₂, T₃, and T₄ not being the carbon atom bonded to *a by a single bond, and T₅, T₆, T₇, T₈, T₉, T_{10,} T₁₁, and T₁₂ are each independently a nitrogen atom or C(R_{A35});
R_{A35} is a hydrogen atom, a halogen atom, a cyano group, a substituted or unsubstituted alkyl group, a substituted or unsubstituted aryl group, -NO₂, -CHO, - CORx, -COORx, or -SO₂Rx; Rx is a substituted or unsubstituted alkyl group; when a plurality of Rx are present, the plurality of Rx are mutually the same or different; and a plurality of R_{A35} are mutually the same or different.

In the compound according to the second aspect of the first exemplary embodiment, it is preferable that at least one selected from the group consisting of T₁, T₂, T₃, and T₄ is a nitrogen atom, and at least one selected from the group consisting of T₉, T_{10,} T₁₁, and T₁₂ is a nitrogen atom.

In the compound according to the second aspect of the first exemplary embodiment, it is preferable that two selected from the group consisting of T₁, T₂, T₃, and T₄ are each a nitrogen atom, and two selected from the group consisting of T₉, T_{10,} T₁₁, and T₁₂ are each a nitrogen atom.

In the compound according to the second aspect of the first exemplary embodiment, it is preferable that T₁, T₄, T₅, T₈, T₉, and T₁₂ are each a nitrogen atom.

In the compound according to the second aspect of the first exemplary embodiment, it is preferable that the acceptor moiety is represented by a formula (A21-1) or (A22-1) below.

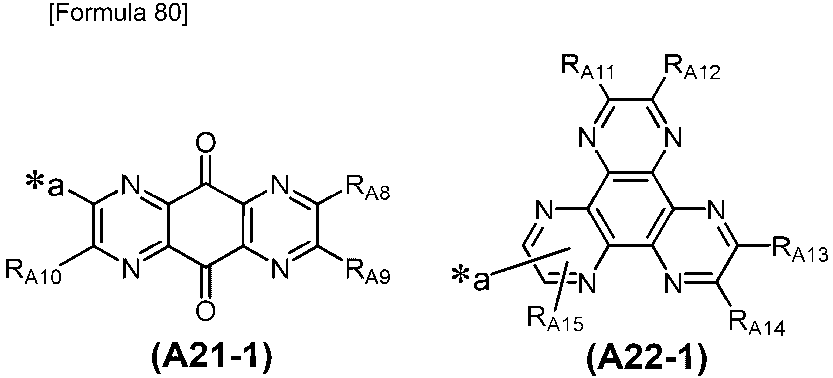

In the formulae (A21-1) and (A22-1):
R_{A8}, R_{A9}, R_{A10}, R_{A11}, R_{A12}, R_{A13}, R_{A14}, and R_{A15} are each independently a hydrogen atom, a halogen atom, a cyano group, a substituted or unsubstituted alkyl group, a substituted or unsubstituted aryl group, -NO₂-, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

In the compound according to the second aspect of the first exemplary embodiment, it is preferable that the acceptor moiety is represented by a formula (A211-1) or (A221-1) below.

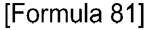

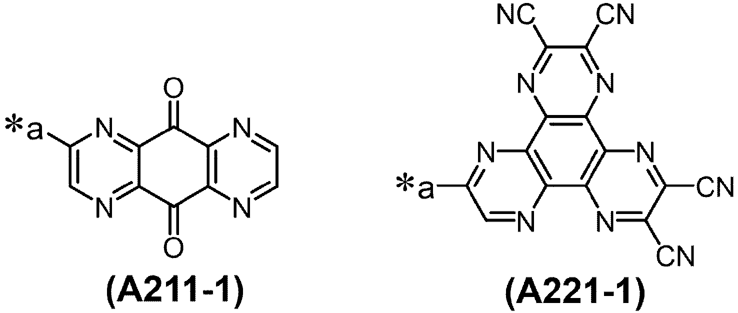

In the formulae (A211-1) and (A221-1), *a represents a bonding position.

In the compound according to the second aspect of the first exemplary embodiment, it is preferable that the acceptor moiety is represented by a formula (A21-2) below.

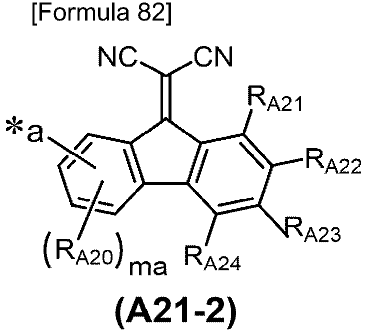

In the formula (A21-2):
ma represents the number of R_{A20} bonded to the ring, ma is 3, and the three R_{A20} are mutually the same or different;
R_{A20}, R_{A21}, R_{A22}, R_{A23}, and R_{A24} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx;
Rx is a substituted or unsubstituted alkyl group, and when a plurality of Rx are present, the plurality of Rx are mutually the same or different; and
*a represents a bonding position.

In the compound according to the second aspect of the first exemplary embodiment, it is preferable that the acceptor moiety is represented by a formula (A212-1) below.

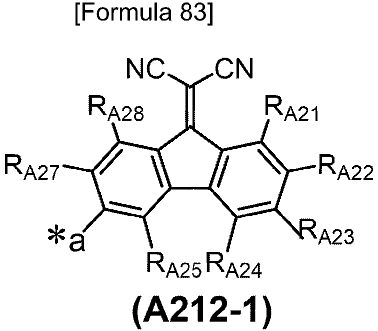

In the formula (A212-1):
R_{A21}, R_{A22}, R_{A23}, R_{A24}, R_{A25}, R_{A27}, and R_{A28} are each independently a hydrogen atom, a halogen atom, a cyano group, a substituted or unsubstituted alkyl group, a substituted or unsubstituted aryl group, -NO₂-, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

In the compound according to the second aspect of the first exemplary embodiment, it is preferable that the acceptor moiety has one or more structures selected from the group consisting of a nitrogen-containing ring, CN, C=O, and C=C(CN)₂.

In the compound according to the second aspect of the first exemplary embodiment, it is preferable that the acceptor moiety is a group having electron-withdrawing properties.

### Donor Moiety in Compound according to Second Aspect

As the donor moiety in the compound according to the second aspect of the first exemplary embodiment, a known donor moiety used in an electro-optic compound or an organic nonlinear optical material can be applied. In the compound according to the second aspect of the first exemplary embodiment, it is preferable that the donor moiety is a group having electron-donating properties.

The donor moiety in the compound according to the second aspect of the first exemplary embodiment is also preferably represented by a formula (DX1) below.

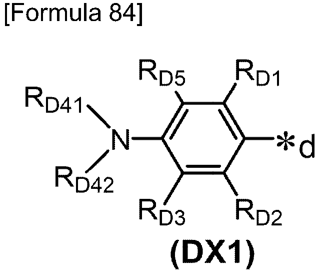

In the formula (DX1):
At least one combination of a combination of R_{D1} and R_{D5} and a combination of R_{D2} and R_{D3} are mutually bonded to form a substituted or unsubstituted monocyclic ring, mutually bonded to form a substituted or unsubstituted fused ring, or not mutually bonded;
R_{D1}, R_{D2}, R_{D3}, and R_{D5} forming neither the substituted or unsubstituted monocyclic ring nor the substituted or unsubstituted fused ring are each independently a hydrogen atom, a substituted or unsubstituted alkoxy group, a substituted or unsubstituted aryloxy group, a substituted or unsubstituted aralkyloxy group, a substituted or unsubstituted silyloxy group, a substituted or unsubstituted alkenyloxy group, a substituted or unsubstituted alkenylcarbonyloxy group, a substituted or unsubstituted alkynyloxy group, or a substituted or unsubstituted hydroxy group;
a combination of R_{D41} and R_{D42} are mutually bonded to form a substituted or unsubstituted monocyclic ring, mutually bonded to form a substituted or unsubstituted fused ring, or not mutually bonded;
when R_{D1} and R_{D2} are each hydrogen atom, a combination of R_{D41} and R_{D42} do not bond to each other,
R_{D41} and R_{D42} forming neither the substituted or unsubstituted monocyclic ring nor the substituted or unsubstituted fused ring are each independently a hydrogen atom, or a substituted or unsubstituted alkyl group; and
*d is a bonding position.

The donor moiety in the compound according to the second aspect of the first exemplary embodiment is also preferably represented by a formula (DX11) or (DX12) below.

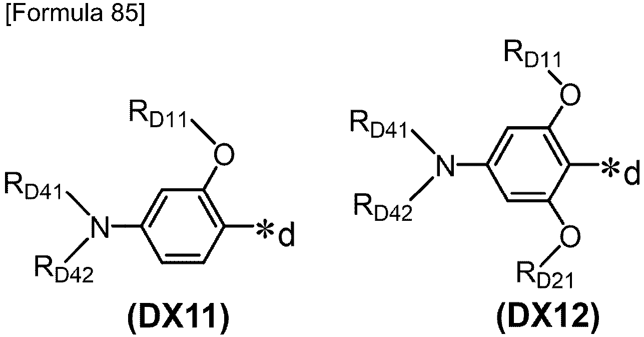

In the formulae (DX11) and (DX12):
R_{D11} and R_{D21} are each independently a hydrogen atom, a substituted or unsubstituted alkyl group, a substituted or unsubstituted aryl group, a substituted or unsubstituted aralkyl group, a substituted or unsubstituted silyl group, a substituted or unsubstituted alkenyl group, a substituted or unsubstituted alkenylcarbonyl group, or a substituted or unsubstituted alkynyl group;
R_{D41} and R_{D42} respectively represent the same as R_{D41} and R_{D42} in the formula (DX1); and
*d is a bonding position.

The donor moiety in the compound according to the second aspect of the first exemplary embodiment is also preferably represented by a formula (DX21) below.

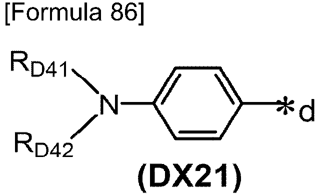

In the formula (DX21), R_{D41} and R_{D42} respectively represent the same as R_{D41} and R_{D42} in the formula (DX1), and *d is a bonding position.

The donor moiety in the compound according to the second aspect of the first exemplary embodiment is also preferably represented by a formula (DX11-1), (DX11-2) or (DX21-1) below.

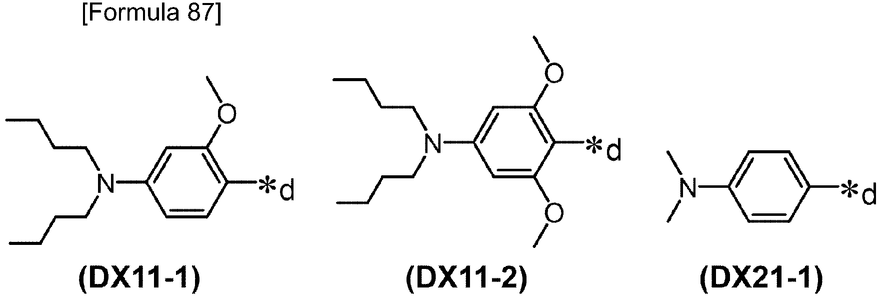

In the donor moiety of the compound according to the second aspect of the first exemplary embodiment, it is preferable that *d is a bonding position to the π-conjugated moiety.

The donor moiety in the compound according to the second aspect of the first exemplary embodiment is also preferably the donor moiety in the compound according to the first aspect described above.

The donor moiety in the compound according to the second aspect of the first exemplary embodiment is also preferably represented by the formula (D1) or (D2) in the compound according to the first aspect described above.

### π-conjugated Moiety in Compound according to Second Aspect

As the π-conjugated moiety in the compound according to the second aspect of the first exemplary embodiment, a known π-conjugated moiety used in an organic nonlinear optical material or an electro-optic compound can be applied. The π-conjugated moiety may also be referred to as a π-conjugated bridge.

In the compound according to the second aspect of the first exemplary embodiment, it is preferable that the π-conjugated moiety has a conjugated system having conjugated multiple bonds. In the compound according to the second aspect of the first exemplary embodiment, for example, as long as π electrons are delocalized from the donor moiety to the acceptor moiety through molecular orbitals within the π-conjugated moiety, the π-conjugated moiety is not particularly limited. The conjugated multiple bonds of the π-conjugated moiety may be either double bonds or triple bonds, and are preferably double bonds. Examples of the π-conjugation include carbon-carbon conjugation and conjugation containing nitrogen.

In the compound according to the second aspect of the first exemplary embodiment, the π-conjugated moiety is also preferably represented by a formula (PX1a), (PX2a), or (PX3a) below.

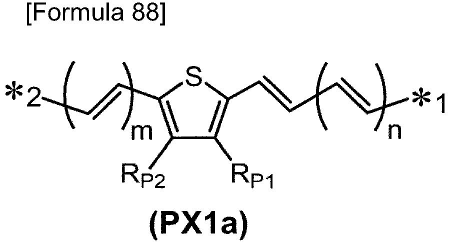

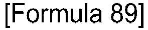

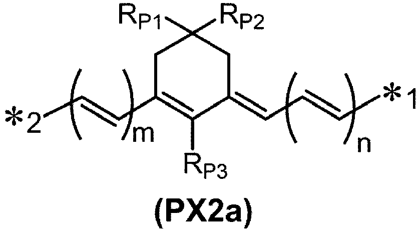

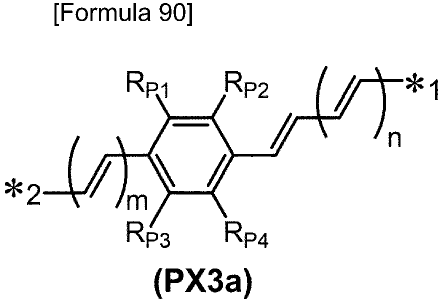

In the formulae (PX1a), (PX2a), and (PX3a):
m and n are each independently 0, 1, 2, or 3;
R_{P1}, R_{P2}, R_{P3}, and R_{P4} are each independently a hydrogen atom, a substituted or unsubstituted alkyl group, a substituted or unsubstituted alkoxy group, a substituted or unsubstituted aryl group, a substituted or unsubstituted alkenyl group, a substituted or unsubstituted cycloalkyl group, a substituted or unsubstituted cycloalkenyl group, a substituted or unsubstituted haloalkyl group, a substituted or unsubstituted aralkyl group, a substituted or unsubstituted aryloxy group, or a substituted or unsubstituted aralkyloxy group; and
*1 and *2 each represent a bonding position.

In the compound according to the second aspect of the first exemplary embodiment, it is also preferable that m is 1 and n is 0.

In the compound according to the second aspect of the first exemplary embodiment, the π-conjugated moiety is also preferably represented by a formula (PX1-1), (PX2-1), or (PX3-1) below. The formula (PX1-1) represents the π-conjugated moiety represented by the formula (PX1a) in which m is 1 and n is 0. The formula (PX2-1) represents the π-conjugated moiety represented by the formula (PX2a) in which m is 1 and n is 0. The formula (PX3-1) represents the π-conjugated moiety represented by the formula (PX3a) in which m is 1 and n is 0.

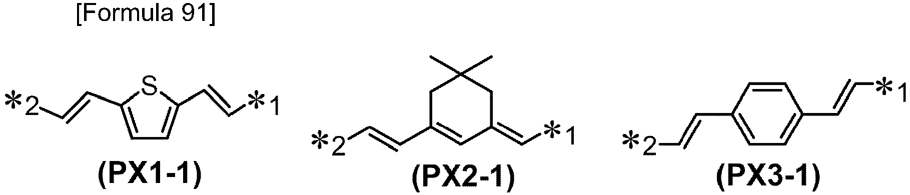

In the π-conjugated moiety of the compound according to the second aspect of the first exemplary embodiment, it is preferable that one of *1 and *2 is a bonding position to the acceptor moiety, and the other of *1 and *2 is a bonding position to the donor moiety.

In the compound according to the second aspect of the first exemplary embodiment, it is also preferable that the π-conjugated moiety is the π-conjugated moiety in the compound according to the third aspect described later.

In the compound according to the second aspect of the first exemplary embodiment, it is also preferable that the π-conjugated moiety is represented by the formula (P1), (P2), or (P3) in the compound according to the third aspect described later.

### Third Aspect

The compound according to the third aspect of the first exemplary embodiment is a compound that satisfies at least the condition (III). Hereinafter, the compound according to the third aspect of the first exemplary embodiment will be described.

### π-conjugated Moiety in Compound according to Third Aspect

In the compound according to the third aspect of the first exemplary embodiment, the π-conjugated moiety is represented by a formula (P1), (P2), or (P3) below.

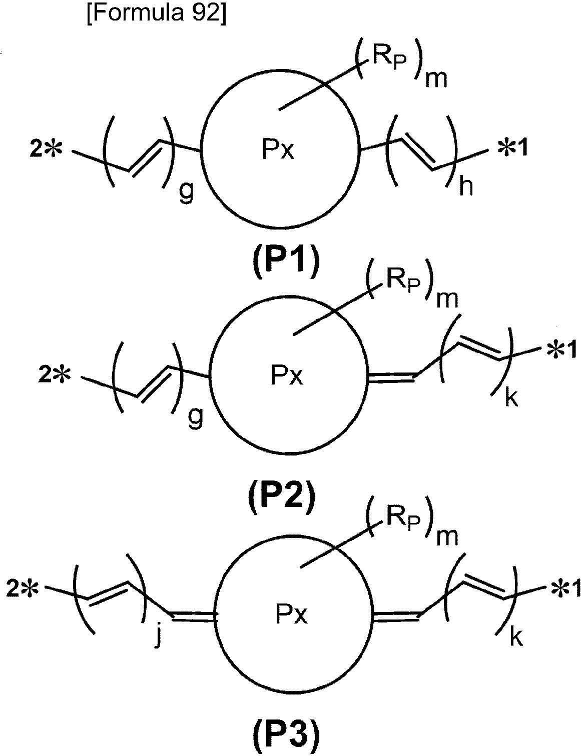

In the formulae (P1), (P2), and (P3):
the ring Px is a monocyclic aromatic hydrocarbon ring having one or more substituents R_{P}, a substituted or unsubstituted polycyclic aromatic hydrocarbon ring, a monocyclic aliphatic hydrocarbon ring having one or more substituents R_{P}, a substituted or unsubstituted polycyclic aliphatic hydrocarbon ring, a substituted or unsubstituted monocyclic heterocycle, or a substituted or unsubstituted polycyclic heterocycle;
m is the number of substituents R_{P} and is 0 or an integer of 1 or more;
a substituent R_{P} is a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
R₁₀₁, R₁₀₂, R₁₀₃, R₁₀₄, R₁₀₅, R₁₀₆, R₁₀₇, R₁₀₈, and R₁₀₉ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when the ring Px is a monocyclic aromatic hydrocarbon ring, a monocyclic aliphatic hydrocarbon ring, or a monocyclic heterocycle having one or more substituents R_{P}, at least one of the substituents R_{P} is a group represented by -O-R_{P1}, and R_{P1} is a substituted or unsubstituted alkyl group or a substituted or unsubstituted aryl group;
when the ring Px is a naphthalene ring, the naphthalene ring as the ring Px has at least one group represented by -O-R_{P1}, and R_{P1} is a substituted or unsubstituted alkyl group or a substituted or unsubstituted aryl group;
when the ring Px is a monocyclic heterocycle having no substituent R_{P}, the monocyclic heterocycle contains two or more heteroatoms as ring atoms;
when the ring Px is a polycyclic heterocycle, at least one ring contains one or more heteroatoms as ring atoms;
g, h, j, and k are each independently 0, 1, 2, or 3; and
*1 and *2 each represent a bonding position.

In the formula (P1), it is also preferable that g is 1 and h is 1. In the formula (P2), it is also preferable that g is 1 and k is 0 or 1. In the formula (P1), it is also preferable that j is 0 or 1 and k is 0 or 1.

In the compound according to the third aspect of the first exemplary embodiment, it is also preferable that the π-conjugated moiety is represented by the formula (P1). In the compound according to the third aspect of the first exemplary embodiment, it is also preferable that the π-conjugated moiety is represented by the formula (P2).

In the compound according to the third aspect of the first exemplary embodiment, when the ring Px is a polycyclic heterocycle, it is also preferable that, at least one ring contains two or more heteroatoms as ring atoms.

In the compound according to the third aspect of the first exemplary embodiment, it is preferable that the π-conjugated moiety is represented by a formula (P11-1), (P12-1), (P13-1), (P14-1), or (P15-1) below.

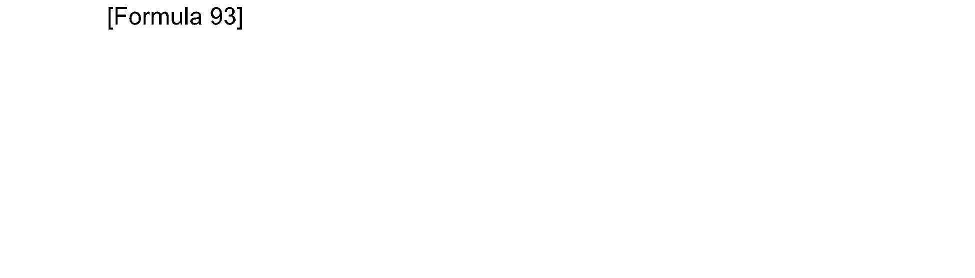

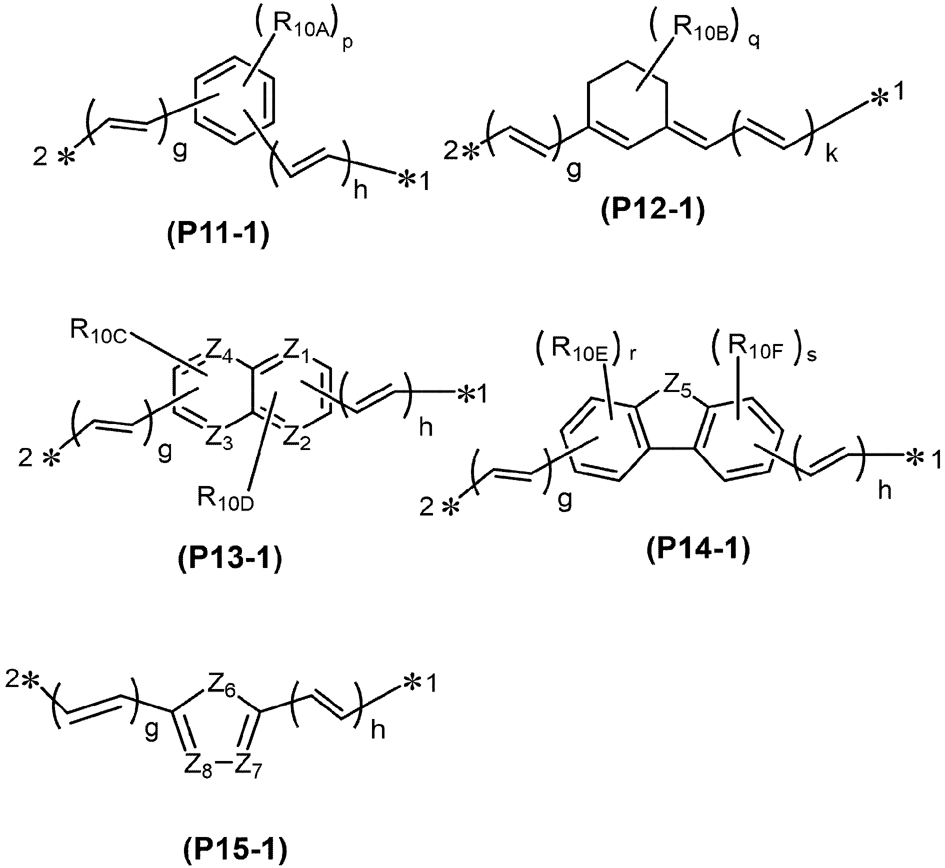

In the formulae (P11-1), (P12-1), (P13-1), (P14-1), and (P15-1):
p, q, r, and s are each an integer of 1 or more;
Z₁, Z₂, Z₃, Z₄, Z₇, and Z₈ are each independently C(R₁₀₀) or a nitrogen atom;
a plurality of R₁₀₀ are mutually the same or different;
Z₅ and Z₆ are each independently an oxygen atom, a sulfur atom, N(R₁₁₀), or C(R₁₁₁)(R₁₁₂);
at least one combination of adjacent two or more of R₁₀₀, R_{10A}, R_{10B}, R_{10C}, R_{10D}, R_{10E}, and R_{10F} are mutually bonded to form a substituted or unsubstituted monocyclic ring, mutually bonded to form a substituted or unsubstituted fused ring, or not mutually bonded;
R₁₀₀, R_{10A}, R_{10B}, R_{10C}, R_{10D}, R_{10E}, and R_{10F} forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
when a plurality of R_{10A} are present, the plurality of R_{10A} are mutually the same or different;
when a plurality of R_{10B} are present, the plurality of R_{10B} are mutually the same or different;
when a plurality of R_{10C} are present, the plurality of R_{10C} are mutually the same or different;
when a plurality of R_{10D} are present, the plurality of R_{10D} are mutually the same or different;
when a plurality of R_{10E} are present, the plurality of R_{10E} are mutually the same or different;
when a plurality of R_{10F} are present, the plurality of R_{10F} are mutually the same or different;
R₁₀₁, R₁₀₂, R₁₀₃, R₁₀₄, R₁₀₅, R₁₀₆, R₁₀₇, R₁₀₈, R₁₀₉, R₁₁₀, R₁₁₁, and R₁₁₂ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when a plurality of R₁₁₀ are present, the plurality of R₁₁₀ are mutually the same or different;
when a plurality of R₁₁₁ are present, the plurality of R₁₁₁ are mutually the same or different;
when a plurality of R₁₁₂ are present, the plurality of R₁₁₂ are mutually the same or different; and
g, h, and k are each independently 0, 1, 2, or 3,
in the formula (P11-1): when p is 1, R_{10A} is a group represented by -O(R₁₀₉); and when p is 2 or more, at least one of R_{10A} is a group represented by -O(R₁₀₉), and R₁₀₉ is a substituted or unsubstituted alkyl group or a substituted or unsubstituted aryl group,
in the formula (P12-1): when q is 1, R_{10B} is a group represented by -O(R₁₀₉); when q is 2 or more, at least one of R_{10B} is a group represented by -O(R₁₀₉), and R₁₀₉ is a substituted or unsubstituted alkyl group or a substituted or unsubstituted aryl group,
in the formula (P13-1): when Z₁, Z₂, Z₃, and Z₄ are C(R₁₀₀), at least one of R_{10C}, R_{10D}, or the four R₁₀₀ is a group represented by -O(R₁₀₉), and R₁₀₉ is a substituted or unsubstituted alkyl group or a substituted or unsubstituted aryl group,
in the formula (P15-1): when Z₇ and Z₈ are C(R₁₀₀), at least one of the two R₁₀₀ is a group represented by -O(R₁₀₉), and R₁₀₉ is a substituted or unsubstituted alkyl group or a substituted or unsubstituted aryl group, and
*1 and *2 each represent a bonding position.

In the compound according to the third aspect of the first exemplary embodiment, R₁₀₁ to R₁₁₂ represent the same as R₁₀₁ to R₁₁₂ in the formulae (P1), (P2), and (D3).

In the formula (P11-1), (P12-1), (P13-1), (P14-1), and (P15-1), it is also preferable that g, h, and k are each independently 1.

In the compound according to the third aspect of the first exemplary embodiment, it is also preferable that the π-conjugated moiety is represented by a formula (P11-1A), (P12-1A), (P13-1A), (P13-1B), (P14-1A), (P14-1B), (P15-1A), or (P15-1B) below.

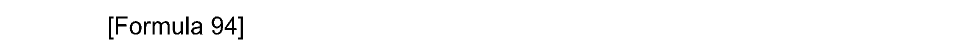

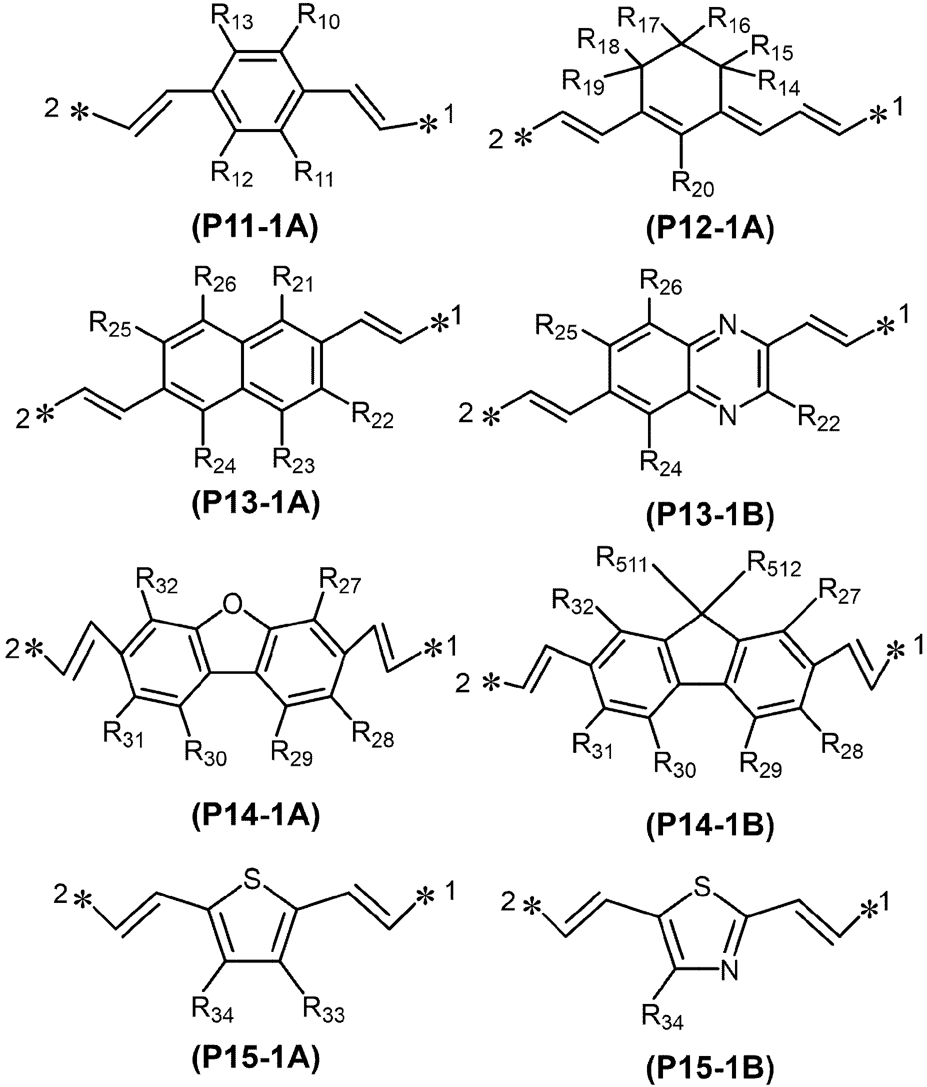

In the formulae (P11-1A), (P12-1A), (P13-1A), (P13-1B), (P14-1A), (P14-1B), (P15-1A), and (P15-1B):
at least one combination of adjacent two or more of R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₅₁₁, and R₅₁₂ are mutually bonded to form a substituted or unsubstituted monocyclic ring, mutually bonded to form a substituted or unsubstituted fused ring, or not mutually bonded; and
R₁₀ to R₃₄, R₅₁₁, and R₅₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉),
in the formula (P11-1A): when at least one of R₁₀ to R₁₃ is a group represented by -O(R₁₀₉), and R₁₀₉ is a substituted or unsubstituted alkyl group or a substituted or unsubstituted aryl group,
in the formula (P12-1A): when at least one of R₁₄ to R₂₀ is a group represented by -O(R₁₀₉), and R₁₀₉ is a substituted or unsubstituted alkyl group or a substituted or unsubstituted aryl group,
   in the formula (P13-1A): when at least one of R₂₁ to R₂₆ is a group represented by - O(R₁₀₉), and R₁₀₉ is a substituted or unsubstituted alkyl group or a substituted or unsubstituted aryl group,
in the formula (P15-1A): when at least one of R₃₃ to R₃₄ is a group represented by -O(R₁₀₉), and R₁₀₉ is a substituted or unsubstituted alkyl group or a substituted or unsubstituted aryl group, and
*1 and *2 each represent a bonding position.

In the compound according to the third aspect of the first exemplary embodiment, when both R₃₃ and R₃₄ in the formula (P15-1A) are groups represented by -O(R₁₀₉), it is also preferable that the group consisting of the two R₁₀₉ are bonded to each other to form a substituted or unsubstituted monocyclic ring, or are bonded to each other to form a substituted or unsubstituted fused ring.

In the compound according to the third aspect of the first exemplary embodiment, when both R₃₃ and R₃₄ in the formula (P15-1A) are groups represented by -O(R₁₀₉), it is also preferable that a combination of the two R₁₀₉ are not bonded to each other.

In the compound according to the third aspect of the first exemplary embodiment, it is also preferable that the π-conjugated moiety is represented by a formula (P11-1A-1), (P11-1A-2), (P11-1A-3), (P12-1A-1), (P13-1A-1), (P13-1A-2), (P13-1A-3), (P13-1B-1), (P14-1A-1), (P14-1B-1), (P15-1A-1), (P15-1A-2), (P15-1A-3), or (P15-1B-1) below.

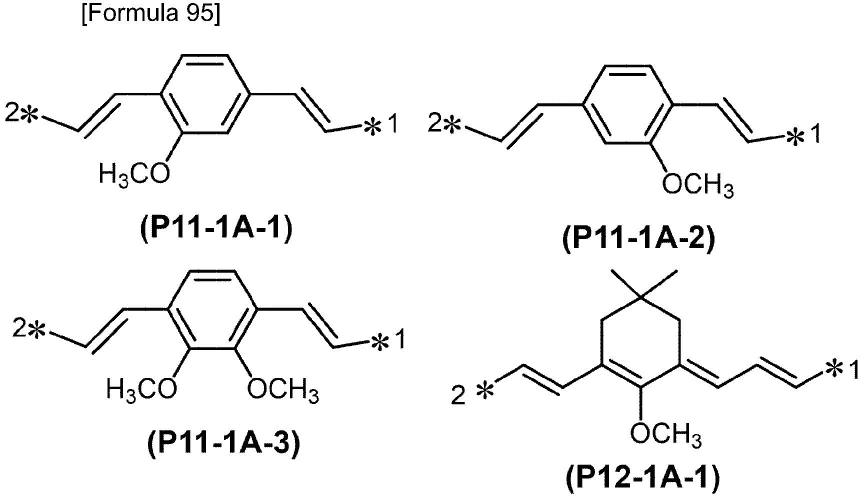

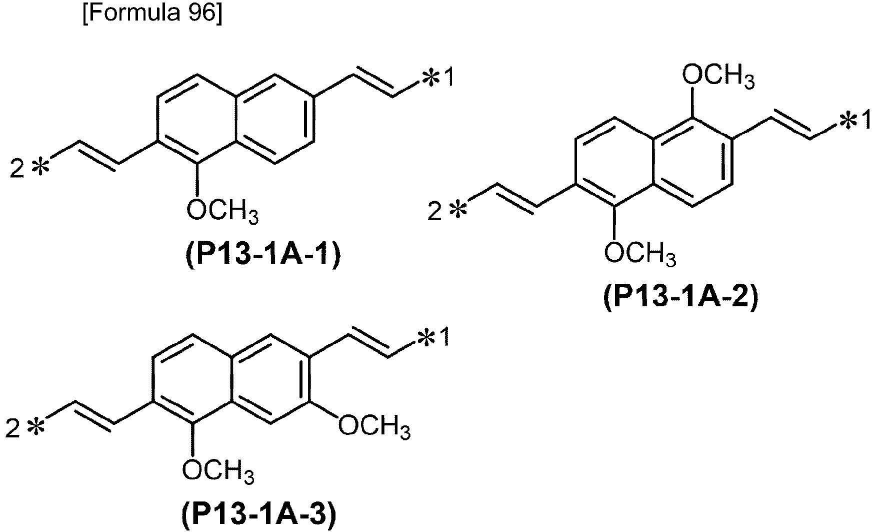

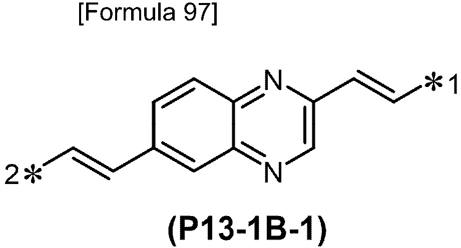

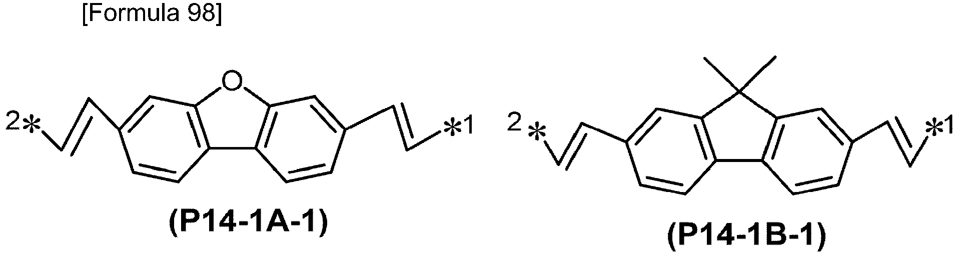

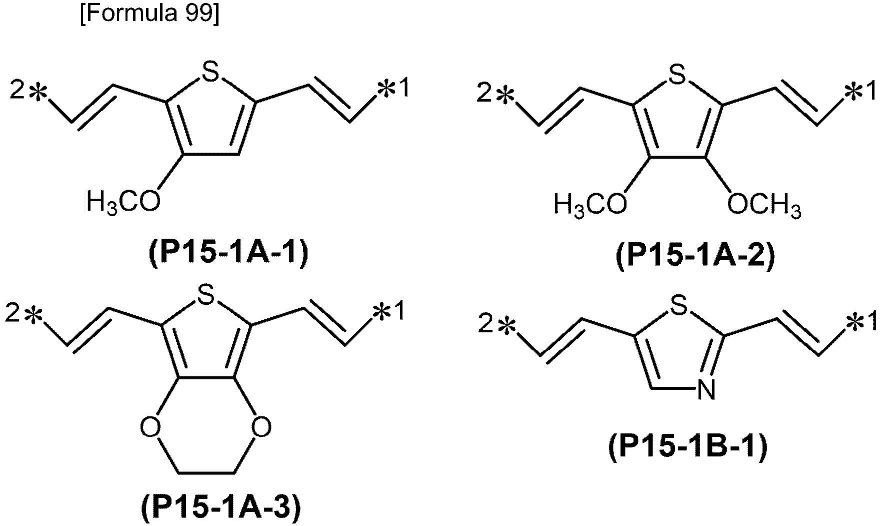

In the formulae (P11-1A-1), (P11-1A-2), (P11-1A-3), (P12-1A-1), (P13-1A-1), (P13-1A-2), (P13-1A-3), (P13-1B-1), (P14-1A-1), (P14-1B-1), (P15-1A-1), (P15-1A-2), (P15-1A-3), and (P15-1B-1), *1 and *2 each represent a bonding position.

In the compound according to the third aspect of the first exemplary embodiment, the π-conjugated moiety is also preferably represented by the formula (PX5).

In the π-conjugated moiety of the compound according to the third aspect of the first exemplary embodiment, it is preferable that one of *1 and *2 is a bonding position to the acceptor moiety, and the other of *1 and *2 is a bonding position to the donor moiety.

### Donor Moiety in Compound according to Third Aspect

As the donor moiety in the compound according to the third aspect of the first exemplary embodiment, a known donor moiety used in an electro-optic compound or an organic nonlinear optical material can be applied. In the compound according to the third aspect of the first exemplary embodiment, it is preferable that the donor moiety is a group having electron-donating properties.

The donor moiety in the compound according to the third aspect of the first exemplary embodiment is also preferably represented by the formula (DX1) below.

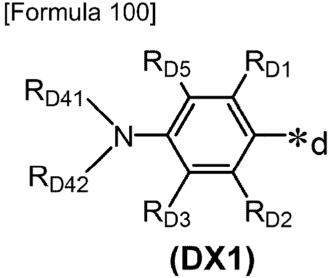

In the formula (DX1):
At least one combination of a combination of R_{D1} and R_{D5} and a combination of R_{D2} and R_{D3} are mutually bonded to form a substituted or unsubstituted monocyclic ring, mutually bonded to form a substituted or unsubstituted fused ring, or not mutually bonded;
R_{D1}, R_{D2,} R_{D3}, and R_{D5} forming neither the substituted or unsubstituted monocyclic ring nor the substituted or unsubstituted fused ring are each independently a hydrogen atom, a substituted or unsubstituted alkoxy group, a substituted or unsubstituted aryloxy group, a substituted or unsubstituted aralkyloxy group, a substituted or unsubstituted silyloxy group, a substituted or unsubstituted alkenyloxy group, a substituted or unsubstituted alkenylcarbonyloxy group, a substituted or unsubstituted alkynyloxy group, or a substituted or unsubstituted hydroxy group;
a combination of R_{D41} and R_{D42} are mutually bonded to form a substituted or unsubstituted monocyclic ring, mutually bonded to form a substituted or unsubstituted fused ring, or not mutually bonded;
when R_{D1} and R_{D2} are each hydrogen atom, a combination of R_{D41} and R_{D42} do not bond to each other;
R_{D41} and R_{D42} forming neither the substituted or unsubstituted monocyclic ring nor the substituted or unsubstituted fused ring are each independently a hydrogen atom, or a substituted or unsubstituted alkyl group; and
*d is a bonding position.

The donor moiety in the compound according to the third aspect of the first exemplary embodiment is also preferably represented by the formula (DX11) or (DX12) below.

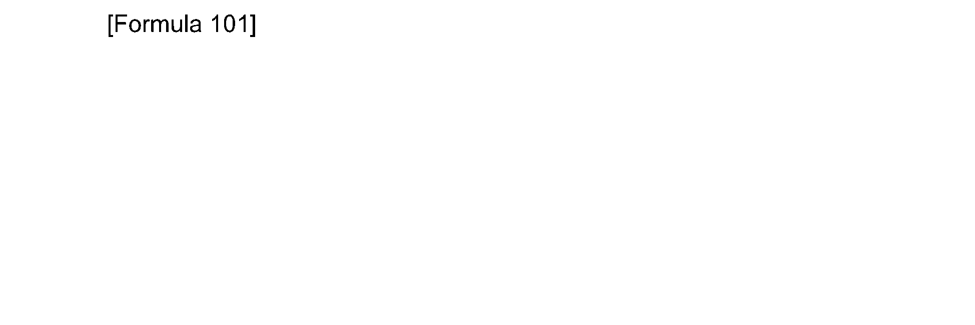

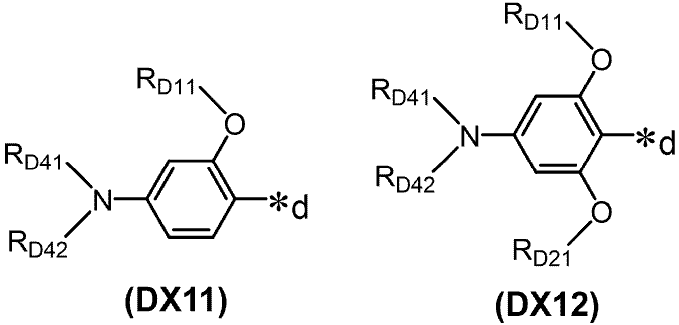

In the formulae (DX11) and (DX12):
R_{D11} and R_{D21} are each independently a hydrogen atom, a substituted or unsubstituted alkyl group, a substituted or unsubstituted aryl group, a substituted or unsubstituted aralkyl group, a substituted or unsubstituted silyl group, a substituted or unsubstituted alkenyl group, a substituted or unsubstituted alkenylcarbonyl group, or a substituted or unsubstituted alkynyl group, R_{D41} and R_{D42} respectively represent the same as R_{D41} and R_{D42} in the formula (DX1); and
*d is a bonding position.

The donor moiety in the compound according to the third aspect of the first exemplary embodiment is also preferably represented by the formula (DX21) below.

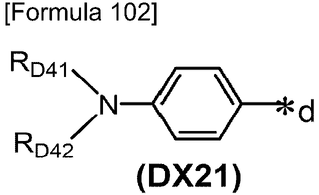

In the formula (DX21), R_{D41} and R_{D42} respectively represent the same as R_{D41} and R_{D42} in the formula (DX1), and *d is a bonding position.

The donor moiety in the compound according to the third aspect of the first exemplary embodiment is also preferably represented by the formula (DX11-1), (DX11-2) or (DX21-1) below.

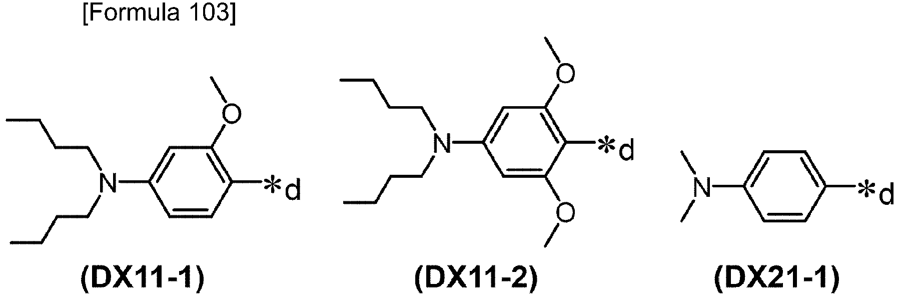

In the donor moiety of the compound according to the third aspect of the first exemplary embodiment, it is preferable that *d is a bonding position to the π-conjugated moiety.

The donor moiety in the compound according to the third aspect of the first exemplary embodiment is also preferably the donor moiety in the compound according to the first aspect described above.

The donor moiety in the compound according to the third aspect of the first exemplary embodiment is also preferably represented by the formula (D1) or (D2) in the compound according to the first aspect described above.

### Acceptor Moiety in Compound according to Third Aspect

As the acceptor moiety in the compound according to the third aspect of the first exemplary embodiment, a known acceptor moiety used in an electro-optic compound or an organic nonlinear optical material can be applied. In the compound according to the third aspect of the first exemplary embodiment, it is preferable that the acceptor moiety is a group having electron-withdrawing properties.

In the compound according to the third aspect of the first exemplary embodiment, it is also preferable that the acceptor moiety is represented by the formula (AX1) or (AX2) below.

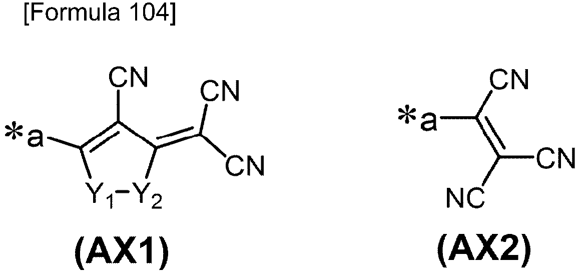

In the formulae (AX1) and (AX2): Y₁ and Y₂ are each independently - C(R_{A30})(R_{A31})-, -C(O)-, -O-, or -NR_{A32}-;
at least one of Y₁ or Y₂ is -O- or -NR_{A32}-;
R_{A30} and R_{A31} are each independently a hydrogen atom, a substituted or unsubstituted alkyl group, or a substituted or unsubstituted aryl group, R_{A32} is a hydrogen atom or a substituted or unsubstituted alkyl group; and
*a represents a bonding position.

In the compound according to the third aspect of the first exemplary embodiment, it is also preferable that the acceptor moiety is represented by the formula (AX11) or (AX21) below.

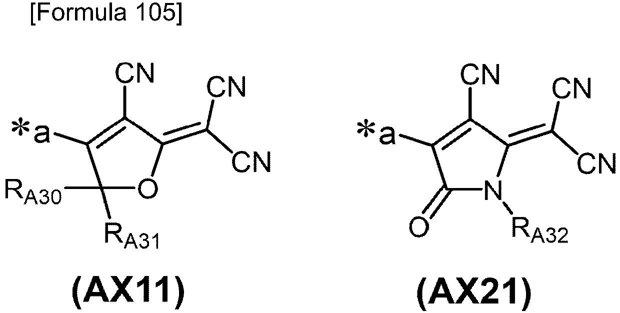

In the formulae (AX11) and (AX21):
R_{A30} and R_{A31} are each independently a hydrogen atom, a substituted or unsubstituted alkyl group, or a substituted or unsubstituted aryl group, R_{A32} is a hydrogen atom or a substituted or unsubstituted alkyl group; and
*a represents a bonding position.

In the acceptor moiety of the compound according to the third aspect of the first exemplary embodiment, at least one of R_{A30} or R_{A31} is preferably a substituted alkyl group, more preferably a haloalkyl group substituted with a halogen atom, and still more preferably a trifluoromethyl group.

In the compound according to the third aspect of the first exemplary embodiment, it is also preferable that the acceptor moiety is represented by the formula (AX11-1), (AX11-2), or (AX21-1) below.

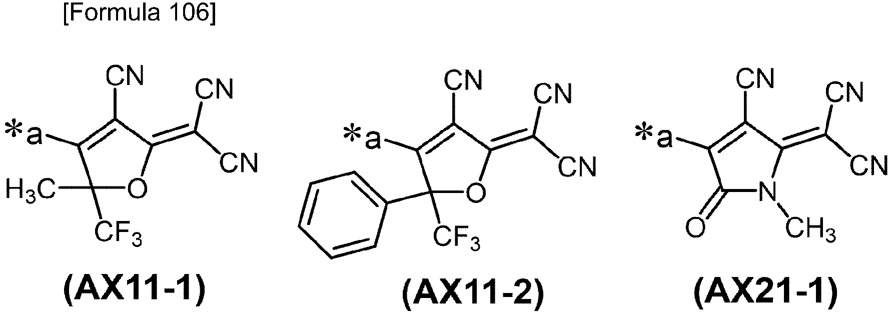

In the acceptor moiety of the compound according to the third aspect of the first exemplary embodiment, it is preferable that *a is a bonding position to the π-conjugated moiety.

In the compound according to the third aspect of the first exemplary embodiment, it is also preferable that the acceptor moiety is the acceptor moiety in the compound according to the first aspect described above.

In the compound according to the third aspect of the first exemplary embodiment, it is also preferable that the acceptor moiety is represented by the formula (A1) or (A2) in the compound according to the second aspect described above.

### Specific Examples of Compound according to First Exemplary Embodiment

The compounds according to the first, second, and third aspects of the first exemplary embodiment are each preferably represented by a formula (1) below.
[Formula 107]

D-L-A (1)

In the formula (1), D is the donor moiety, L is the π-conjugated moiety, and A is the acceptor moiety.

In the compound according to the first exemplary embodiment, it is preferable that the donor moiety is a group having electron-donating properties and the acceptor moiety is a group having electron-withdrawing properties.

In one form of the compound according to the first exemplary embodiment, it is preferable that the compound is represented by a formula (D-DP1-1), (D-DP1-2), (D-DP2-1), (D-DP2-2), (D-DP3-1), or (D-DP3-2) below.

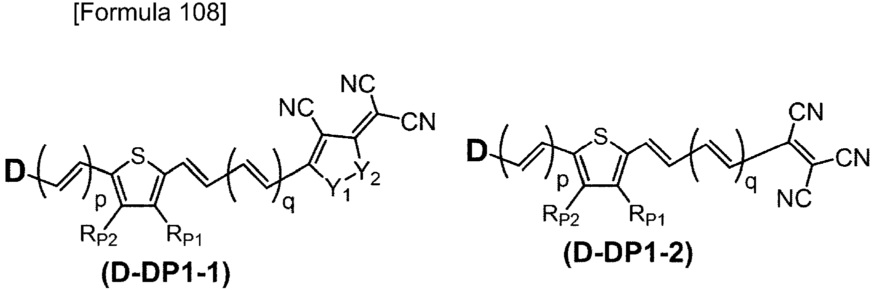

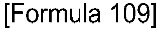

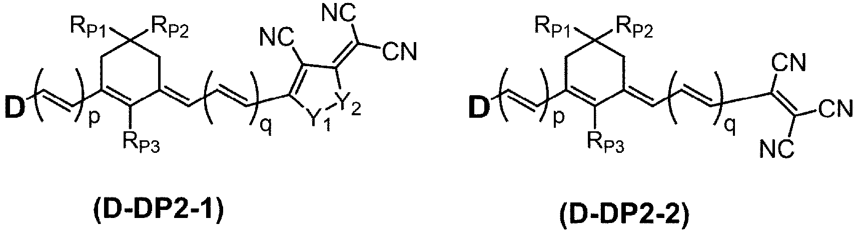

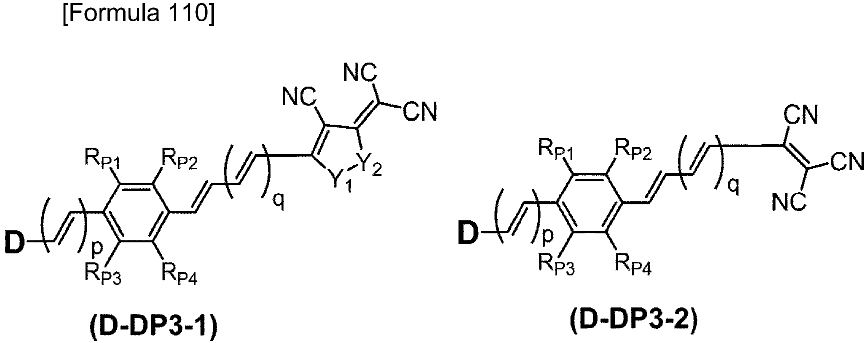

In the formulae (D-DP1-1), (D-DP1-2), (D-DP2-1), (D-DP2-2), (D-DP3-1), and (D-DP3-2):
D is the donor moiety;
R_{P1}, R_{P2}, R_{P3}, R_{P4}, p, and q respectively represent the same as R_{P1}, R_{P2}, R_{P3}, R_{P4}, p, and q in the formula (PX1), (PX2), or (PX3); and
Y₁ and Y₂ each independently represent the same as Y₁ and Y₂ in the formula (AX1).

In one form of the compound according to the first exemplary embodiment, it is also preferable that the compound is represented by a formula (D-DP11-1) or (D-DP4-1) below.

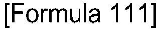

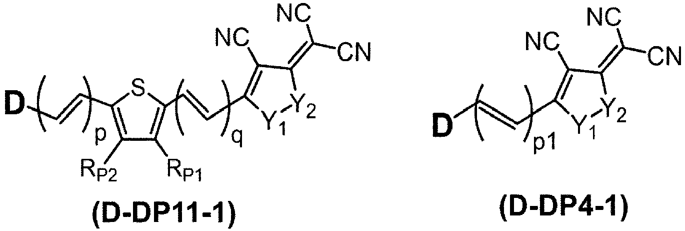

In the formulae (D-DP11-1) and (D-DP4-1):
D is the donor moiety;
R_{P1}, R_{P2}, p, and q respectively represent the same as R_{P1}, R_{P2}, p, and q in the formula (PX11), and p1 represents the same as p1 in the formula (PX4); and
Y₁ and Y₂ each independently represent the same as Y₁ and Y₂ in the formula (AX1).

In one form of the compound according to the first exemplary embodiment, it is also preferable that the compound is represented by a formula (D-DP11-A11) below.

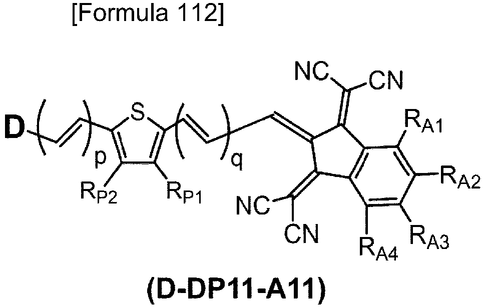

In the formula (D-DP11-A11):
D is the donor moiety;
R_{P1}, R_{P2}, p, and q respectively represent the same as R_{P1}, R_{P2}, p, and q in the formula (PX11); and
R_{A1}, R_{A2}, R_{A3}, and R_{A4} each independently represent the same as R_{A1}, R_{A2}, R_{A3}, and R_{A4} in the formula (A11-2).

In one form of the compound represented by a formula (D-DP1-1), (D-DP1-2), (D-DP2-1), (D-DP2-2), (D-DP3-1), or (D-DP3-2) below, D is the donor moiety in the compound according to the first aspect, the donor moiety in the compound according to the second aspect, or the donor moiety in the compound according to the third aspect.

The compound according to the first aspect of the first exemplary embodiment is also preferably represented by a formula (D-DPA1-1), (D-DPA1-2), (D-DPA2-1), (D-DPA2-2), (D-DPA3-1), or (D-DPA3-2) below.

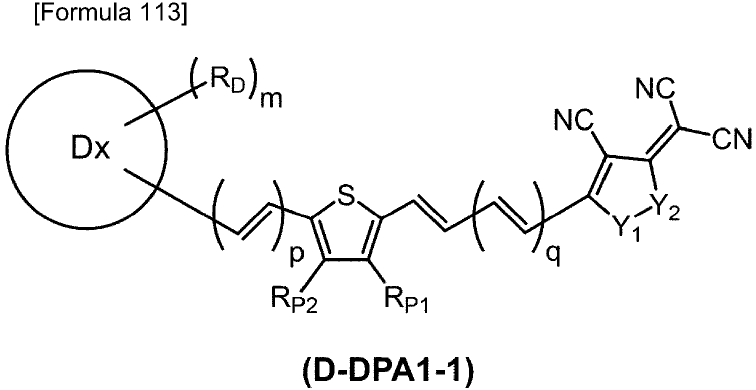

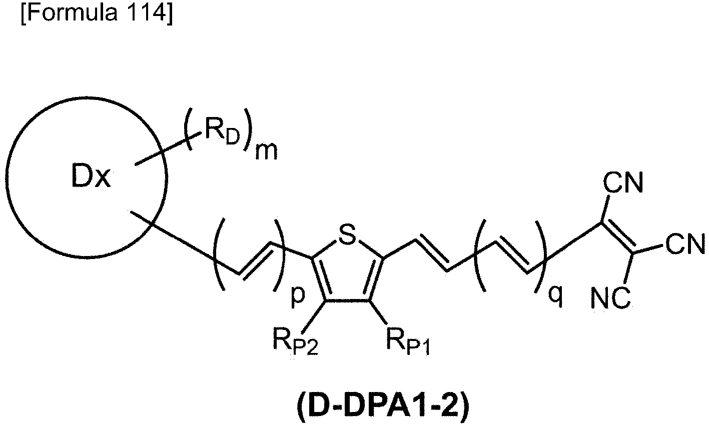

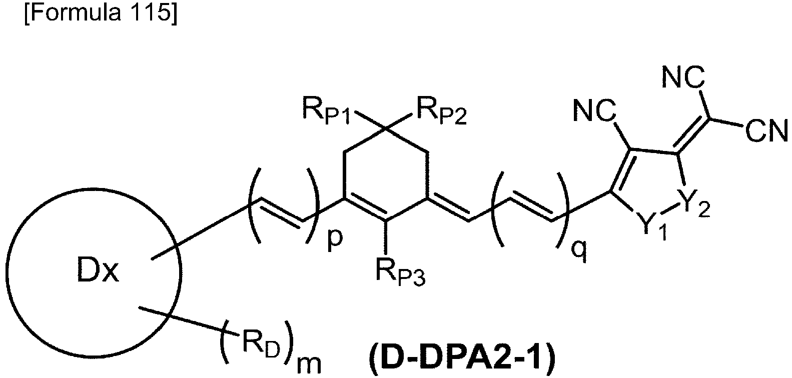

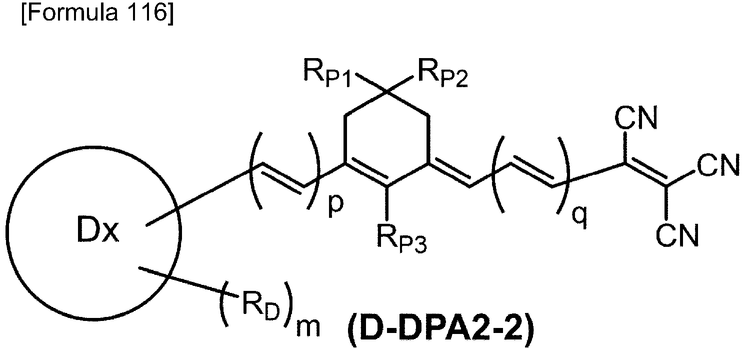

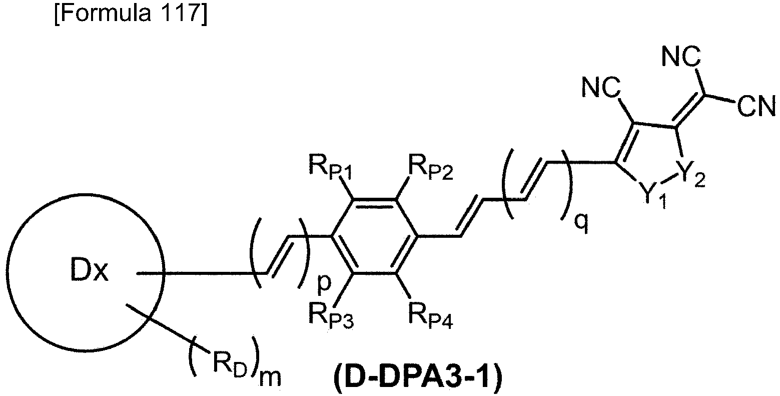

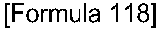

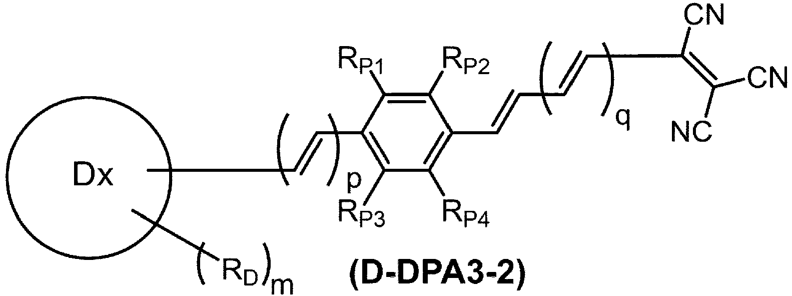

In the formulae (D-DPA1-1), (D-DPA1-2), (D-DPA2-1), (D-DPA2-2), (D-DPA3-1), and (D-DPA3-2): Dx, R_{D}, and m each independently represent the same as Dx, R_{D}, and m in the formula (D1); R_{P1}, R_{P2}, R_{P3}, R_{P4}, p, and q each independently represent the same as R_{P1}, R_{P2}, R_{P3}, R_{P4}, p, and q in the formula (PX1), (PX2), or (PX3); and Y₁ and Y₂ each independently represent the same as Y₁ and Y₂ in the formula (AX1).

The compound represented by the formula (D-DPA1-1) or (D-DPA1-2) corresponds to a compound in which the donor moiety represented by the formula (D1), the π-conjugated moiety represented by the formula (PX1), and the acceptor moiety represented by the formula (AX1) or (AX2) are bonded.

The compound represented by the formula (D-DPA2-1) or (D-DPA2-2) corresponds to a compound in which the donor moiety represented by the formula (D1), the π-conjugated moiety represented by the formula (PX2), and the acceptor moiety represented by the formula (AX1) or (AX2) are bonded.

The compound represented by the formula (D-DPA3-1) or (D-DPA3-2) corresponds to a compound in which the donor moiety represented by the formula (D1), the π-conjugated moiety represented by the formula (PX3), and the acceptor moiety represented by the formula (AX1) or (AX2) are bonded.

The compound according to the first aspect of the first exemplary embodiment is also preferably represented by a formula (D-DPA11-1) or (D-DPA4-1) below.

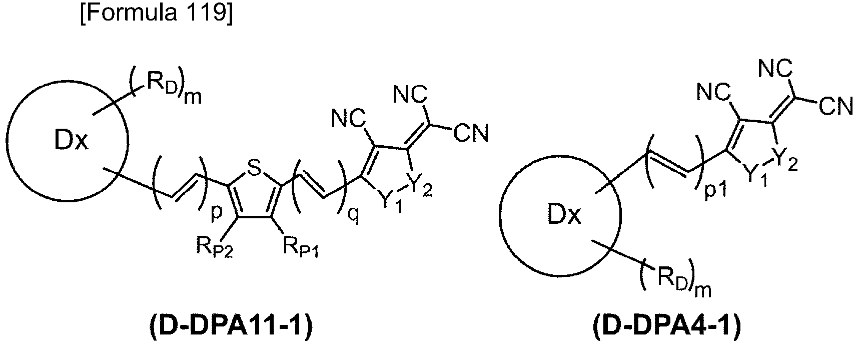

In the formulae (D-DPA11-1) and (D-DPA4-1): Dx, R_{D}, and m each independently represent the same as Dx, R_{D}, and m in the formula (D1); R_{P1}, R_{P2}, p, and q each independently represent the same as R_{P1}, R_{P2}, p, and q in the formula (PX11); p1 represents the same as p1 in the formula (PX4); and Y₁ and Y₂ each independently represent the same as Y₁ and Y₂ in the formula (AX1).

The compound according to the first aspect of the first exemplary embodiment is also preferably represented by a formula (D-DPA1-A11) or (D-DPA11-A11) below.

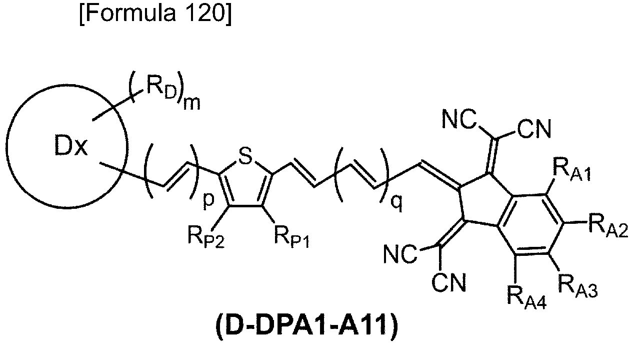

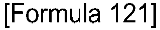

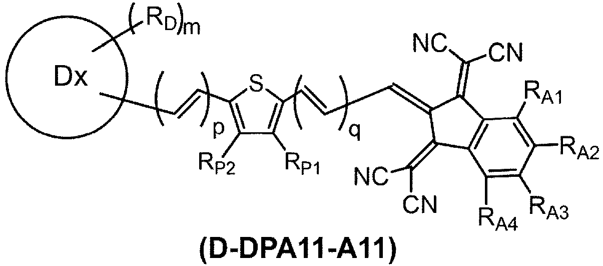

In the formulae (D-DPA1-A11) and (D-DPA11-A11):
Dx, R_{D}, and m each independently represent the same as Dx, R_{D}, and m in the formula (D1);
R_{P1}, R_{P2}, p, and q respectively represent the same as R_{P1}, R_{P2}, p, and q in the formulae (PX1) and (PX11); and
R_{A1}, R_{A2}, R_{A3}, and R_{A4} each independently represent the same as R_{A1}, R_{A2}, R_{A3}, and R_{A4} in the formula (A11-2).

The compound represented by the formula (D-DPA11-1) or (D-DPA4-1) corresponds to a compound in which the donor moiety represented by the formula (D1), the π-conjugated moiety represented by the formula (PX11) or (PX4), and the acceptor moiety represented by the formula (AX1) are bonded.

The compound represented by the formula (D-DPA1-A11) or (D-DPA11-A11) corresponds to a compound in which the donor moiety represented by the formula (D1), the π-conjugated moiety represented by the formula (PX1) or (PX11), and the acceptor moiety represented by the formula (A11-2) are bonded.

The compound according to the first aspect of the first exemplary embodiment also preferably corresponds to a compound in which the donor moiety represented by the formula (D1), the π-conjugated moiety represented by the formula (PX5), and the acceptor moiety represented by the formula (AX1) are bonded.

The compound according to the first aspect of the first exemplary embodiment also preferably corresponds to a compound in which the donor moiety represented by the formula (D1), the π-conjugated moiety represented by the formula (P15-1A), and the acceptor moiety represented by the formula (AX1) are bonded.

The compound according to the first aspect of the first exemplary embodiment is also preferably represented by a formula (D-DPA1-3), (D-DPA1-4), (D-DPA2-3), (D-DPA2-4), (D-DPA3-3), or (D-DPA3-4) below.

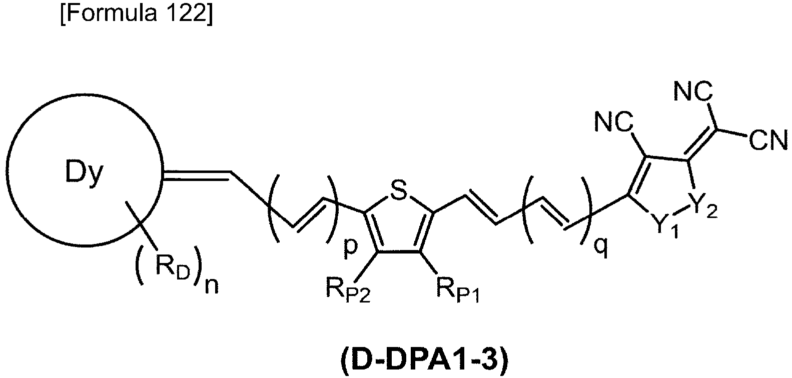

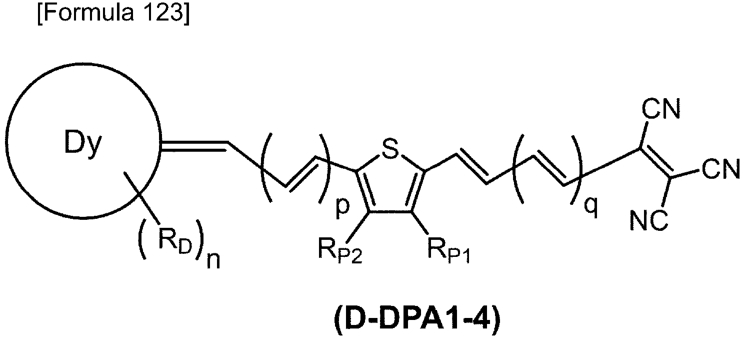

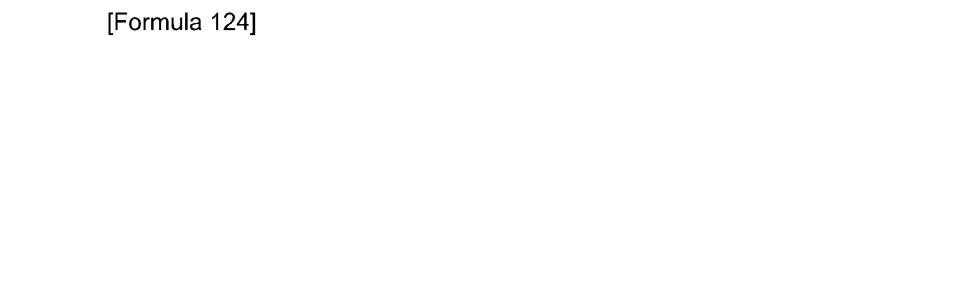

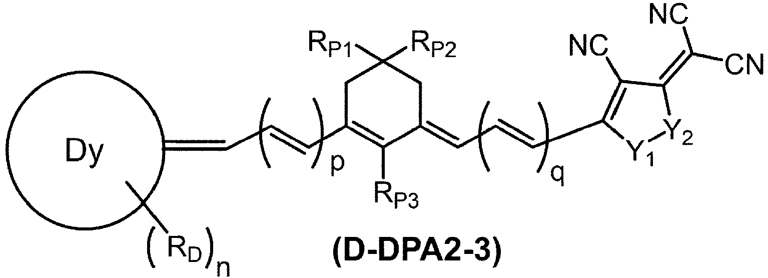

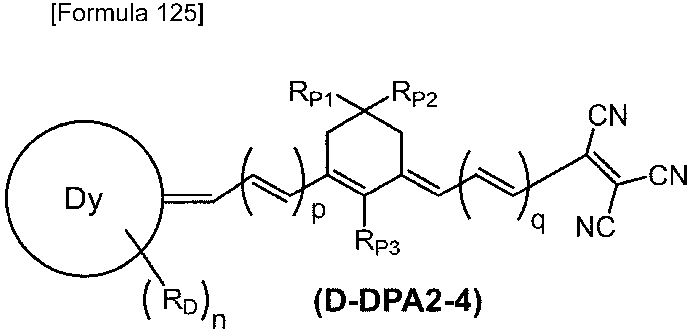

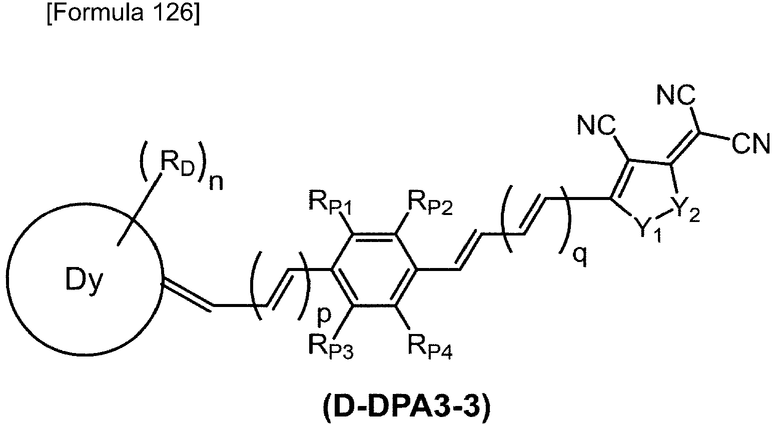

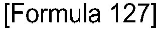

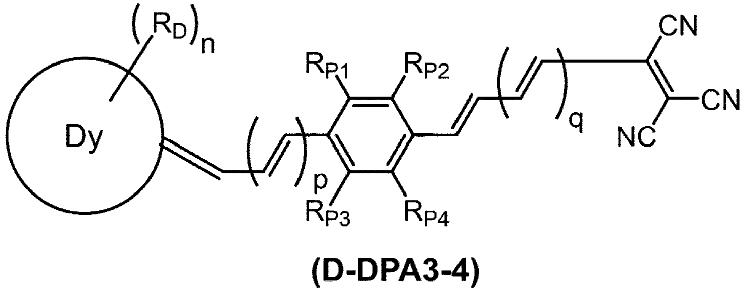

In the formulae (D-DPA1-3), (D-DPA1-4), (D-DPA2-3), (D-DPA2-4), (D-DPA3-3), and (D-DPA3-4): Dy, R_{D}, and n each independently represent the same as Dy, R_{D}, and n in the formula (D2); R_{P1}, R_{P2}, R_{P3}, R_{P4}, p, and q respectively represent the same as R_{P1}, R_{P2}, R_{P3}, R_{P4}, p, and q in the formula (PX1), (PX2), or (PX3); and Y₁ and Y₂ respectively represent the same as Y₁ and Y₂ in the formula (AX1).

The compound represented by the formula (D-DPA1-3) or (D-DPA1-4) corresponds to a compound in which the donor moiety represented by the formula (D2), the π-conjugated moiety represented by the formula (PX1), and the acceptor moiety represented by the formula (AX1) or (AX2) are bonded.

The compound represented by the formula (D-DPA2-3) or (D-DPA2-4) corresponds to a compound in which the donor moiety represented by the formula (D2), the π-conjugated moiety represented by the formula (PX2), and the acceptor moiety represented by the formula (AX1) or (AX2) are bonded.

The compound represented by the formula (D-DPA3-3) or (D-DPA3-4) corresponds to a compound in which the donor moiety represented by the formula (D2), the π-conjugated moiety represented by the formula (PX3), and the acceptor moiety represented by the formula (AX1) or (AX2) are bonded.

The compound according to the first aspect of the first exemplary embodiment is also preferably represented by a formula (D123-DPA1-1), (D123-DPA11-1), (D123-DPA4-1), (D123-DPA1-A11), or (D123-DPA11-A11) below.

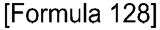

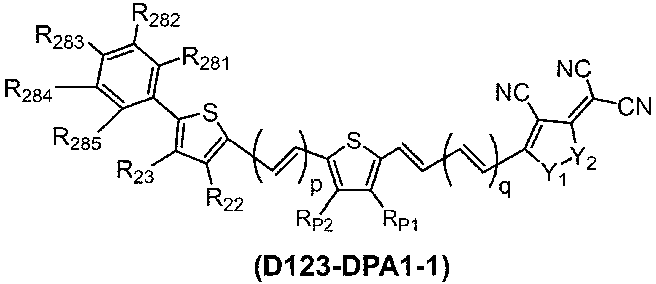

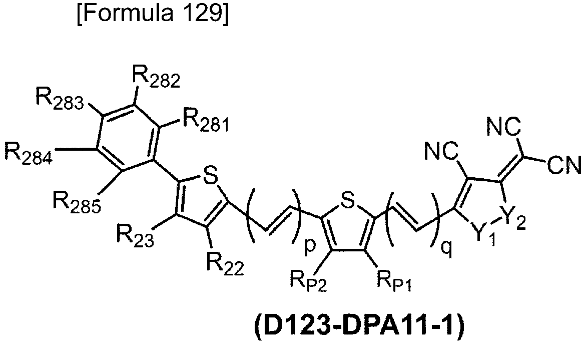

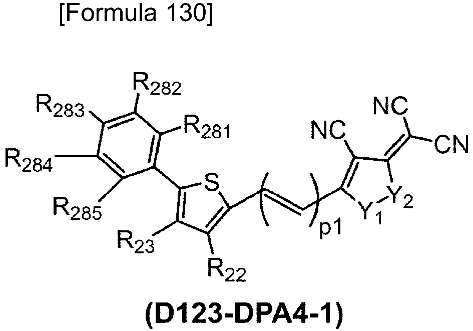

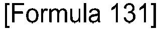

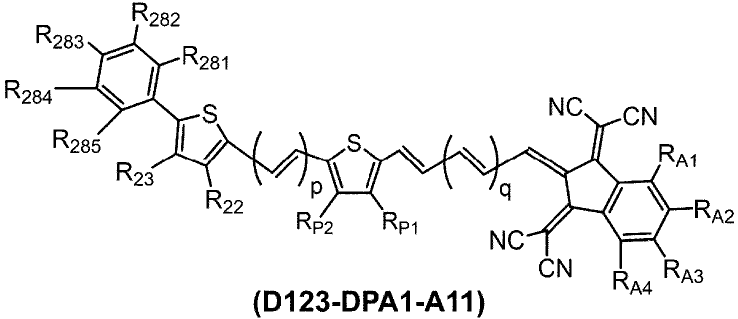

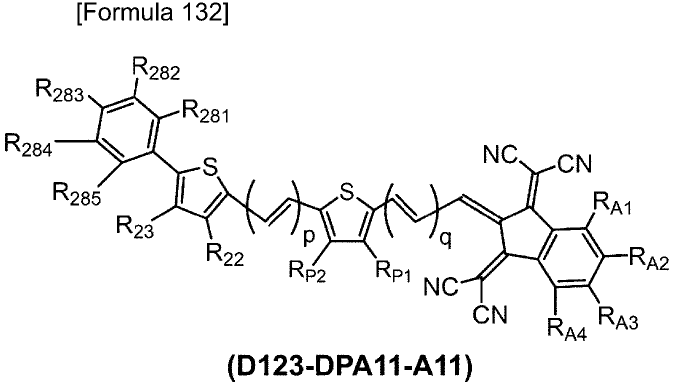

In the formulae (D123-DPA1-1), (D123-DPA11-1), (D123-DPA4-1), (D123-DPA1-A11), and (D123-DPA11-A11):
R₂₂, R₂₃, and R₂₈₁ to R₂₈₅ each independently represent the same as R₂₂, R₂₃, and R₂₈₁ to R₂₈₅ in the formula (D123-1B);
R_{P1} and R_{P2} each independently represent the same as R_{P1} and R_{P2} in the formulae (PX1) and (PX11);
p is 1, 2, or 3;
p1 represents the same as p1 in the formula (PX4);
Y₁ and Y₂ each independently represent the same as Y₁ and Y₂ in the formula (AX1); and
R_{A1}, R_{A2}, R_{A3}, and R_{A4} each independently represent the same as R_{A1}, R_{A2}, R_{A3}, and R_{A4} in the formula (A11-2).

The compound represented by the formula (D123-DPA1-1), (D123-DPA11-1), or (D123-DPA4-1) corresponds to a compound in which the donor moiety represented by the formula (D123-1B), the π-conjugated moiety represented by the formula (PX1), (PX11) or (PX4), and the acceptor moiety represented by the formula (AX1) are bonded.

The compound represented by the formula (D123-DPA1-A11) or (D123-DPA11-A11) corresponds to a compound in which the donor moiety represented by the formula (D123-1B), the π-conjugated moiety represented by the formula (PX1) or (PX11), and the acceptor moiety represented by the formula (A11-2) are bonded.

The compound according to the first aspect of the first exemplary embodiment is also preferably represented by a formula (D123-DPA5-1) below.

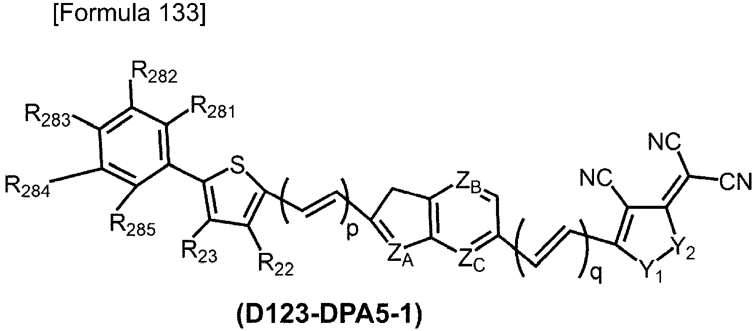

In the formula (D123-DPA5-1):
R₂₂, R₂₃, and R₂₈₁ to R₂₈₅ each independently represent the same as R₂₂, R₂₃, and R₂₈₁ to R₂₈₅ in the formula (D123-1B);
Z_{A}, Z_{B}, and Z_{C} each independently represent the same as Z_{A}, Z_{B}, and Z_{C} in the formula (PX5);
p is 1, 2, or 3; and
Y₁ and Y₂ each independently represent the same as Y₁ and Y₂ in the formula (AX1).

The compound represented by the formula (D123-DPA5-1) corresponds to a compound in which the donor moiety represented by the formula (D123-1B), the π-conjugated moiety represented by the formula (PX5), and the acceptor moiety represented by the formula (AX1) are bonded.

The compound according to the first aspect of the first exemplary embodiment is also preferably represented by a formula (D123-DPA15-1) below.

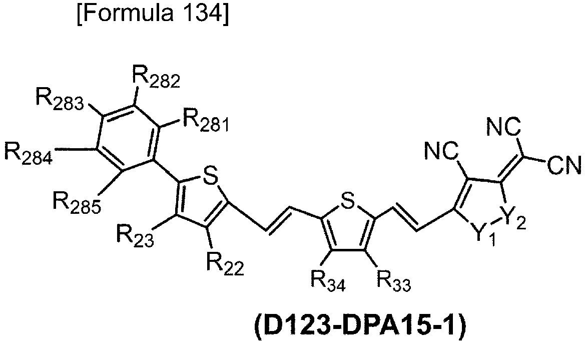

In the formula (D123-DPA15-1):
R₂₂, R₂₃, and R₂₈₁ to R₂₈₅ each independently represent the same as R₂₂, R₂₃, and R₂₈₁ to R₂₈₅ in the formula (D123-1B);
R₃₃ and R₃₄ each independently represent the same as R₃₃ and R₃₄ in the formula (P15-1A); and
Y₁ and Y₂ each independently represent the same as Y₁ and Y₂ in the formula (AX1).

The compound represented by the formula (D123-DPA15-1) corresponds to a compound in which the donor moiety represented by the formula (D123-1B), the π-conjugated moiety represented by the formula (P15-1A), and the acceptor moiety represented by the formula (AX1) are bonded.

The compound according to the first aspect of the first exemplary embodiment is also preferably represented by a formula (D111-DPA11-1) or (D111-DPA11-A11) below.

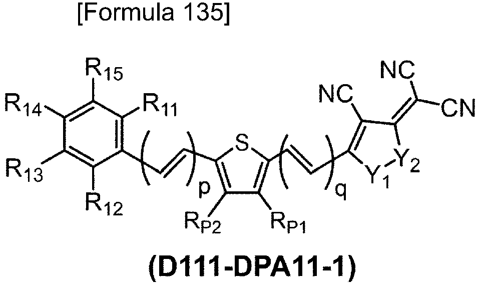

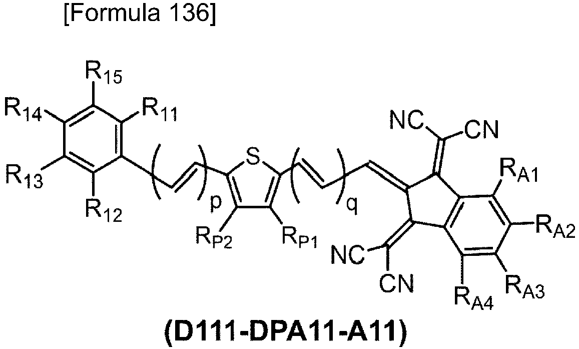

In the formulae (D111-DPA11-1) and (D111-DPA11-A11):
R₁₁ to R₁₅ are each independently a hydrogen atom, a group represented by -N(R₁₀₁)(R₁₀₂), a group represented by -Si(R₁₀₃)(R₁₀₄)(R₁₀₅), a group represented by -C(=O)R₁₀₆, a group represented by -O(R₁₁₃), a substituted or unsubstituted alkyl group, a substituted or unsubstituted cycloalkyl group, a substituted or unsubstituted aryl group, or a substituted or unsubstituted heterocyclic group;
R_{P1}, R_{P2}, p, and q each independently represent the same as R_{P1}, R_{P2}, p, and q in the formula (PX11);
Y₁ and Y₂ each independently represent the same as Y₁ and Y₂ in the formula (AX1); and
R_{A1}, R_{A2}, R_{A3}, and R_{A4} each independently represent the same as R_{A1}, R_{A2}, R_{A3}, and R_{A4} in the formula (A11-2).

The compound represented by the formula (D111-DPA11-1) or (D111-DPA11-A11) corresponds to a compound in which the donor moiety represented by the formula (D111), the π-conjugated moiety represented by the formula (PX11), and the acceptor moiety represented by the formula (AX1) or (A11-2) are bonded.

In one form of the compound according to the first exemplary embodiment, it is also preferable that the compound is represented by a formula (A-DP1), (A-DP2), or (A-DP3) below.

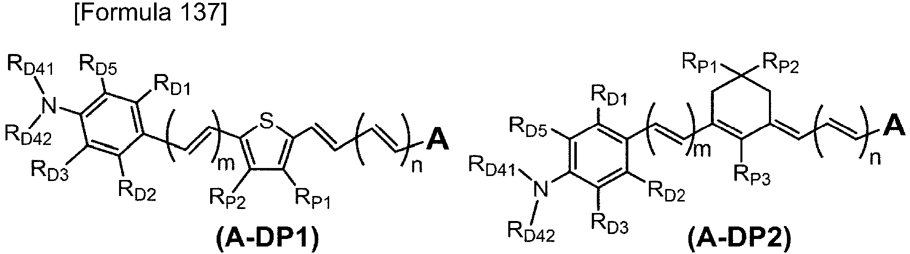

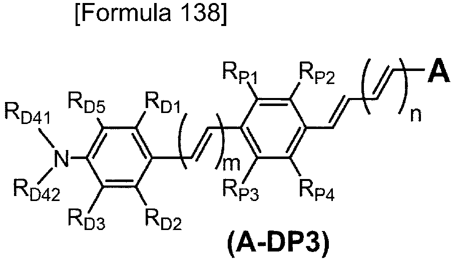

In the formulae (A-DP1), (A-DP2), and (A-DP3):
A is the acceptor moiety;
R_{D1}, R_{D2}, R_{D3}, R_{D5}, R_{D41}, and R_{D42} respectively represent the same as R_{D1}, R_{D2}, R_{D3}, R_{D5}, R_{D41}, and R_{D42} in the formula (DX1); and
R_{P1}, R_{P2}, R_{P3}, R_{P4}, m, and n respectively represent the same as R_{P1}, R_{P2}, R_{P3}, R_{P4}, m, and n in the formula (PX1a), (PX2a), or (PX3a).

In one form of the compound represented by the formula (A-DP1), (A-DP2), or (A-DP3), A is the acceptor moiety in the compound according to the first aspect, the acceptor moiety in the compound according to the second aspect, or the acceptor moiety in the compound according to the third aspect.

The compound according to the second aspect of the first exemplary embodiment is preferably represented by a formula (A-DPA1), (A-DPA2), (A-DPA3), (A-DPA4), (A-DPA5), or (A-DPA6) below.

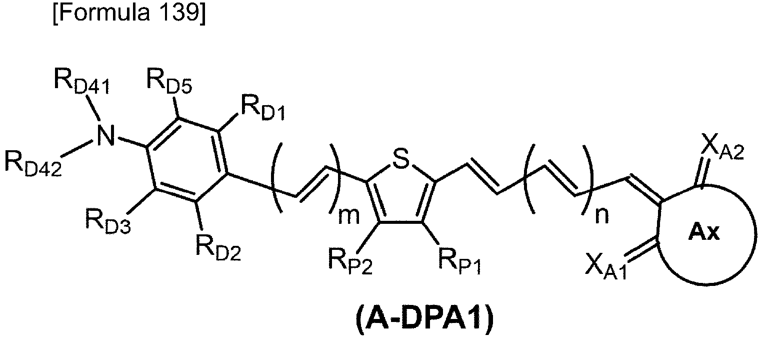

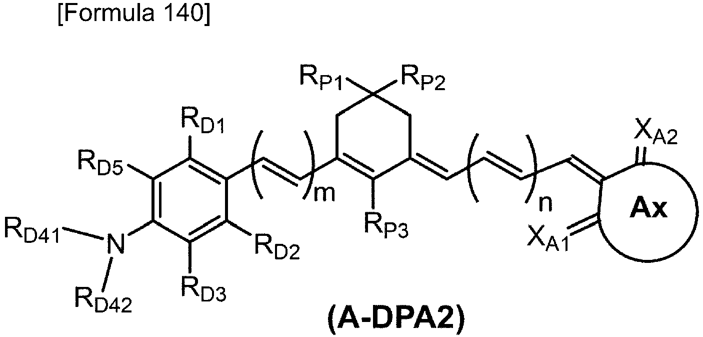

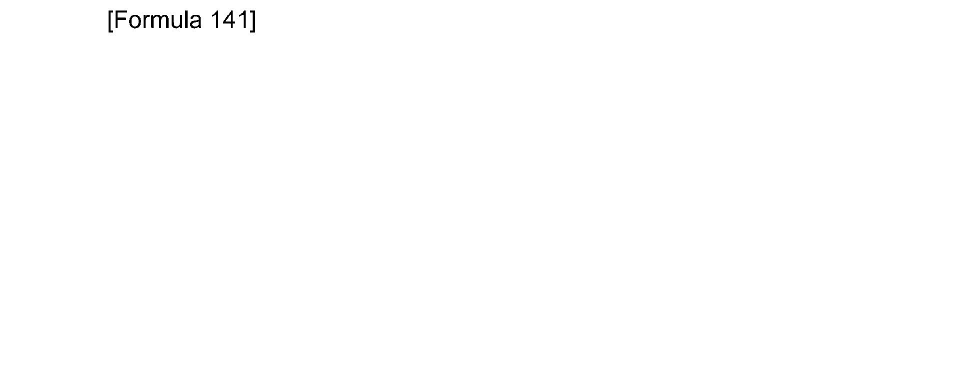

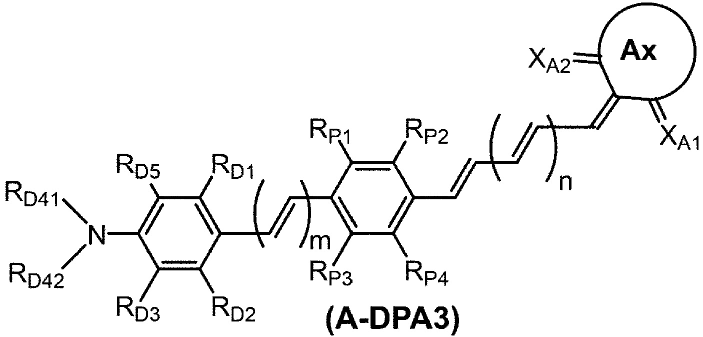

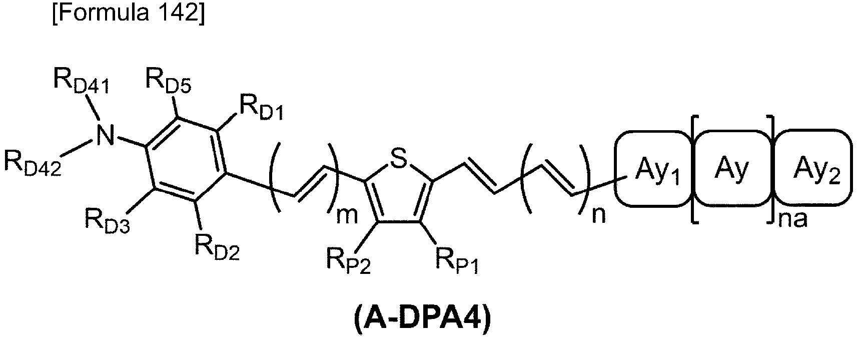

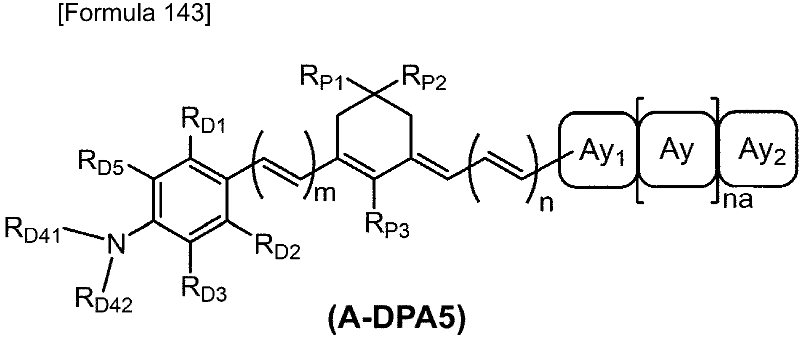

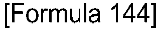

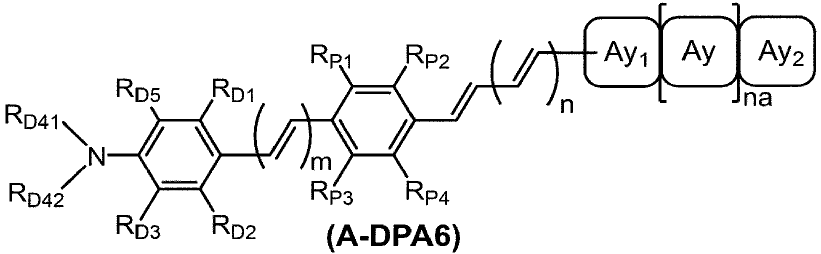

In the formulae (A-DPAT1), (A-DPA2), (A-DPA3), (A-DPA4), and (A-DPA6):
R_{P1}, R_{P2}, R_{D3}, R_{D5}, R_{D41}, and R_{D42} respectively represent the same as R_{D1}, R_{D2}, R_{D3}, R_{D5}, R_{D41}, and R_{D42} in the formula (DX1); and
R_{P1}, R_{P2}, R_{P3}, R_{P4}, m, and n respectively represent the same as R_{P1}, R_{P2}, R_{P3}, R_{P4}, m, and n in the formula (PX1a), (PX2a), or (PX3a);
X_{A1}, X_{A2}, and the ring Ax respectively represent the same as X_{A1}, X_{A2}, and the ring Ax in the formula (A1); and
na, the ring Ay, the ring Ay₁, and the ring Ay₂ respectively represent the same as na, the ring Ay, the ring Ay₁, and the ring Ay₂ in the formula (A2).

One form of the compound according to the first exemplary embodiment is also preferably a compound represented by a formula (P-DP1) or (P-DP2) below.

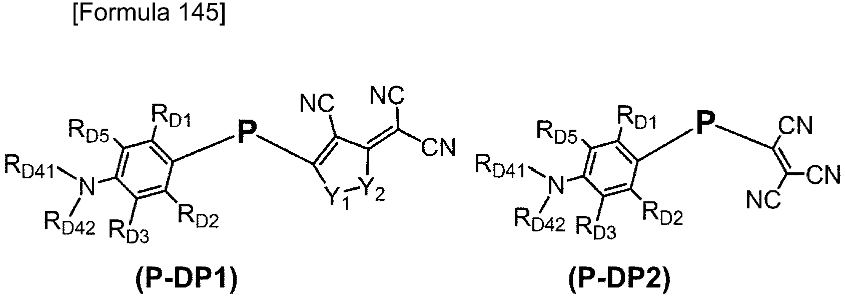

In one form of the compound represented by the formula (P-DP1) or (P-DP2), P is the π-conjugated moiety in the compound according to the first aspect, the π-conjugated moiety in the compound according to the second aspect, or the π-conjugated moiety in the compound according to the third aspect.

In the formulae (P-DP1) and (P-DP2):
P is the π-conjugated moiety;
R_{D1}, R_{D2}, R_{D3}, R_{D5}, R_{D41}, and R_{D42} respectively represent the same as R_{D1}, R_{D2}, R_{D3}, R_{D5}, R_{D41}, and R_{D42} in the formula (DX1); and
Y₁ and Y₂ respectively represent the same as Y₁ and Y₂ in the formula (AX1).

The compound according to the third aspect of the first exemplary embodiment is also preferably represented by a formula (P-DPA1-1), (P-DPA1-2), (P-DPA2-1), (P-DPA2-2), (P-DPA3-1), or (P-DPA3-2) below.

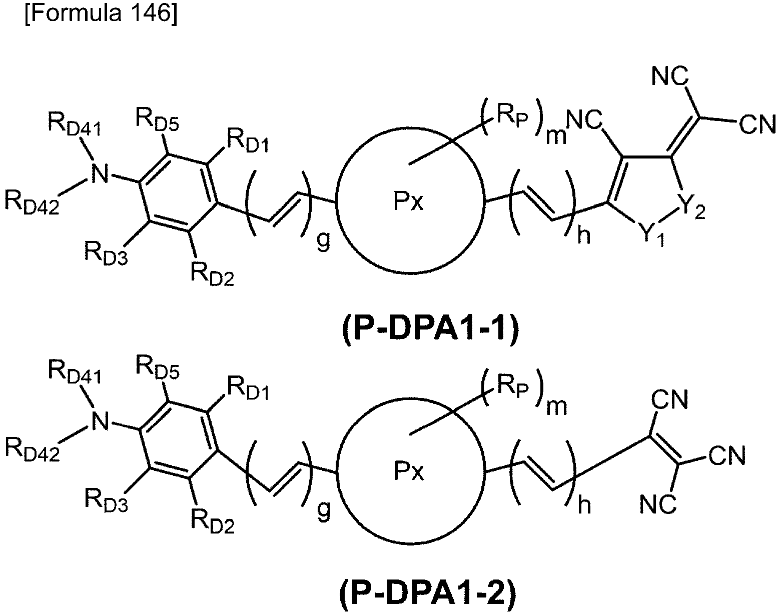

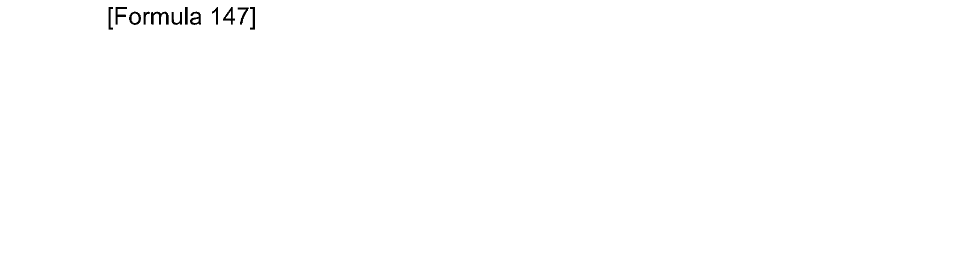

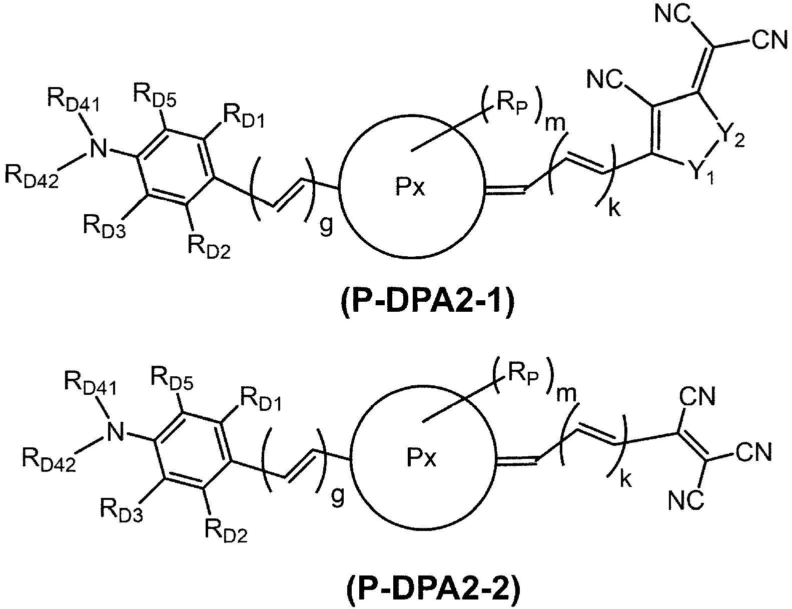

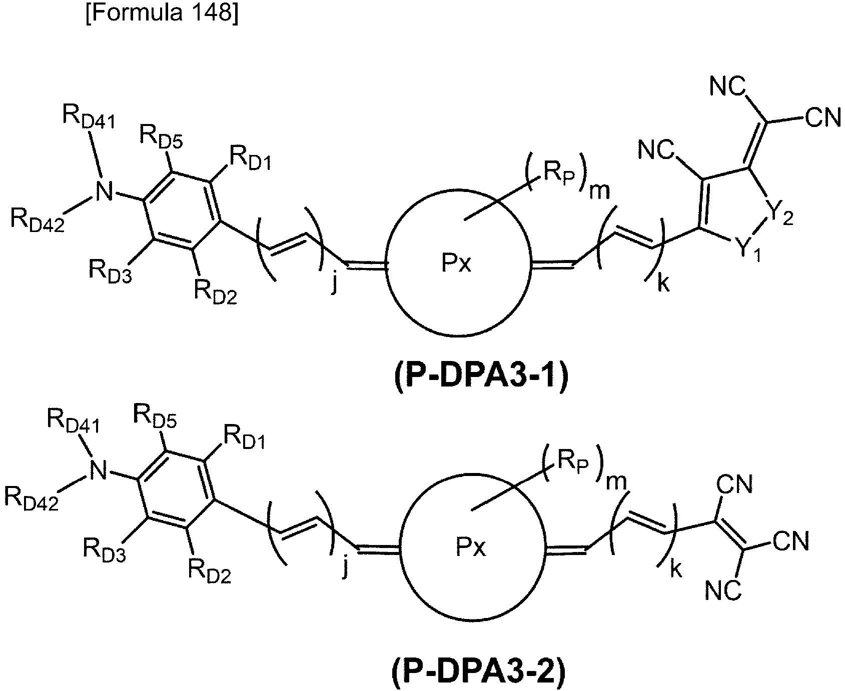

In the formulae (P-DPA1-1), (P-DPA1-2), (P-DPA2-1), (P-DPA2-2), (P-DPA3-1), and (P-DPA3-2):
R_{D1}, R_{D2}, R_{D3}, R_{D5}, R_{D41}, and R_{D42} respectively represent the same as R_{D1}, R_{D2}, R_{D3}, R_{D5}, R_{D41}, and R_{D42} in the formula (DX1); and
R_{P}, m, g, h, j, and k respectively represent the same as R_{P}, m, g, h, j, and k in the formula (P1), (P2), or (P3); and
Y₁ and Y₂ respectively represent the same as Y₁ and Y₂ in the formula (AX1).

The compound represented by the formula (P-DPA1-1) or (P-DPA1-2) corresponds to a compound in which the donor moiety represented by the formula (DX1), the π-conjugated moiety represented by the formula (P1), and the acceptor moiety represented by the formula (AX1) or (AX2) are bonded.

The compound represented by the formula (P-DPA2-1) or (P-DPA2-2) corresponds to a compound in which the donor moiety represented by the formula (DX1), the π-conjugated moiety represented by the formula (P2), and the acceptor moiety represented by the formula (AX1) or (AX2) are bonded.

The compound represented by the formula (P-DPA3-1) or (P-DPA3-2) corresponds to a compound in which the donor moiety represented by the formula (DX1), the π-conjugated moiety represented by the formula (P3), and the acceptor moiety represented by the formula (AX1) or (AX2) are bonded.

### Method of Producing Compound according to First Exemplary Embodiment

The respective compounds according to the first aspect, the second aspect, and the third aspect of the first exemplary embodiment can each be produced according to a known method (for example, a method described in Patent Literature 1 and Patent Literature 2 described above). The respective compounds according to the first aspect, the second aspect, and the third aspect of the first exemplary embodiment can also be produced, in accordance with known methods, by using known alternative reactions and raw materials suitable for the target compound. Furthermore, the respective compounds according to the first aspect, the second aspect, and the third aspect of the first exemplary embodiment can also be produced according to synthesis methods described in Examples described later, or in accordance with these synthesis methods, by using known alternative reactions and raw materials suitable for the target compound.

For example, the following compound (D101) according to the first aspect of the first exemplary embodiment or the following compound (A101) according to the second aspect can also be produced according to the following methods; however, the production methods of the compounds according to the first aspect and the second aspect of the first exemplary embodiment are not limited to the following production methods.

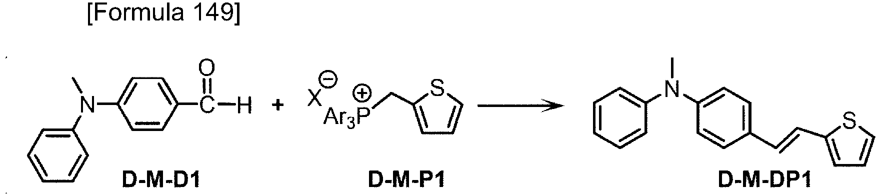

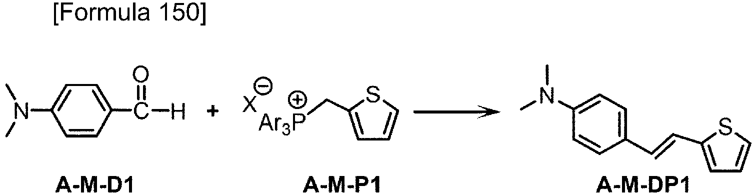

As shown in the above reaction scheme, a compound D-M-DP1 can be obtained by reacting a compound D-M-D1 with a compound D-M-P1 in the presence of a base. A compound A-M-DP1 can also be obtained by reacting a compound AM-D1 with a compound A-M-P1 in the presence of a base. Here, Ar in the compound D-M-P1 and the compound A-M-P1 represents an aryl group, for example, a phenyl group, and X represents a halogen atom, for example, a chlorine atom. The compound D-M-DP1 can also be obtained, as shown in the following reaction scheme, by using the following compound D-M-P2 instead of the compound D-M-P1. The compound A-M-DP1 can also be obtained, as shown in the following reaction scheme, by using a compound A-M-P2 instead of the compound A-M-P1.

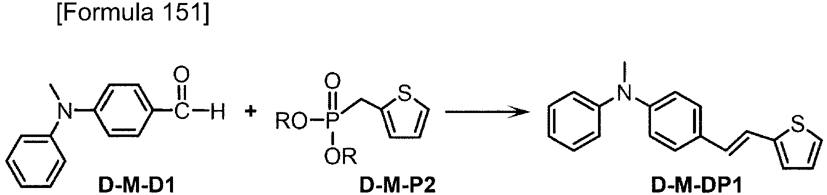

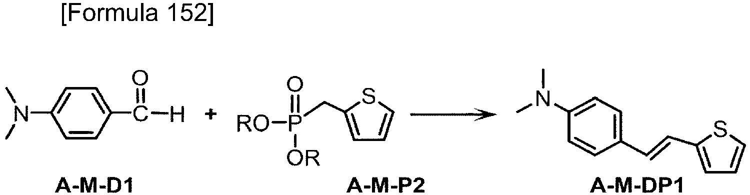

After obtaining the compound D-M-DP1, the compound D-M-DP2 can be obtained by reacting the compound D-M-DP1 with alkyllithium (e.g., n-butyllithium) or the like in an organic solvent (e.g., tetrahydrofuran), and then reacting with N,N-dimethylformamide, N-formylpiperidine, or the like. Alternatively, after obtaining the compound A-M-DP1, the compound A-M-DP2 can be obtained by reacting the compound A-M-DP1 with alkyllithium (e.g., n-butyllithium) or the like in an organic solvent (e.g., tetrahydrofuran), and then reacting with N,N-dimethylformamide, N-formylpiperidine, or the like.

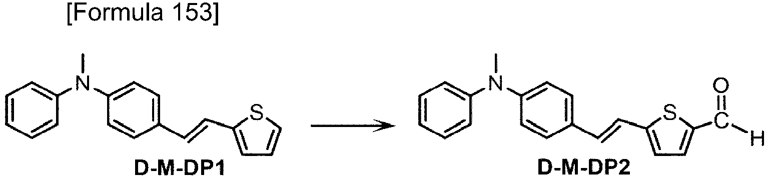

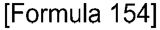

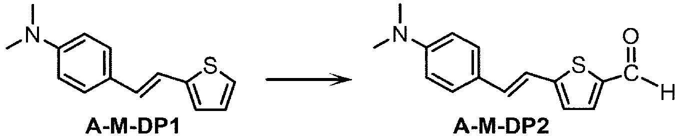

Next, the compound (D101) can be obtained by reacting the compound D-M-DP2 with a compound D-M-A1 below. Alternatively, the compound (A101) can be obtained by reacting the compound A-M-DP2 with a compound A-M-A1 below.

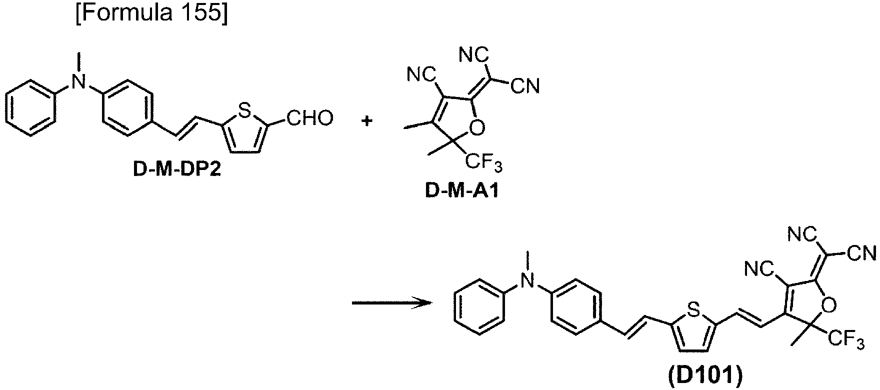

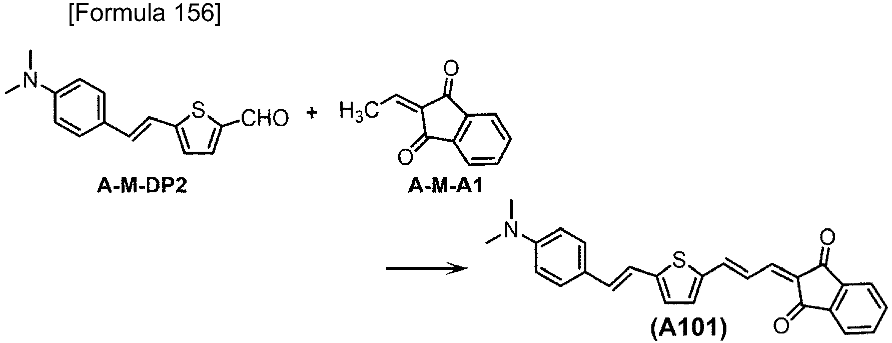

The reaction between the compound D-M-DP2 and the compound D-M-A1, or the reaction between the compound A-M-DP2 and the compound A-M-A1, is usually carried out in a polar solvent. The polar solvent may be a polar protic solvent, a polar aprotic solvent, or a mixed polar solvent thereof. As the polar protic solvent, for example, at least one selected from the group consisting of water, methanol, ethanol, 1-propanol, 2-propanol, 1-butanol, acetic acid, and formic acid may be used, or a mixed solvent obtained by mixing two or more of these polar protic solvents may be used. As the polar aprotic solvent, for example, at least one selected from the group consisting of tetrahydrofuran (THF), acetone, acetonitrile, N,N-dimethylformamide (DMF), dimethyl sulfoxide (DMSO), methylene chloride, chloroform, dioxane, and N-methylpyrrolidone may be used, or a mixed solvent obtained by mixing these polar aprotic solvents may be used. After completion of the reaction, the compound (D101) or the compound (A101) can be obtained by purifying a crude product obtained by a conventional work-up according to a conventional method, or without purification.

In the compound according to the third aspect of the first exemplary embodiment, for example, a compound (P101) below can also be produced by the following method; however, the production method of the compound according to the third aspect of the first exemplary embodiment is not limited to the following production method.

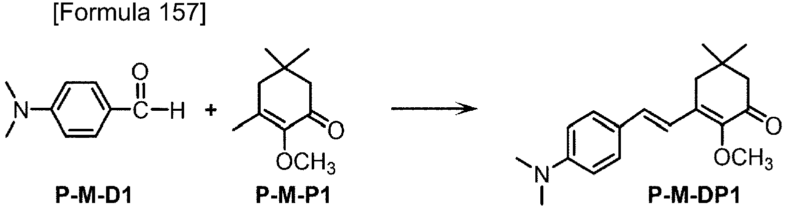

As shown in the above reaction scheme, a compound P-M-DP1 can be obtained by reacting a compound P-M-D1 with a compound P-M-P1. After obtaining the compound P-M-DP1, a compound P-M-DP2 below can be obtained by reacting the compound P-M-DP1 with alkylideneimine (e.g., C6H11N=CHCH2Li), followed by treatment with water.

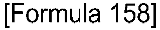

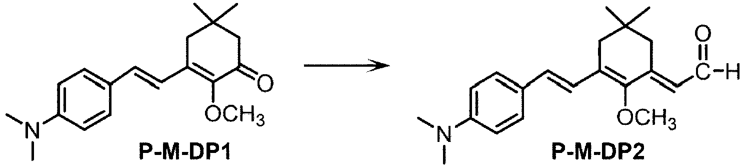

After obtaining the compound P-M-DP2, the compound (P101) below can be obtained by reacting the compound P-M-DP2 with the compound P-M-A1.

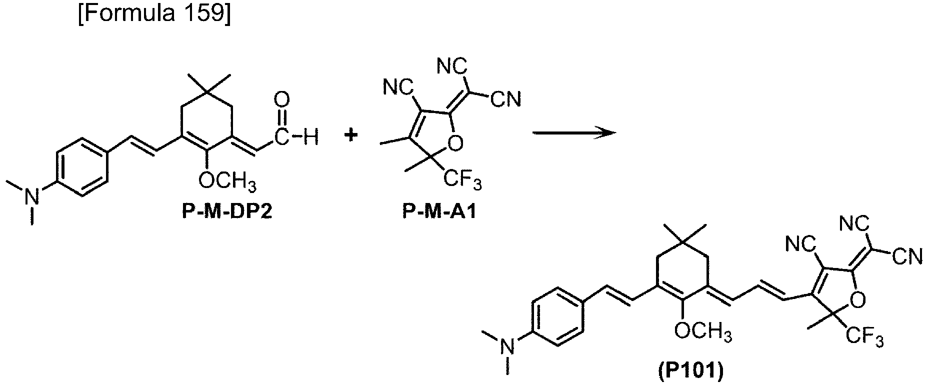

In the reaction scheme of the compound according to the third aspect, the reaction between P-M-DP2 and the compound P-M-A1 is usually carried out in a polar solvent. The polar solvent may be a polar protic solvent, a polar aprotic solvent, or a mixed polar solvent thereof. As the polar protic solvent, for example, at least one selected from the group consisting of water, methanol, ethanol, 1-propanol, 2-propanol, 1-butanol, acetic acid, and formic acid may be used, or a mixed solvent obtained by mixing two or more of these polar protic solvents may be used. As the polar aprotic solvent, for example, at least one selected from the group consisting of tetrahydrofuran (THF), acetone, acetonitrile, N,N-dimethylformamide (DMF), dimethyl sulfoxide (DMSO), methylene chloride, chloroform, dioxane, and N-methylpyrrolidone may be used, or a mixed solvent obtained by mixing these polar aprotic solvents may be used. After completion of the reaction, the compound (P101) can be obtained by purifying a crude product obtained by a conventional work-up according to a conventional method, or without purification.

In the reaction schemes for the compounds according to the first aspect, the second aspect, and the third aspect described above, the compound (D101), (A101), or (P101) is obtained by bonding the acceptor moiety to the intermediate obtained by bonding the donor moiety and the π-conjugated moiety; however, the production methods of the compounds according to the first aspect, the second aspect, and the third aspect of the first exemplary embodiment are not limited to such reaction schemes. For example, the compound (D101), (A101), or (P101) may also be obtained by bonding the donor moiety to the intermediate obtained by bonding the acceptor moiety and the π-conjugated moiety.

### Specific Examples of Compound according to First Exemplary Embodiment

Specific examples of the compound according to the first aspect of the first exemplary embodiment include the following compounds. However, the invention is by no means limited to these specific examples.

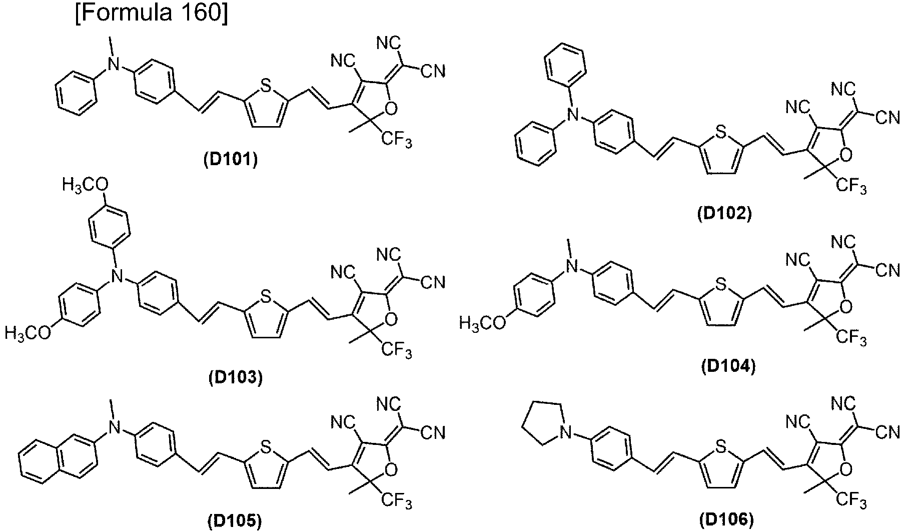

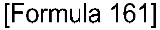

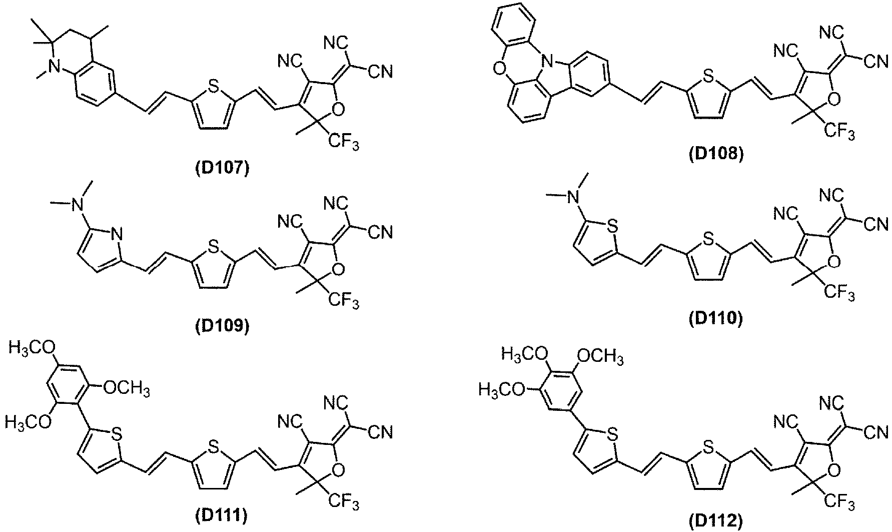

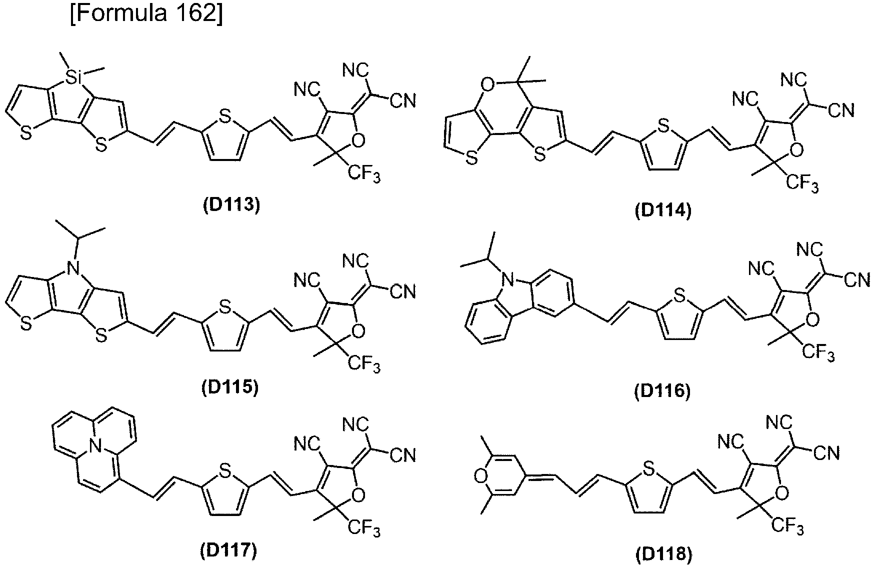

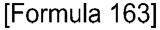

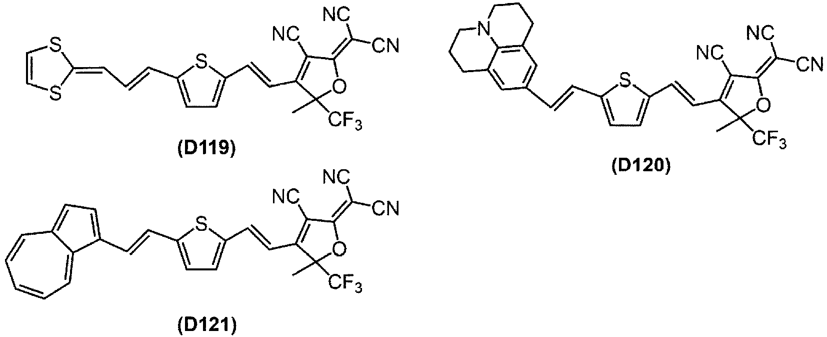

Specific examples of the compound according to the second aspect of the first exemplary embodiment include the following compounds. However, the invention is by no means limited to these specific examples.

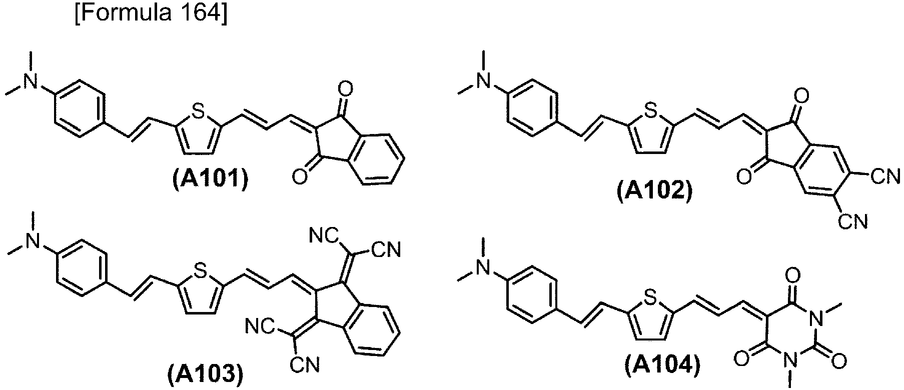

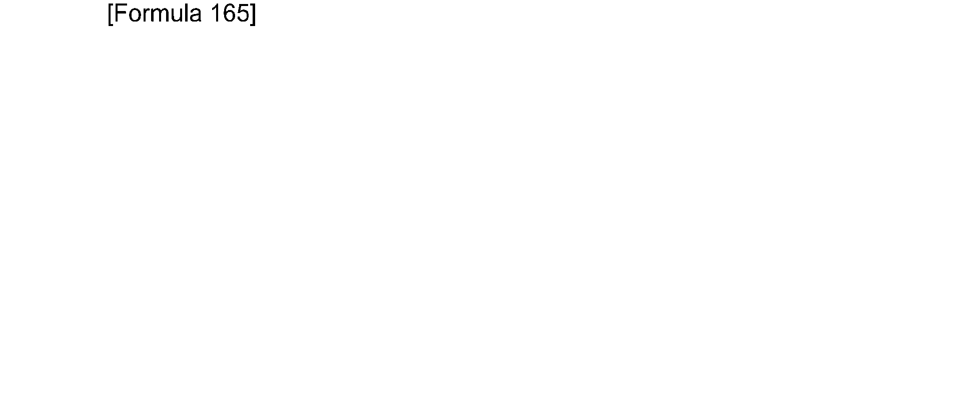

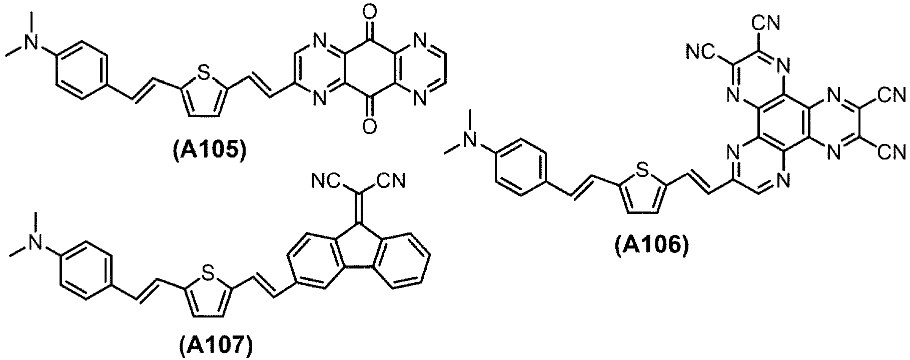

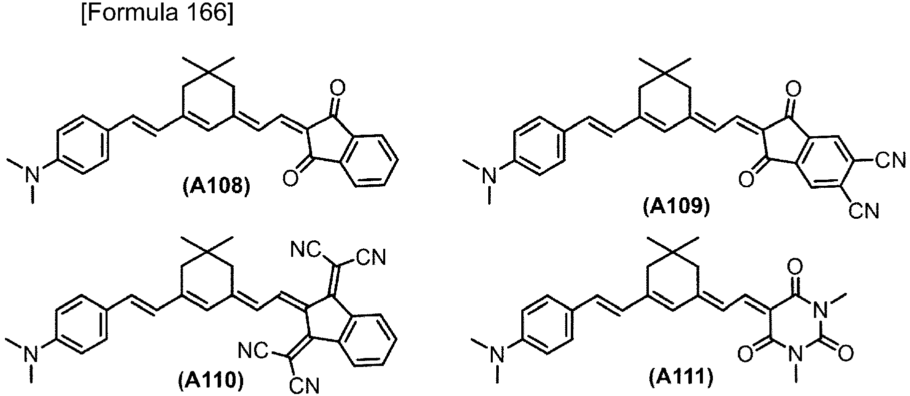

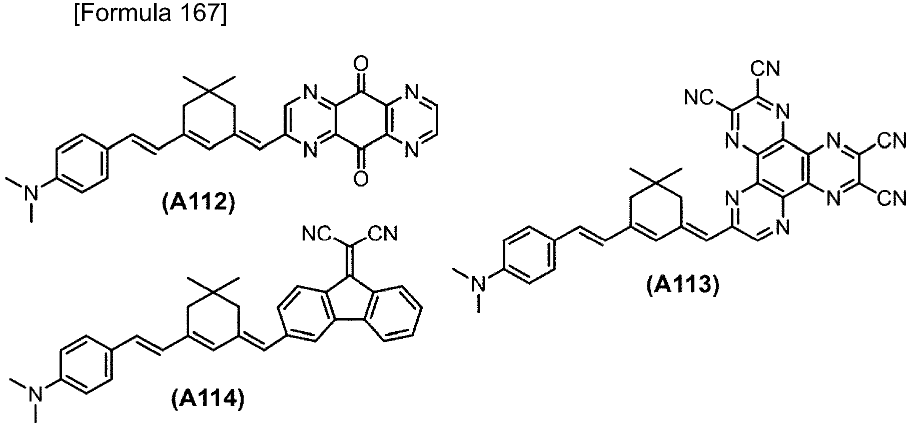

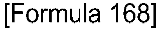

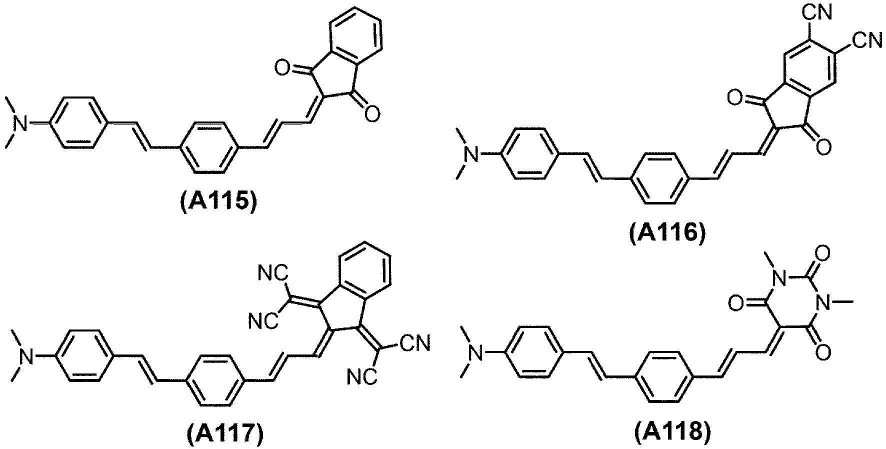

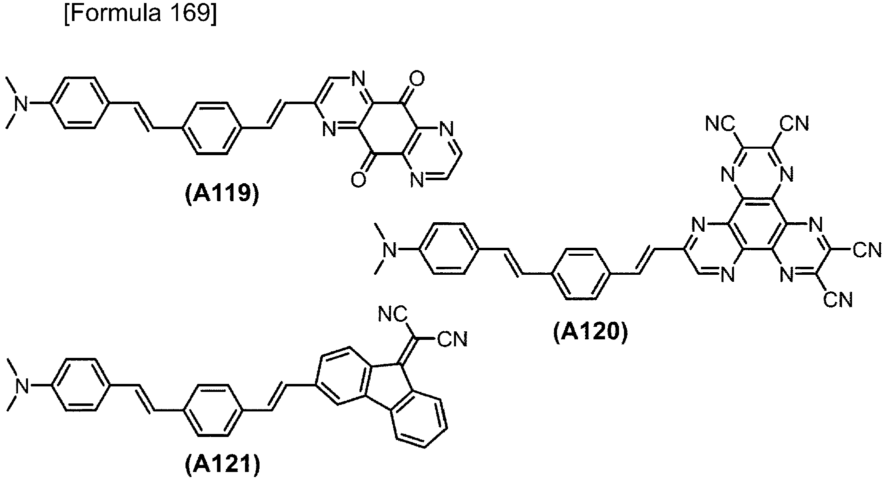

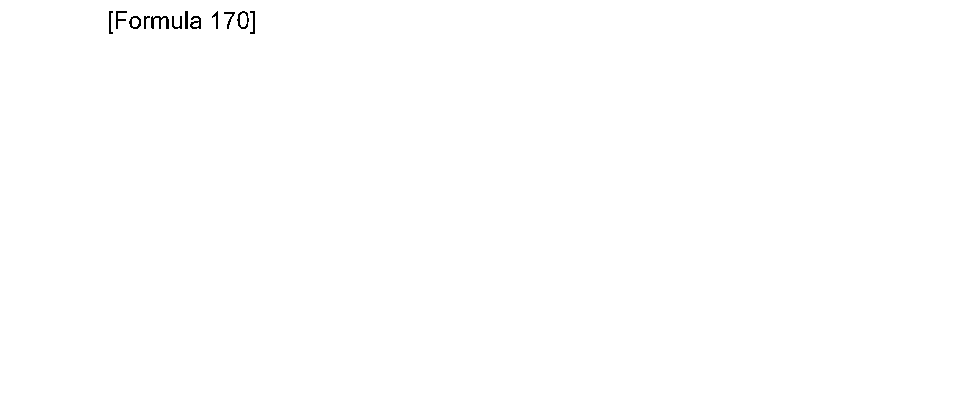

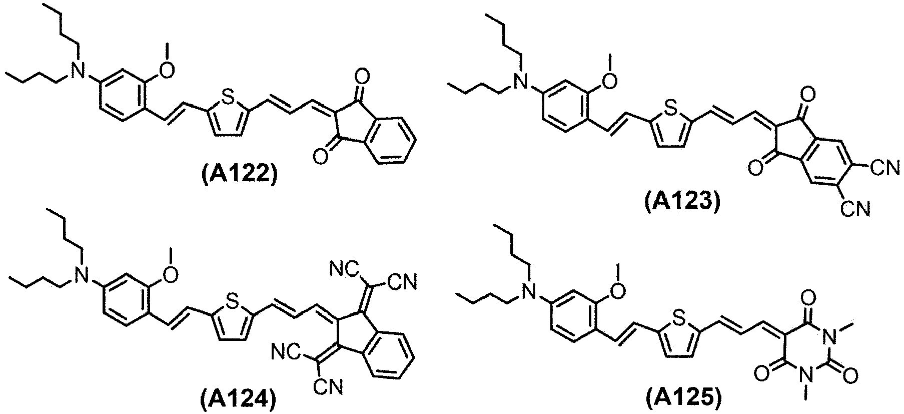

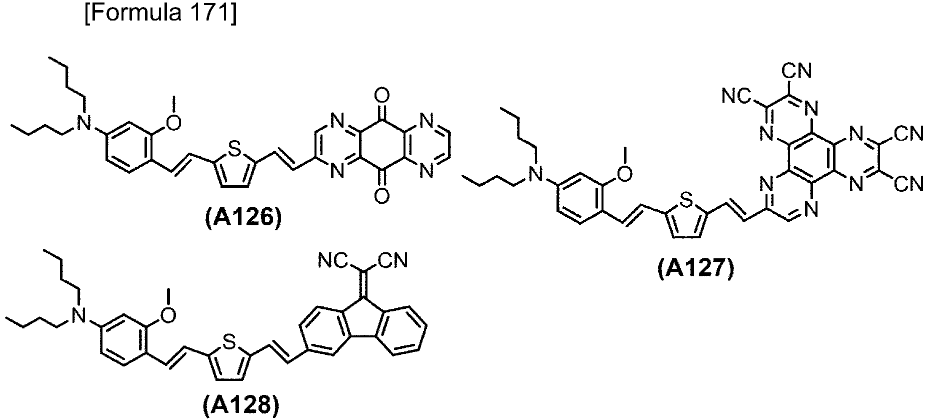

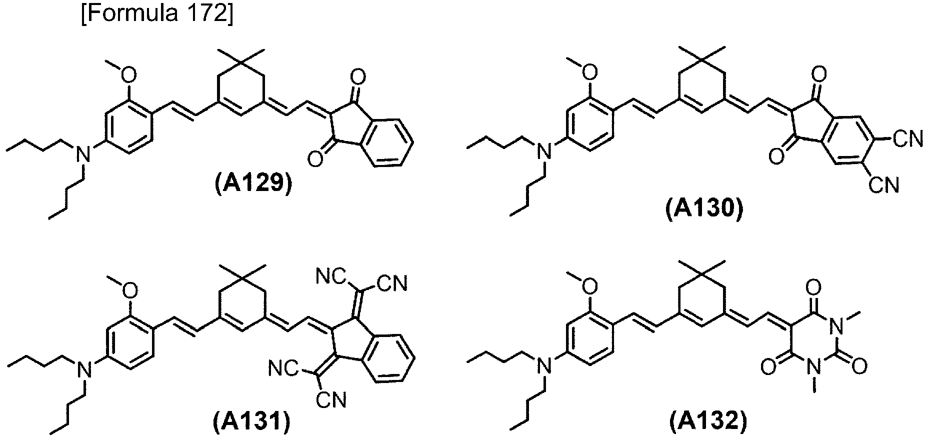

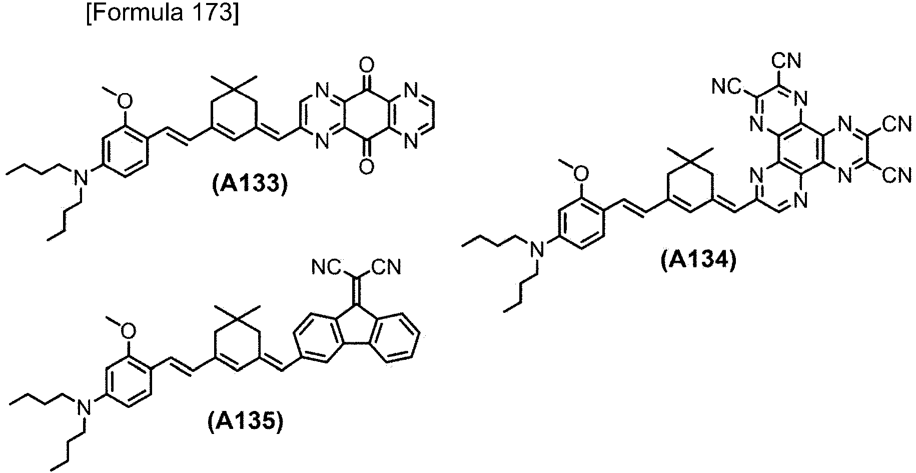

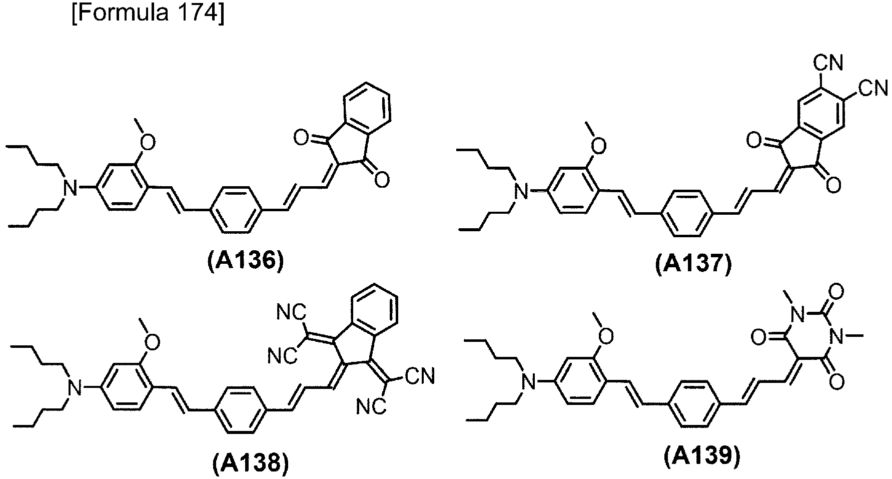

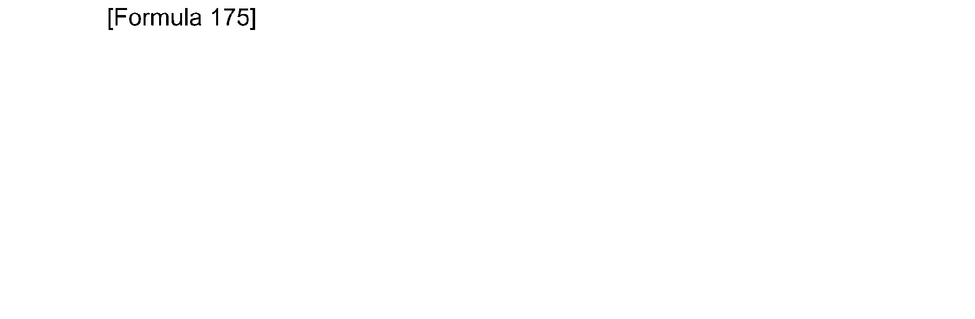

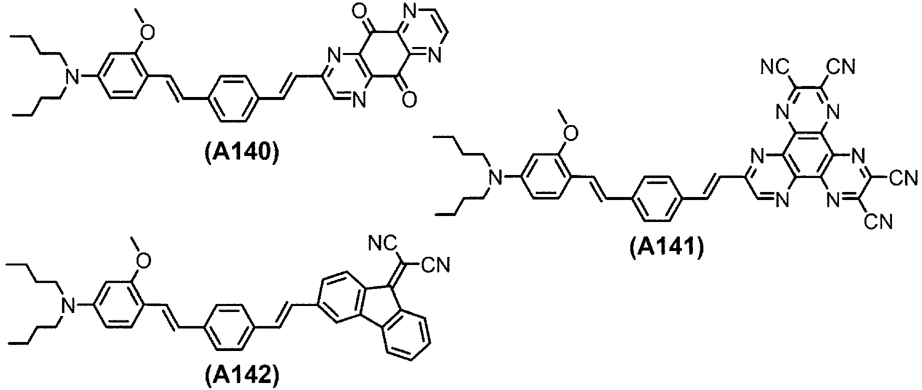

Specific examples of the compound according to the third aspect of the first exemplary embodiment include the following compounds. However, the invention is by no means limited to these specific examples.

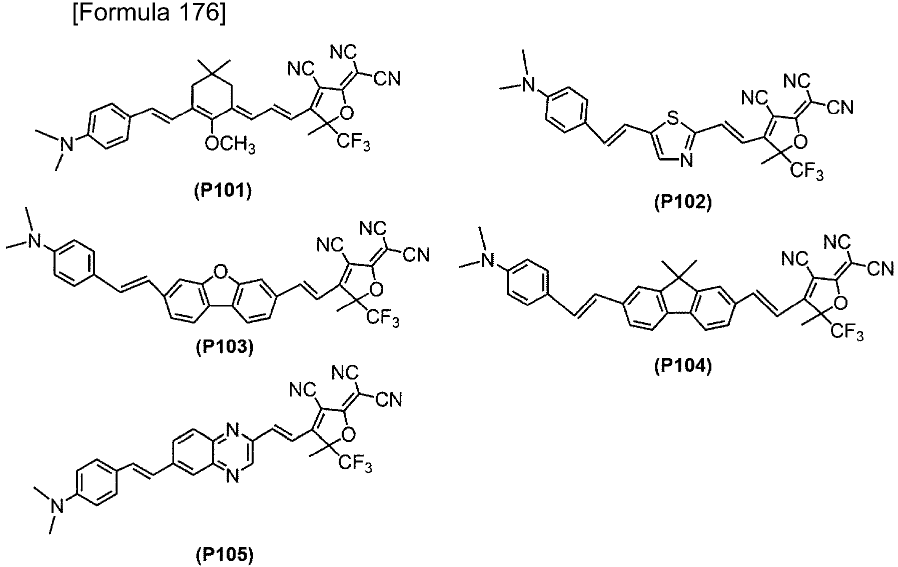

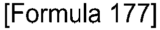

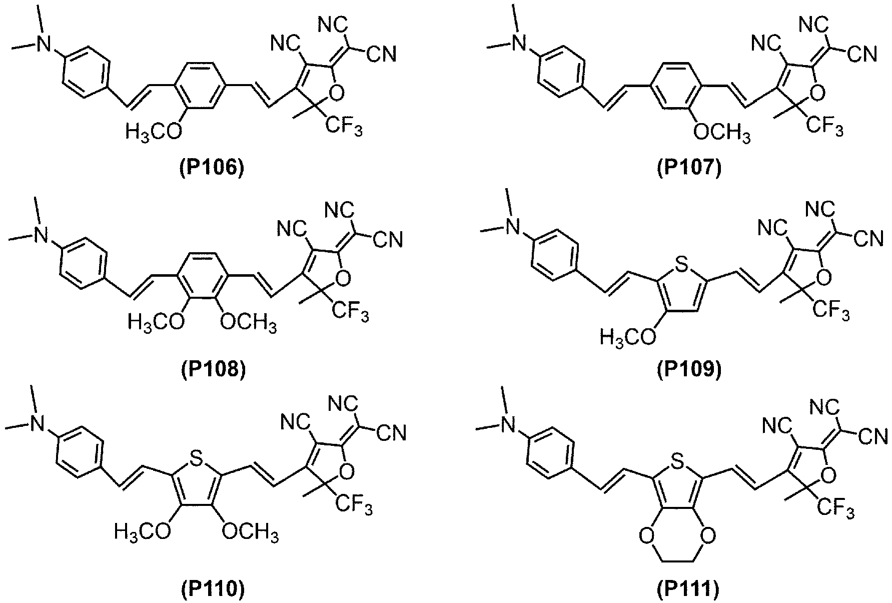

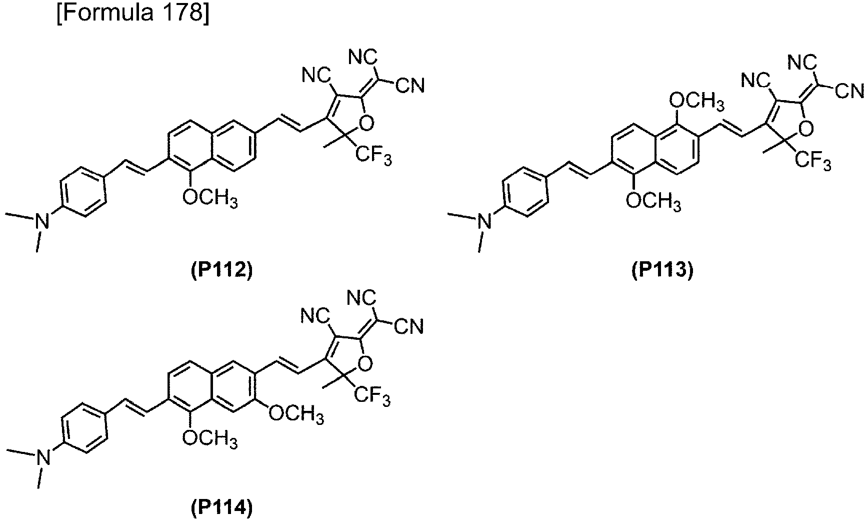

According to the first exemplary embodiment, a novel compound applicable to an electro-optic material can be provided. According to the compound according to the first exemplary embodiment, an improvement in hyperpolarizability can be expected. The hyperpolarizability can be calculated by a method described later. It is also possible to determine whether the compound is applicable to the electro-optic material based on the calculated hyperpolarizability of the compound.

### Hyperpolarizability

The hyperpolarizability can be calculated, for example, by quantum chemical calculations using Gaussian 16, which is a quantum chemical calculation program. Gaussian 16 is manufactured by Gaussian, Inc. Specifically, the hyperpolarizability can be calculated by the calculation method described in Examples.

### Description of Ring Structures and Substituents, etc.

### Aromatic hydrocarbon ring

In the first exemplary embodiment, the monocyclic aromatic hydrocarbon ring is preferably a benzene ring. The polycyclic aromatic hydrocarbon ring is preferably a fused polycyclic aromatic hydrocarbon ring. The number of ring carbon atoms in the polycyclic aromatic hydrocarbon ring is preferably from 9 to 30, more preferably from 9 to 18. Examples of the fused polycyclic aromatic hydrocarbon ring include a naphthalene ring, an indene ring, an azulene ring, a fluorene ring, an anthracene ring, a phenanthrene ring, a triphenylene ring, a naphthacene ring, a phenalene ring, a pyrene ring, and a chrysene ring.

### Aliphatic hydrocarbon ring

In the first exemplary embodiment, the aliphatic hydrocarbon ring means a ring that is non-aromatic and in which the ring atoms consist only of carbon atoms. The aliphatic hydrocarbon ring may be monocyclic or polycyclic. The polycyclic aliphatic hydrocarbon ring is, for example, a fused aliphatic ring, a bridged aliphatic ring, or a spiro aliphatic ring. The aliphatic hydrocarbon ring may be a saturated ring or an unsaturated ring. The number of ring carbon atoms in the monocyclic aliphatic hydrocarbon ring is preferably from 3 to 30, more preferably from 3 to 15, and still more preferably from 3 to 8. Specific examples of the monocyclic aliphatic hydrocarbon ring include saturated monocyclic aliphatic rings such as a cyclobutane ring, a cyclopentane ring, a cyclopentadiene ring, a cyclohexane ring, and a cycloheptane ring, and unsaturated monocyclic aliphatic rings such as a cyclobutene ring, a cyclopentene ring, a cyclohexene ring, and a cycloheptene ring. The number of ring carbon atoms in the polycyclic aliphatic hydrocarbon ring is preferably from 7 to 30, more preferably from 7 to 18, and still more preferably from 7 to 10. Specific examples of the polycyclic aliphatic hydrocarbon ring include fused aliphatic rings such as an indane ring, a decahydronaphthalene ring, and an octahydronaphthalene ring, and bridged aliphatic rings such as an adamantane ring and a norbornene ring.

### Heterocycle

In the first exemplary embodiment, the heterocycle is a ring containing one or more heteroatoms as ring atoms. Examples of the ring atoms of the heterocycle include a carbon atom, a nitrogen atom, an oxygen atom, a sulfur atom, a silicon atom, a boron atom, and a selenium atom. The heterocycle may contain two or more kinds of heteroatoms. Examples of the heterocycle include a nitrogen-containing heterocycle, a sulfur-containing heterocycle, and an oxygen-containing heterocycle. The heterocycle is a saturated ring or an unsaturated ring. The number of ring atoms in the polycyclic heterocycle is preferably from 3 to 9, more preferably 5 or 6. The number of ring atoms in the polycyclic aromatic hydrocarbon ring is preferably from 9 to 30, more preferably from 9 to 18.

Specific examples of the monocyclic heterocycle include a pyrrole ring, an imidazole ring, a pyrazole ring, an isoxazole ring, a triazole ring, a tetrazole ring, a pyridine ring, a pyrimidine ring, a pyridazine ring, a pyrazine ring, a triazine ring, a piperazine ring, a furan ring, an oxazole ring, an isoxazole ring, an oxadiazole ring, a dioxolane ring, a thiophene ring, a thiazole ring, an isothiazole ring, a thiadiazole ring, a dithiolane ring, and a morpholine ring.

Specific examples of the polycyclic heterocycle include an indole ring, a benzimidazole ring, an indazole ring, a purine ring, a quinoline ring, an isoquinoline ring, a phthalazine ring, a naphthyridine ring, a quinoxaline ring, a quinazoline ring, a cinnoline ring, a pteridine ring, a carbazole ring, a 1,4,5,8,9,11-hexaazatriphenylene ring, a benzofuran ring, a dibenzofuran ring, a benzoxazole ring, a benzisoxazole ring, a benzothiophene ring, a dibenzothiophene ring, a benzothiadiazole ring, and a benzisothiadiazole ring.

The polycyclic heterocycle may be a ring formed by fusion of one or more monocyclic or polycyclic aliphatic hydrocarbon rings and one or more monocyclic or polycyclic heterocycles, or a ring formed by fusion of one or more monocyclic or polycyclic aromatic hydrocarbon rings and one or more monocyclic or polycyclic heterocycles.

### Halogen atom

In the first exemplary embodiment, examples of the halogen atom include a fluorine atom, a chlorine atom, a bromine atom, and an iodine atom.

### Alkyl group

In the first exemplary embodiment, the alkyl group is preferably, for example, a linear or branched alkyl group having 1 to 20 carbon atoms, 1 to 18 carbon atoms, 1 to 10 carbon atoms, 1 to 6 carbon atoms, or 1 to 5 carbon atoms. In the first exemplary embodiment, examples of the alkyl group include a methyl group, an ethyl group, an n-propyl group, an isopropyl group, an n-butyl group, an isobutyl group, a sec-butyl group, a tert-butyl group, various pentyl groups, various hexyl groups, various heptyl groups, various octyl groups, various nonyl groups, various decyl groups, various undecyl groups, various dodecyl groups, various tridecyl groups, various tetradecyl groups, various pentadecyl groups, various hexadecyl groups, various heptadecyl groups, various octadecyl groups, various nonadecyl groups, and various eicosyl groups. Preferable examples of the alkyl group include an alkyl group having 1 to 6 carbon atoms. More preferable examples of the alkyl group include a methyl group, an ethyl group, an n-propyl group, an isopropyl group, an n-butyl group, an isobutyl group, a sec-butyl group, a tert-butyl group, a pentyl group, an isopentyl group, a hexyl group, and an isohexyl group. Herein, when the name of a substituent is preceded by the term "various," the "various" indicates that both linear and all branched chain groups are included, and the same applies hereinafter.

### Haloalkyl group

In the first exemplary embodiment, examples of the substituted alkyl group include a haloalkyl group. Examples of the haloalkyl group include a linear or branched alkyl group having 1 to 20 carbon atoms substituted with at least one identical or different halogen atom (e.g., at least one halogen atom selected from the group consisting of a fluorine atom, a chlorine atom, a bromine atom, and an iodine atom). Preferable examples of the haloalkyl group include a haloalkyl group having 1 to 6 carbon atoms, and more preferably a fluoromethyl group, a difluoromethyl group, a trifluoromethyl group, a 2-fluoroethyl group, a 1,2-difluoroethyl group, a chloromethyl group, a 2-chloroethyl group, a 1,2-dichloroethyl group, a bromomethyl group, a 2-bromoethyl group, a 1-bromopropyl group, a 2-bromopropyl group, a 3-bromopropyl group, and an iodomethyl group.

### Alkenyl group

In the first exemplary embodiment, the alkenyl group is preferably, for example, a linear or branched alkenyl group having 2 to 10 carbon atoms or 2 to 6 carbon atoms. In the first exemplary embodiment, examples of the alkenyl group include an ethenyl group (vinyl group), a 1-propenyl group, a 2-propenyl group (allyl group), a 1-butenyl group, a 2-butenyl group, a 3-butenyl group, a 1-methyl-1-propenyl group, a 1-methyl-2-propenyl group, a 2-methyl-1-propenyl group, a 2-methyl-2-propenyl group, various butenyl groups, various pentenyl groups, and various hexenyl groups.

### Alkynyl group

In the first exemplary embodiment, the alkynyl group is preferably, for example, a linear or branched alkynyl group having 2 to 10 carbon atoms or 2 to 6 carbon atoms. In the first exemplary embodiment, examples of the alkynyl group include an ethynyl group, a 1-propynyl group, a 2-propynyl group, a 1-methyl-2-propynyl group, a 1,1-dimethyl-2-propynyl group, a 1-butynyl group, a 2-butynyl group, a 3-butynyl group, a 1-pentynyl group, a 2-pentynyl group, a 3-pentynyl group, a 4-pentynyl group, and a 3-hexynyl group.

### Cycloalkyl group

In the first exemplary embodiment, the cycloalkyl group is preferably, for example, a monocyclic or polycyclic cycloalkyl group having 3 to 26 ring carbon atoms, 3 to 20 ring carbon atoms, or 6 to 20 ring carbon atoms. In the first exemplary embodiment, examples of the cycloalkyl group include a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, an adamantyl group, and a norbornyl group.

### Cycloalkenyl group

In the first exemplary embodiment, the cycloalkenyl group is preferably, for example, a monocyclic or polycyclic unsaturated aliphatic cyclic group having 3 to 26 ring carbon atoms, 3 to 20 ring carbon atoms, or 6 to 20 ring carbon atoms. Examples of the cycloalkenyl group include a cyclopropenyl group, a cyclopentenyl group, a cyclohexenyl group, a cycloheptenyl group, a cyclooctenyl group, a cyclopentadienyl group, a cyclohexadienyl group, a cycloheptadienyl group, and a cyclooctadienyl group.

### Aryl group

In the first exemplary embodiment, the aryl group is preferably, for example, a monocyclic or polycyclic aryl group having 6 to 20 ring carbon atoms, 6 to 14 ring carbon atoms, or 6 to 10 ring carbon atoms. In the first exemplary embodiment, examples of the aryl group include a phenyl group, a biphenyl group, a naphthyl group, an anthryl group, and a phenanthryl group. In the first exemplary embodiment, examples of the substituted aryl group include a 2-methylphenyl group, a 3-methylphenyl group, a 4-methylphenyl group, a 4-ethylphenyl group, a dichlorophenyl group, and a methylnaphthyl group.

### Alkoxy group

In the first exemplary embodiment, the alkoxy group is preferably, for example, an alkoxy group having 1 to 20 carbon atoms, 1 to 18 carbon atoms, 1 to 10 carbon atoms, or 1 to 6 carbon atoms. In the first exemplary embodiment, examples of the alkoxy group include a methoxy group, an ethoxy group, a propoxy group, an isopropoxy group, a butoxy group, an isobutoxy group, a sec-butoxy group, a tert-butoxy group, various pentyloxy groups, and various hexyloxy groups.

### Cycloalkoxy group

In the first exemplary embodiment, the cycloalkoxy group is preferably a cycloalkoxy group having 3 to 20 ring carbon atoms or 6 to 20 ring carbon atoms. In the first exemplary embodiment, examples of the cycloalkoxy group include a case where the cycloalkyl group moiety is the cycloalkyl group described above, that is, a case where Arx in a group represented by -O-Arx is the cycloalkyl group described above.

### Aryloxy group

In the first exemplary embodiment, examples of the aryloxy group include a monocyclic or polycyclic aryloxy group having 6 to 12 ring carbon atoms, and preferably include a phenoxy group and a naphthyloxy group. In the first exemplary embodiment, examples of the substituted aryloxy group include a tolyloxy group.

### Aralkyl group

In the first exemplary embodiment, the aralkyl group is preferably, for example, an aralkyl group having 7 to 20 carbon atoms or 7 to 15 carbon atoms. In the first exemplary embodiment, examples of the aralkyl group include a benzyl group, a 1-phenylethyl group, a phenethyl group, a 1-naphthylmethyl group, a 2-naphthylmethyl group, a 1-naphthylethyl group, and a 2-naphthylethyl group.

### Aralkyloxy group

In the first exemplary embodiment, the aralkyloxy group is preferably, for example, an aralkyloxy group having 7 to 20 carbon atoms or 7 to 15 carbon atoms. In the first exemplary embodiment, examples of the aralkyloxy group include a case where the aralkyl group moiety is the aralkyl group described above, that is, a case where Ary in a group represented by -O-Ary is the aralkyl group described above. In the first exemplary embodiment, preferable examples of the aralkyloxy group include a benzyloxy group, a phenethyloxy group, a 1-naphthylmethoxy group, and a 2-naphthylmethoxy group, more preferable examples thereof include a benzyloxy group.

### Silyloxy group

In the first exemplary embodiment, examples of the silyloxy group include a tert-butyldiphenylsilyloxy group and a tert-butyldimethylsilyloxy group.

### Alkenyloxy group

In the first exemplary embodiment, examples of the alkenyloxy group include a linear or branched alkenyloxy group having 2 to 20 carbon atoms, preferably an alkenyloxy group having 2 to 6 carbon atoms, and more preferably, for example, an ethenyloxy group, a 1-propenyloxy group, a 2-propenyloxy group, a 1-methylethenyloxy group, a 1-butenyloxy group, a 2-butenyloxy group, a 3-butenyloxy group, a 1-methyl-1-propenyloxy group, a 1-methyl-2-propenyloxy group, a 2-methyl-1-propenyloxy group, and a 2-methyl-2-propenyloxy group.

### Alkenylcarbonyl group

In the first exemplary embodiment, examples of the alkenyl moiety in the alkenylcarbonyl group include the alkenyl groups described above.

### Alkenylcarbonyloxy group

In the first exemplary embodiment, examples of the alkenylcarbonyloxy group include a linear or branched alkenylcarbonyloxy group having 2 to 20 carbon atoms, preferably, for example, an alkenylcarbonyloxy group having 2 to 6 carbon atoms, and more preferably, for example, an ethenylcarbonyloxy group, a 1-propenylcarbonyloxy group, a 2-propenylcarbonyloxy group, a 1-methylethenylcarbonyloxy group, a 1-butenylcarbonyloxy group, a 2-butenylcarbonyloxy group, a 3-butenylcarbonyloxy group, a 1-methyl-1-propenylcarbonyloxy group, a 1-methyl-2-propenylcarbonyloxy group, a 2-methyl-1-propenylcarbonyloxy group, and a 2-methyl-2-propenylcarbonyloxy group.

### Alkynyloxy group

In the first exemplary embodiment, examples of the alkynyloxy group include a linear or branched alkynyloxy group having 2 to 20 carbon atoms, preferably an alkynyloxy group having 3 to 6 carbon atoms, and more preferably, for example, a 2-propynyloxy group, a 1-methyl-2-propynyloxy group, a 1,1-dimethyl-2-propynyloxy group, a 2-butynyloxy group, a 3-butynyloxy group, a 1-pentynyloxy group, a 2-pentynyloxy group, a 3-pentynyloxy group, and a 4-pentynyloxy group.

### Silyl group

In the first exemplary embodiment, examples of the silyl group include an alkylsilyl group and an arylsilyl group. Examples of the alkylsilyl group include a trimethylsilyl group and a tert-butyldimethylsilyl group. Examples of the arylsilyl group include a triphenylsilyl group.

### Heteroaryl group

In the first exemplary embodiment, the heteroaryl group is preferably a heteroaryl group having 5 to 20 ring atoms, 5 to 14 ring atoms, or 5 to 10 ring atoms. In the first exemplary embodiment, the heteroaryl group contains at least one heteroatom as a ring atom. For example, the heteroaryl group may contain one, two, or three heteroatoms as ring atoms. The heteroatom as a ring atom contained in the heteroaryl group is at least one atom selected from the group consisting of a nitrogen atom, an oxygen atom, a sulfur atom, a silicon atom, a phosphorus atom, and a boron atom. When the heteroaryl group contains a plurality of heteroatoms as ring atoms, the plurality of heteroatoms are mutually the same or different. In the first exemplary embodiment, examples of the heteroaryl group include a pyrrolyl group, an imidazolyl group, a furyl group, a thienyl group, a pyridinyl group, a pyrazinyl group, a pyrimidinyl group, a pyridazinyl group, a triazinyl group, an indolyl group, a benzofuranyl group, a benzothienyl group, a quinolyl group, an isoquinolyl group, a phenanthrolinyl group, and an acridinyl group.

Herein, a numerical range expressed as "AA to BB" means a range from the lower limit value AA described in the former part of "AA to BB" to the upper limit value BB described in the latter part of "AA to BB."

Herein, substituents as used in "substituted or unsubstituted" are bonded to each other to form a monocyclic ring, bonded to each other to form a fused ring, or not bonded to each other, Here, a case where substituents as used in "substituted or unsubstituted" are bonded to each other to form a monocyclic ring, or bonded to each other to form a fused ring also includes a case where substituents are bond to each other to form a polycyclic group. The polycyclic group is preferably a bicyclic group, a tricyclic group, or a tetracyclic group.

With respect to groups derived from cycloalkanes (such as a cycloalkyl group, cycloalkylene group, and cycloalkylidene group), a case where substituents as used in "substituted or unsubstituted" are bonded to each other to form a polycyclic group is exemplified by monovalent or divalent groups derived from bicycloalkanes, tricycloalkanes, and tetracycloalkanes.

Herein, the substituents in "substituted" as used in "substituted or unsubstituted" are each independently preferably at least one substituent selected from the group consisting of an alkyl group, a haloalkyl group, an alkenyl group, an alkynyl group, a cycloalkyl group, an alkoxy group, an aryl group, an aryloxy group, a hydroxy group, an oxiranyl group, a mercapto group, an amino group, a carbamoyl group, a sulfamoyl group, a carboxy group, an alkoxycarbonyl group, a sulfo group, a sulfino group, a phosphono group, a nitro group, a cyano group, an amidino group, an imino group, a dihydroborono group, a sulfinyl group, a sulfonyl group, an acyl group, an oxo group, a thioxo group, and a halogen atom (e.g., a fluorine atom, a chlorine atom, a bromine atom, and an iodine atom).

Specific examples of these substituents are the same as the specific examples described above. The number of the substituents in "substituted" as used in "substituted or unsubstituted" may be one or two or more, and when two or more substituents are present, the substituents are mutually the same or different. Further, herein, "unsubstituted" as used in "substituted or unsubstituted" means that the group is not substituted with the substituents described above and that a hydrogen atom is bonded thereto.

Herein, in specific examples of compounds, D may represent a deuterium atom, Me may represent a methyl group, Et may represent a ethyl group, Ph may represent a phenyl group, and tBu may represent a tert-butyl group.

Herein, the preferred requirements can be optionally adopted, and combinations of the preferred ones can be said to be more preferable.

### Second Exemplary Embodiment

A precursor according to a second exemplary embodiment is represented by a formula (DP-XM1), (DP-XM2), (DP-XM3), (DP-XM4), (DP-YM1), (DP-YM2), (DP-YM3), or (DP-YM4) below.

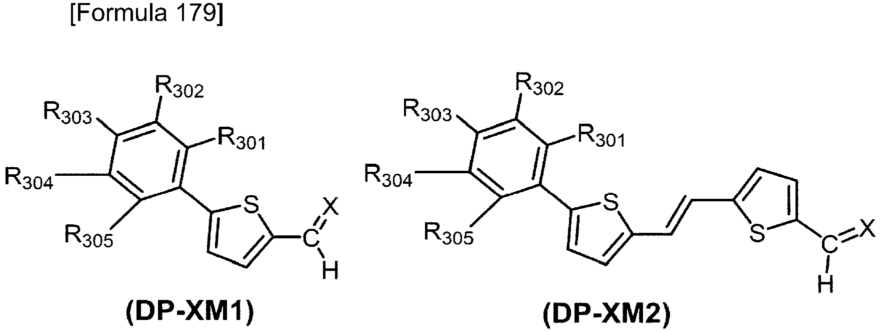

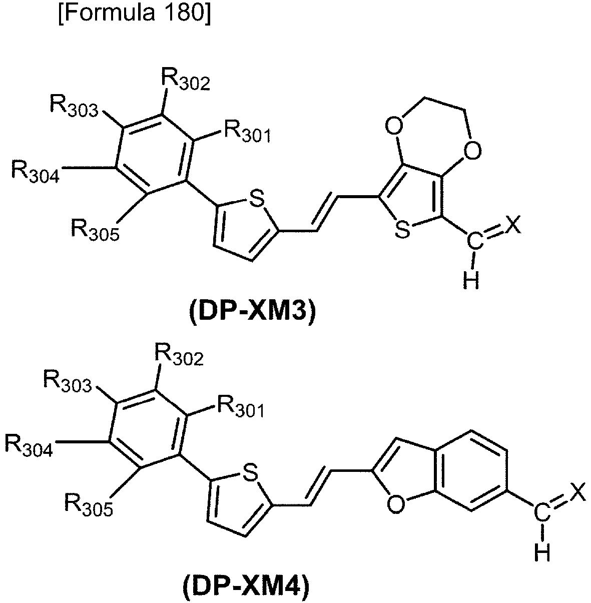

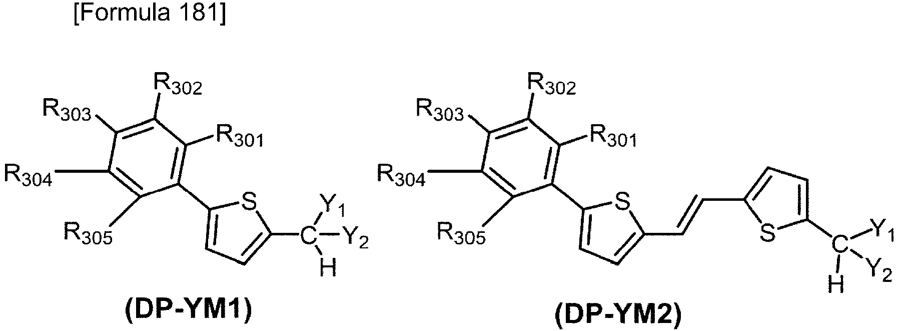

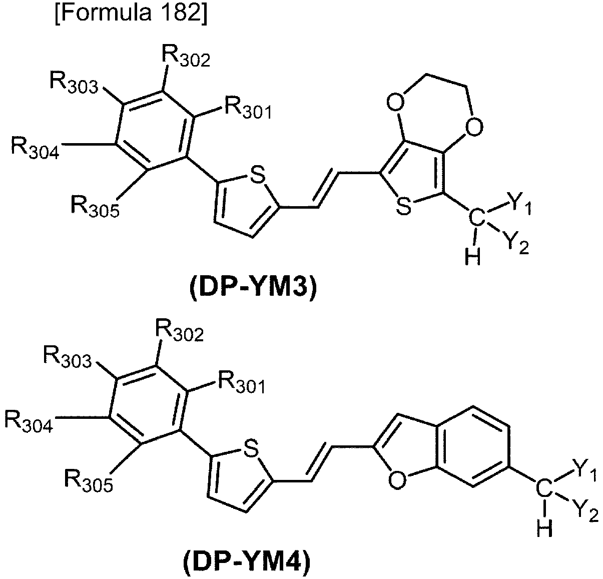

In the formulae (DP-XM1), (DP-XM2), (DP-XM3), (DP-XM4), (DP-YM1), (DP-YM2), (DP-YM3), and (DP-YM4):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
in the formulae (DP-XM1), (DP-XM2), (DP-XM3), and (DP-XM4): X is an oxygen atom or a sulfur atom,
in the formulae (DP-YM1), (DP-YM2), (DP-YM3), and (DP-YM4):
   a combination of Y₁ and Y₂ are mutually bonded to form a substituted or unsubstituted monocyclic ring, mutually bonded to form a substituted or unsubstituted fused ring, or not mutually bonded;
   Y₁ and Y₂ forming neither the substituted or unsubstituted monocyclic ring nor the substituted or unsubstituted fused ring are each independently a substituted or unsubstituted alkoxy group or a group represented by -S(R₁₁₄);
   R₁₁₄ is a substituted or unsubstituted alkyl group;
   when a plurality of R₁₀₁ are present, the plurality of R₁₀₁ are mutually the same or different;
   when a plurality of R₁₀₂ are present, the plurality of R₁₀₂ are mutually the same or different;
   when a plurality of R₁₁₃ are present, the plurality of R₁₁₃ are mutually the same or different; and
   when a plurality of R₁₁₄ are present, the plurality of R₁₁₄ are mutually the same or different.

R₁₀₁ to R₁₀₃, R₁₁₃, and R₁₁₄ in the precursor according to the second exemplary embodiment represent the same as R₁₀₁ to R₁₀₃, R₁₁₃, and R₁₁₄ in the formulae (DP-XM1), (DP-XM2), (DP-XM3), (DP-XM4), (DP-YM1), (DP-YM2), (DP-YM3), and (DP-YM4).

In one form of the precursor according to the second exemplary embodiment, it is also preferable that X in the formulae (DP-XM1), (DP-XM2), (DP-XM3), and (DP-XM4) is an oxygen atom.

In one form of the precursor according to the second exemplary embodiment, when one of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by - N(R₁₀₁)(R₁₀₂), it is also preferable that each of the remaining R₃₀₁ to R₃₀₅ is not a group represented by -O(R₁₁₃).

In one form of the precursor according to the second exemplary embodiment, it is also preferable that R₃₀₁ to R₃₀₅ in the formulae (DP-XM1), (DP-XM2), (DP-XM3), (DP-XM4), (DP-YM1), (DP-YM2), (DP-YM3), and (DP-YM4) are each independently a hydrogen atom, a group represented by -N(R₁₀₁)(R₁₀₂), or a group represented by -O(R₁₁₃).

In one form of the precursor according to the second exemplary embodiment, it is also preferable that R₃₀₁ to R₃₀₅ in the formulae (DP-XM1), (DP-XM2), (DP-XM3), (DP-XM4), (DP-YM1), (DP-YM2), (DP-YM3), and (DP-YM4) are each independently a hydrogen atom or a group represented by -O(R₁₁₃).

In one form of the precursor according to the second exemplary embodiment, it is also preferable that R₃₀₁ to R₃₀₅ in the formulae (DP-XM1), (DP-XM2), (DP-XM3), (DP-XM4), (DP-YM1), (DP-YM2), (DP-YM3), and (DP-YM4) are each independently a hydrogen atom or a group represented by -N(R₁₀₁)(R₁₀₂).

In one form of the precursor according to the second exemplary embodiment, it is also preferable that at least one of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -O(R₁₁₃).

In one form of the precursor according to the second exemplary embodiment, it is also preferable that three of R₃₀₁ to R₃₀₅ in the formulae (DP-XM1), (DP-XM2), (DP-XM3), (DP-XM4), (DP-YM1), (DP-YM2), (DP-YM3), and (DP-YM4) are groups represented by -O(R₁₁₃).

In one form of the precursor according to the second exemplary embodiment, it is also preferable that three of R₃₀₁ to R₃₀₅ in the formulae (DP-XM1), (DP-XM2), (DP-XM3), (DP-XM4), (DP-YM1), (DP-YM2), (DP-YM3), and (DP-YM4) are groups represented by -O(R₁₁₃), and the remaining two of R₃₀₁ to R₃₀₅ are hydrogen atoms.

In one form of the precursor according to the second exemplary embodiment, it is also preferable that, in the formulae (DP-XM1), (DP-XM2), (DP-XM3), (DP-XM4), (DP-YM1), (DP-YM2), (DP-YM3), and (DP-YM4), R₃₀₃ is a group represented by - O(R₁₁₃), one of R₃₀₁ and R₃₀₂ is a group represented by -O(R₁₁₃) and the other of R₃₀₁ and R₃₀₂ is a hydrogen atom, and one of R₃₀₄ and R₃₀₅ is a group represented by - O(R₁₁₃) and the other of R₃₀₄ and R₃₀₅ is a hydrogen atom.

In one form of the precursor according to the second exemplary embodiment, it is also preferable that R₃₀₁, R₃₀₃, and R₃₀₅ in the formulae (DP-XM1), (DP-XM2), (DP-XM3), (DP-XM4), (DP-YM1), (DP-YM2), (DP-YM3), and (DP-YM4) are groups represented by -O(R₁₁₃), and R₃₀₂ and R₃₀₄ are hydrogen atoms.

In one form of the precursor according to the second exemplary embodiment, when at least one selected from the group consisting of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -O(R₁₁₃), it is also preferable that R₁₁₃ in R₃₀₁ to R₃₀₅ is an unsubstituted alkyl group.

In one form of the precursor according to the second exemplary embodiment, when at least one selected from the group consisting of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -O(R₁₁₃), R₁₁₃ in R₃₀₁ to R₃₀₅ is also preferably an unsubstituted alkyl group having 1 to 20 carbon atoms, also preferably an unsubstituted alkyl group having 1 to 10 carbon atoms, also preferably an unsubstituted alkyl group having 1 to 8 carbon atoms, also preferably an unsubstituted alkyl group having 1 to 5 carbon atoms, also preferably an unsubstituted alkyl group having 1 to 3 carbon atoms, and also preferably a methyl group.

In one form of the precursor according to the second exemplary embodiment, it is also preferable that one of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -N(R₁₀₁)(R₁₀₂), and the remaining four of R₃₀₁ to R₃₀₅ are hydrogen atoms.

In one form of the precursor according to the second exemplary embodiment, when at least one selected from the group consisting of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -N(R₁₀₁)(R₁₀₂), it is also preferable that R₁₀₁ and R₁₀₂ in R₃₀₁ to R₃₀₅ are each independently a substituted or unsubstituted aryl group.

In one form of the precursor according to the second exemplary embodiment, when at least one selected from the group consisting of R₃₀₁ to R₃₀₅ in the formulae (DP-XM1), (DP-XM2), (DP-XM3), (DP-XM4), (DP-YM1), (DP-YM2), (DP-YM3), and (DP-YM4) is a group represented by -N(R₁₀₁)(R₁₀₂), it is also preferable that R₁₀₁ and R₁₀₂ in R₃₀₁ to R₃₀₅ are each an unsubstituted phenyl group.

In one form of the precursor according to the second exemplary embodiment, the precursor represented by the formulae (DP-XM1), (DP-XM2), (DP-XM3), (DP-XM4), (DP-YM1), (DP-YM2), (DP-YM3), and (DP-YM4) is used in production of an electro-optic compound.

One form of the precursor according to the second exemplary embodiment is a precursor represented by the formulae (DP-XM1), (DP-XM2), (DP-XM3), (DP-XM4), (DP-YM1), (DP-YM2), (DP-YM3), and (DP-YM4), used in production of an electro-optic compound.

In one form of the precursor according to the second exemplary embodiment, it is also preferable that Y₁ and Y₂ in the formulae (DP-YM1), (DP-YM2), (DP-YM3), and (DP-YM4) are not bonded to each other.

In one form of the precursor according to the second exemplary embodiment, when Y₁ and Y₂ in the formulae (DP-YM1), (DP-YM2), (DP-YM3), and (DP-YM4) are each independently a substituted or unsubstituted alkoxy group or a group represented by -S(R₁₁₄), it is also preferable that a combination of Y₁ and Y₂ are bonded to each other to form a substituted or unsubstituted monocyclic ring, or are bonded to each other to form a substituted or unsubstituted fused ring.

The precursor according to the second exemplary embodiment is also preferably represented by a formula (DP-XM11), (DP-XM21), (DP-XM31), or (DP-XM41) below.

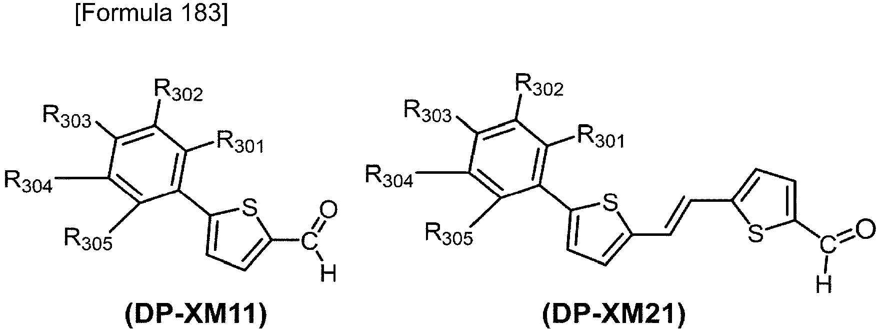

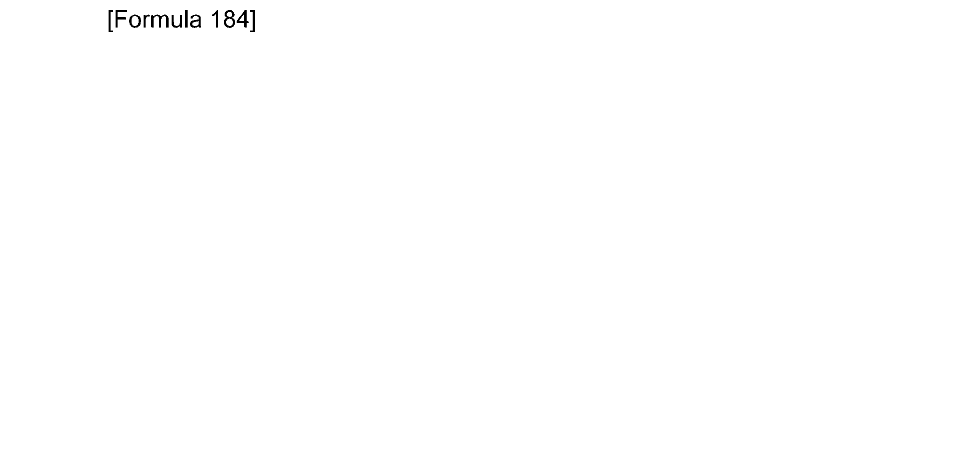

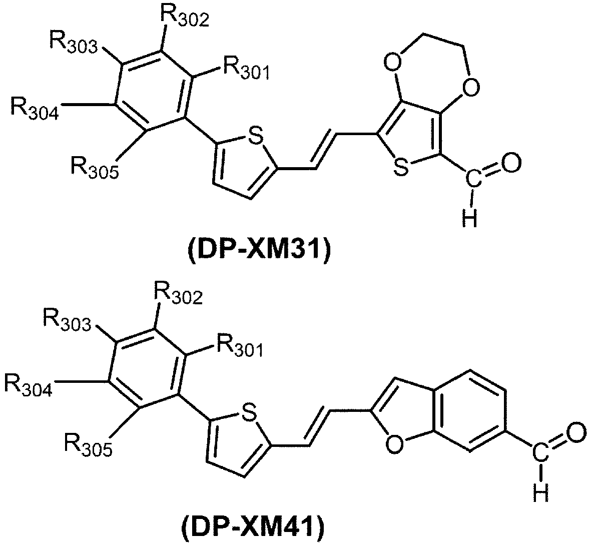

In the formulae (DP-XM11), (DP-XM21), (DP-XM31), and (DP-XM41):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different; and
when a plurality of R₁₁₃ are present, the plurality of R₁₁₃ are mutually the same or different.

In one form of the precursor according to the second exemplary embodiment, when one of R₃₀₁ to R₃₀₅ in the formulae (DP-XM11), (DP-XM21), (DP-XM31), and (DP-XM41) is a group represented by -N(R₁₀₁)(R₁₀₂), it is also preferable that each of the remaining R₃₀₁ to R₃₀₅ is not a group represented by -O(R₁₁₃).

In one form of the precursor according to the second exemplary embodiment, it is also preferable that R₃₀₁ to R₃₀₅ in the formulae (DP-XM11), (DP-XM21), (DP-XM31), and (DP-XM41) are each independently a hydrogen atom, a group represented by -N(R₁₀₁)(R₁₀₂), or a group represented by -O(R₁₁₃).

In one form of the precursor according to the second exemplary embodiment, it is also preferable that R₃₀₁ to R₃₀₅ in the formulae (DP-XM11), (DP-XM21), (DP-XM31), and (DP-XM41) are each independently a hydrogen atom or a group represented by -O(R₁₁₃).

In one form of the precursor according to the second exemplary embodiment, it is also preferable that R₃₀₁ to R₃₀₅ in the formulae (DP-XM11), (DP-XM21), (DP-XM31), and (DP-XM41) are each independently a hydrogen atom or a group represented by -N(R₁₀₁)(R₁₀₂).

In one form of the precursor according to the second exemplary embodiment, it is also preferable that at least one of R₃₀₁ to R₃₀₅ in the formulae (DP-XM11), (DP-XM21), (DP-XM31), and (DP-XM41) is a group represented by -O(R₁₁₃).

In one form of the precursor according to the second exemplary embodiment, it is also preferable that three of R₃₀₁ to R₃₀₅ in the formulae (DP-XM11), (DP-XM21), (DP-XM31), and (DP-XM41) are groups represented by -O(R₁₁₃).

In one form of the precursor according to the second exemplary embodiment, it is also preferable that three of R₃₀₁ to R₃₀₅ in the formulae (DP-XM11), (DP-XM21), (DP-XM31), and (DP-XM41) are groups represented by -O(R₁₁₃), and the remaining two of R₃₀₁ to R₃₀₅ are hydrogen atoms.

In one form of the precursor according to the second exemplary embodiment, it is also preferable that, in the formulae (DP-XM11), (DP-XM21), (DP-XM31), and (DP-XM41), R₃₀₃ is a group represented by -O(R₁₁₃), one of R₃₀₁ and R₃₀₂ is a group represented by -O(R₁₁₃) and the other of R₃₀₁ and R₃₀₂ is a hydrogen atom, and one of R₃₀₄ and R₃₀₅ is a group represented by -O(R₁₁₃) and the other of R₃₀₄ and R₃₀₅ is a hydrogen atom.

In one form of the precursor according to the second exemplary embodiment, it is also preferable that R₃₀₁, R₃₀₃, and R₃₀₅ in the formulae (DP-XM11), (DP-XM21), (DP-XM31), and (DP-XM41) are groups represented by -O(R₁₁₃), and R₃₀₂ and R₃₀₄ are hydrogen atoms.

In one form of the precursor according to the second exemplary embodiment, when at least one selected from the group consisting of R₃₀₁ to R₃₀₅ in the formulae (DP-XM11), (DP-XM21), (DP-XM31), and (DP-XM41) is a group represented by - O(R₁₁₃), it is also preferable that R₁₁₃ in R₃₀₁ to R₃₀₅ is an unsubstituted alkyl group.

In one form of the precursor according to the second exemplary embodiment, when at least one selected from the group consisting of R₃₀₁ to R₃₀₅ in the formulae (DP-XM11), (DP-XM21), (DP-XM31), and (DP-XM41) is a group represented by - O(R₁₁₃), R₁₁₃ in R₃₀₁ to R₃₀₅ is also preferably an unsubstituted alkyl group having 1 to 20 carbon atoms, also preferably an unsubstituted alkyl group having 1 to 10 carbon atoms, also preferably an unsubstituted alkyl group having 1 to 8 carbon atoms, also preferably an unsubstituted alkyl group having 1 to 5 carbon atoms, also preferably an unsubstituted alkyl group having 1 to 3 carbon atoms, and also preferably a methyl group.

In one form of the precursor according to the second exemplary embodiment, it is also preferable that one of R₃₀₁ to R₃₀₅ in the formulae (DP-XM11), (DP-XM21), (DP-XM31), and (DP-XM41) is a group represented by -N(R₁₀₁)(R₁₀₂), and the remaining four of R₃₀₁ to R₃₀₅ are hydrogen atoms.

In one form of the precursor according to the second exemplary embodiment, when at least one selected from the group consisting of R₃₀₁ to R₃₀₅ in the formulae (DP-XM11), (DP-XM21), (DP-XM31), and (DP-XM41) is a group represented by - N(R₁₀₁)(R₁₀₂), it is also preferable that R₁₀₁ and R₁₀₂ in R₃₀₁ to R₃₀₅ are each independently a substituted or unsubstituted aryl group.

In one form of the precursor according to the second exemplary embodiment, when at least one selected from the group consisting of R₃₀₁ to R₃₀₅ in the formulae (DP-XM11), (DP-XM21), (DP-XM31), and (DP-XM41) is a group represented by - N(R₁₀₁)(R₁₀₂), it is also preferable that R₁₀₁ and R₁₀₂ in R₃₀₁ to R₃₀₅ is an unsubstituted phenyl group.

In the precursor according to the second exemplary embodiment, the precursor represented by the formula (DP-XM11), (DP-XM21), (DP-XM31), or (DP-XM41) is also preferably represented by a formula (DP-M1), (DP-M2), (DP-M3), (DP-M4), (DP-M5), (DP-M6), (DP-M7), or (DP-M8) below.

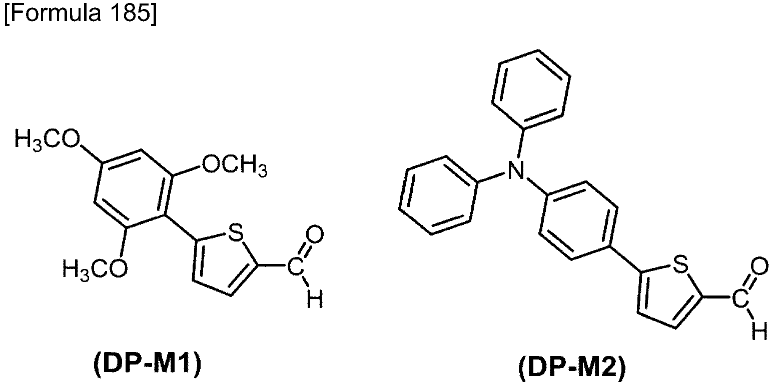

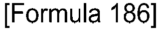

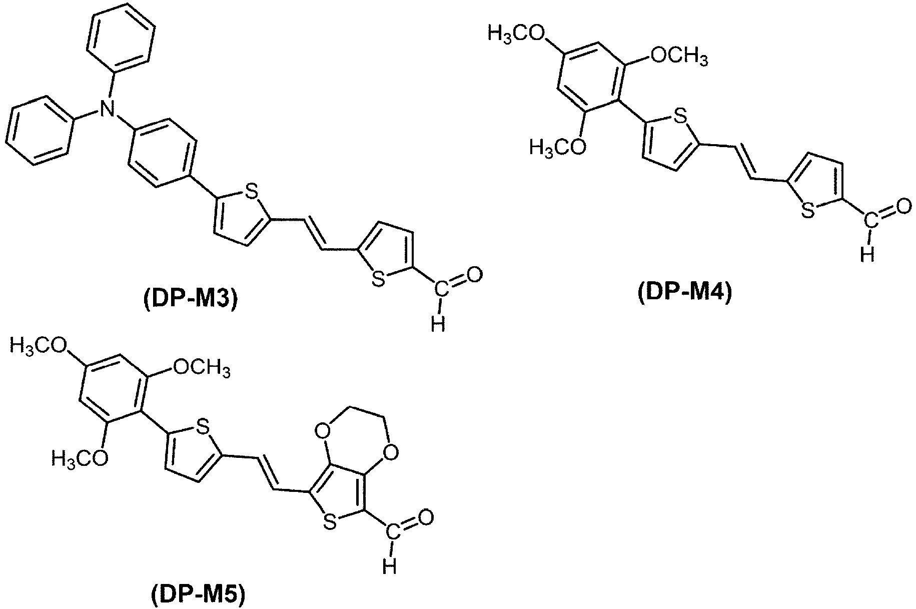

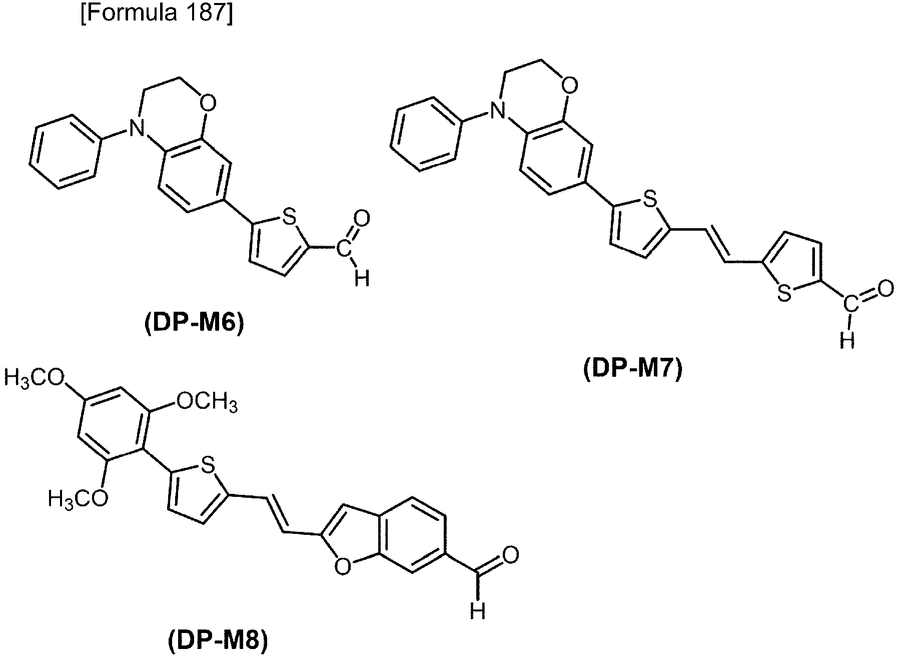

In one form of the precursor according to the second exemplary embodiment, the precursor represented by the formula (DP-XM11), (DP-XM21), (DP-XM31), or (DP-XM41) is used in production of an electro-optic compound.

One form of the precursor according to the second exemplary embodiment is the precursor represented by the formula (DP-XM11), (DP-XM21), (DP-XM31), or (DP-XM41), used in production of the compound according to the first exemplary embodiment.

In one form of the precursor according to the second exemplary embodiment, the precursor represented by the formula (DP-XM11), (DP-XM21), (DP-XM31), or (DP-XM41) is used in production of the compound according to the first exemplary embodiment.

One form of the precursor according to the second exemplary embodiment is the precursor represented by the formula (DP-XM11), (DP-XM21), (DP-XM31), or (DP-XM41), used in production of an electro-optic compound.

In one form of the precursor according to the second exemplary embodiment, the precursor represented by the formula (DP-XM11), (DP-XM21), (DP-XM31), or (DP-XM41) is used in production of an electro-optic compound.

In one form of the precursor according to the second exemplary embodiment, the precursor represented by the formula (DP-XM11), (DP-XM21), (DP-XM31), or (DP-XM41) is used in a production method of the compound according to the first exemplary embodiment.

Specific examples of the production method of the compound according to the first exemplary embodiment include the production method described in the first exemplary embodiment described above. For example, the precursor represented by the formula (DP-M1), (DP-M2), (DP-M3), (DP-M4), (DP-M5), (DP-M6), (DP-M7), or (DP-M8) can be used in place of the compound D-M-D1, the compound P-M-D1, the compound (A-M-D1), or the like described above, but is not limited thereto.

In one form of the precursor according to the second exemplary embodiment, it is also preferable that the precursor is used for production of the compound according to the first exemplary embodiment having the donor moiety represented by the formula (D123-1B), the π-conjugated moiety, and the acceptor moiety.

In one form of the precursor according to the second exemplary embodiment, it is also preferable that the precursor is used for production of the compound according to the first exemplary embodiment having the donor moiety represented by the formula (D111), the π-conjugated moiety, and the acceptor moiety. In one form of the precursor according to the second exemplary embodiment, it is also preferable that the precursor is used for production of the compound according to the first exemplary embodiment having the donor moiety represented by the formula (D111), the π-conjugated moiety in which p and q in the π-conjugated moiety represented by the formula (PX1) are 0, and the acceptor moiety.

### Third Exemplary Embodiment

### Electro-optic Material

An electro-optic material according to a third exemplary embodiment contains an electro-optic compound and a host material, and the electro-optic compound is the compound according to the first exemplary embodiment. In the electro-optic material according to the third exemplary embodiment, the host material may also be referred to as a matrix material.

In one form of the electro-optic material according to the third exemplary embodiment, it is also preferable that the electro-optic material contains an electro-optic compound and a host material, the electro-optic compound is the compound according to the first exemplary embodiment, and the electro-optic compound is dispersed in the host material.

In one form of the electro-optic material according to the third exemplary embodiment, it is also preferable that the electro-optic material contains an electro-optic compound and a host material, the electro-optic compound is the compound according to the first exemplary embodiment, the host material is a polymer, and the electro-optic compound is dispersed in the host material without being bonded to the polymer.

In one form of the electro-optic material according to the third exemplary embodiment, it is also preferable that the electro-optic material contains an electro-optic compound and a host material, the electro-optic compound is the compound according to the first exemplary embodiment, the host material is a polymer, and the electro-optic compound is bonded to the polymer as the host material.

The mixing ratio of the electro-optic compound and the host material is not particularly limited. When the total of the mass parts M_{EO} of the electro-optic compound and the mass parts M_{HOST} of the host material contained in the electro-optic material according to the third exemplary embodiment is 100 mass parts, for example, the mass ratio M_{EO}/M_{HOST} of the electro-optic compound to the host material may be 5/95 or more and 95/5 or less. The mass ratio M_{EO}/M_{HOST} of the electro-optic compound to the host material may be 10/90 or more, 20/80 or more, 30/70 or more, 40/60 or more, or 50/50 or more. The mass ratio M_{EO}/M_{HOST} of the electro-optic compound to the host material may be 90/10 or less, 85/15 or less, 80/20 or less, or 70/30 or less.

The host material is not particularly limited. From the viewpoint of dispersing the electro-optic compound in the host material in a nearly uniform state, it is preferable that the host material has high compatibility with the electro-optic compound.

In the electro-optic material according to the third exemplary embodiment, the host material is preferably a polymer. The host material is preferably a polymer soluble in an organic solvent. From the viewpoint that excellent transparency and excellent moldability (including film-forming properties) are easily obtained when used in an electro-optic film or an electro-optic device, the host material is more preferably, for example, specifically, at least one polymer selected from the group consisting of a poly(meth)acrylate, a polyimide, a polycarbonate, a norbornene polymer, a polystyrene, a polysulfone, a polyethersulfone, a silicone resin, and an epoxy resin.

As one form of the method for dispersing the electro-optic compound in the host material, there is a method in which the electro-optic compound and the host material are introduced into an organic solvent, and the electro-optic compound and the host material are dispersed or dissolved in the organic solvent using a known stirrer, disperser, or the like. As one form of the method for dispersing the electro-optic compound in the host material, there is a method in which a mixed raw material obtained by mixing the electro-optic compound and the host material is dispersed using a known kneader.

The organic solvent is not particularly limited as long as the electro-optic compound and the host material can be dispersed or dissolved in the organic solvent. Examples of the organic solvent include ethers (ether-based solvents), ketones (ketone-based solvents), esters (ester-based solvents), alcohols (alcohol-based solvents), halides (chlorine-containing solvents), hydrocarbon-based solvents (aromatic hydrocarbons and aliphatic hydrocarbons), and polar aprotic solvents (amides and sulfoxides), and one or two or more of these may be used. Specifically, the organic solvent is exemplified by at least one selected from the group consisting of ether-based solvents such as tetrahydrofuran, methyltetrahydrofuran, 1,4-dioxane, and cyclopentyl methyl ether; ketone-based solvents such as acetone, methyl ethyl ketone, 2-heptanone, cyclopentanone, and cyclohexanone; ester-based solvents such as ethyl acetate, butyl acetate, and propylene glycol monomethyl ether acetate; alcohol-based solvents such as methanol, ethanol, isopropyl alcohol, 1-butanol, 2-butanol, 2-pentanol, benzyl alcohol, and alkoxy alcohols (e.g., 1-methoxy-2-propanol and 3-methoxy-1-butanol); chlorine-containing solvents such as dichlorobenzene, chlorobenzene, chloroform, and 1,2-dichloroethane; hydrocarbon-based solvents such as toluene, xylene, tetralin, ethylbenzene, 1,3,5-trimethylbenzene, hexane, and ethylcyclohexane; and N,N-dimethylformamide, N,N-dimethylacetamide, N-methyl-2-pyrrolidone, and dimethyl sulfoxide. These organic solvents may be used alone or in combination of two or more. One form of the electro-optic material according to the third exemplary embodiment may be a composition for forming an electro-optic film described in a fourth exemplary embodiment. The organic solvent is not limited to the specific examples described above, but from the viewpoint of process applicability such as coating and drying of the composition for forming an electro-optic film, the boiling point of the organic solvent is preferably 50 degrees C or higher, more preferably 80 degrees C or higher, and still more preferably 100 degrees C or higher. From the same viewpoint, the boiling point is preferably 200 degrees C or lower, more preferably 180 degrees C or lower, and still more preferably 150 degrees C or lower.

The method for bonding the electro-optic compound to the polymer as the host material is not particularly limited. The method for bonding the electro-optic compound to the polymer as the host material may include reacting the electro-optic compound and the host material under conditions in which the electro-optic compound and the host material react with each other. The method for bonding the electro-optic compound to the polymer serving as the host material may include, for example, mixing the electro-optic compound and the host material, and then reacting them under heating conditions, reacting them in the presence of a catalyst, or reacting them under a combination thereof (in the presence of a catalyst and under heating conditions). Alternatively, after the electro-optic compound and the host material are dispersed or dissolved in an organic solvent, the electro-optic compound and the host material may be reacted under conditions in which they can react with each other.

In the electro-optic material according to the third exemplary embodiment, the host material is preferably a polymer having a reactive group capable of forming a covalent bond with the compound according to the first exemplary embodiment (electro-optic compound) (the reactive group capable of forming a covalent bond with the electro-optic compound may be abbreviated as RG_{P}). In the electro-optic material according to the third exemplary embodiment, it is preferable that at least a part of the electro-optic compound is bonded to the polymer having the reactive group RG_{P} as the host material. Such an electro-optic material allows the compound according to the first exemplary embodiment to be dispersed in the host material at high density, and excellent EO properties can be obtained. Examples of the host material having a reactive group include the above-described polymers having a reactive group.

The reactive group RG_{P} of the polymer as the host material is not particularly limited as long as it can form a covalent bond with the electro-optic compound. Examples of the reactive group RG_{P} of the host material include a haloalkyl group, a halogenated acyl group, an alkoxycarbonyl group, an aryloxycarbonyl group, a hydroxy group, a thiol group, an amino group, an isocyanate group, an epoxy group, a carboxy group, and an acid anhydride group. The reactive group RG_{P} can react with a reactive group or the like in the electro-optic compound to form a covalent bond. The reactive group of the electro-optic compound is not particularly limited as long as it is a reactive group capable of reacting with the reactive group of the polymer as the host material (the reactive group capable of forming a covalent bond with the polymer as the host material may be abbreviated as RG_{EO}). The reactive group RG_{EO} of the electro-optic compound is, for example, at least one selected from the group consisting of a hydroxy group, an amino group, and an alkoxycarbonyl group.

In the electro-optic material according to the third exemplary embodiment, when the electro-optic compound is bonded to the polymer as the host material, the electro-optic compound may be covalently bonded to a side chain of the polymer as the host material, the electro-optic compound may be covalently bonded to a main chain of the polymer as the host material, or the polymer as the host material and the electro-optic compound may be crosslinked. Examples of the bonding manner when the electro-optic compound is bonded to the polymer as the host material include: a bonding manner in which the donor moiety in the compound according to the first exemplary embodiment as the electro-optic compound may be bonded to a side chain of the polymer; a bonding manner in which the acceptor moiety in the compound according to the first exemplary embodiment may be bonded to a side chain of the polymer; a bonding manner in which the donor moiety in the compound according to the first exemplary embodiment may be bonded to a main chain of the polymer; and a bonding manner in which the acceptor moiety in the compound according to the first exemplary embodiment may be bonded to a main chain of the polymer.

Since the electro-optic material according to the third exemplary embodiment contains a host material and the compound according to the first exemplary embodiment as the electro-optic compound, the electro-optic effect of an electro-optic film or an electro-optic device can be expected to be improved by using the electro-optic material according to the third exemplary embodiment.

### Fourth Exemplary Embodiment

### Electro-optic Film

One form of the electro-optic film according to the fourth exemplary embodiment contains the compound according to the first exemplary embodiment. One form of the electro-optic film according to the fourth exemplary embodiment contains the electro-optic material according to the third exemplary embodiment.

The electro-optic film according to the fourth exemplary embodiment can be obtained, for example, from a composition for forming an electro-optic film containing the compound according to the first exemplary embodiment or from a composition for forming an electro-optic film containing the electro-optic material according to the third exemplary embodiment. Specifically, for example, the electro-optic film according to the fourth exemplary embodiment can be formed by preparing a composition for forming an electro-optic film in which the compound according to the first exemplary embodiment or the electro-optic material according to the third exemplary embodiment is dispersed or dissolved in an organic solvent, applying the composition for forming an electro-optic film onto a substrate, and evaporating the organic solvent. The organic solvent may be evaporated by a known drying means, or may be evaporated by heating as necessary. Examples of the organic solvent include the organic solvent described in the third exemplary embodiment. The composition for forming an electro-optic film may contain one or more kinds of various additives as necessary.

The method for applying the composition for forming an electro-optic film onto a substrate is not particularly limited, and examples of the method include known coating methods such as spin coating, dip coating, roll coating, spray coating, doctor blade coating, wire bar coating, and slit coating.

The electro-optic film according to the fourth exemplary embodiment may be subjected to a poling treatment. The poling treatment is a treatment in which a predetermined electric field is applied to the electro-optic film, and the electro-optic compound (the compound according to the first exemplary embodiment) is oriented in the direction of the applied electric field by a Coulomb force between a dipole moment of the electro-optic compound and the applied electric field. The poling treatment may also be carried out, for example, while heating the electro-optic film formed on a substrate. When performing the poling treatment, the electro-optic film is preferably heated, for example, to a temperature of not lower than a temperature 25 degrees C lower than the glass transition temperature Tg of the electro-optic film and not higher than the melting temperature Tm (from Tg - 25 degrees C to Tm). By heating to such a temperature, molecular motion of the electro-optic compound is promoted, and thus, by performing the poling treatment while heating, the compound according to the first exemplary embodiment or the electro-optic material according to the third exemplary embodiment in the electro-optic film is more easily oriented. Furthermore, by cooling the electro-optic film while maintaining the electric field, the above orientation is more easily fixed. By orienting the compound according to the first exemplary embodiment or the electro-optic material according to the third exemplary embodiment in the electro-optic film, the compound according to the first exemplary embodiment or the electro-optic material according to the third exemplary embodiment in the electro-optic film can more easily exhibit a larger electro-optic effect in the electro-optic film.

### Fifth Exemplary Embodiment

### Electro-optic Device

One form of an electro-optic device according to a fifth exemplary embodiment contains the compound according to the first exemplary embodiment. One form of the electro-optic device according to the fifth exemplary embodiment contains the electro-optic material according to the third exemplary embodiment. One form of the electro-optic device according to the fifth exemplary embodiment contains the electro-optic film according to the fourth exemplary embodiment.

The application of the electro-optic device according to the fifth exemplary embodiment is not particularly limited as long as it contains the compound according to the first exemplary embodiment, the electro-optic material according to the third exemplary embodiment, or the electro-optic film according to the fourth exemplary embodiment. The electro-optic device according to the fifth exemplary embodiment can be applied to various devices such as an optical modulator, an optical switch, an optical transceiver, an optical interconnect, an optical memory, a wavelength converter, an electric field sensor, a bioelectric potential sensor, a spatial light modulator, and an optical scanner. Further, the electro-optic device according to the fifth exemplary embodiment can also be used for, for example, optical signal transmission between electronic circuits by combination with electronic circuits.

An example of the electro-optic device is an electro-optic device applied to a Mach-Zehnder optical modulator. The Mach-Zehnder optical modulator has a structure of a Mach-Zehnder interferometer in which light emitted from one light source is split into two, passes through different paths, and is then recombined to cause interference. The electro-optic device according to the fifth exemplary embodiment is not limited thereto, and can also be used in optical modulators such as directional coupler-type optical modulators, in addition to the Mach-Zehnder optical modulator.

Since the electro-optic device according to the fifth exemplary embodiment contains at least one of the compound according to the first exemplary embodiment, the electro-optic material according to the third exemplary embodiment, and the electro-optic film according to the fourth exemplary embodiment, improvement in device performance can be expected.

In addition, specific structures, shapes, and the like in the implementation of the invention may be other structures and the like as long as the object of the invention can be achieved.

### Examples

The invention will be described in further detail with reference to Examples. The invention is by no means limited to these Examples.

### Compound

The hyperpolarizability and the maximum absorption wavelength λmax of the following compounds were calculated in accordance with the later-described methods for calculating the hyperpolarizability and the maximum absorption wavelength λmax .

### Donor Moiety

In Examples, compounds having a donor moiety represented by the formula (D1) or the formula (D2) and used for the calculation of hyperpolarizability are represented by the formulae (D101) to (D121).

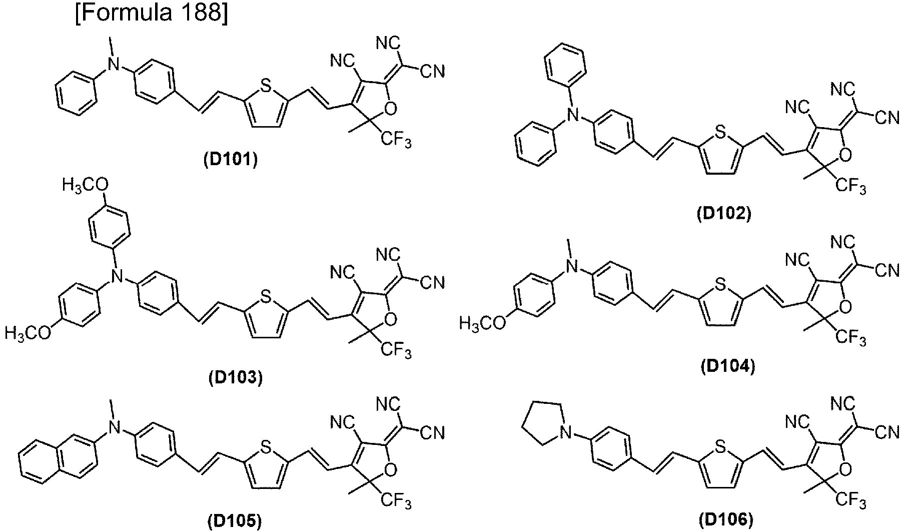

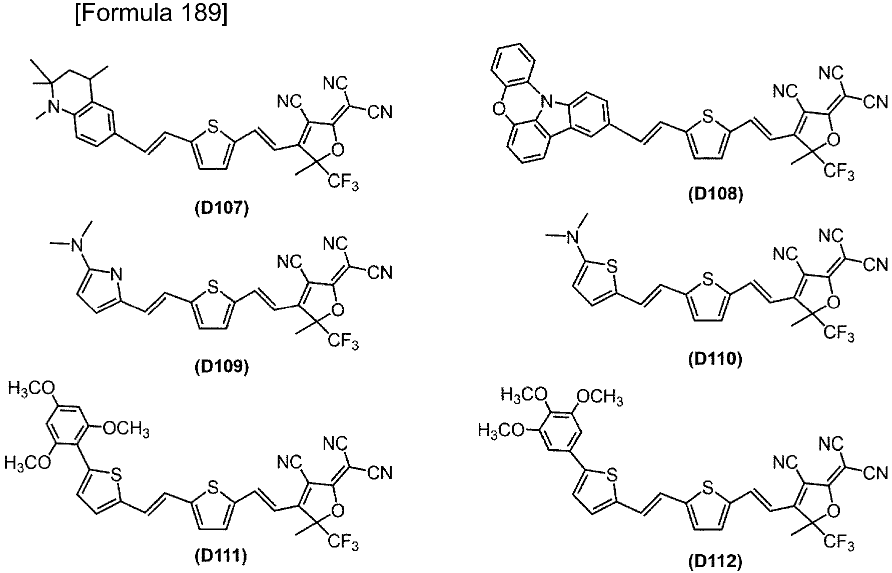

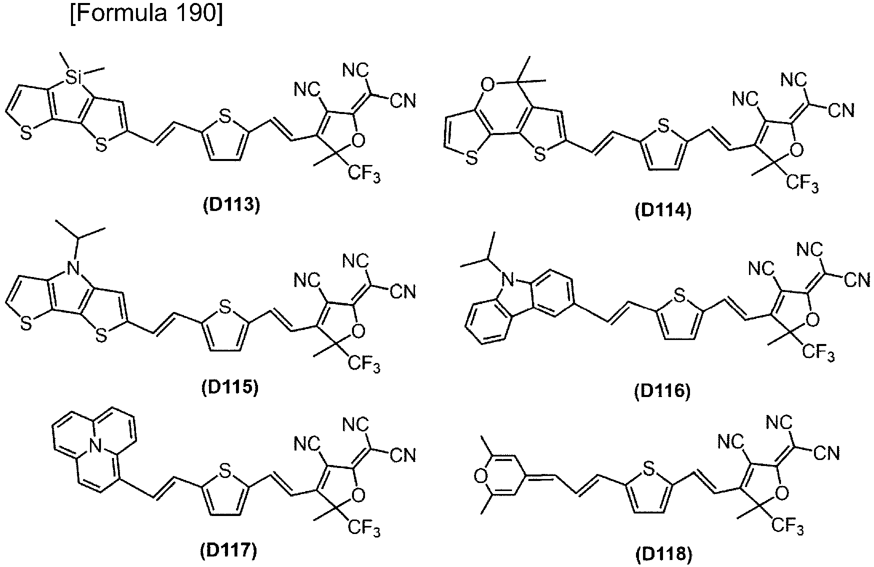

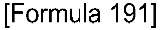

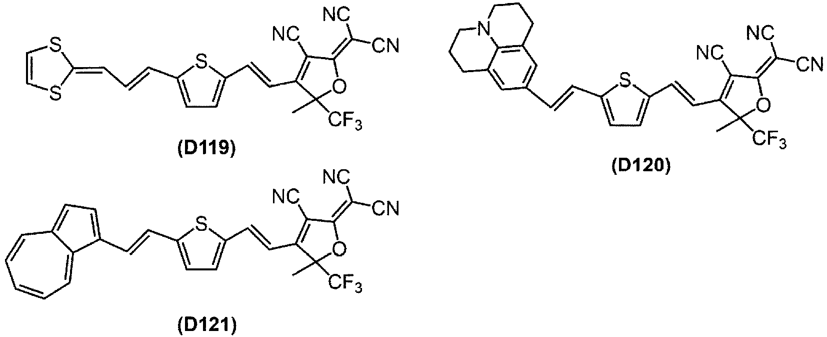

### Acceptor Moiety

In Examples, compounds having the acceptor moiety represented by the formula (A1) or the formula (A2) and used for the calculation of hyperpolarizability are represented by formulae (A101) to (A107) below.

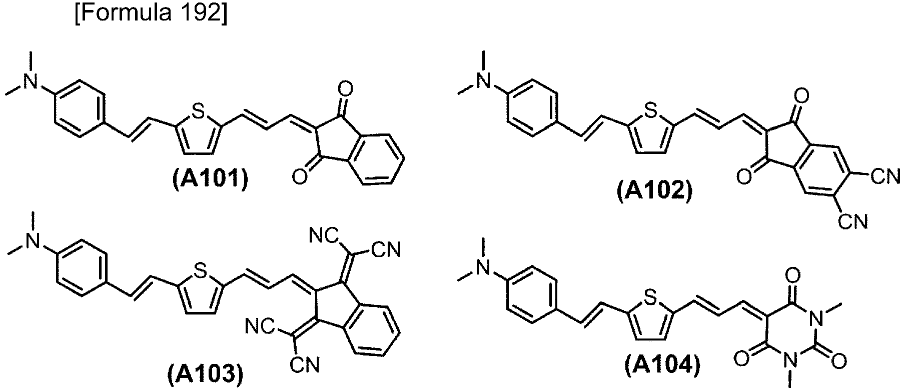

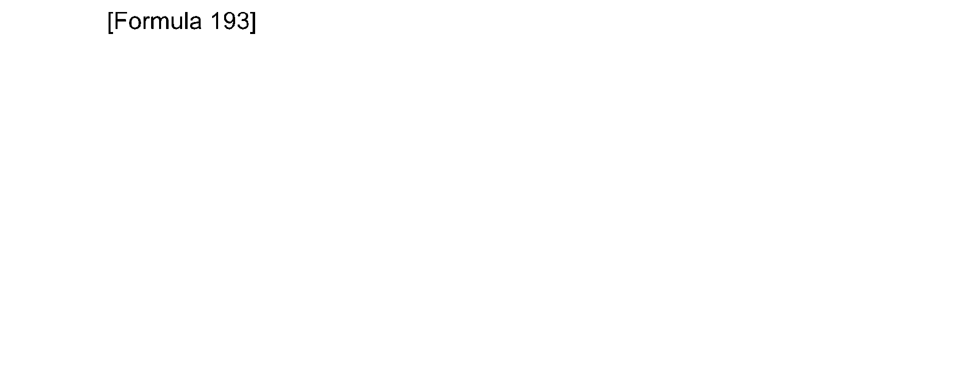

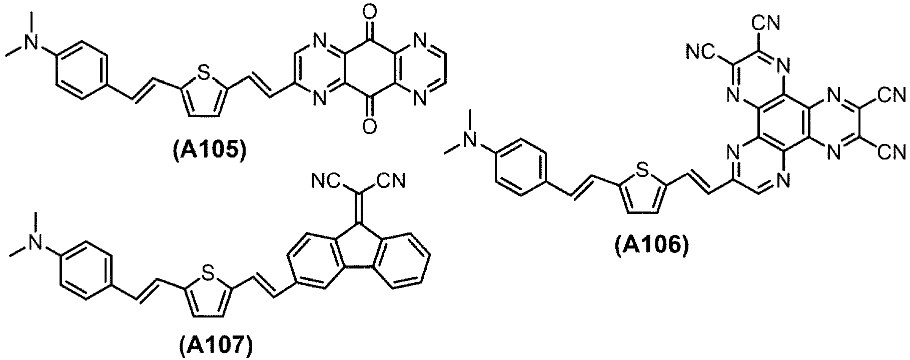

### π-conjugated Moiety

In Examples, compounds having a π-conjugated moiety represented by the formulae (P1), (P2), or (P3) and used for the calculation of hyperpolarizability are represented by the formulae (P101) to (P114) below.

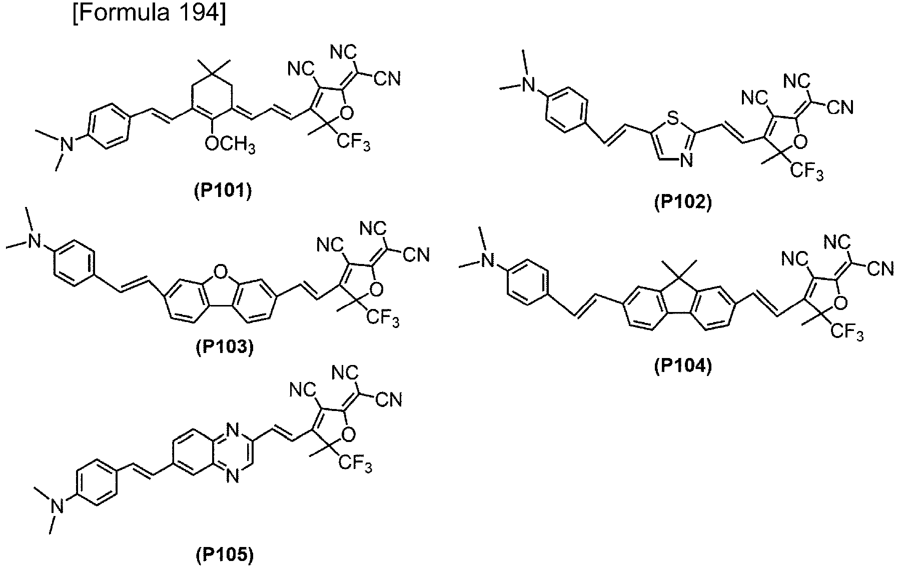

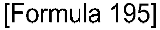

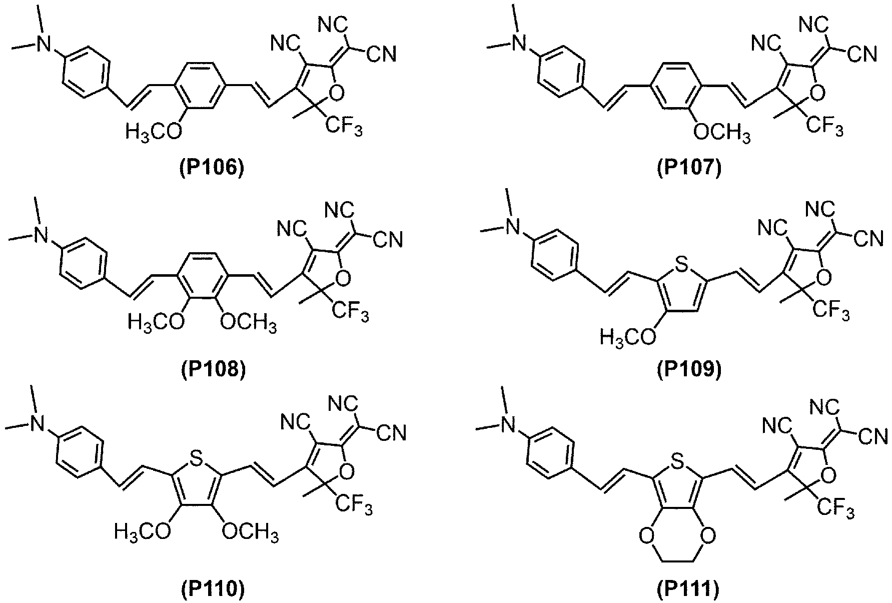

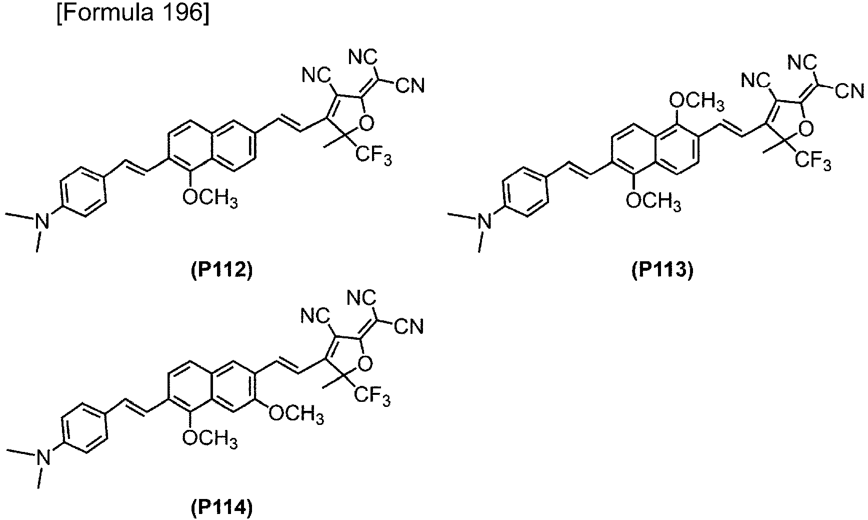

As a reference example, a compound used for the calculation of hyperpolarizability is represented by a formula (Ref-1) below.

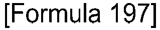

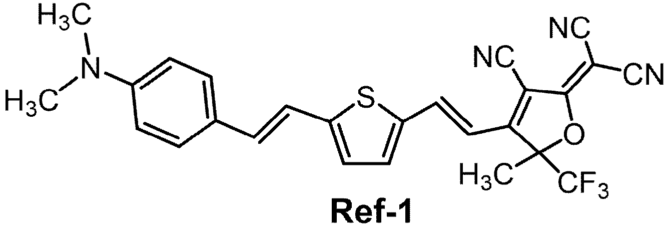

### Evaluation of Compounds

### Hyperpolarizability

In Examples, the hyperpolarizability was calculated by quantum chemical calculations using Gaussian16, which is a quantum chemical calculation program manufactured by Gaussian, Inc. Specifically, first, the molecular structure was optimized by a density functional theory using a functional B3LYP with Grimme's empirical dispersion correction D3BJ and a basis set 6-31+G(d). Next, the hyperpolarizability in the optimized molecular structure was calculated using the hyperpolarizability tensor and the dipole moment values by applying the following numerical formula (Numerical Formula 1). The hyperpolarizability tensor and the dipole moment were calculated by a calculation using CAM-B3LYP/6-31+G(d) with the keyword polar specified.
Numerical Formula 1 $β = \frac{{\sum }_{i = x , y , z} \left({μ}_{i}{β}_{i}\right)}{\sqrt{\left({{μ}_{x}}^{2}+{{μ}_{y}}^{2}+{{μ}_{z}}^{2}\right)}}$ (Numerical Formula 1)

Here, β is the hyperpolarizability, and µᵢ is a dipole moment component in the i direction. βi is the hyperpolarizability in the i direction and was determined from the following numerical formula (Numerical Formula 2) using each tensor component of the hyperpolarizability. µx, µy, and µz represent vector components in the x direction, the y direction, and the z direction, respectively, of the dipole moment µ. The dipole moment µ is represented by the square root of (µx² + µy² + µz²).
Numerical Formula 2 ${β}_{i} = {β}_{iii} + \frac{1}{3} {\sum }_{i \neq j} \left({β}_{ijj}+{β}_{jij}+{β}_{jji}\right) , i , j ∈ \left(x, y, z\right)$ (Numerical Formula 2)

### Maximum Absorption Wavelength λmax

The maximum absorption wavelength λmax of each compound was calculated by time-dependent density functional theory using the functional B3LYP and the basis set 6-31+G(d), based on the molecular structure optimized by the same method as that used for the calculation of the hyperpolarizability described above.

When the hyperpolarizability was calculated for the compounds represented by the formulae (D101) to (D121), the formulae (A101) to (A107), the formulae (P101) to (P114), and the formula (Ref-1), the compounds having a donor moiety represented by the formula (D1) or the formula (D2), the compounds having the acceptor moiety represented by the formula (A1) or the formula (A2), and the compounds having the π-conjugated moiety represented by the formula (P1), (P2), or (P3) all exhibited hyperpolarizability applicable to electro-optic materials.

### Synthesis of Compounds

Next, the following compounds were synthesized. The following compounds each contain at least the donor moiety of the compound represented by the formula (D1).

The materials used in the following synthesis examples are as follows.
2-bromo-1,3,5-trimethoxybenzene (manufactured by Tokyo Chemical Industry Co., Ltd.)
5-formyl-2-thiopheneboronic acid (manufactured by Tokyo Chemical Industry Co., Ltd.)
[1,1'-bis(diphenylphosphino)ferrocene]dichloropalladium(II) (manufactured by FUJIFILM Wako Pure Chemical Corporation)
1,3-bis(dicyanomethylidene)indane (manufactured by Tokyo Chemical Industry Co., Ltd.)
bis(dibenzylideneacetone)palladium(0) (manufactured by FUJIFILM Wako Pure Chemical Corporation)
diethyl 2-thienylmethylphosphonate (manufactured by Leap Chem Co., Ltd.)
2-[3-cyano-4-methyl-5-phenyl-5-trifluoromethyl-2(5H)-furanylidene]propanedinitrile (manufactured by Tokyo Chemical Industry Co., Ltd.)
5-bromo-2-thiophenecarboxaldehyde (manufactured by Tokyo Chemical Industry Co., Ltd.)
4-(diphenylamino)phenylboronic acid (manufactured by Tokyo Chemical Industry Co., Ltd.)

### Example 1

### 1. Synthesis of Compound C1

A synthesis method of a compound C1 is described below.

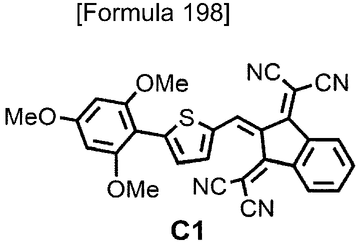

### 1-1. Synthesis of Precursor S1

First, a precursor S1 as an intermediate compound was synthesized.

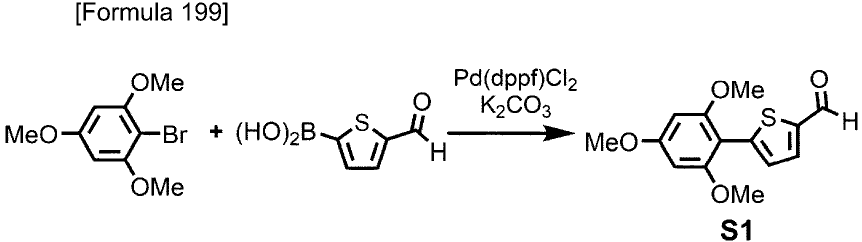

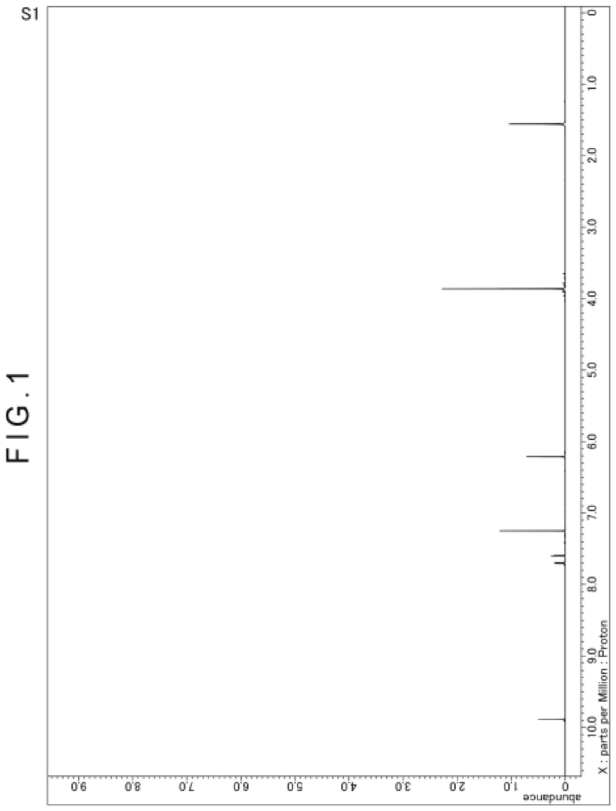

Into a 100 mL side-arm eggplant-shaped flask equipped with a Dimroth condenser, 2-bromo-1,3,5-trimethoxybenzene (2.47 g, 10.0 mmol), 5-formyl-2-thiopheneboronic acid (2.03 g, 13.0 mmol), [1,1'-bis(diphenylphosphino)ferrocene]dichloropalladium(II) (Pd(dppf)Cl₂) (366 mg, 0.50 mmol), and potassium carbonate (K₂CO₃) (6.91 g, 50.0 mmol) were charged, and the mixture was dissolved in toluene (20 mL) and methanol (20 mL), followed by stirring at 75 degrees C for 6 hours under a nitrogen atmosphere using an aluminum heat-block magnetic stirrer. After cooling the reaction mixture to room temperature, water was added, and the mixture was extracted with ethyl acetate, washed with saturated brine, and then dried over sodium sulfate. The solvent was removed, and the residue was purified by silica gel chromatography, followed by vacuum drying at 40 degrees C for 10 hours to obtain 1.06 g (3.8 mmol, yield: 38%) of the precursor S1 as a solid powder. As a result of ¹H-NMR measurement (see Fig. 1), the obtained solid powder was confirmed to be the target precursor S1.

### 1-2. Synthesis of Compound C1

Next, a compound C1 was synthesized.

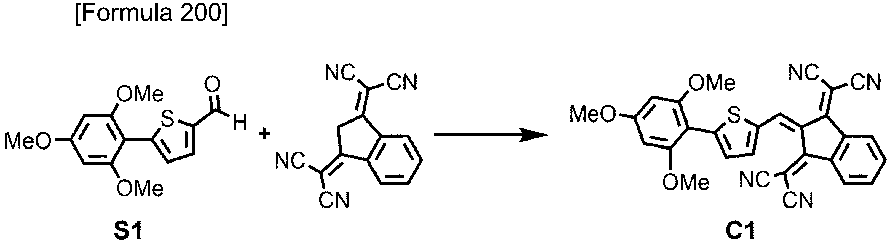

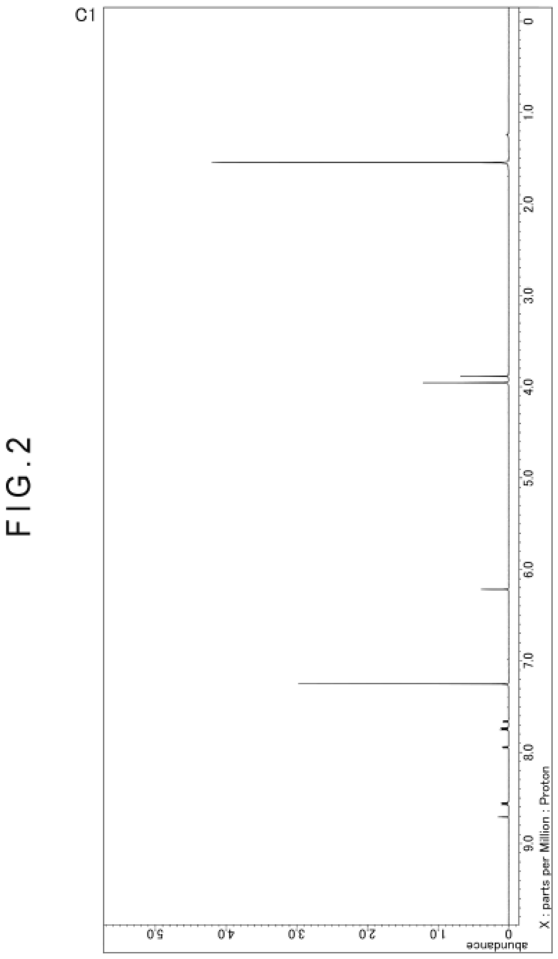

Into a 100 mL side-arm eggplant-shaped flask equipped with a Dimroth condenser were charged the precursor S1 (1.50 mmol) and 1,3-bis(dicyanomethylidene)indane (1.50 mmol). Ethanol (24.0 mL) and tetrahydrofuran (THF) (6.0 mL) were added thereto to dissolve the mixture, followed by stirring while heating at 75 degrees C for 4.5 hours under a nitrogen atmosphere using an aluminum heat-block magnetic stirrer. After cooling the reaction mixture to room temperature, hexane was added, and the precipitated solid was separated by suction filtration. The solid was further washed with ethanol and vacuum-dried at 40 degrees C for 10 hours to obtain 460 mg (0.915 mmol, yield: 61%) of the compound C1. As a result of ¹H-NMR measurement (see Fig. 2), the obtained compound was confirmed to be the target compound C1.

### Example 2

### 2. Synthesis of Compound C3

A synthesis method of a compound C3 is described below.

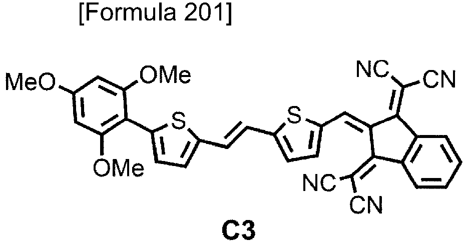

### 2-1. Synthesis of Precursor S3

First, a precursor S3 as an intermediate compound was synthesized.

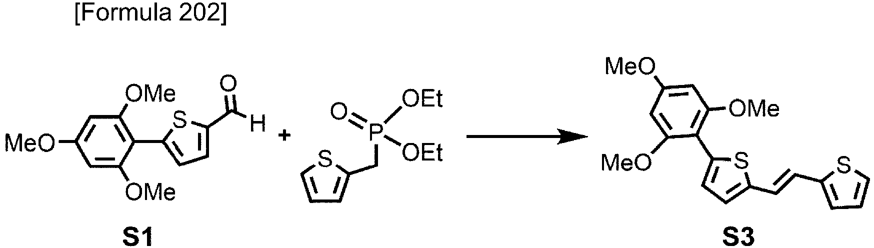

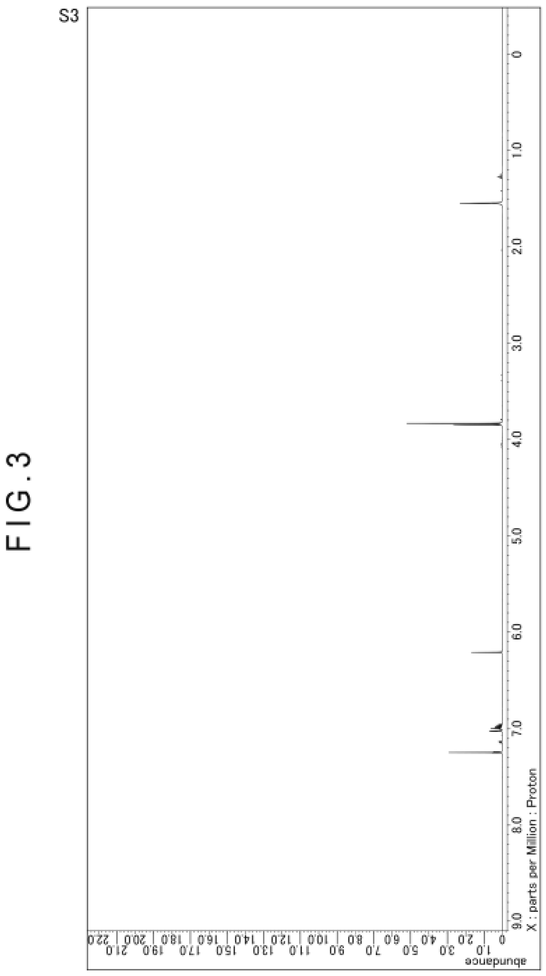

The precursor S1 (595 mg, 2.14 mmol) and diethyl 2-thienylmethylphosphonate (752 mg, 3.21 mmol) were dissolved in THF (26 mL) in a 100 mL side-arm eggplant-shaped flask, and while cooling to 0 degrees C under a nitrogen atmosphere, a 1.0 M potassium tert-butoxide (tBuOK)/tetrahydrofuran (THF) solution (4 mL, 4.0 mmol) was added dropwise, followed by stirring at room temperature for 5 hours after completion of the addition. Water was added, and the mixture was extracted with ethyl acetate, washed with saturated brine, and then dried over sodium sulfate. The solvent was removed, followed by vacuum drying at 40 degrees C for 10 hours to obtain 565 mg (1.58 mmol, yield: 74%) of the precursor S3 as a solid powder. As a result of ¹H-NMR measurement (see Fig. 3), the obtained solid powder was confirmed to be the target precursor S3.

### 2-2. Synthesis of Precursor S4

Next, a precursor S4 as an intermediate compound was synthesized.

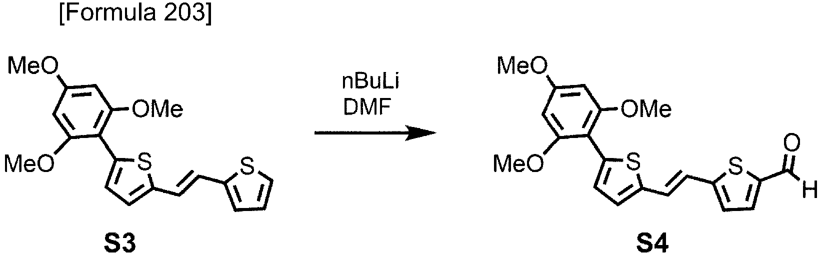

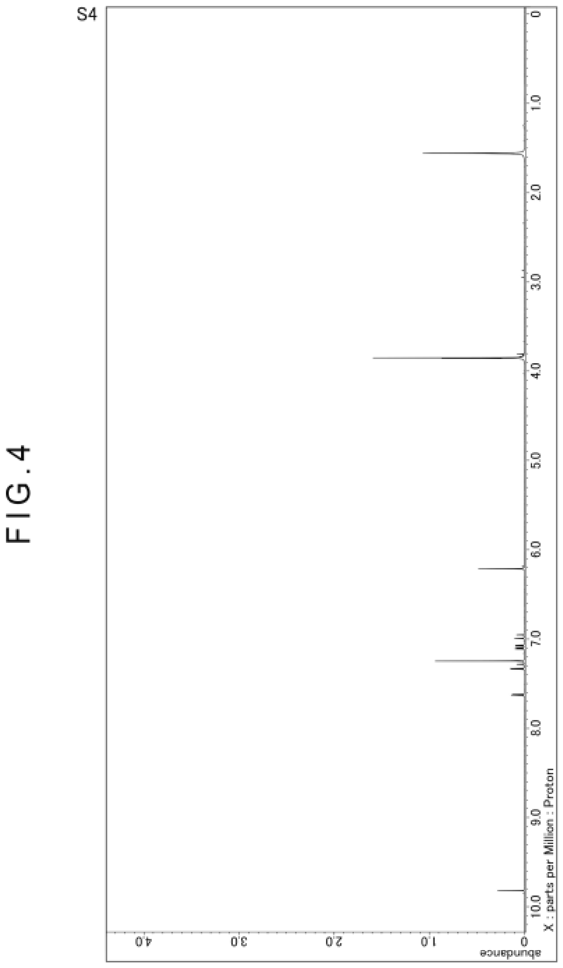

The precursor S3 (1.16 g, 3.24 mmol) was dissolved in tetrahydrofuran (THF) (20 mL) in a 100 mL side-arm eggplant-shaped flask, and while cooling to -78 degrees C under a nitrogen atmosphere, a 1.6 M n-butyllithium (nBuLi)/n-hexane solution (2.4 mL, 3.9 mmol) was added dropwise, followed by stirring for 30 minutes after completion of the addition. Thereafter, dimethylformamide (DMF) (1.3 mL, 16 mmol) was added dropwise, and the mixture was stirred at room temperature for 3 hours. Water was added, and the mixture was extracted with ethyl acetate, washed with saturated brine, and then dried over sodium sulfate. The solvent was removed, and the obtained residue was purified by recrystallization, followed by vacuum drying at 40 degrees C for 10 hours to obtain 641 mg (1.66 mmol, yield: 51%) of the precursor S4 as a solid powder. As a result of ¹H-NMR measurement (see Fig. 4), the obtained solid powder was confirmed to be the target precursor S4.

### 2-3. Synthesis of Compound C3

Next, a compound C3 was synthesized.

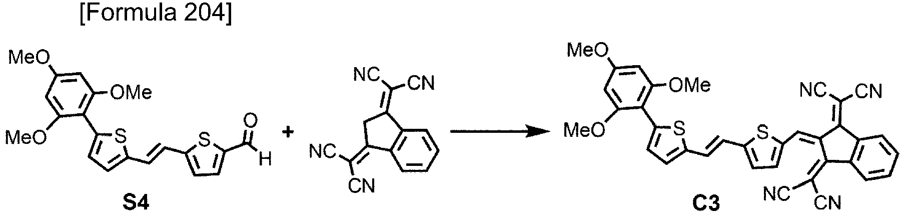

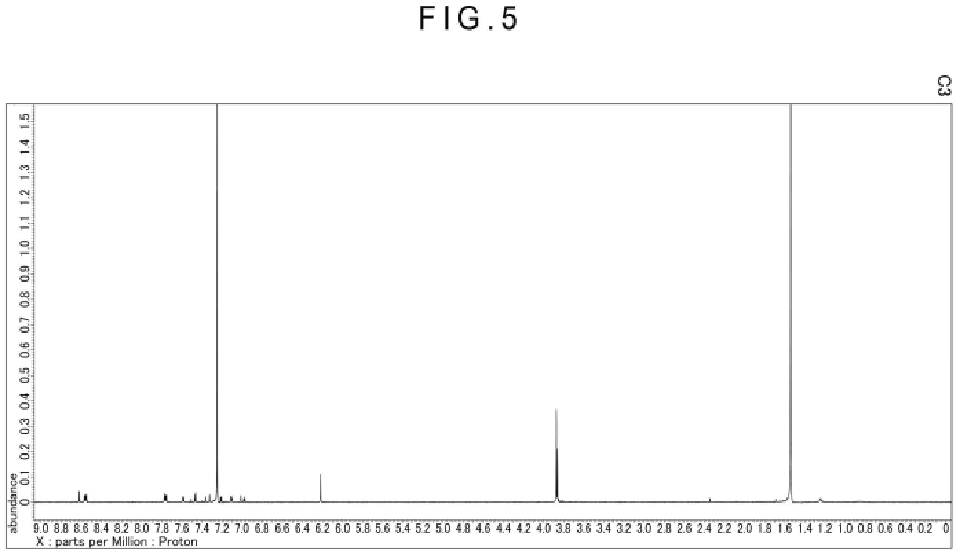

Into a 100 mL side-arm eggplant-shaped flask equipped with a Dimroth condenser were charged precursor S4 (0.983 mmol) and 1,3-bis(dicyanomethylidene)indane (0.983 mmol), and ethanol (16.0 mL) and tetrahydrofuran (THF) (4.0 mL) were added to dissolve the mixture, followed by stirring while heating at 75 degrees C for 6 hours under a nitrogen atmosphere using an aluminum heat-block magnetic stirrer. After cooling the reaction mixture to room temperature, hexane was added, and the precipitated solid was separated by suction filtration. The obtained solid was purified by recrystallization, followed by vacuum drying at 40 degrees C for 10 hours to obtain 438 mg (0.717 mmol, yield: 73%) of the compound C3 as a solid powder. As a result of ¹H-NMR measurement (see Fig. 5), the obtained solid powder was confirmed to be the target compound C3.

### Example 3

### 3. Synthesis of Compound C4

A synthesis method of a compound C4 is described below.

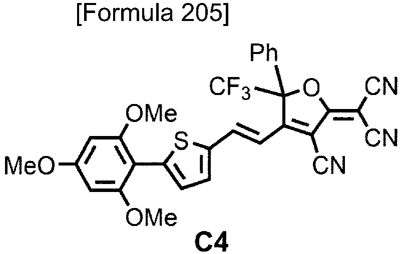

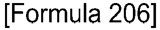

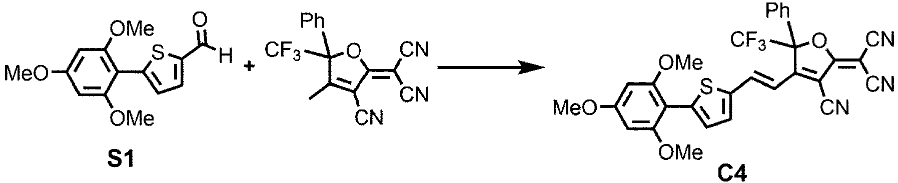

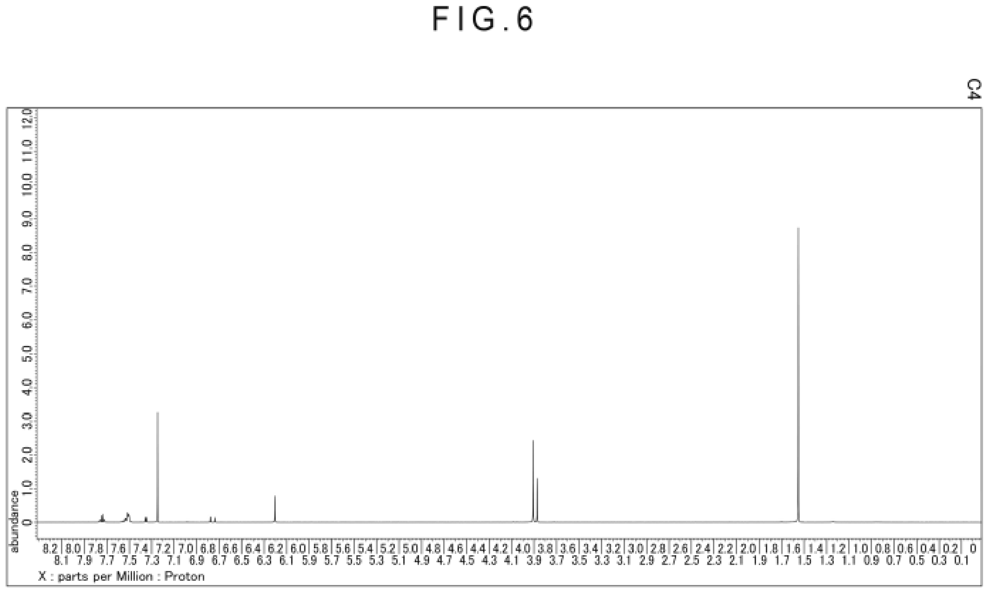

Into a 100 mL side-arm eggplant-shaped flask equipped with a Dimroth condenser were charged the precursor S1 (139 mg, 0.50 mmol) and 2-[3-cyano-4-methyl-5-phenyl-5-trifluoromethyl-2(5H)-furanylidene]propanedinitrile (189 mg, 0.60 mmol), and ethanol (8.0 mL) and tetrahydrofuran (THF) (2.0 mL) were added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 240 mg (0.42 mmol, yield: 83%) of the compound C4 as a solid powder. As a result of ¹H-NMR measurement (see Fig. 6), the obtained solid powder was confirmed to be the target compound C4.

### Example 4

### 4. Synthesis of Compound C6

A synthesis method of a compound C6 is described below.

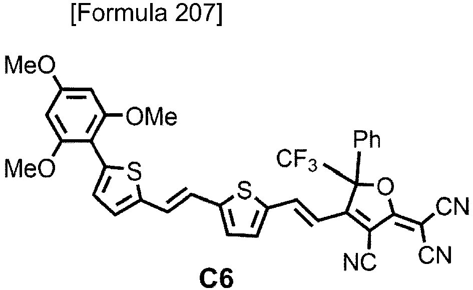

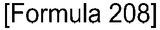

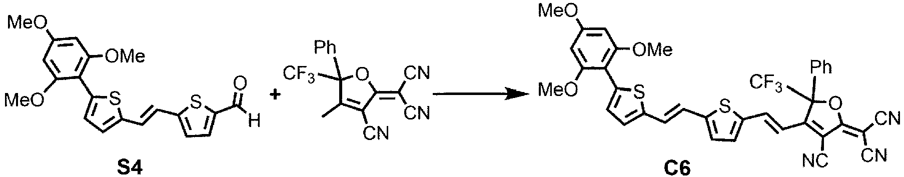

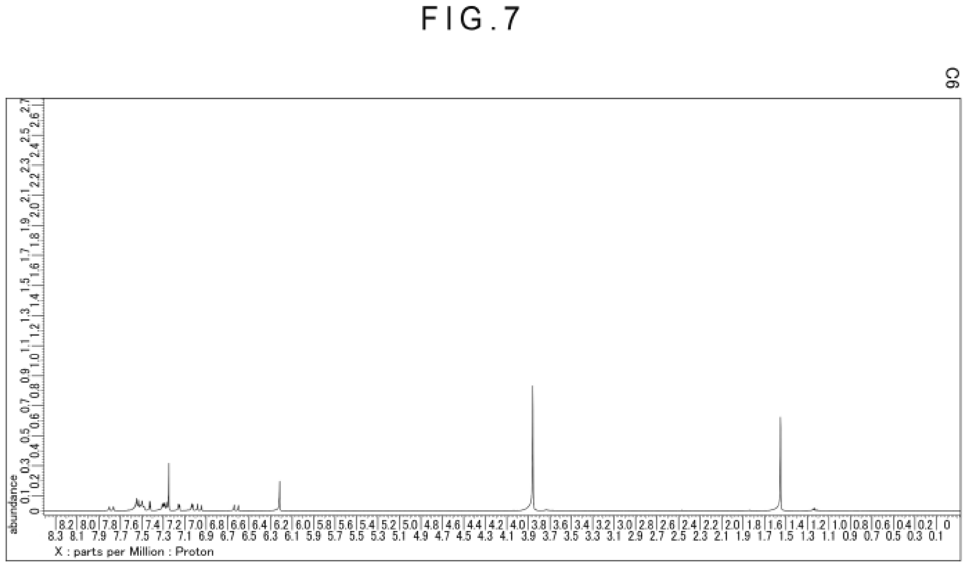

Into a 100 mL side-arm eggplant-shaped flask equipped with a Dimroth condenser were charged the precursor S4 (193 mg, 0.50 mmol) and 2-[3-cyano-4-methyl-5-phenyl-5-trifluoromethyl-2(5H)-furanylidene]propanedinitrile (189 mg, 0.60 mmol), and ethanol (8.0 mL) and tetrahydrofuran (THF) (2.0 mL) were added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 302 mg (0.44 mmol, yield: 88%) of compound C6 as a solid powder. As a result of ¹H-NMR measurement (see Fig. 7), the obtained solid powder was confirmed to be the target compound C6.

### Example 5

### 5. Synthesis of Compound C7

A synthesis method of a compound C7 is described below.

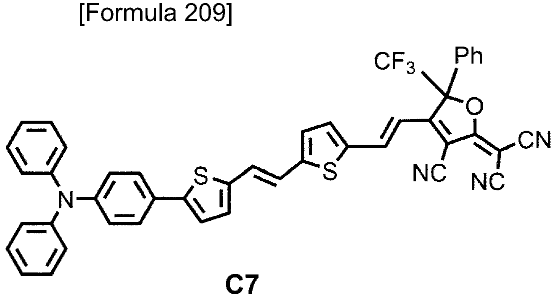

### 5-1. Synthesis of Precursor S5

First, a precursor S5 as an intermediate compound was synthesized.

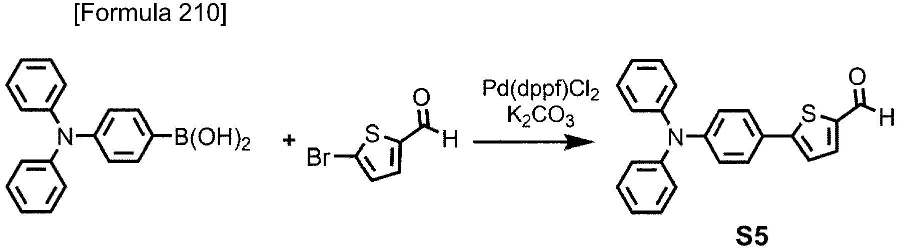

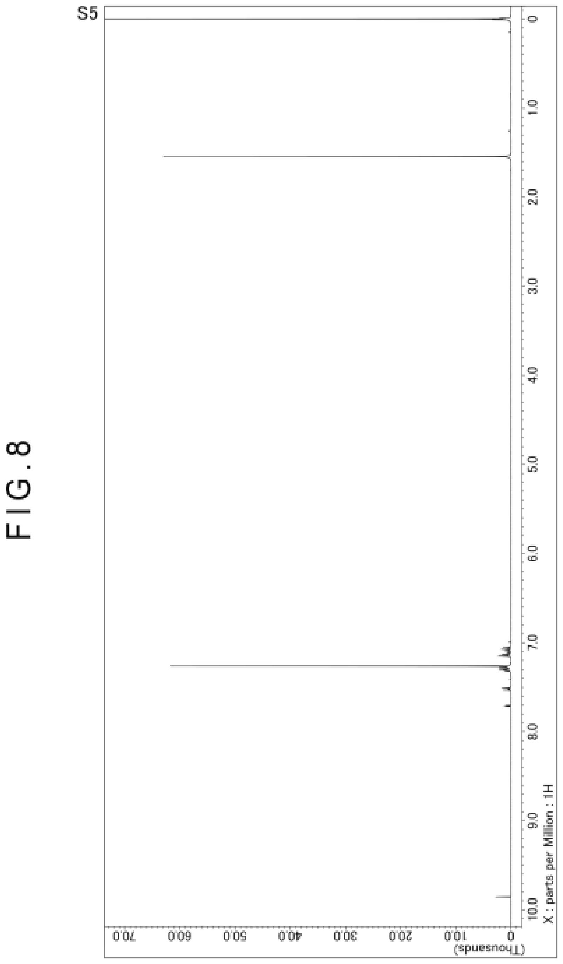

5-Bromo-2-thiophenecarboxaldehyde (1.91 g, 10 mmol), 4-(diphenylamino)phenylboronic acid (3.76 g, 13 mmol), [1,1'-bis(diphenylphosphino)ferrocene]dichloropalladium(II) (Pd(dppf)Cl2) (0.277 g, 0.50 mmol), and potassium carbonate (6.91 g, 50.0 mmol) were dissolved in toluene (20 mL) and methanol (20 mL) in a 100 mL side-arm eggplant-shaped flask equipped with a Dimroth condenser, and the mixture was stirred at 60°C for 1 hour under a nitrogen atmosphere. The reaction mixture was filtered through neutral silica using ethyl acetate, washed with saturated aqueous sodium bicarbonate, and the organic layer was separated. The obtained organic layer was dried over sodium sulfate. The solvent was removed, and the residue was purified by silica gel chromatography, followed by vacuum drying at 40 degrees C for 10 hours to obtain 3.27 g (9.2 mmol, yield: 92%) of precursor S5 as a solid powder. As a result of ¹H-NMR measurement (see Fig. 8), the obtained solid powder was confirmed to be the target precursor S5.

### 5-2. Synthesis of Precursor S6

Next, a precursor S6 as an intermediate compound was synthesized.

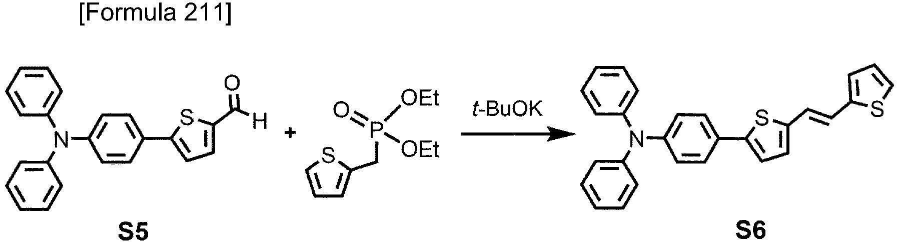

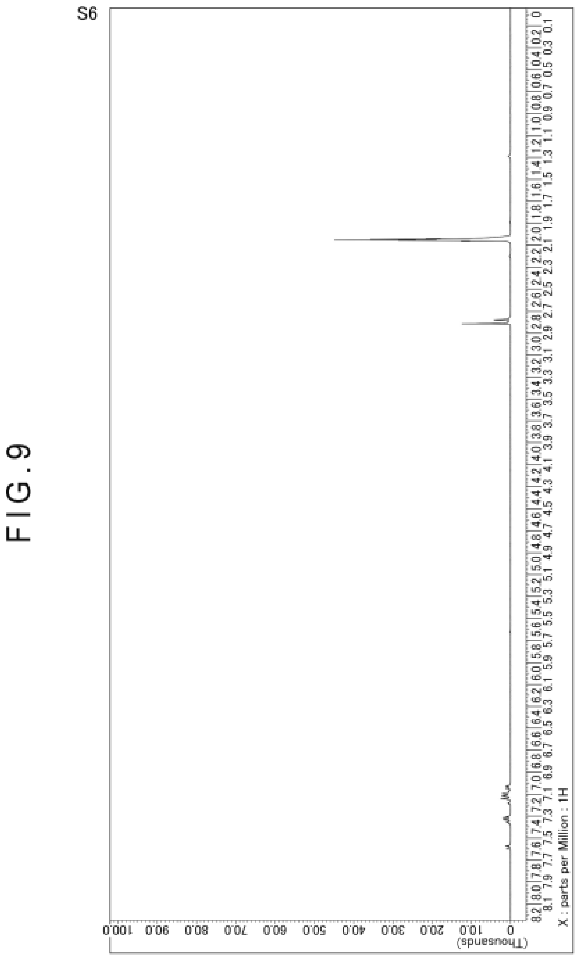

The precursor S5 (1.07 g, 3.0 mmol) and diethyl 2-thienylmethylphosphonate (1.05 g, 4.5 mmol) were dissolved in tetrahydrofuran (THF) (20 mL) in a 100 mL side-arm eggplant-shaped flask, and while cooling to 0 degrees C under a nitrogen atmosphere, a 1.0 M potassium tert-butoxide (tBuOK)/tetrahydrofuran (THF) solution (6 mL, 6.0 mmol) was added dropwise, followed by stirring at room temperature for 5 hours after completion of the addition. Water was added, and the mixture was extracted with ethyl acetate, washed with saturated brine, and then dried over sodium sulfate. The solvent was removed, followed by vacuum drying at 40 degrees C for 10 hours to obtain 1.05 g (2.43 mmol, yield: 81%) of precursor S6 as a solid powder. As a result of ¹H-NMR measurement (see Fig. 9), the obtained solid powder was confirmed to be the target precursor S6.

### 5-3. Synthesis of Precursor S7

Next, a precursor S7 as an intermediate compound was synthesized.

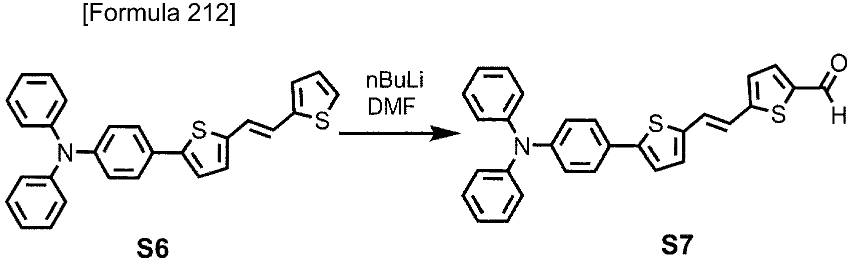

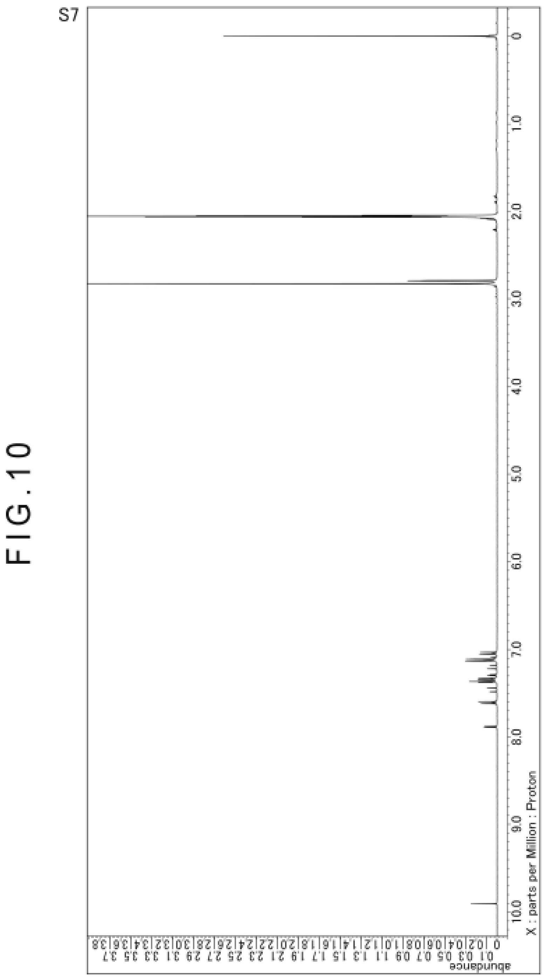

The precursor S6 (0.958 g, 2.2 mmol) was dissolved in tetrahydrofuran (THF) (20 mL) in a 100 mL side-arm eggplant-shaped flask, and while cooling to -78 degrees C under a nitrogen atmosphere, a 1.6 M n-butyllithium (nBuLi)/n-hexane solution (1.6 mL, 2.6 mmol) was added dropwise, followed by stirring for 30 minutes after completion of the addition. Thereafter, dimethylformamide (DMF) (0.9 mL, 11 mmol) was added dropwise, and the mixture was stirred at room temperature for 3 hours. Water was added, and the mixture was extracted with ethyl acetate, washed with saturated brine, and then dried over sodium sulfate. The solvent was removed, and the obtained residue was purified by recrystallization, followed by vacuum drying at 40 degrees C for 10 hours to obtain 788 mg (1.7 mmol, yield: 77%) of the precursor S7 as a solid powder. As a result of ¹H-NMR measurement (see Fig. 10), the obtained solid powder was confirmed to be the target precursor S7.

### 5-4. Synthesis of Compound C7

Next, a compound C7 was synthesized.

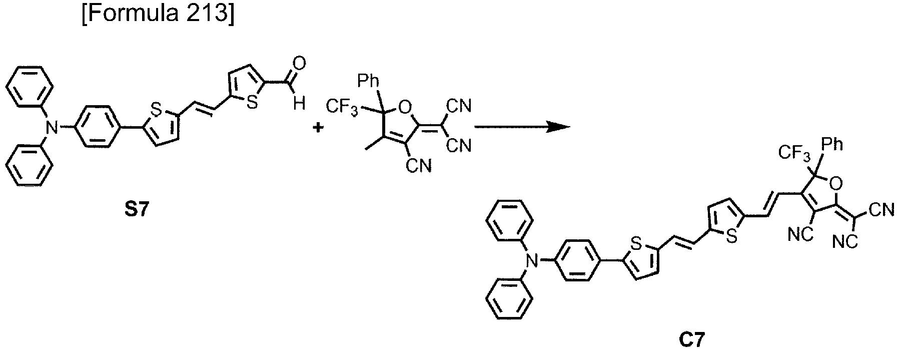

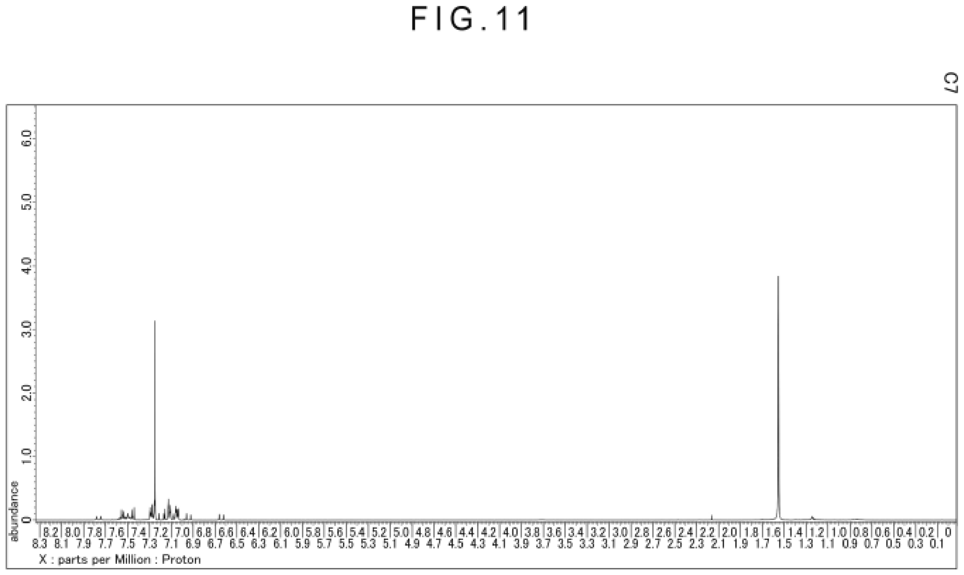

Into a 100 mL side-arm eggplant-shaped flask equipped with a Dimroth condenser were charged the precursor S7 (218 mg, 0.50 mmol) and 2-[3-cyano-4-methyl-5-phenyl-5-trifluoromethyl-2(5H)-furanylidene]propanedinitrile (189 mg, 0.60 mmol), and ethanol (8.0 mL) and tetrahydrofuran (THF) (2.0 mL) were added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 342 mg (0.45 mmol, yield: 90%) of compound C7. As a result of ¹H-NMR measurement (see Fig. 11), the obtained solid powder was confirmed to be the target compound C7.

### Example 6

### 6. Synthesis of Compound C8

A synthesis method of a compound C8 is described below.

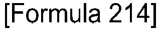

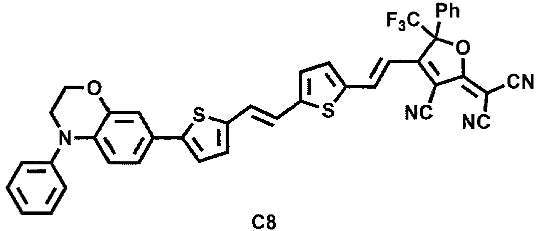

### 6-1. Synthesis of Precursor S8

First, a precursor S8 as an intermediate compound was synthesized.

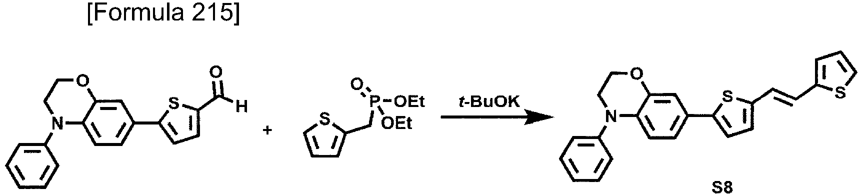

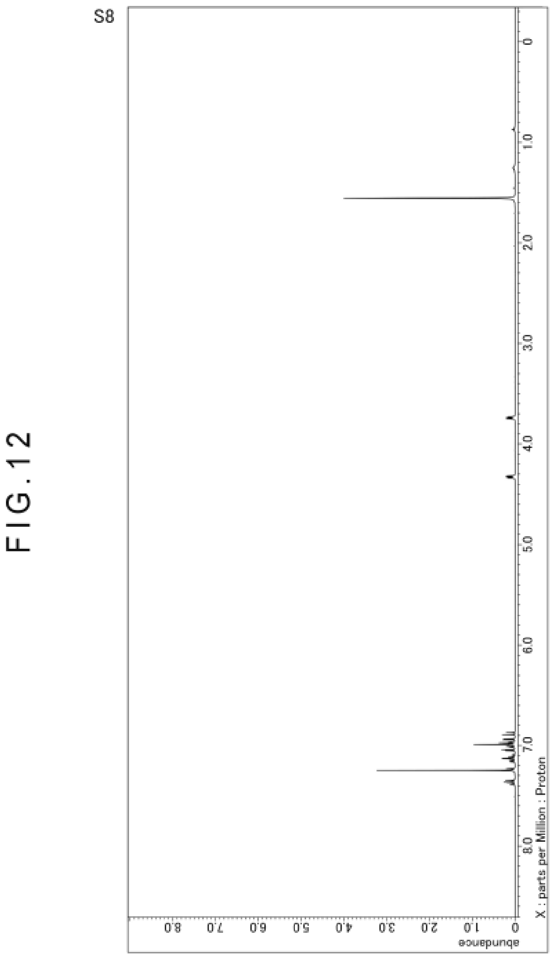

5-(4-phenyl-2,3-dihydro-1,4-benzoxazin-7-yl)thiophene-2-carbaldehyde (643 mg, 2.00 mmol) and diethyl 2-thienylmethylphosphonate (703 mg, 3.00 mmol) were dissolved in tetrahydrofuran (THF) (16 mL) in a 100 mL side-arm eggplant-shaped flask, and while cooling to 0 degrees C under a nitrogen atmosphere, a 1.0 M potassium tert-butoxide (t-BuOK)/tetrahydrofuran (THF) solution (3.6 mL, 3.6 mmol) was added dropwise, followed by stirring at room temperature for 5 hours after completion of the addition. Water was added, and the mixture was extracted with ethyl acetate, washed with saturated brine, and then dried over sodium sulfate. The solvent was removed, followed by vacuum drying at 40 degrees C for 10 hours to obtain 793 mg (1.98 mmol, yield: 99%) of the precursor S8 as a solid powder. As a result of ¹H-NMR measurement (see Fig. 12), the obtained solid powder was confirmed to be the target precursor S8.

### 6-2. Synthesis of Precursor S9

Next, a precursor S9 as an intermediate compound was synthesized.

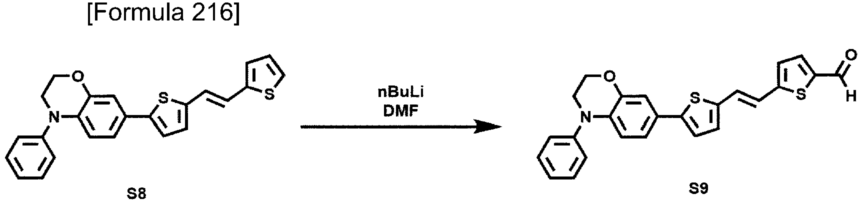

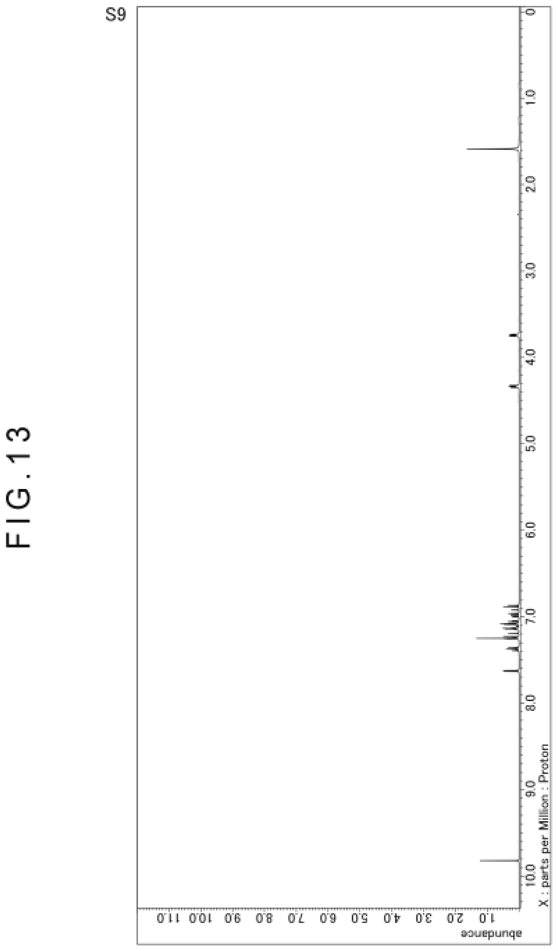

The precursor S8 (757 mg, 1.89 mmol) was dissolved in tetrahydrofuran (THF) (15 mL) in a 100 mL side-arm eggplant-shaped flask, and while cooling to -78 degrees C under a nitrogen atmosphere, a 1.6 M potassium tert-butoxide (t-BuOK)/n-hexane solution (1.4 mL, 2.3 mmol) was added dropwise, followed by stirring for 30 minutes after completion of the addition. Thereafter, dimethylformamide (DMF) (0.7 mL, 9.5 mmol) was added dropwise, and the mixture was stirred at room temperature for 3 hours. Water was added, and the mixture was extracted with ethyl acetate, washed with saturated brine, and then dried over sodium sulfate. The solvent was removed, and the obtained residue was purified by recrystallization, followed by vacuum drying at 40 degrees C for 10 hours to obtain 658 mg (1.53 mmol, yield: 81%) of the precursor S9 as a solid powder. As a result of ¹H-NMR measurement (see Fig. 13), the obtained solid powder was confirmed to be the target precursor S9.

### 6-3. Synthesis of Compound C8

Next, a compound C8 was synthesized.

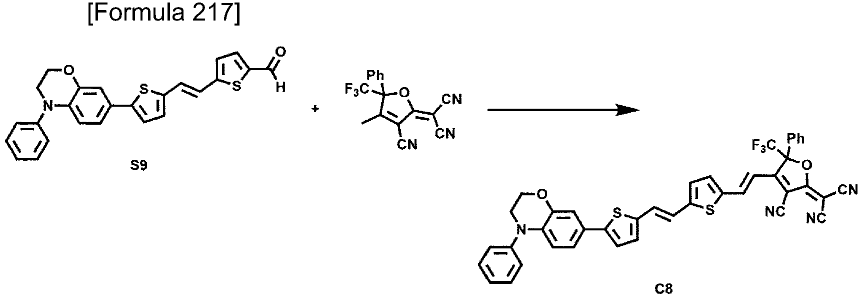

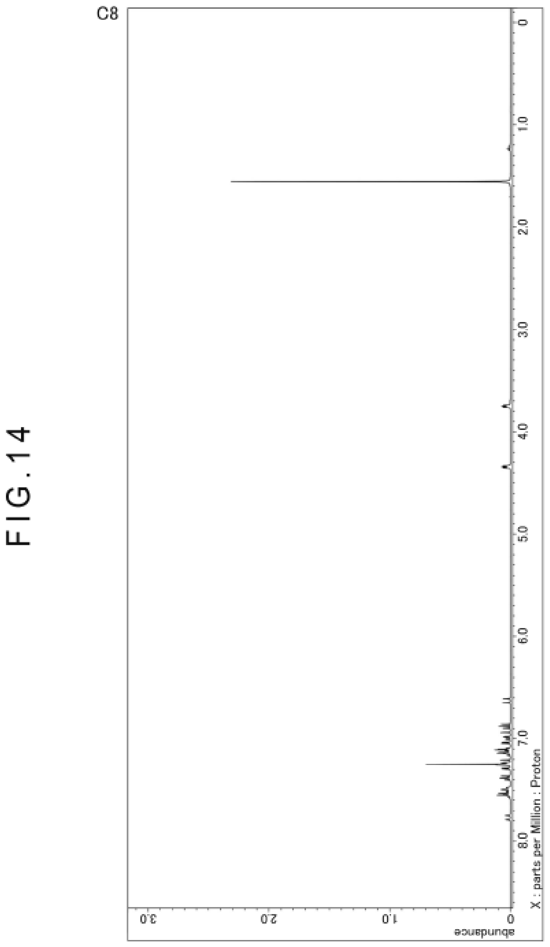

Into a 100 mL side-arm eggplant-shaped flask equipped with a Dimroth condenser were charged the precursor S9 (322 mg, 0.75 mmol) and 2-[3-cyano-4-methyl-5-phenyl-5-trifluoromethyl-2(5H)-furanylidene]propanedinitrile (284 mg, 0.90 mmol), and ethanol (15 mL) was added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 531 mg (0.73 mmol, yield: 97%) of compound C8. As a result of ¹H-NMR measurement (see Fig. 14), the obtained solid powder was confirmed to be the target compound C8.

### Example 7

### 7. Synthesis of Compound C9

A synthesis method of a compound C9 is described below.

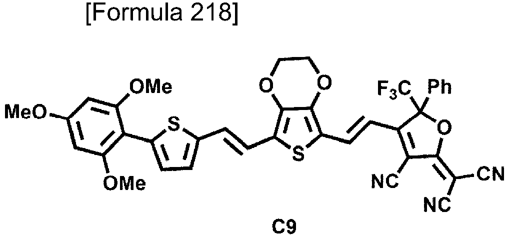

### 7-1. Synthesis of Precursor S10

First, a precursor S10 as an intermediate compound was synthesized.

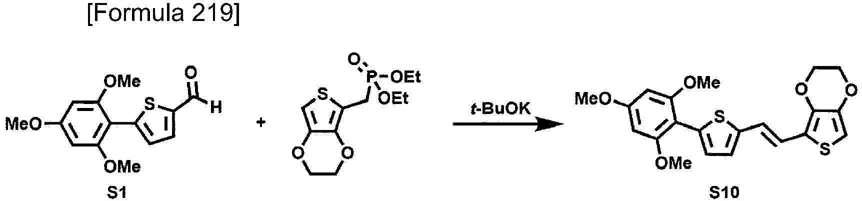

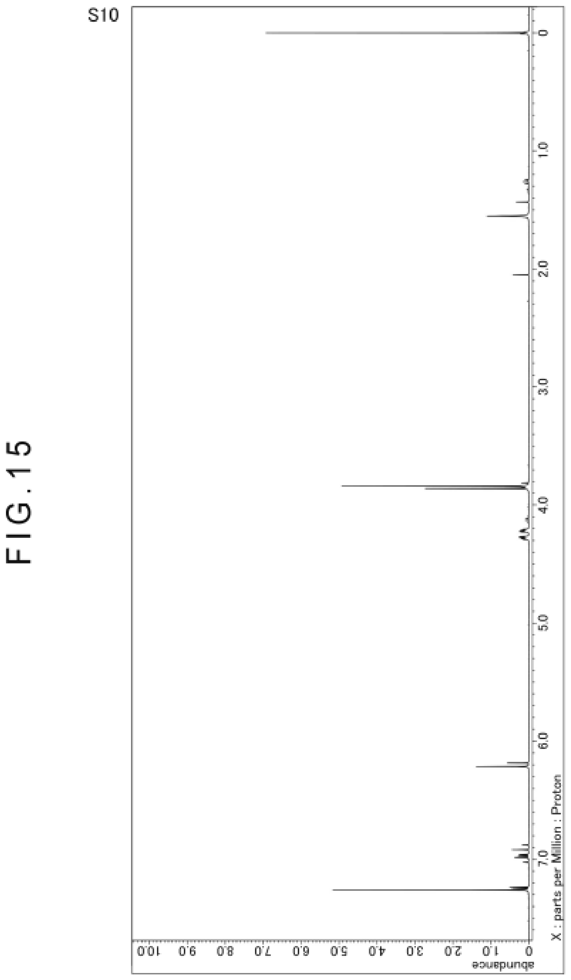

The precursor S1 (595 mg, 2.14 mmol) and 5-(diethoxyphosphorylmethyl)-2,3-dihydrothieno[3,4-b][1,4]dioxine (938 mg, 3.21 mmol) were dissolved in tetrahydrofuran (THF) (16 mL) in a 100 mL side-arm eggplant-shaped flask, and while cooling to 0 degrees C under a nitrogen atmosphere, a 1.0 M potassium tert-butoxide (t-BuOK)/tetrahydrofuran (THF) solution (3.9 mL, 3.9 mmol) was added dropwise, followed by stirring at room temperature for 5 hours after completion of the addition. Water was added, and the mixture was extracted with ethyl acetate, washed with saturated brine, and then dried over sodium sulfate. The solvent was removed, followed by vacuum drying at 40 degrees C for 10 hours to obtain 856 mg (2.05 mmol, yield: 96%) of the precursor S10 as a solid powder. As a result of ¹H-NMR measurement (see Fig. 15), the obtained solid powder was confirmed to be the target precursor S10.

### 7-2. Synthesis of Precursor S11

Next, a precursor S11 as an intermediate compound was synthesized.

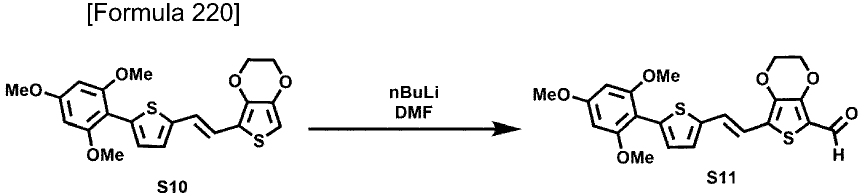

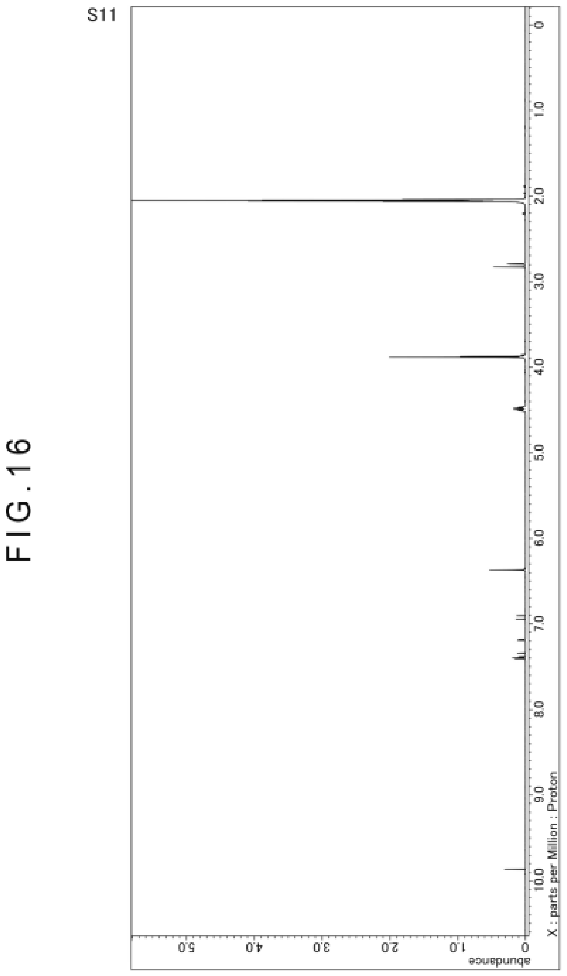

The precursor S10 (625 mg, 1.50 mmol) was dissolved in tetrahydrofuran (THF) (15 mL) in a 100 mL side-arm eggplant-shaped flask, and while cooling to -78 degrees C under a nitrogen atmosphere, a 1.6 M n-butyllithium (nBuLi)/n-hexane solution (1.1 mL, 1.80 mmol) was added dropwise, followed by stirring for 30 minutes after completion of the addition. Thereafter, dimethylformamide (DMF) (0.6 mL, 7.5 mmol) was added dropwise, and the mixture was stirred at room temperature for 3 hours. Water was added, and the mixture was extracted with ethyl acetate, washed with saturated brine, and then dried over sodium sulfate. The solvent was removed, and the obtained residue was purified by recrystallization, followed by vacuum drying at 40 degrees C for 10 hours to obtain 533 mg (1.20 mmol, yield: 80%) of the precursor S11 as a solid powder. As a result of ¹H-NMR measurement (see Fig. 16), the obtained solid powder was confirmed to be the target precursor S11.

### 7-3. Synthesis of Compound C9

Next, a compound C9 was synthesized.

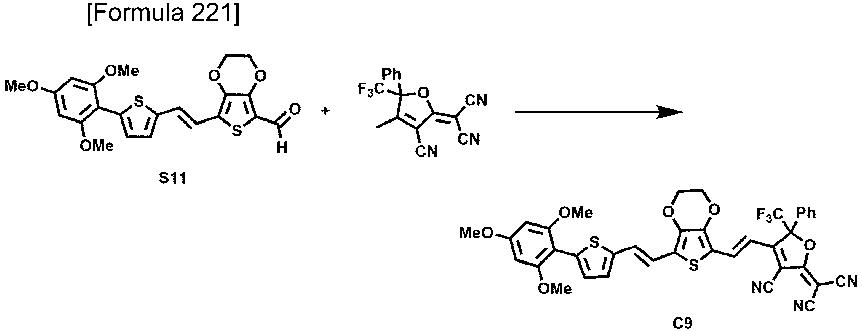

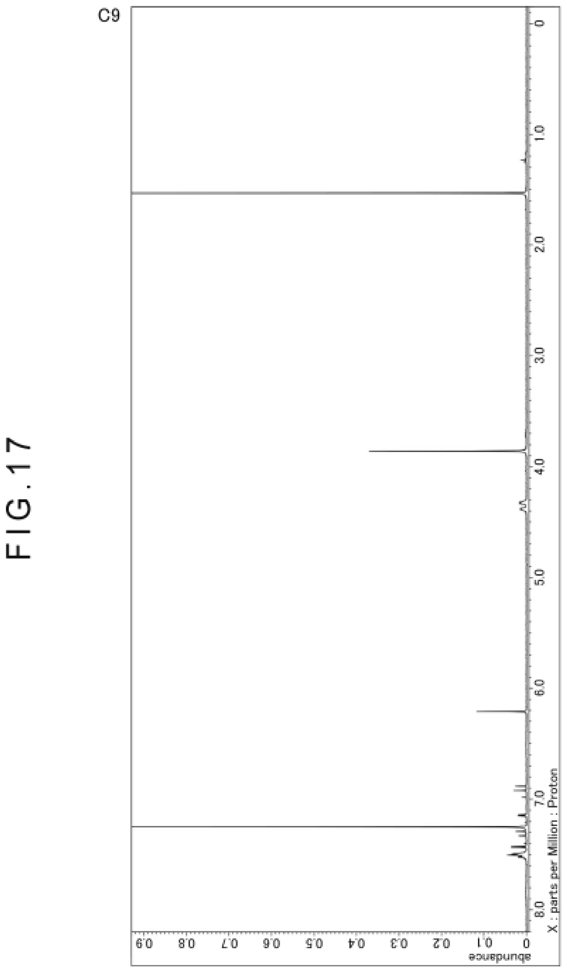

Into a 100 mL side-arm eggplant-shaped flask equipped with a Dimroth condenser were charged the precursor S11 (333 mg, 0.75 mmol) and 2-[3-cyano-4-methyl-5-phenyl-5-trifluoromethyl-2(5H)-furanylidene]propanedinitrile (284 mg, 0.90 mmol), and ethanol (16 mL) was added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 526 mg (0.71 mmol, yield: 94%) of compound C9. As a result of ¹H-NMR measurement (see Fig. 17), the obtained solid powder was confirmed to be the target compound C9.

### Example 8

### 8. Synthesis of Compound C10

A synthesis method of a compound C10 is described below.

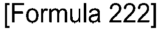

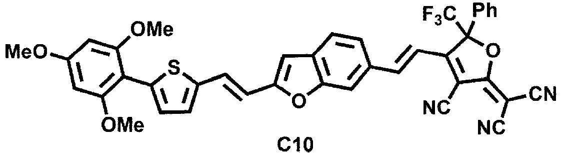

### 8-1. Synthesis of Precursor S12

First, a precursor S12 as an intermediate compound was synthesized.

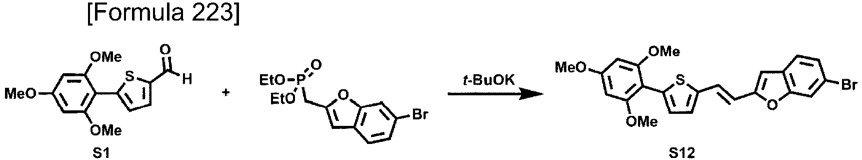

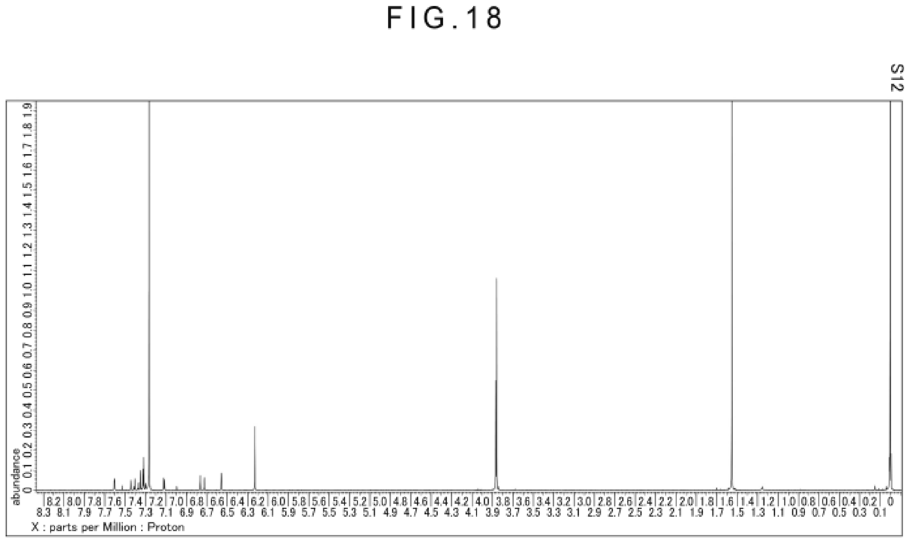

The precursor S1 (557 mg, 2.00 mmol) and 6-bromo-2-(diethoxyphosphorylmethyl)benzofuran (1.04 g, 3.00 mmol) were dissolved in tetrahydrofuran (THF) (16 mL) in a 100 mL side-arm eggplant-shaped flask, and while cooling to 0 degrees C under a nitrogen atmosphere, a 1.0 M potassium tert-butoxide (t-BuOK)/tetrahydrofuran (THF) solution (3.6 mL, 3.6 mmol) was added dropwise, followed by stirring at room temperature for 5 hours after completion of the addition. Water was added, and the mixture was extracted with ethyl acetate, washed with saturated brine, and then dried over sodium sulfate. The solvent was removed, followed by vacuum drying at 40 degrees C for 10 hours to obtain 745 mg (1.58 mmol, yield: 79%) of the precursor S12 as a solid powder. As a result of ¹H-NMR measurement (see Fig. 18), the obtained solid powder was confirmed to be the target precursor S12.

### 8-2. Synthesis of Precursor S13

Next, a precursor S13 was synthesized.

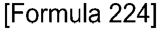

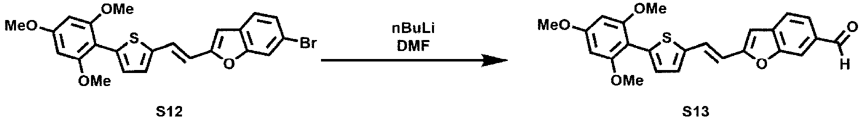

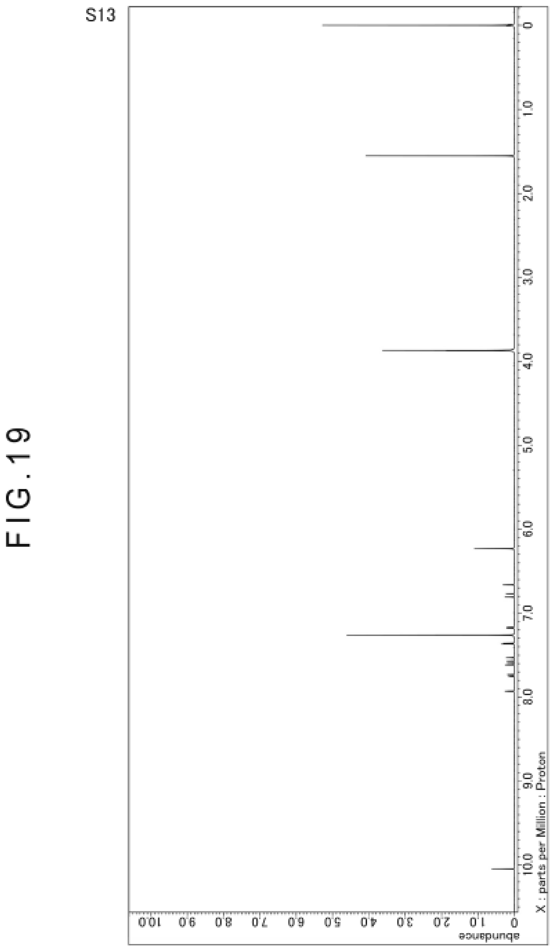

The precursor S12 (750 mg, 1.59 mmol) was dissolved in THF (15 mL) in a 100 mL side-arm eggplant-shaped flask, and while cooling to -78 degrees C under a nitrogen atmosphere, a 1.6 M n-butyllithium (nBuLi)/n-hexane solution (1.2 mL, 1.9 mmol) was added dropwise, followed by stirring for 30 minutes after completion of the addition. Thereafter, dimethylformamide (DMF) (0.6 mL, 8.0 mmol) was added dropwise, and the mixture was stirred at room temperature for 3 hours. Water was added, and the mixture was extracted with ethyl acetate, washed with saturated brine, and then dried over sodium sulfate. The solvent was removed, and the obtained residue was purified by recrystallization, followed by vacuum drying at 40 degrees C for 10 hours to obtain 576 mg (1.37 mmol, yield: 86%) of the precursor S13 as a solid powder. As a result of ¹H-NMR measurement (see Fig. 19), the obtained solid powder was confirmed to be the target precursor S13.

### 8-3. Synthesis of Compound C10

Next, a compound C10 was synthesized.

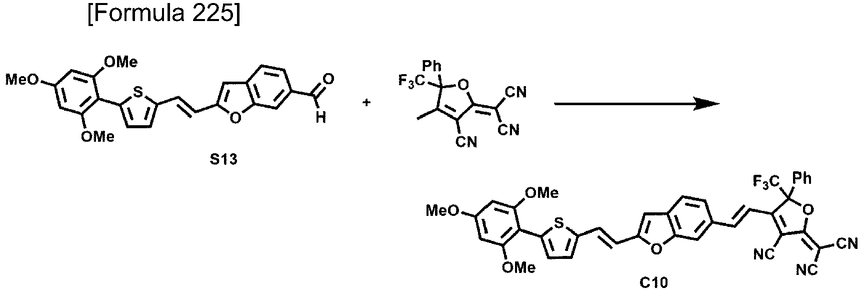

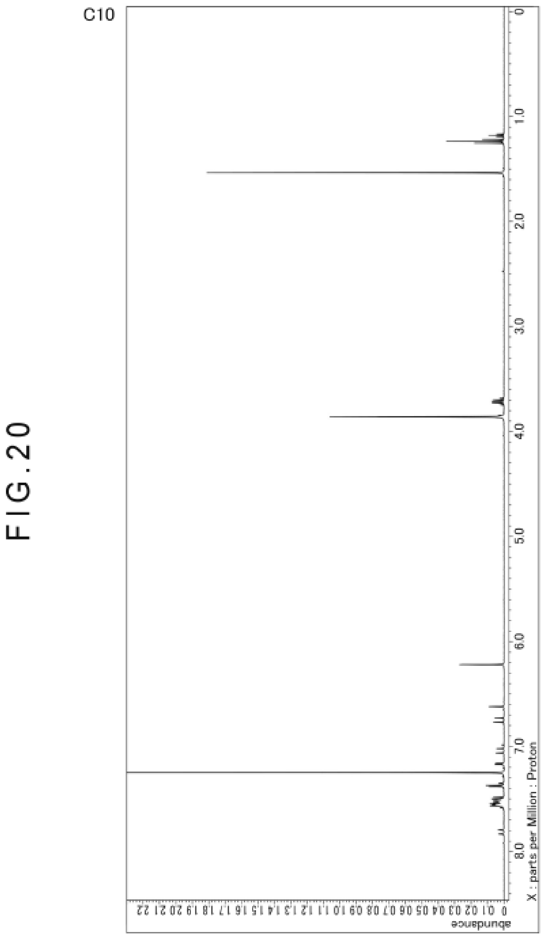

Into a 100 mL side-arm eggplant-shaped flask equipped with a Dimroth condenser were charged the precursor S13 (336 mg, 0.80 mmol) and 2-[3-cyano-4-methyl-5-phenyl-5-trifluoromethyl-2(5H)-furanylidene]propanedinitrile (303 mg, 0.96 mmol), and ethanol (16 mL) was added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 534 mg (0.75 mmol, yield: 94%) of compound C10. As a result of ¹H-NMR measurement (see Fig. 20), the obtained solid powder was confirmed to be the target compound C10.

### Example 9

### 9. Synthesis of Compound C11

A synthesis method of a compound C11 is described below.

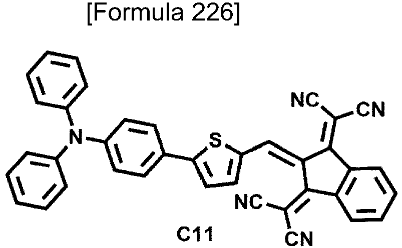

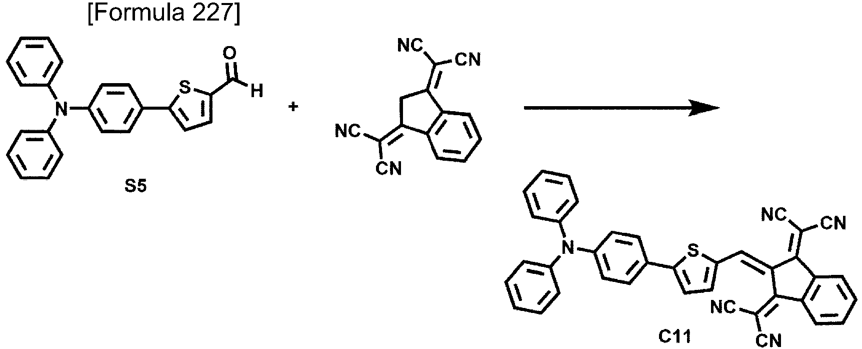

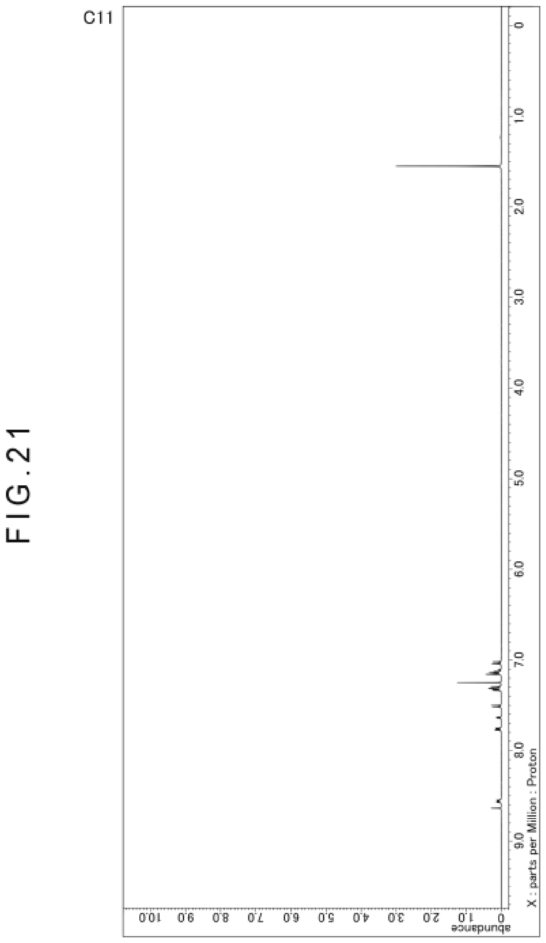

Into a 100 mL side-arm eggplant-shaped flask equipped with a Dimroth condenser were charged the precursor S5 (0.50 mmol) and 1,3-bis(dicyanomethylidene)indane (0.60 mmol), and ethanol (8.0 mL) and tetrahydrofuran (THF) (2.0 mL) were added to dissolve the mixture, followed by stirring while heating at 75 degrees C for 4.5 hours under a nitrogen atmosphere using an aluminum heat-block magnetic stirrer. After cooling the reaction mixture to room temperature, hexane was added, and the precipitated solid was separated by suction filtration. The solid was further washed with ethanol and vacuum-dried at 40 degrees C for 10 hours to obtain 141 mg (0.24 mmol, yield: 48%) of the compound C11. As a result of ¹H-NMR measurement (see Fig. 21), the obtained solid powder was confirmed to be the target compound C11.

Example 10

### 10. Synthesis of Compound C12

A synthesis method of a compound C12 is described below.

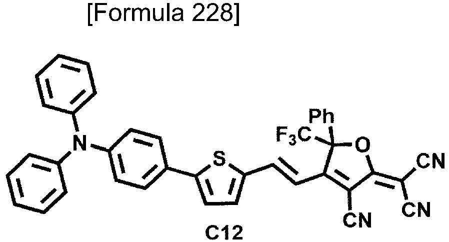

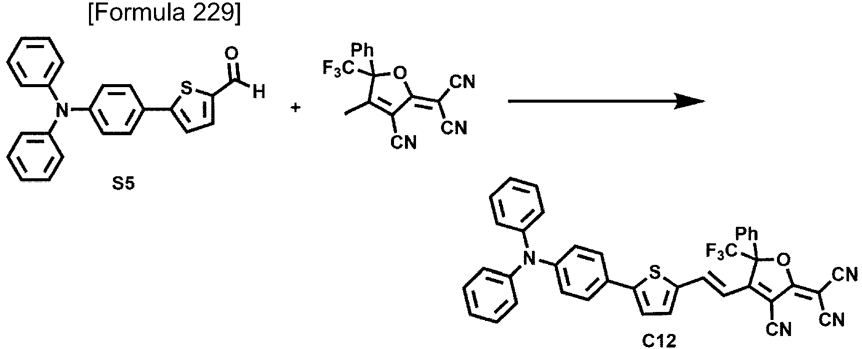

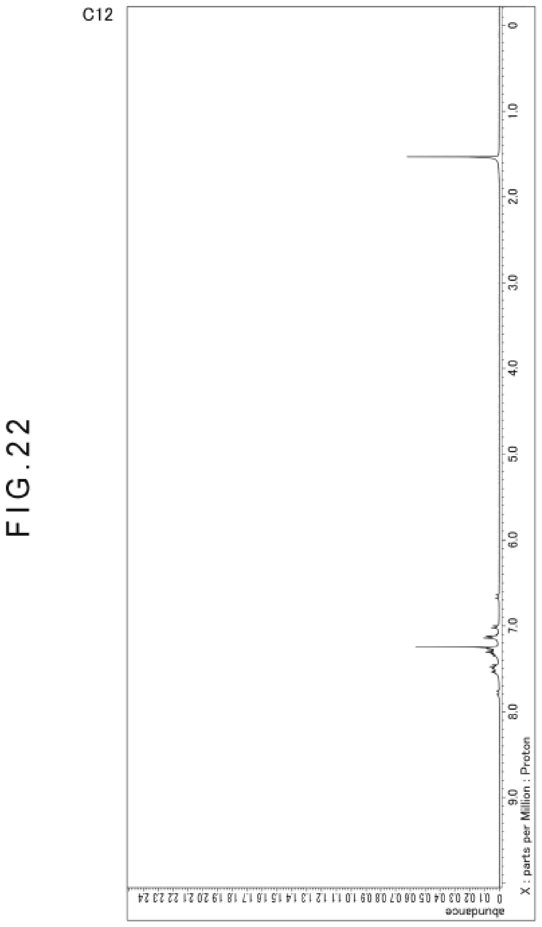

Into a 100 mL side-arm eggplant-shaped flask equipped with a Dimroth condenser were charged the precursor S5 (336 mg, 0.80 mmol) and 2-[3-cyano-4-methyl-5-phenyl-5-trifluoromethyl-2(5H)-furanylidene]propanedinitrile (303 mg, 0.96 mmol), and ethanol (16 mL) was added to dissolve the mixture, followed by stirring while heating at 75 degrees C for 3 hours under a nitrogen atmosphere using an aluminum heat-block magnetic stirrer. After cooling the reaction mixture to room temperature, hexane was added, and the precipitated solid was separated by suction filtration. The solid was further washed with hexane and vacuum-dried at 40 degrees C for 10 hours to obtain 534 mg (0.75 mmol, yield: 94%) of compound C12. As a result of ¹H-NMR measurement (see Fig. 22), the obtained solid powder was confirmed to be the target compound C12.

### Example 11

### 11. Synthesis of Compound C13

A synthesis method of a compound C13 is described below.

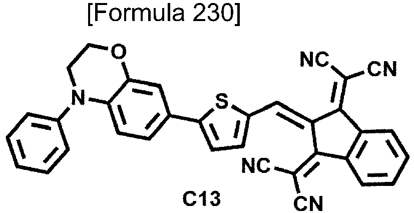

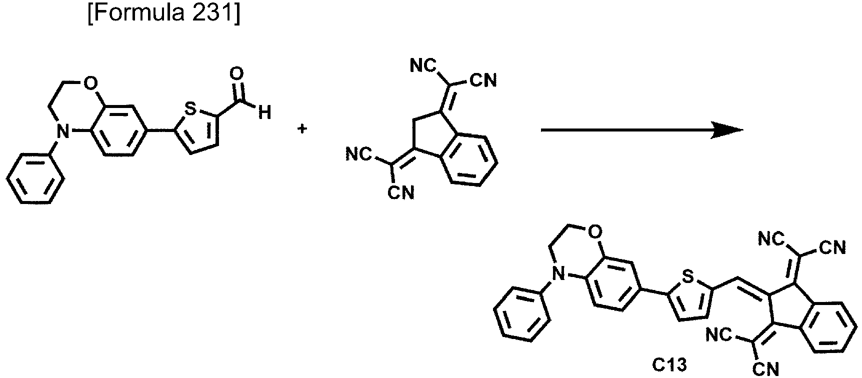

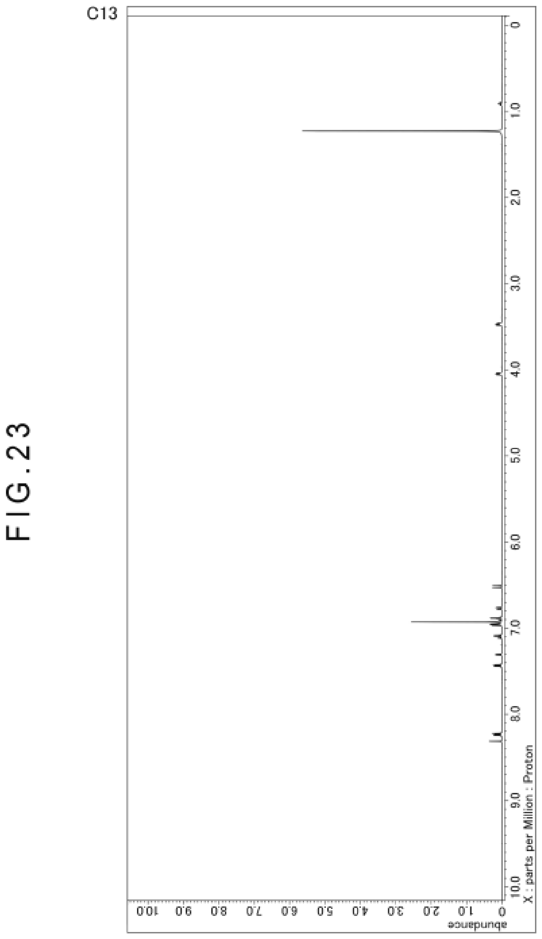

Into a 100 mL side-arm eggplant-shaped flask equipped with a Dimroth condenser were charged 5-(4-phenyl-2,3-dihydro-1,4-benzoxazin-7-yl)thiophene-2-carbaldehyde (0.50 mmol) and 1,3-bis(dicyanomethylidene)indane (0.60 mmol). Ethanol (8.0 mL) and tetrahydrofuran (THF) (2.0 mL) were added thereto to dissolve the mixture, followed by stirring while heating at 75 degrees C for 4.5 hours under a nitrogen atmosphere using an aluminum heat-block magnetic stirrer. After cooling the reaction mixture to room temperature, hexane was added, and the precipitated solid was separated by suction filtration. The solid was further washed with ethanol and vacuum-dried at 40 degrees C for 10 hours to obtain 242 mg (0.45 mmol, yield: 89%) of the compound C13. As a result of ¹H-NMR measurement (see Fig. 23), the obtained solid powder was confirmed to be the target compound C13.

### Evaluation of Compounds Synthesized in Examples

The following evaluations were performed on the compounds C1, C3, C4, and C6 to C13 synthesized in Examples 1 to 11 (hereinafter, these may be collectively referred to as the compounds C1 to C13). In addition, as a reference compound, NEO823 (Reference Example 1) manufactured by Tokyo Chemical Industry Co., Ltd. was evaluated as follows.

### Hyperpolarizability

The hyperpolarizability was calculated by quantum chemical calculations using Gaussian16, which is a quantum chemical calculation program manufactured by Gaussian, Inc., as described above.

### Preparation of Electro-optic Film

The electro-optic film was prepared in accordance with the following procedure.

### Reference Example A1

To 1.8 g of cyclopentanone (manufactured by Tokyo Chemical Industry Co., Ltd.), 0.2 g of a polycarbonate resin represented by a formula (PC-X) below (x = 55, y = 45, Mw 86,000) was added to prepare a 10 mass% polymer solution.

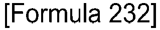

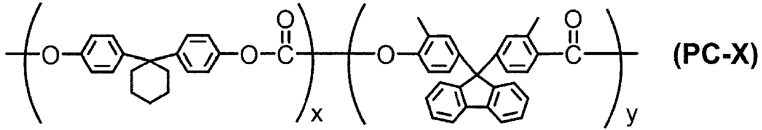

To 0.479 g of cyclopentanone (manufactured by Tokyo Chemical Industry Co., Ltd.), 0.0214 g (0.029 mmol) of NEO823 (molecular weight: 744.88, manufactured by Tokyo Chemical Industry Co., Ltd.), which is a compound as an organic chromophore, was added, and the mixture was stirred at room temperature for 2 hours to prepare a compound solution as an organic chromophore solution. To the prepared compound solution, 0.5 g of the 10 mass% polymer solution prepared above was added to prepare a solution of a composition for forming an electro-optic film in which 0.575 mmol of the compound as an organic chromophore was dissolved per 1 g of the polymer.

The solution of the composition for forming an electro-optic film prepared above was applied onto a hot plate before heating by a spin coating method (rotation speed: 1,000 rpm, 15 sec), and dried on the hot plate at 100 degrees C for 10 minutes to produce an electro-optic film of Reference Example A1.

### Examples A1 to A11

Electro-optic films of Examples A1 to A11 were produced in the same manner as in Reference Example A1, except that NEO823, a compound as an organic chromophore, was replaced with any one of the compounds C1 to C13 synthesized above. The compounds C1 to C13 are also compounds as the organic chromophore.

The structure of NEO823, which is the compound as the organic chromophore used in Reference Example A1, is represented by a formula (Ref-A1) below.

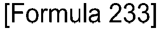

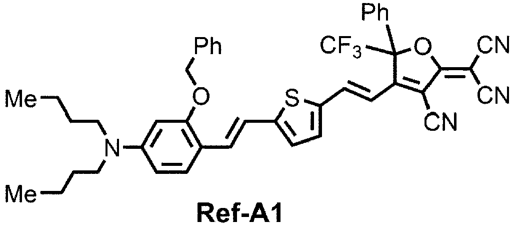

The structures of the compounds C1 to C13 used as compounds as the organic chromophore in Examples A1 to A11 are shown below.

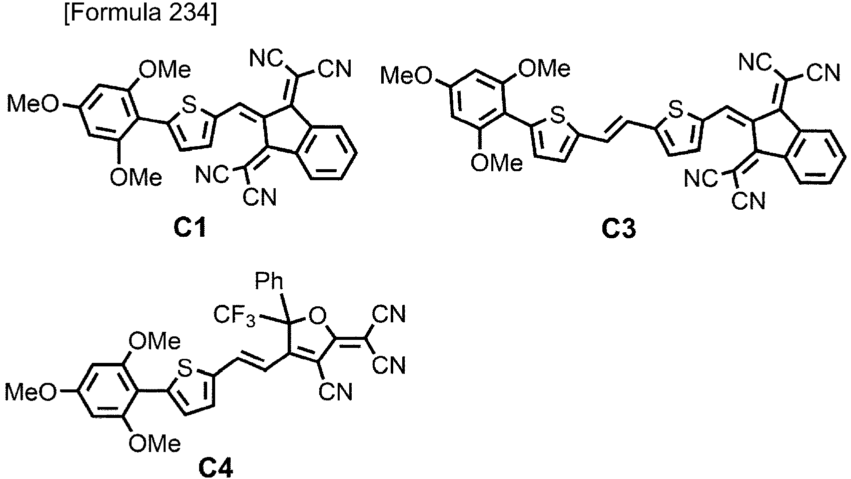

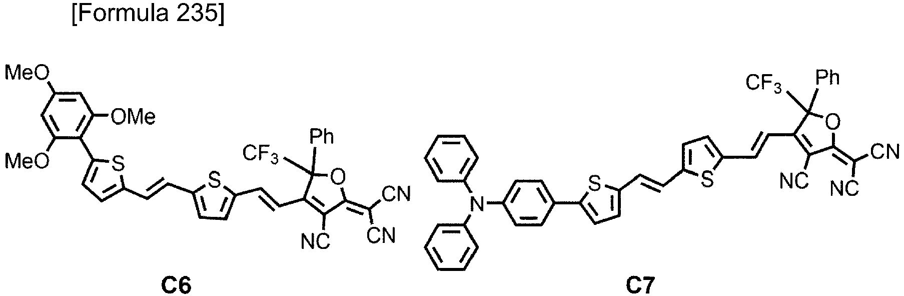

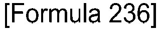

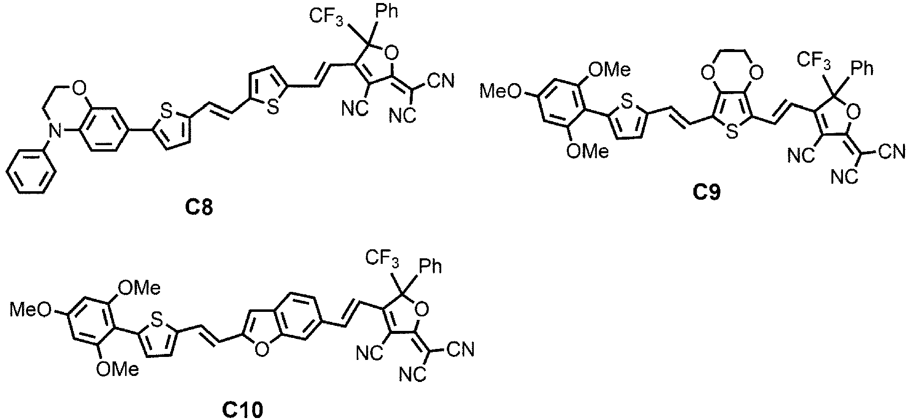

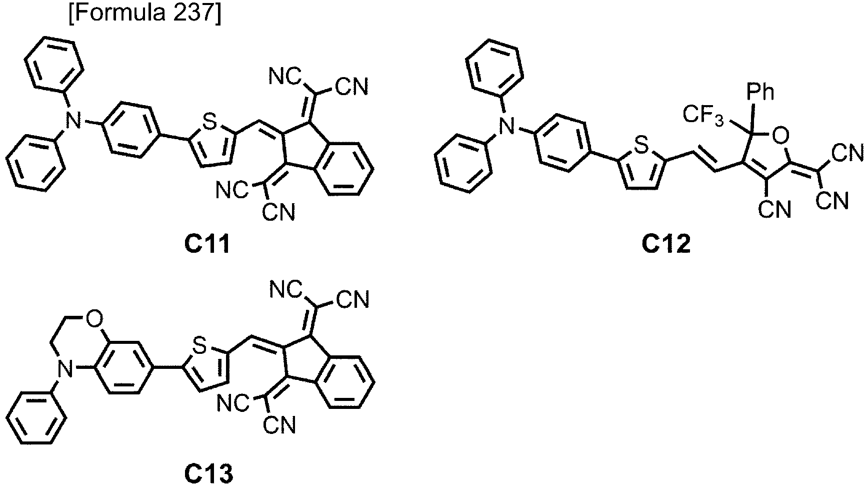

### Evaluation of Electro-optic Films

### Maximum Absorption Wavelength λmax

The absorption spectra of the electro-optic films prepared in Examples A1 to A11 and Reference Example A1 were measured using SHIMADZU UV-3600 manufactured by Shimadzu Corporation, and the maximum absorption wavelength λmax was measured.

### Light Resistance Test

Light resistance test was performed on the electro-optic films prepared in Examples A1 to A11 and Reference Example A1.

The light resistance test was carried out in the same manner as the method described in the reference (Photochemical Stability of Organic Electro-Optic Polymer at 1310-nm Wavelength, IEICE Trans. Electron., Vol. E106-C, No. 6, June 2023).

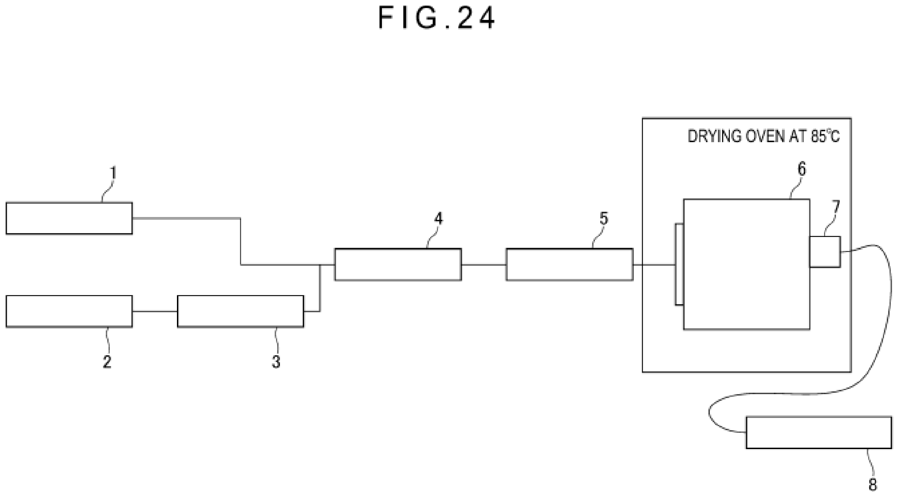

A schematic diagram of the apparatus used for the measurement is shown in Fig. 24. The reference numerals shown in Fig. 24 are as follows.
1: Laser light source for degradation (FPL1053S, laser driver CLD1015 (manufactured by THORLABS))
2: Laser light source for probe (appropriately changed depending on chromophore, laser driver CLD1010P (manufactured by THORLABS))
3: Laser light source for probe (attenuator V600F (manufactured by THORLABS) in the case of 600 nm to 780 nm, or attenuator V450F (manufactured by THORLABS) in the case of 488 nm to 600 nm)
4: WDM coupler (WD6513B-SP-JP (manufactured by THORLABS))
5: MFD conversion fiber (UHNA3) (manufactured by deltafiber.jp)
6: Integrating sphere (2P3/M)
7: Photodiode (SM05PD3A (manufactured by THORLABS))
8: Source meter (2612A (manufactured by KEITHLEY))

An FPL1053S (wavelength: 1,310 nm, 80 mW) manufactured by THORLABS was used as a laser light source for degradation testing, and an LP730-SF15 (wavelength: 730 nm) manufactured by THORLABS was used as a laser light source for probe (note that the wavelength and intensity of the laser light source for probe were appropriately changed depending on a compound as an organic chromophore to be evaluated).

A sample of the electro-optic film for light resistance test was prepared. The prepared sample for the light resistance test was continuously irradiated with a degradation laser light source (wavelength: 1,310 nm) at a laser light intensity of 80 mW at 85 degrees C. The light source was periodically switched to a laser light source for probe, and the light transmitted through the sample was measured as a current value using a photodiode and a source meter. The current value before irradiation with the degradation laser light was defined as A0, and the current value after irradiation for 120 minutes was defined as A120, and the ratio (A120/A0) of A120 to A0 was calculated.

**Table 1**

| | Compound No. | Hyperpolarizability | Maximum absorption wavelength λmax | Light resistance test |
|---|---|---|---|---|
| | | (×10⁻³⁰esu) | (nm) | (-) |
| Ex. A1 | C1 | 157 | 620 | - |
| Ex. A2 | C3 | 856 | - | - |
| Ex. A3 | C4 | 262 | 622 | - |
| Ex. A4 | C6 | 1092 | 696 | 2.6 |
| Ex. A5 | C7 | 1233 | 692 | 3.3 |
| Ex. A6 | C8 | 1234 | 722 | - |
| Ex. A7 | C9 | 969 | - | - |
| Ex. A8 | C10 | 953 | - | - |
| Ex. A9 | C11 | 389 | - | - |
| Ex. A10 | C12 | 573 | - | - |
| Ex. A11 | C13 | 362 | - | - |
| Ref. Ex. A1 | NEO823 | 863 | 812 | 6.0 |

The compounds C1, C3, C4, and C6 to C13 synthesized in Examples 1 to 11 all exhibited hyperpolarizability and maximum absorption wavelengths applicable to electro-optic materials. A compound having hyperpolarizability not less than 100 × 10⁻³⁰ esu can be suitably applied as an electro-optic material. Further, the electro-optic films of Examples A1 to A11 prepared using the compounds C1 to C13 synthesized in Examples 1 to 11 exhibited excellent light resistance. The results of the light resistance test indicate that a smaller numerical value represents better light resistance. Since the results of the light resistance test are considered to depend on the compounds C1 to C13, the electro-optic device including the electro-optic film using the compounds C1 to C13 is expected to exhibit excellent light resistance. For example, when the electro-optic device is a device applied to optical communication technology, the compound contained in the device is required to have stability to light from the viewpoint of communication stability and the like. Therefore, the electro-optic device including the electro-optic film using the compounds C1 to C13 can be suitably applied as a device applied to optical communication technology. The numbers of Examples 1 to 11 and the numbers of Examples A1 to A11 correspond to each other.

### Supplementary Notes

The gist of the invention may also encompass the following configurations.

[Configuration 1-1] A compound having a donor moiety, a π-conjugated moiety, and an acceptor moiety, in which the donor moiety is represented by a formula (D1) or a formula (D2) below.

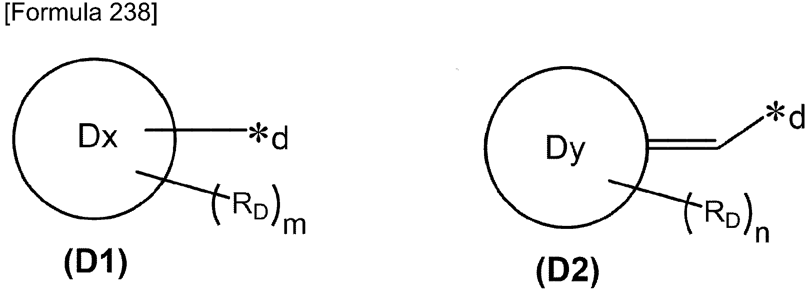

In the formulae (D1) and (D2):
a ring Dx and a ring Dy are each independently a five-membered or six-membered aromatic hydrocarbon ring, a five-membered or six-membered aliphatic hydrocarbon ring, or a five-membered or six-membered heterocycle;
the ring Dx has one or more substituents R_{D};
the ring Dy has one or more substituents R_{D} or has no substituent R_{D};
m is the number of the substituents R_{D} on the ring Dx and is an integer of 1 or more;
n is the number of the substituents R_{D} on the ring Dy and is 0 or an integer of 1 or more;
the substituents R_{D} on the ring Dx are bonded with the ring Dx to form a substituted or unsubstituted monocyclic ring, bonded with the ring Dx to form a substituted or unsubstituted fused ring, or not bonded with the ring Dx;
the substituents R_{D} on the ring Dy are bonded with the ring Dy to form a substituted or unsubstituted monocyclic ring, bonded with the ring Dy to form a substituted or unsubstituted fused ring, or not bonded with the ring Dy;
when the ring Dx has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
when the ring Dy has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
the substituents R_{D} on the ring Dx and the ring Dy are each independently a group represented by -N(R₁₀₁)(R₁₀₂), a group represented by -Si(R₁₀₃)(R₁₀₄)(R₁₀₅), a group represented by -C(=O)R₁₀₆, a substituted or unsubstituted alkyl group, a substituted or unsubstituted cycloalkyl group, a substituted or unsubstituted aryl group, or a substituted or unsubstituted heterocyclic group;
R₁₀₁, R₁₀₂, R₁₀₃, R₁₀₄, R₁₀₅, and R₁₀₆ are each independently a hydrogen atom, a substituted or unsubstituted alkyl group, a substituted or unsubstituted cycloalkyl group, a substituted or unsubstituted aryl group, or a substituted or unsubstituted heterocyclic group;
when the number of substituents R_{D} on the ring Dx and the ring Dy is 1 and the one substituent R_{D} is a group represented by -N(R₁₀₁)(R₁₀₂), R₁₀₁ and R₁₀₂ are neither hydrogen atoms nor substituted or unsubstituted alkyl groups;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different; and
*d is a bonding position.

[Configuration 1-2] The compound according to [Configuration 1-1], in which the rings Dx and Dy in the formula (D1) or the formula (D2) are each independently a five-membered or six-membered aromatic hydrocarbon ring, or a five-membered or six-membered heterocycle.

[Configuration 1-3] The compound according to [Configuration 1-1] or [Configuration 1-2], in which the donor moiety is represented by a formula (D11), (D12), (D13), (D21), or (D22) below.

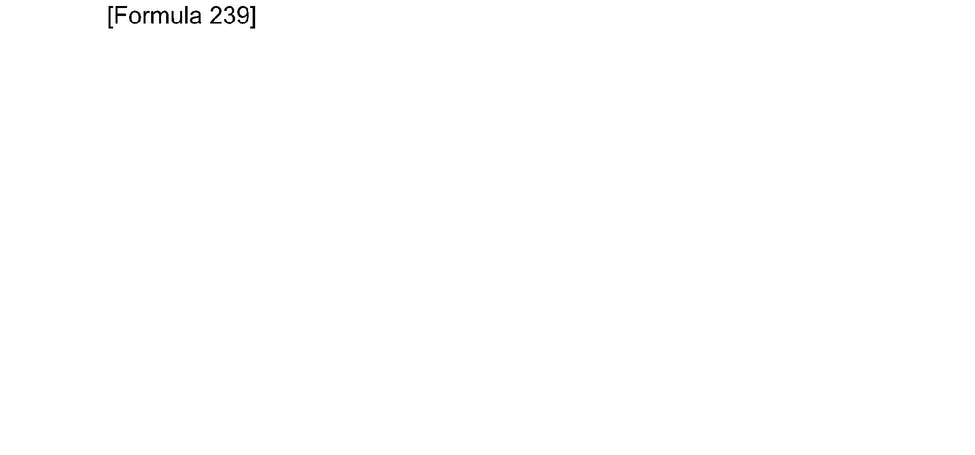

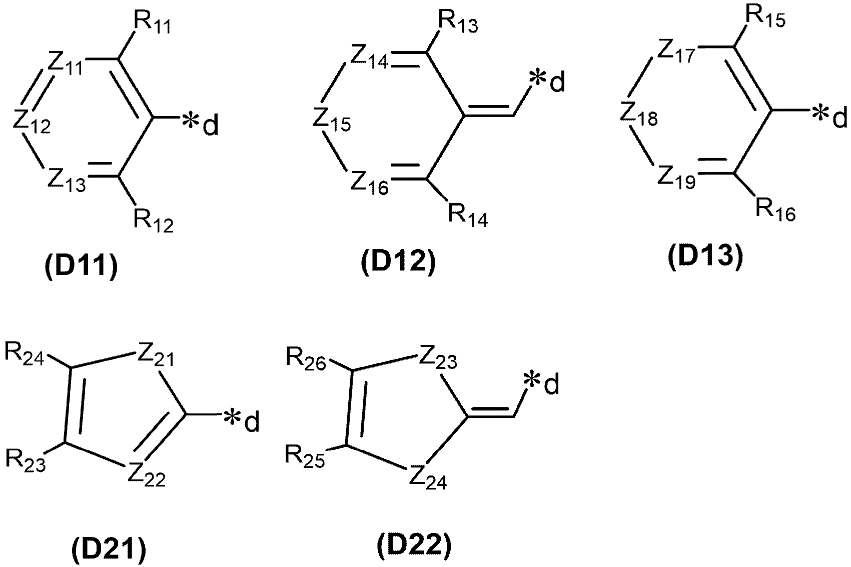

In the groups represented by the formulae (D11), (D12), (D13), (D21), and (D22):
Z₁₁, Z₁₂, Z₁₃, Z₁₄, Z₁₆, and Z₁₉ are each independently c(R₁₀₀);
Z₁₅, Z₁₇, and Z₁₈ are each independently an oxygen atom, a sulfur atom, C=R₁₀₀, N(R₁₁₀), or C(R₁₁₁)(R₁₁₂);
Z₂₁, Z₂₃, and Z₂₄ are each independently an oxygen atom, a sulfur atom, N(R₁₁₀), or C(R₁₁₁)(R₁₁₂);
Z₂₂ is c(R₁₀₀);
at least one combination of adjacent two or more of R₁₁, R₁₂, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₃, R₁₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₅, R₁₆, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₃, R₂₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₅, R₂₆, R₁₁₀, R₁₁₁, and R₁₁₂ are mutually bonded to form a substituted or unsubstituted monocyclic ring, mutually bonded to form a substituted or unsubstituted fused ring, or not mutually bonded;
R₁₁ to R₁₆, R₂₃ to R₂₆, R₁₀₀, and R₁₁₀ to R₁₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), or a group represented by -C(R₁₀₇)(R₁₀₈)(R₁₀₉);
R₁₀₁ to R₁₀₅ respectively represent the same as R₁₀₁ to R₁₀₅ in the formula (D1);
R₁₀₇, R₁₀₈, and R₁₀₉ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different; and
*d is a bonding position.

[Configuration 1-4] The compound according to any one of [Configuration 1-1] to [Configuration 1-3], in which the donor moiety is a compound represented by a formula (D111), (D112), (D113), (D121), (D122), (D123), or (D124) below.

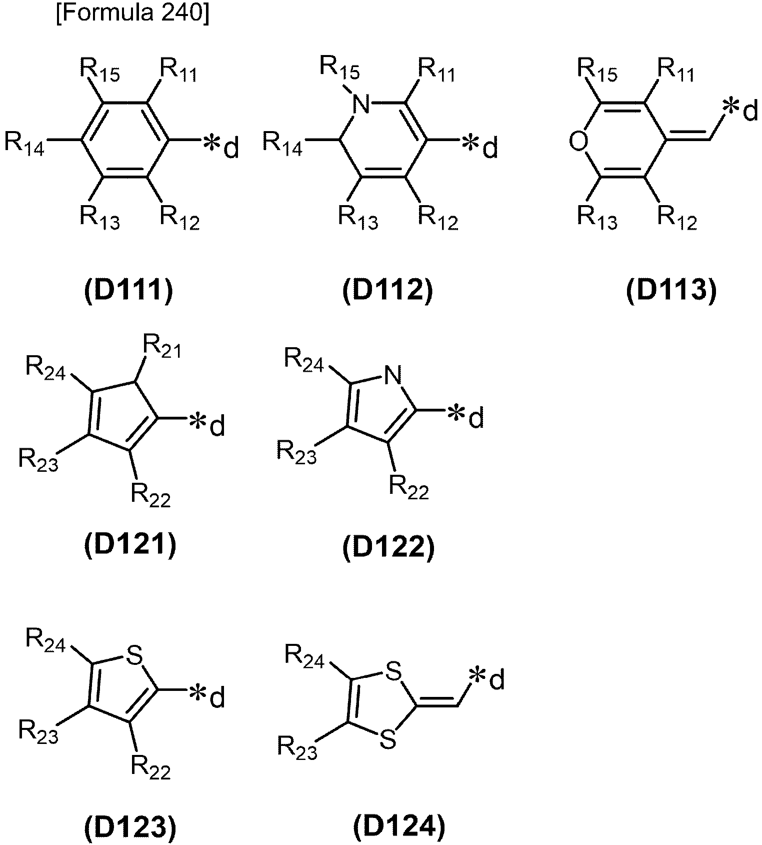

In the groups represented by the formulae (D111), (D112), (D113), (D121), (D122), (D123), and (D124):
at least one combination of adjacent two or more of R₁₁ to R₁₅ are mutually bonded to form a substituted or unsubstituted monocyclic ring, mutually bonded to form a substituted or unsubstituted fused ring, or not mutually bonded;
at least one combination of adjacent two or more of R₂₁ to R₂₄ are mutually bonded to form a substituted or unsubstituted monocyclic ring, mutually bonded to form a substituted or unsubstituted fused ring, or not mutually bonded;
R₁₁ to R₁₅ and R₂₁ to R₂₄ forming neither the substituted or unsubstituted monocyclic ring nor the substituted or unsubstituted fused ring are each independently a hydrogen atom, a group represented by -N(R₁₀₁)(R₁₀₂), a group represented by -Si(R₁₀₃)(R₁₀₄)(R₁₀₅), a group represented by C(=O)R₁₀₆, a substituted or unsubstituted alkyl group, a substituted or unsubstituted cycloalkyl group, a substituted or unsubstituted aryl group, or a substituted or unsubstituted heterocyclic group; and
*d represents a bonding position.

[Configuration 1-5] The compound according to any one of [Configuration 1-1] to [Configuration 1-4], in which the donor moiety is represented by a formula (D111-1A), (D111-1B), (D111-1C), (D111-1D), (D111-1E), (D111-1F), (D111-1G), or (D111-1H) below.

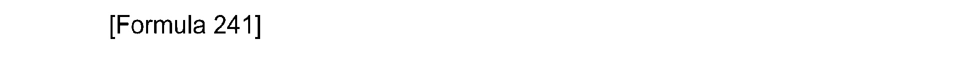

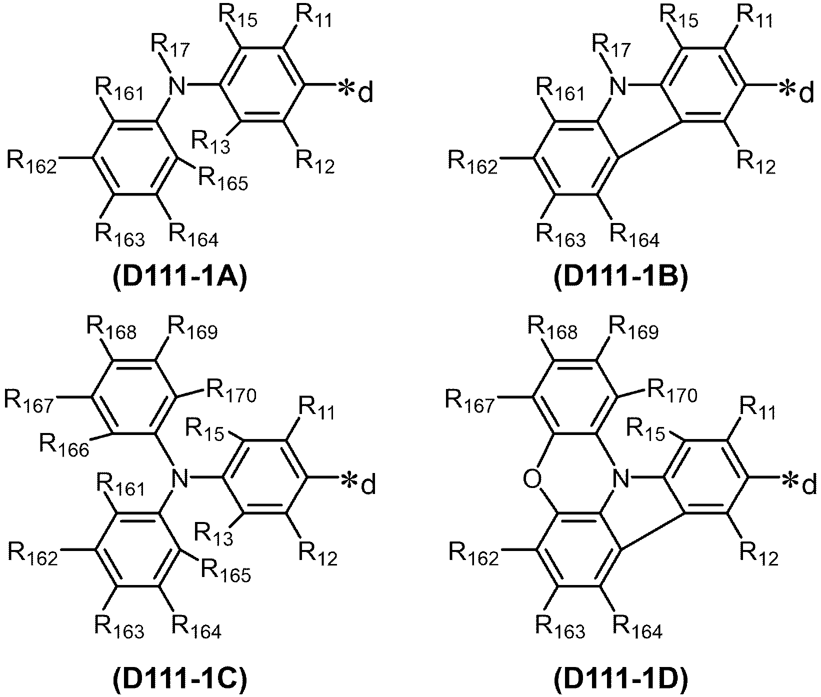

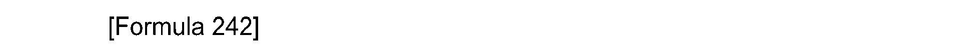

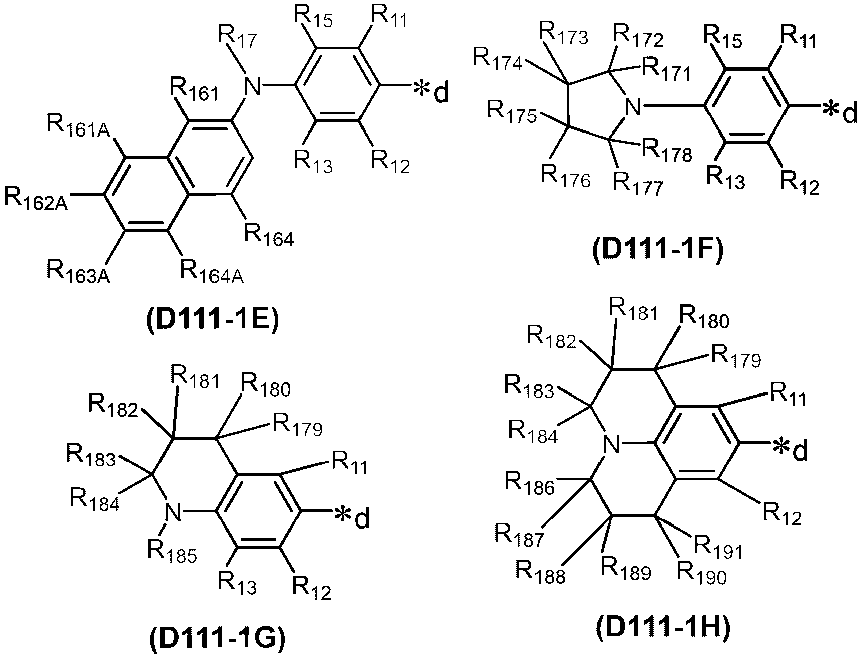

In the formulae (D111-1A), (D111-1B), (D111-1C), (D111-1D), (D111-1E), (D111-1F), (D111-1G), and (D111-1H):
R₁₁ to R₁₃, R₁₅, R₁₇, R₁₆₁ to R₁₉₁, R_{161A} to R_{164A} are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃);
R₁₁₃ is a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₁₃ are present, the plurality of R₁₁₃ are mutually the same or different; and
*d represents a bonding position.

[Configuration 1-6] The compound according to any one of [Configuration 1-1] to [Configuration 1-5], in which the donor moiety is represented by a formula (D122-1), (D123-1A), or (D123-1B) below.

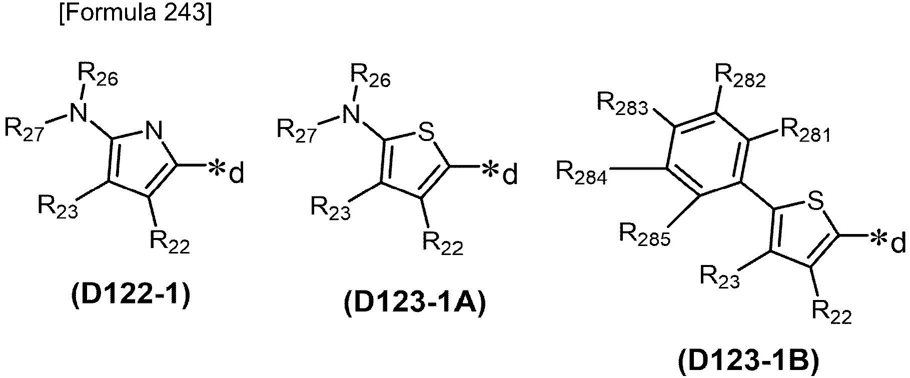

In the formulae (D122-1), (D123-1A), and (D123-1B):
R₂₂, R₂₃, R₂₆, R₂₇, and R₂₈₁ to R₂₈₅ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

[Configuration 1-7] The compound according to any one of [Configuration 1-1] to [Configuration 1-6], in which the donor moiety is represented by a formula (D123-2A), (D123-3A), or (D123-4A) below.

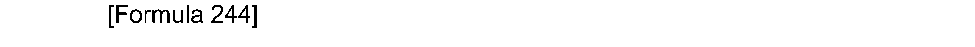

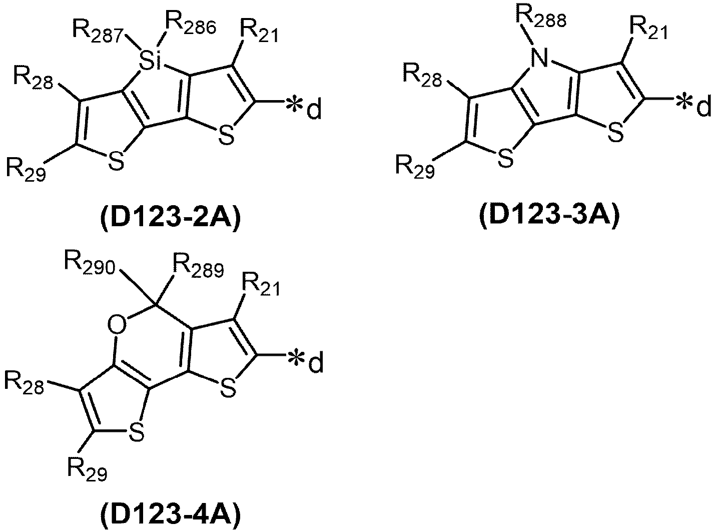

In the formulae (D123-2A), (D123-3A), and (D123-4A):
R₂₁, R₂₈, R₂₉, R₂₈₆, R₂₈₇, R₂₈₈, R₂₈₉, and R₂₉₀ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

[Configuration 1-8] The compound according to any one of [Configuration 1-1] to [Configuration 1-7], in which the donor moiety is represented by a formula (D112-1) or (D121-1) below.

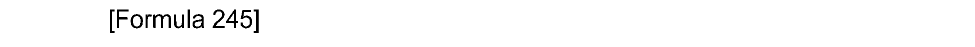

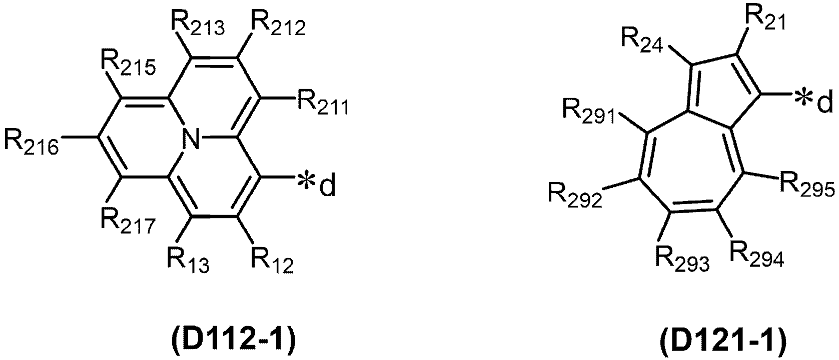

In the formulae (D112-1) and (D121-1):
R₁₂, R₁₃, R₂₁, R₂₄, R₂₁₁ to R₂₁₇, and R₂₉₁ to R₂₉₅ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

[Configuration 1-9] The compound according to any one of [Configuration 1-1] to [Configuration 1-8], represented by a formula (1) below.
[Formula 246]

D-L-A **(1)**

In the formula (1), D is the donor moiety, L is the π-conjugated moiety, and A is the acceptor moiety.

[Configuration 1-10] The compound according to any one of [Configuration 1-1] to [Configuration 1-9], in which the donor moiety is a group having electron-donating properties, and the acceptor moiety is a group having electron-withdrawing properties.

[Configuration 1-11] An electro-optic material containing an electro-optic compound and a host material, in which the electro-optic compound is the compound according to any one of [Configuration 1-1] to [Configuration 1-10].

[Configuration 1-12] The electro-optic material according to [Configuration 1-11], in which the host material is a polymer, and the electro-optic compound is bonded to the polymer as the host material.

[Configuration 1-13] The electro-optic material according to [Configuration 1-11], in which the host material is a polymer, and the electro-optic compound is not bonded to the polymer and is dispersed in the host material.

[Configuration 1-13] An electro-optic film containing the compound according to any one of [Configuration 1-1] to [Configuration 1-10].

[Configuration 1-15] An electro-optic film containing the electro-optic material according to any one of [Configuration 1-11] to [Configuration 1-13].

[Configuration 1-16] An electro-optic device including the compound according to any one of [Configuration 1-1] to [Configuration 1-10].

[Configuration 1-17] An electro-optic device including the electro-optic material according to any one of [Configuration 1-11] to [Configuration 1-13].

[Configuration 1-18] An electro-optic device including the electro-optic film according to any one of [Configuration 1-14] or [Configuration 1-15].

The gist of the invention may also encompass the following configurations.

[Configuration 2-1] A compound having a donor moiety, a π-conjugated moiety, and an acceptor moiety, in which the acceptor moiety is represented by a formula (A1) or a formula (A2) below.

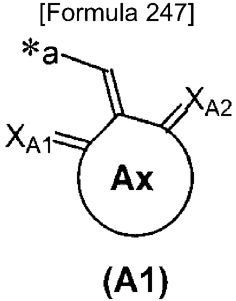

In the formula (A1):
X_{A1} and X_{A2} are each independently an oxygen atom (O) or C(CN)₂;
when one of X_{A1} and X_{A2} is O and the other of X_{A1} and X_{A2} is C(CN)₂, the ring to which X_{A1} and X_{A2} are directly bonded is not a five-membered ring;
the ring Ax is a substituted or unsubstituted monocyclic aromatic hydrocarbon ring, a substituted or unsubstituted polycyclic aromatic hydrocarbon ring, a substituted or unsubstituted monocyclic aliphatic hydrocarbon ring, a substituted or unsubstituted polycyclic aliphatic hydrocarbon ring, a substituted or unsubstituted monocyclic heterocycle, or a substituted or unsubstituted polycyclic heterocycle; and
*a represents a bonding position.

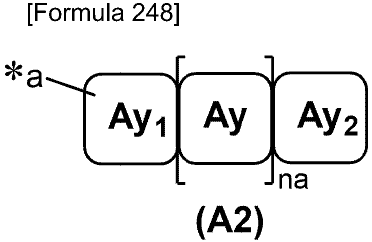

In the formula (A2):
na represents the number of rings Ay, and na is 0 or an integer of 1 or more;
the rings Ay, Ay₁, and Ay₂ are each independently a substituted or unsubstituted monocyclic aromatic hydrocarbon ring, a substituted or unsubstituted monocyclic aliphatic hydrocarbon ring, or a substituted or unsubstituted monocyclic heterocycle;
when na is 0, the ring Ay₁ and the ring Ay₂ are fused to each other;
when na is an integer of 1 or more, the ring Ay, the ring Ay₁, and the ring Ay₂ are each independently fused to an adjacent ring;
when na is an integer of 2 or more, the ring Ay₁, the ring Ay₂, and two or more rings Ay include a fused ring structure in which the rings are fused in series to adjacent rings or a fused ring structure in which the rings are fused in a branched manner to adjacent rings; and
*a represents a bonding position.

[Configuration 2-2] The compound according to [Configuration 2-1], in which the acceptor moiety is represented by a formula (A11) or (A12) below.

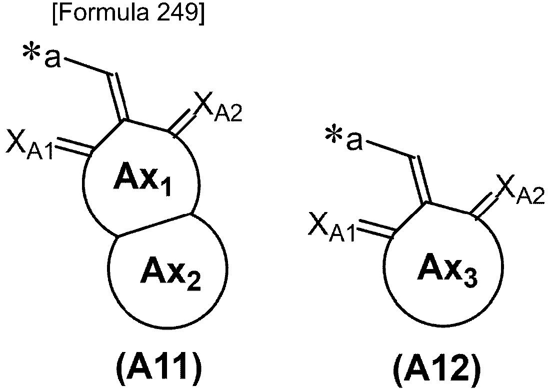

In the formulae (A11) and (A12):
X_{A1} and X_{A2} respectively represent the same as X_{A1} and X_{A2} in the formula (A1);
the rings Ax₁, Ax₂, and Ax₃ are each independently a substituted or unsubstituted monocyclic aromatic hydrocarbon ring, a substituted or unsubstituted monocyclic aliphatic hydrocarbon ring, or a substituted or unsubstituted monocyclic heterocycle; and
*a represents a bonding position.

[Configuration 2-3] The compound according to [Configuration 2-1] or [Configuration 2-2], in which the acceptor moiety is represented by a formula (A11-1), (A11-2), or (A12-1) below.

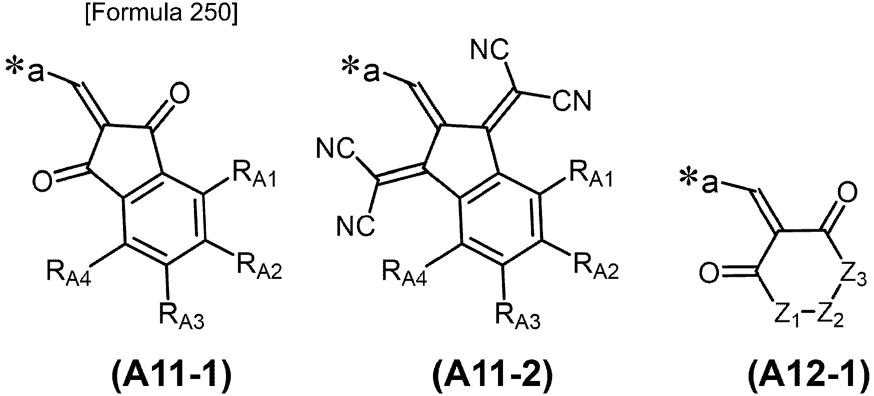

In the formulae (A11-1) and (A11-2):
R_{A1}, R_{A2}, R_{A3}, and R_{A4} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx;
in the formula (A12-1):
   Z₁, Z₂, and Z₃ are each independently N(R_{A5}), C=O, or C(R_{A6})(R_{A7});
   R_{A5}, R_{A6}, and R_{A7} are each independently a hydrogen atom, a halogen atom, a cyano group, a substituted or unsubstituted alkyl group, a substituted or unsubstituted aryl group, -NO₂, -CHO, -CORx, -COORx, or -SO₂Rx;
   Rx is a substituted or unsubstituted alkyl group, and when a plurality of Rx are present, the plurality of Rx are mutually the same or different; and
   *a represents a bonding position.

[Configuration 2-4] The compound according to any one of [Configuration 2-1] to [Configuration 2-3], in which the acceptor moiety is represented by a formula (A111-1), (A111-2), (A112-1), or (A121-1) below.

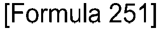

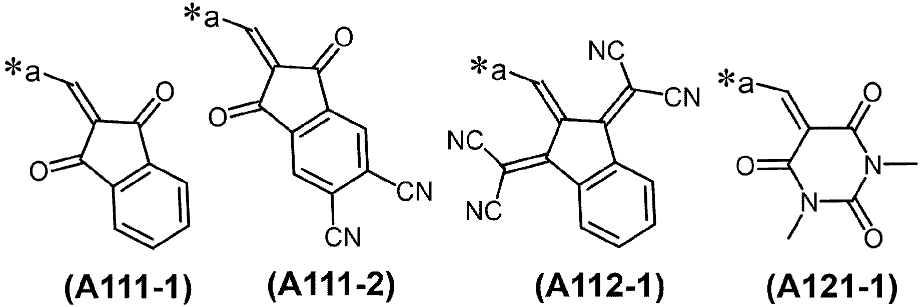

In the formulae (A111-1), (A111-2), (A112-1), and (A121-1), *a represents a bonding position.

[Configuration 2-5] The compound according to [Configuration 2-4], in which na in the formula (A2) is 1 or 2.

[Configuration 2-6] The compound according to [Configuration 2-1] or [Configuration 2-5], in which the acceptor moiety is represented by a formula (A21) or (A22) below.

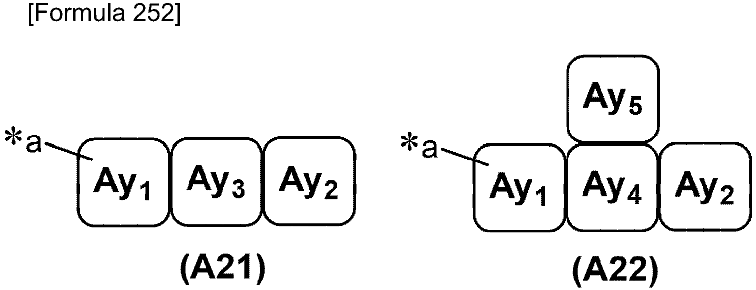

In the formula (A21):
the ring Ay₁ and the ring Ay₂ represent the same as the ring Ay₁ and the ring Ay₂ in the formula (A2), respectively; and
the ring Ay₃ represents the same as the ring Ay in the formula (A2),
in the formula (A22):
   the ring Ay₁ and the ring Ay₂ represent the same as the ring Ay₁ and the ring Ay₂ in the formula (A2), respectively; and
   the ring Ay₄ and the ring Ay₅ each independently represent the same as the ring Ay in the formula (A2), and
   *a represents a bonding position.

[Configuration 2-7] The compound according to any one of [Configuration 2-1], [Configuration 2-5], and [Configuration 2-6], in which the ring Ay₁ and the ring Ay₂ are each independently a nitrogen-containing ring.

[Configuration 2-8] The compound according to any one of [Configuration 2-1], [Configuration 2-5], [Configuration 2-6], and [Configuration 2-7], in which the ring Ay₁ and the ring Ay₂ are each independently a nitrogen-containing six-membered ring.

[Configuration 2-9] The compound according to any one of [Configuration 2-1], [Configuration 2-5], [Configuration 2-6], [Configuration 2-7], and [Configuration 2-8], in which the acceptor moiety is represented by a formula (A21-1) or (A22-1) below.

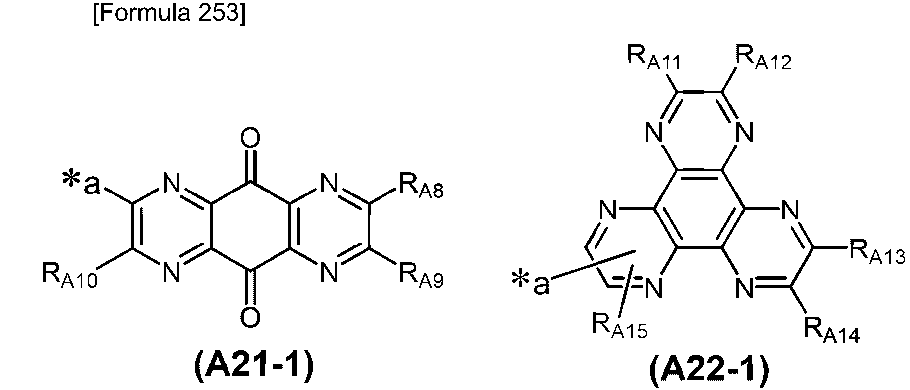

In the formulae (A21-1) and (A22-1):
R_{A8}, R_{A9}, R_{A10}, R_{A11}, R_{A12}, R_{A13}, R_{A14}, and R_{A15} are each independently a hydrogen atom, a halogen atom, a cyano group, a substituted or unsubstituted alkyl group, a substituted or unsubstituted aryl group, -NO₂, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

[Configuration 2-10] The compound according to any one of [Configuration 2-1], [Configuration 2-5], [Configuration 2-6], [Configuration 2-7], [Configuration 2-8], and [Configuration 2-9], in which the acceptor moiety is represented by a formula (A211-1) or (A221-1) below.

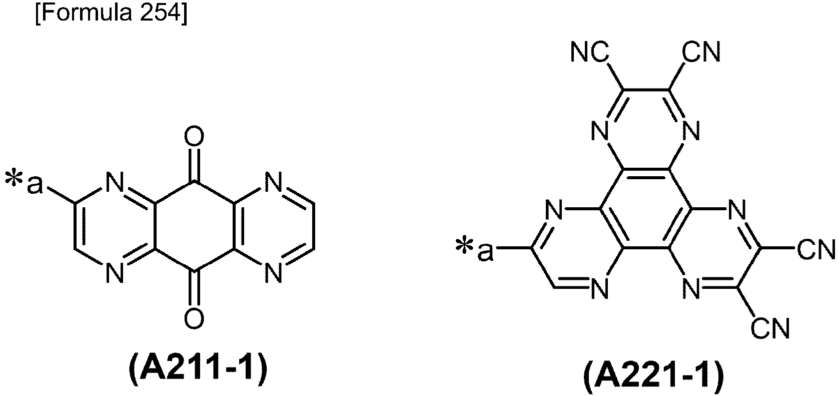

In the formulae (A211-1) and (A221-1), *a represents a bonding position.

[Configuration 2-11] The compound according to any one of [Configuration 2-1], [Configuration 2-5], and [Configuration 2-6], in which the acceptor moiety is represented by a formula (A21-2) below.

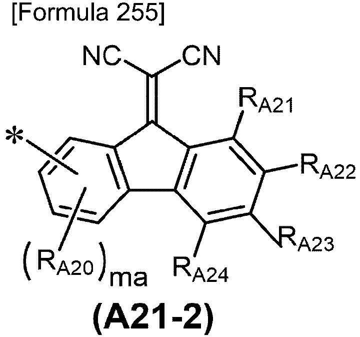

In the formula (A21-2):
ma represents the number of R_{A20} bonded to the ring, ma is 3, and the three R_{A20} are mutually the same or different;
R_{A20}, R_{A21}, R_{A22}, R_{A23}, and R_{A24} are each independently a hydrogen atom, a halogen atom, a cyano group, a substituted or unsubstituted alkyl group, a substituted or unsubstituted aryl group, -NO₂, -CHO, -CORx, -COOR_{X}, or -SO₂Rx;
Rx is a substituted or unsubstituted alkyl group, and when a plurality of Rx are present, the plurality of Rx are mutually the same or different; and
*a represents a bonding position.

[Configuration 2-12] The compound according to any one of [Configuration 2-1], [Configuration 2-5], [Configuration 2-6], and [Configuration 2-11], in which the acceptor moiety is represented by a formula (A212-1) below.

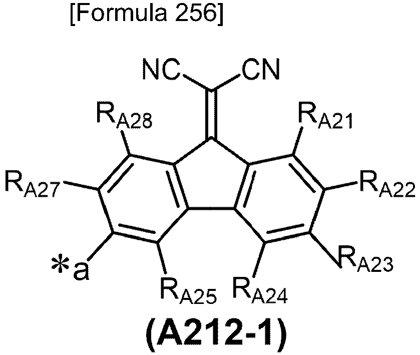

In the formula (A212-1):
R_{A21}, R_{A22}, R_{A23}, R_{A24}, R_{A25}, R_{A27}, and R_{A28} are each independently a hydrogen atom, a halogen atom, a cyano group, a substituted or unsubstituted alkyl group, a substituted or unsubstituted aryl group, -NO₂, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

[Configuration 2-13] The compound according to any one of [Configuration 2-1] to [Configuration 2-12], in which the acceptor moiety has at least one structure selected from the group consisting of a nitrogen-containing ring, CN, C=O, and C=C(CN)₂.

[Configuration 2-14] The compound according to any one of [Configuration 2-1] to [Configuration 2-13], represented by a formula (1) below.
[Formula 257]

D-L-A **(1)**

In the formula (1), D is the donor moiety, L is the π-conjugated moiety, and A is the acceptor moiety.

[Configuration 2-15] The compound according to any one of [Configuration 2-1] to [Configuration 2-14], in which the donor moiety is a group having electron-donating properties, and the acceptor moiety is a group having electron-withdrawing properties.

[Configuration 2-16] An electro-optic material containing an electro-optic compound and a host material, in which the electro-optic compound is the compound according to any one of [Configuration 2-1] to [Configuration 2-15].

[Configuration 2-17] The electro-optic material according to [Configuration 2-16], in which the host material is a polymer, and the electro-optic compound is bonded to the polymer as the host material.

[Configuration 2-18] The electro-optic material according to [Configuration 2-16], in which the host material is a polymer, and the electro-optic compound is not bonded to the polymer and is dispersed in the host material.

[Configuration 2-19] An electro-optic film containing the compound according to any one of [Configuration 2-1] to [Configuration 2-15].

[Configuration 2-20] An electro-optic film containing the electro-optic material according to any one of [Configuration 2-16] to [Configuration 2-18].

[Configuration 2-21] An electro-optic device including the compound according to any one of [Configuration 2-1] to [Configuration 2-15].

[Configuration 2-22] An electro-optic device including the electro-optic material according to any one of [Configuration 2-16] to [Configuration 2-18].

[Configuration 2-23] An electro-optic device including the electro-optic film according to any one of [Configuration 2-19] or [Configuration 2-20].

The gist of the invention may also encompass the following configurations.

[Configuration 3-1] A compound having a donor moiety, a π-conjugated moiety, and an acceptor moiety, in which the π-conjugated moiety is represented by a formula (P1), (P2), or (P3) below.

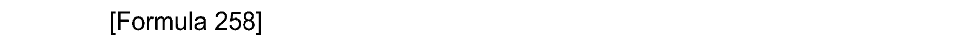

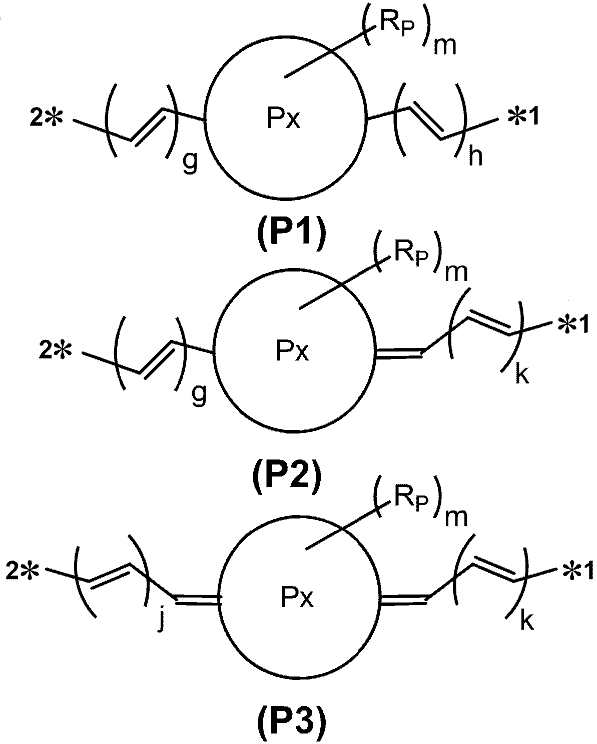

In the formulae (P1), (P2), and (P3):
the ring Px is a monocyclic aromatic hydrocarbon ring having one or more substituents R_{P}, a substituted or unsubstituted polycyclic aromatic hydrocarbon ring, a monocyclic aliphatic hydrocarbon ring having one or more substituents R_{P}, a substituted or unsubstituted polycyclic aliphatic hydrocarbon ring, a substituted or unsubstituted monocyclic heterocycle, or a substituted or unsubstituted polycyclic heterocycle;
m is the number of substituents R_{P} and is 0 or an integer of 1 or more;
a substituent R_{P} is a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
when the ring Px is a monocyclic aromatic hydrocarbon ring, a monocyclic aliphatic hydrocarbon ring, or a monocyclic heterocycle having one or more substituents R_{P}, at least one of the substituents R_{P} is a group represented by -O-R_{P1}, and R_{P1} is a substituted or unsubstituted alkyl group or a substituted or unsubstituted aryl group;
when the ring Px is a naphthalene ring, the naphthalene ring as the ring Px has at least one group represented by -O-R_{P1}, and R_{P1} is a substituted or unsubstituted alkyl group or a substituted or unsubstituted aryl group;
when the ring Px is a monocyclic heterocycle having no substituent R_{P}, the monocyclic heterocycle contains two or more heteroatoms as ring atoms;
when the ring Px is a polycyclic heterocycle, at least one ring contains one or more heteroatoms as ring atoms;
g, h, j, and k are each independently 0, 1, 2, or 3; and
*1 and *2 each represent a bonding position.

[Configuration 3-2] The compound according to [Configuration 3-1], in which the π-conjugated moiety is represented by a formula (P11-1), (P12-1), (P13-1), (P14-1), or (P15-1) below.

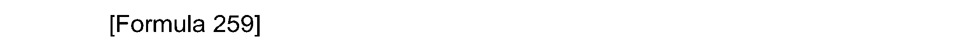

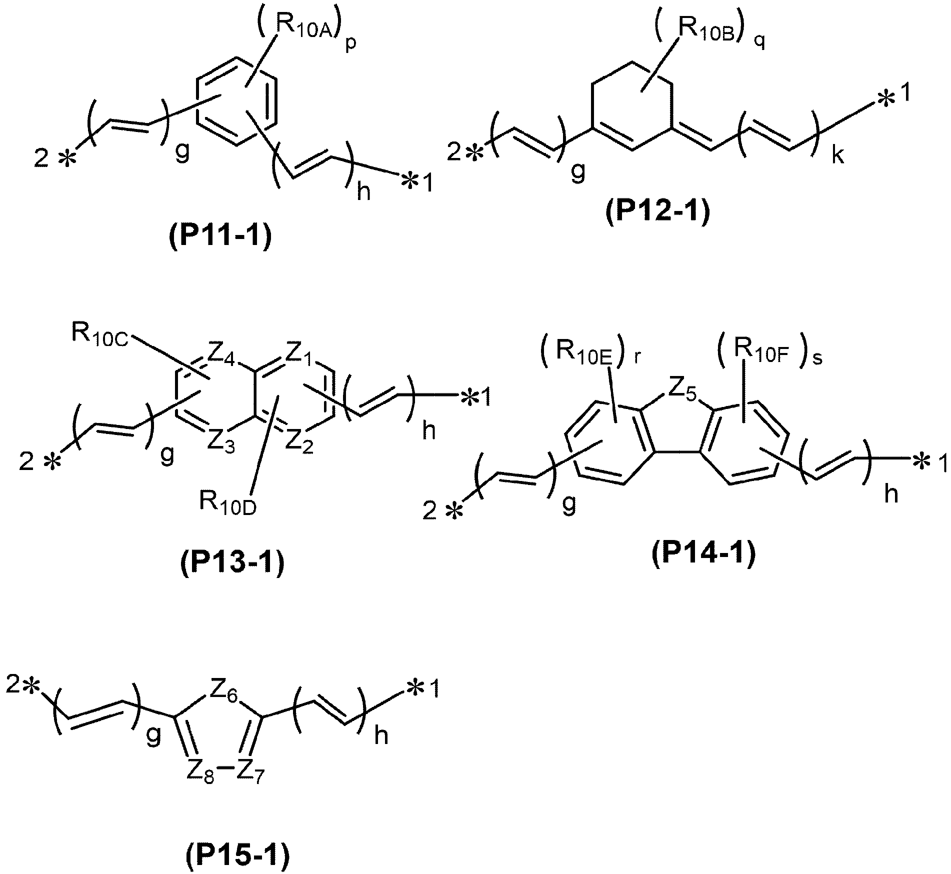

In the formulae (P11-1), (P12-1), (P13-1), (P14-1), and (P15-1):
p, q, r, and s are each an integer of 1 or more;
Z₁, Z₂, Z₃, Z₄, Z₇, and Z₈ are each independently C(R₁₀₀) or a nitrogen atom;
a plurality of R₁₀₀ are mutually the same or different;
Z₅ and Z₆ are each independently an oxygen atom, a sulfur atom, N(R₁₁₀), or C(R₁₁₁)(R₁₁₂);
at least one combination of adjacent two or more of R₁₀₀, R_{10A}, R_{10B}, R_{10C}, R₁₀₀, R_{10E}, and R_{10F} are mutually bonded to form a substituted or unsubstituted monocyclic ring, mutually bonded to form a substituted or unsubstituted fused ring, or not mutually bonded;
R₁₀₀, R_{10A}, R_{10B}, R_{10C}, R_{10D}, R_{10E}, and R_{10F} forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
R₁₀₁, R₁₀₂, R₁₀₃, R₁₀₄, R₁₀₅, R₁₀₆, R₁₀₇, R₁₀₈, R₁₀₉, R₁₁₀, R₁₁₁, and R₁₁₂ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when a plurality of R₁₁₀ are present, the plurality of R₁₁₀ are mutually the same or different;
when a plurality of R₁₁₁ are present, the plurality of R₁₁₁ are mutually the same or different;
when a plurality of R₁₁₂ are present, the plurality of R₁₁₂ are mutually the same or different; and
g, h, and k are each independently 0, 1, 2, or 3,
in the formula (P11-1): when p is 1, R_{10A} is a group represented by -O(R₁₀₉); and when p is 2 or more, at least one of R_{10A} is a group represented by -O(R₁₀₉), and R₁₀₉ is a substituted or unsubstituted alkyl group or a substituted or unsubstituted aryl group,
in the formula (P12-1): when q is 1, R_{10B} is a group represented by -O(R₁₀₉); and when q is 2 or more, at least one of R_{10B} is a group represented by -O(R₁₀₉), and R₁₀₉ is a substituted or unsubstituted alkyl group or a substituted or unsubstituted aryl group,
in the formula (P13-1): when Z₁, Z₂, Z₃, and Z₄ are C(R₁₀₀), at least one of R_{10C}, R_{10D}, or the four R₁₀₀ is a group represented by -O(R₁₀₉), and R₁₀₉ is a substituted or unsubstituted alkyl group or a substituted or unsubstituted aryl group,
in the formula (P15-1): when Z₇ and Z₈ are C(R₁₀₀), at least one of the two R₁₀₀ is a group represented by -O(R₁₀₉), and R₁₀₉ is a substituted or unsubstituted alkyl group or a substituted or unsubstituted aryl group, and
*1 and *2 each represent a bonding position.

[Configuration 3-3] The compound according to [Configuration 3-1] or [Configuration 3-2], in which the π-conjugated moiety is represented by a formula (P11-1A), (P12-1A), (P13-1A), (P13-1B), (P14-1A), (P14-1B), (P15-1A), or (P15-1B) below.

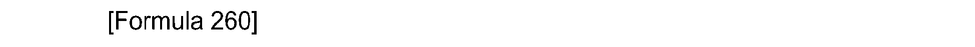

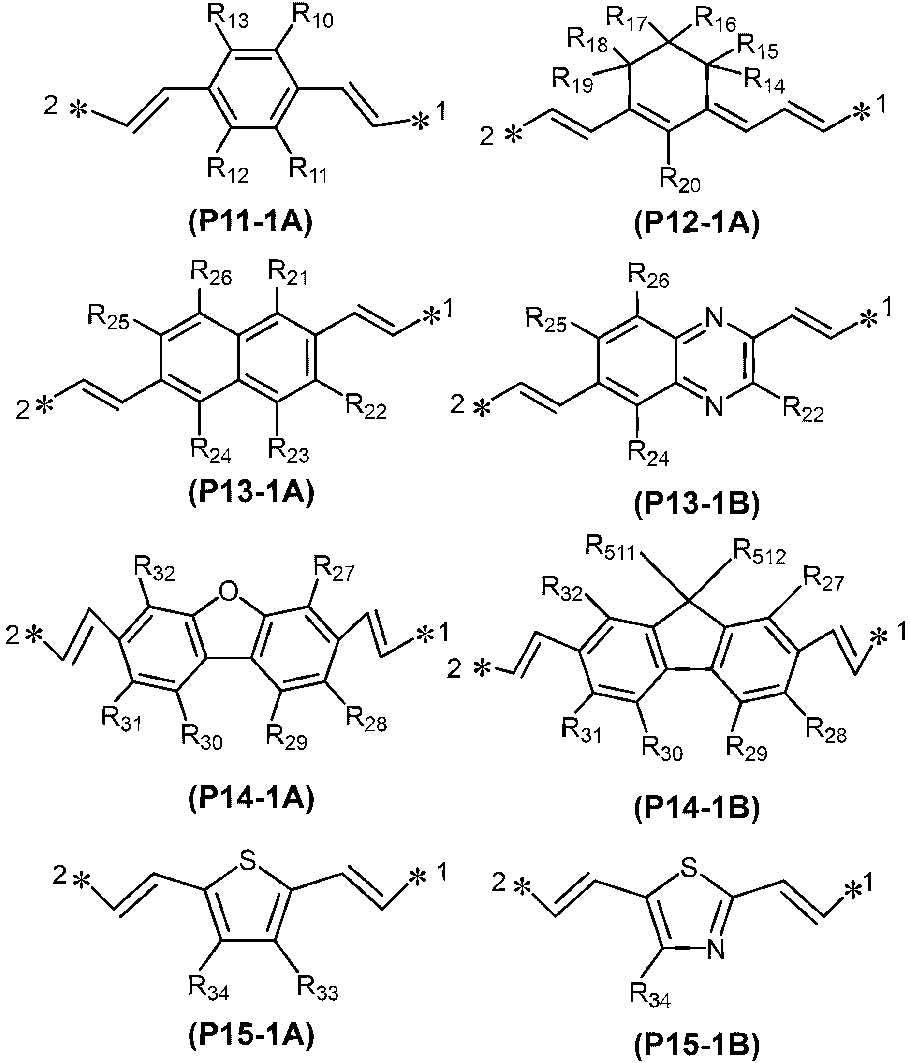

In the formulae (P11-1A), (P12-1A), (P13-1A), (P13-1B), (P14-1A), (P14-1B), (P15-1A), and (P15-1B):
at least one combination of adjacent two or more of R₁₀ to R₃₄, R₅₁₁, and R₅₁₂ are mutually bonded to form a substituted or unsubstituted monocyclic ring, mutually bonded to form a substituted or unsubstituted fused ring, or not mutually bonded;
R₁₀ to R₃₄, R₅₁₁, and R₅₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
in the formula (P11-1A): when at least one of R₁₀ to R₁₃ is a group represented by -O(R₁₀₉), and R₁₀₉ is a substituted or unsubstituted alkyl group or a substituted or unsubstituted aryl group,
in the formula (P12-1A): when at least one of R₁₄ to R₂₀ is a group represented by -O(R₁₀₉), and R₁₀₉ is a substituted or unsubstituted alkyl group or a substituted or unsubstituted aryl group,
   in the formula (P13-1A): when at least one of R₂₁ to R₂₆ is a group represented by - O(R₁₀₉), and R₁₀₉ is a substituted or unsubstituted alkyl group or a substituted or unsubstituted aryl group,
in the formula (P15-1A): when at least one of R₃₃ to R₃₄ is a group represented by -O(R₁₀₉), and R₁₀₉ is a substituted or unsubstituted alkyl group or a substituted or unsubstituted aryl group, and
*1 and *2 each represent a bonding position.

[Configuration 3-4] The compound according to any one of [Configuration 3-1] to [Configuration 3-3], represented by a formula (1) below.
[Formula 261]

D-L-A **(1)**

In the formula (1), D is the donor moiety, L is the π-conjugated moiety, and A is the acceptor moiety.

[Configuration 3-5] The compound according to any one of [Configuration 3-1] to [Configuration 3-4], in which the donor moiety is a group having electron-donating properties, and the acceptor moiety is a group having electron-withdrawing properties.

[Configuration 3-6] An electro-optic material containing an electro-optic compound and a host material, in which the electro-optic compound is the compound according to any one of [Configuration 3-1] to [Configuration 3-5].

[Configuration 3-7] The electro-optic material according to [Configuration 3-6], in which the host material is a polymer, and the electro-optic compound is bonded to the polymer as the host material.

[Configuration 3-8] The electro-optic material according to [Configuration 3-6], in which the host material is a polymer, and the electro-optic compound is not bonded to the polymer and is dispersed in the host material.

[Configuration 3-9] An electro-optic film containing the compound according to any one of [Configuration 3-1] to [Configuration 3-5].

[Configuration 3-10] An electro-optic film containing the electro-optic material according to any one of [Configuration 3-6] to [Configuration 3-8].

[Configuration 3-11] An electro-optic device including the compound according to any one of [Configuration 3-1] to [Configuration 3-5].

[Configuration 3-12] An electro-optic device including the electro-optic material according to any one of [Configuration 3-6] to [Configuration 3-8].

[Configuration 3-13] An electro-optic device including the electro-optic film according to any one of [Configuration 3-9] or [Configuration 3-10].

## Claims

1. A compound comprising:
a donor moiety,
a π-conjugated moiety, and
an acceptor moiety, wherein
the compound satisfies at least one selected from the group consisting of conditions (I), (II), and (III) below,
Condition (I): the donor moiety is represented by a formula (D1) or a formula (D2) below,
Condition (II): the acceptor moiety is represented by a formula (A1) or a formula (A2) below, and
Condition (III): the π-conjugated moiety is represented by a formula (P1), a formula (P2), or a formula (P3) below, 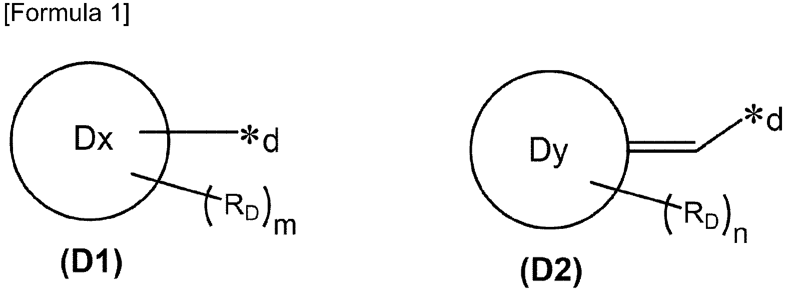
where, in the formulae (D1) and (D2):
a ring Dx and a ring Dy are each independently a five-membered or six-membered aromatic hydrocarbon ring, a five-membered or six-membered aliphatic hydrocarbon ring, or a five-membered or six-membered heterocycle;
the ring Dx has one or more substituents R_{D};
the ring Dy has one or more substituents R_{D} or has no substituent R_{D};
m is the number of the substituents R_{D} on the ring Dx and is an integer of 1 or more;
n is the number of the substituents R_{D} on the ring Dy and is 0 or an integer of 1 or more;
the substituents R_{D} on the ring Dx are bonded with the ring Dx to form a substituted or unsubstituted monocyclic ring, bonded with the ring Dx to form a substituted or unsubstituted fused ring, or not bonded with the ring Dx;
the substituents R_{D} on the ring Dy are bonded with the ring Dy to form a substituted or unsubstituted monocyclic ring, bonded with the ring Dy to form a substituted or unsubstituted fused ring, or not bonded with the ring Dy;
when the ring Dx has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
when the ring Dy has two or more substituents R_{D}, at least one combination of adjacent two or more of substituents R_{D} are mutually bonded to form a substituted or unsubstituted monocyclic ring, mutually bonded to form a substituted or unsubstituted fused ring, or not mutually bonded;
the substituents R_{D} on the ring Dx and the ring Dy are each independently a group represented by -N(R₁₀₁)(R₁₀₂), a group represented by -Si(R₁₀₃)(R₁₀₄)(R₁₀₅), a group represented by -C(=O)R₁₀₆, a group represented by -C(R₁₀₇)(R₁₀₈)(R₁₀₉), a group represented by -O(R₁₁₃), a substituted or unsubstituted alkyl group, a substituted or unsubstituted cycloalkyl group, a substituted or unsubstituted aryl group, or a substituted or unsubstituted heterocyclic group;
R₁₀₁, R₁₀₂, R₁₀₃, R₁₀₄, R₁₀₅, R₁₀₆, R₁₀₇, R₁₀₈, R₁₀₉, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when the number of substituents R_{D} on the ring Dx and the ring Dy is 1 and the one substituent R_{D} is a group represented by -N(R₁₀₁)(R₁₀₂), R₁₀₁ and R₁₀₂ are neither hydrogen atoms nor substituted or unsubstituted alkyl groups;
when the number of the substituents R_{D} on the ring Dx is 2 and one of the two substituents R_{D} is a group represented by -N(R₁₀₁)(R₁₀₂), the other thereof is not a group represented by -O(R₁₁₃);
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when a plurality of R₁₁₃ are present, the plurality of R₁₁₃ are mutually the same or different; and
*d is a bonding position, 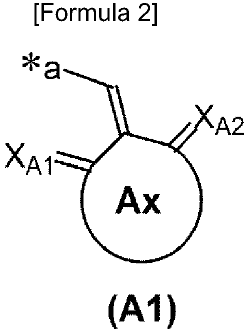 where, in the formula (A1):
X_{A1} and X_{A2} are each independently an oxygen atom (O) or C(CN)₂;
when one of X_{A1} and X_{A2} is O and the other of X_{A1} and X_{A2} is C(CN)₂, the ring to which X_{A1} and X_{A2} are directly bonded is not a five-membered ring;
the ring Ax is a substituted or unsubstituted monocyclic aromatic hydrocarbon group, a substituted or unsubstituted polycyclic aromatic hydrocarbon ring, a substituted or unsubstituted monocyclic aliphatic hydrocarbon ring, a substituted or unsubstituted polycyclic aliphatic hydrocarbon ring, a substituted or unsubstituted monocyclic heterocycle, or a substituted or unsubstituted polycyclic heterocycle; and
*a represents a bonding position, 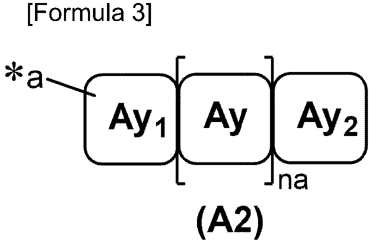 where, in the formula (A2):
na represents the number of rings Ay, and na is 0 or an integer of 1 or more;
the rings Ay, a ring Ay₁ and a ring Ay₂ are each independently a substituted or unsubstituted monocyclic aromatic hydrocarbon group, a substituted or unsubstituted monocyclic aliphatic hydrocarbon ring, or a substituted or unsubstituted monocyclic heterocycle;
when na is 0, the ring Ay₁ and the ring Ay₂ are fused to each other;
when na is an integer of 1 or more, the ring Ay, the ring Ay₁, and the ring Ay₂ are each independently fused to an adjacent ring;
when na is an integer of 2 or more, the ring Ay₁, the ring Ay₂, and two or more rings Ay include a fused ring structure in which the rings are fused in series to adjacent rings or a fused ring structure in which the rings are fused in a branched manner to adjacent rings; and
*a represents a bonding position, 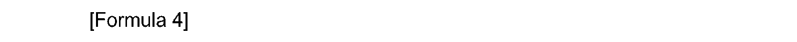 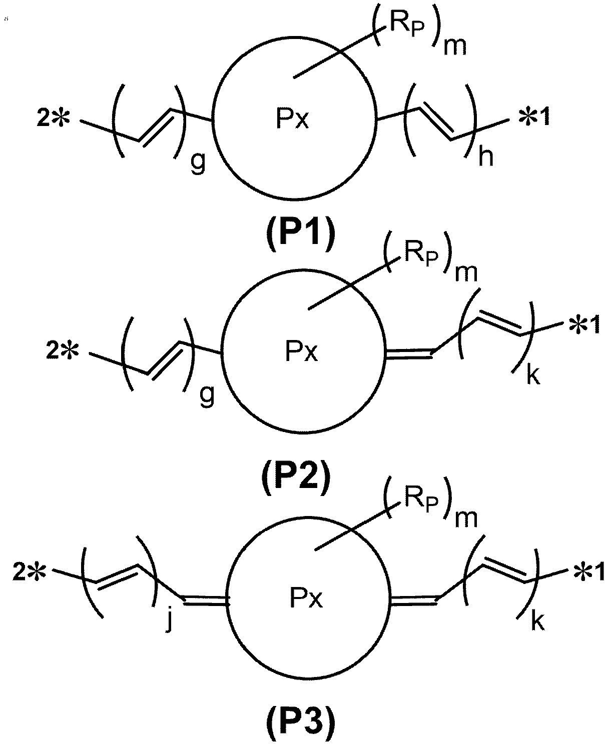 where, in the formulae (P1), (P2), and (P3):
the ring Px is a monocyclic aromatic hydrocarbon ring having one or more substituents R_{P}, a substituted or unsubstituted polycyclic aromatic hydrocarbon ring, a monocyclic aliphatic hydrocarbon ring having one or more substituents R_{P}, a substituted or unsubstituted polycyclic aliphatic hydrocarbon ring, a substituted or unsubstituted monocyclic heterocycle, or a substituted or unsubstituted polycyclic heterocycle;
m is the number of substituents R_{P} and is 0 or an integer of 1 or more;
a substituent R_{P} is a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₀)(R₁₀₇)(R₁₀₃), or a group represented by -O(R₁₀₉);
R₁₀₁, R₁₀₂, R₁₀₃, R₁₀₄, R₁₀₅, R₁₀₆, R₁₀₇, R₁₀₈, and R₁₀₉ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when the ring Px is a monocyclic aromatic hydrocarbon ring, a monocyclic aliphatic hydrocarbon ring, or a monocyclic heterocycle having one or more substituents R_{P}, at least one of the substituents R_{P} is a group represented by -O-R_{P1}, and R_{P1} is a substituted or unsubstituted alkyl group or a substituted or unsubstituted aryl group;
when the ring Px is a naphthalene ring, the naphthalene ring as the ring Px has at least one group represented by -O-R_{P1}, and R_{P1} is a substituted or unsubstituted alkyl group or a substituted or unsubstituted aryl group;
when the ring Px is a monocyclic heterocycle having no substituent R_{P}, the monocyclic heterocycle contains two or more heteroatoms as ring atoms;
when the ring Px is a polycyclic heterocycle, at least one ring contains one or more heteroatoms as ring atoms;
g, h, j, and k are each independently 0, 1, 2, or 3; and
*1 and *2 each represent a bonding position.

2. The compound according to claim 1, wherein the donor moiety is represented by the formula (D1) or the formula (D2).

3. The compound according to claim 1 or 2, wherein the rings Dx and Dy in the formulae (D1) and (D2) are each independently a five-membered or six-membered aromatic hydrocarbon ring, or a five-membered or six-membered heterocycle.

4. The compound according to any one of claims 1 to 3, wherein the donor moiety is represented by a formula (D11), (D12), (D13), (D21), or (D22) below, 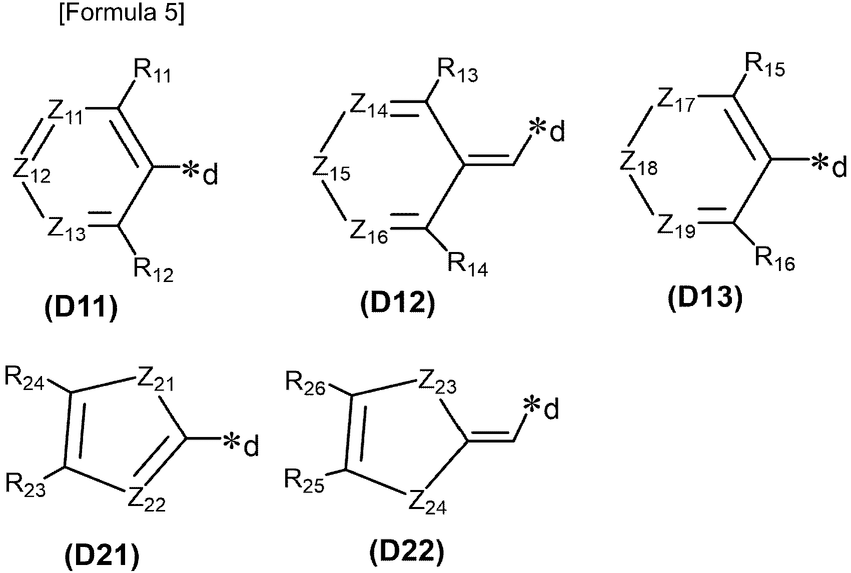 where, in the groups represented by the formulae (D11), (D12), (D13), (D21), and (D22):
Z₁₁, Z₁₂, Z₁₃, Z₁₄, Z₁₆, and Z₁₉ are each independently c(R₁₀₀);
Z₁₅, Z₁₇, and Z₁₈ are each independently an oxygen atom, a sulfur atom, C=R₁₀₀, N(R₁₁₀), or C(R₁₁₁)(R₁₁₂);
Z₂₁, Z₂₃, and Z₂₄ are each independently an oxygen atom, a sulfur atom, N(R₁₁₀), or C(R₁₁₁)(R₁₁₂);
Z₂₂ is C(R₁₀₀);
at least one combination of adjacent two or more of R₁₁, R₁₂, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₃, R₁₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₁₅, R₁₆, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₃, R₂₄, R₁₀₀, R₁₁₀, R₁₁₁, and R₁₁₂ are mutually bonded to form a substituted or unsubstituted monocyclic ring, mutually bonded to form a substituted or unsubstituted fused ring, or not mutually bonded;
at least one combination of adjacent two or more of R₂₅, R₂₆, R₁₁₀, R₁₁₁, and R₁₁₂ are mutually bonded to form a substituted or unsubstituted monocyclic ring, mutually bonded to form a substituted or unsubstituted fused ring, or not mutually bonded;
R₁₁ to R₁₆, R₂₃ to R₂₆, R₁₀₀, and R₁₁₀ to R₁₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₇)(R₁₀₈)(R₁₀₉), or a group represented by -O(R₁₁₃); and
*d is a bonding position.

5. The compound according to any one of claims 1 to 4, wherein the donor moiety is a compound represented by a formula (D111), (D112), (D113), (D121), (D122), (D123), or (D124) below, 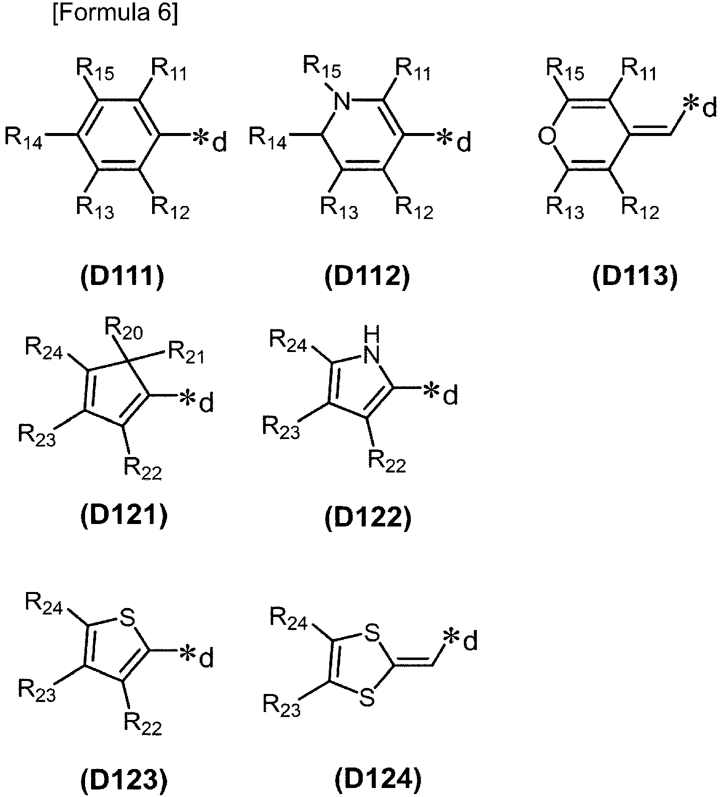 where, in the groups represented by the formulae (D111), (D112), (D113), (D121), (D122), (D123), and (D124):
at least one combination of adjacent two or more of R₁₁ to R₁₅ are mutually bonded to form a substituted or unsubstituted monocyclic ring, mutually bonded to form a substituted or unsubstituted fused ring, or not mutually bonded;
at least one combination of adjacent two or more of R₂₀ to R₂₄ are mutually bonded to form a substituted or unsubstituted monocyclic ring, mutually bonded to form a substituted or unsubstituted fused ring, or not mutually bonded;
R₁₁ to R₁₅ and R₂₀ to R₂₄ forming neither the substituted or unsubstituted monocyclic ring nor the substituted or unsubstituted fused ring are each independently a hydrogen atom, a group represented by -N(R₁₀₁)(R₁₀₂), a group represented by -Si(R₁₀₃)(R₁₀₄)(R₁₀₅), a group represented by -C(=O)R₁₀₆, a group represented by -O(R₁₁₃), a substituted or unsubstituted alkyl group, a substituted or unsubstituted cycloalkyl group, a substituted or unsubstituted aryl group, or a substituted or unsubstituted heterocyclic group; and
*d represents a bonding position.

6. The compound according to any one of claims 1 to 5, wherein the donor moiety is represented by a formula (D111-1A), (D111-1B), (D111-1C), (D111-1D), (D111-1E), (D111-1F), (D111-1G), or (D111-1H) below, 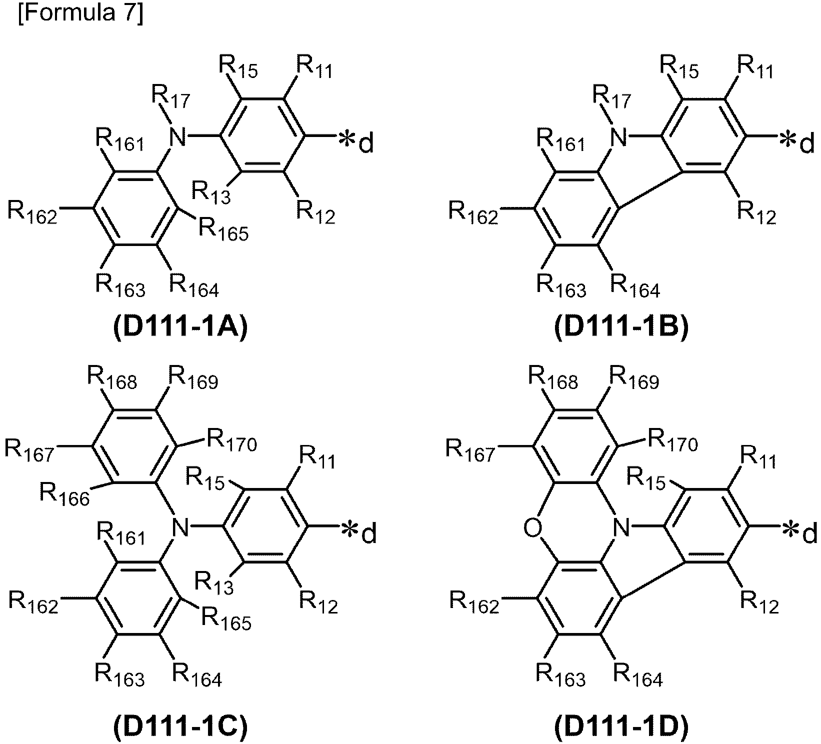 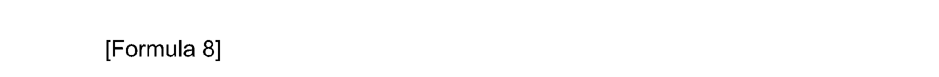 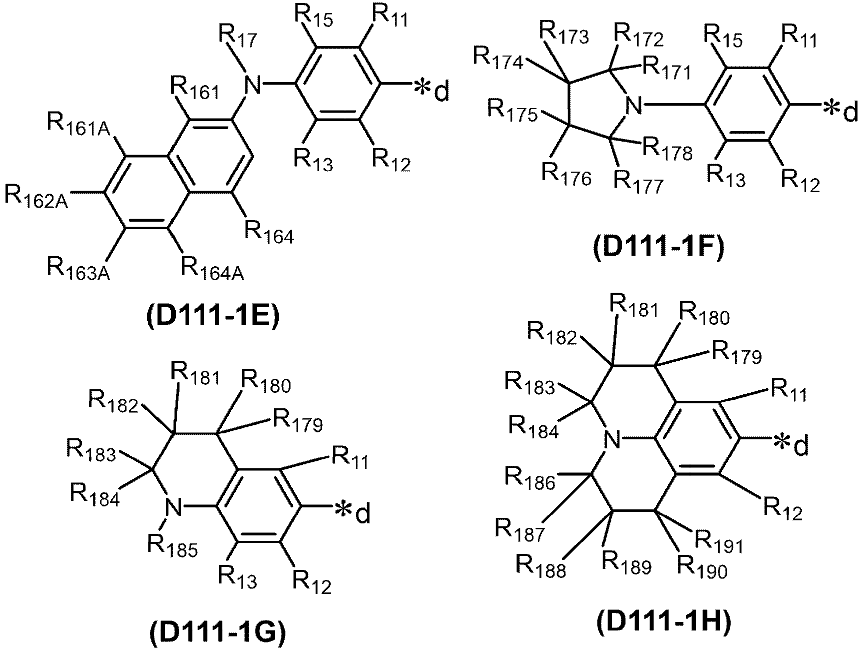 where, in the formulae (D111-1A), (D111-1B), (D111-1C), (D111-1D), (D111-1E), (D111-1F), (D111-1G), and (D111-1H):
R₁₁ to R₁₃ and R₁₅ are each independently a hydrogen atom, a substituted or unsubstituted alkyl group, a substituted or unsubstituted cycloalkyl group, a substituted or unsubstituted aryl group, or a substituted or unsubstituted heterocyclic group;
R₁₇, R₁₆₁ to R₁₉₁, and R_{161A} to R_{164A} are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

7. The compound according to any one of claims 1 to 6, wherein the donor moiety is represented by a formula (D122-1), (D123-1A), or (D123-1B) below, 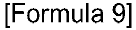 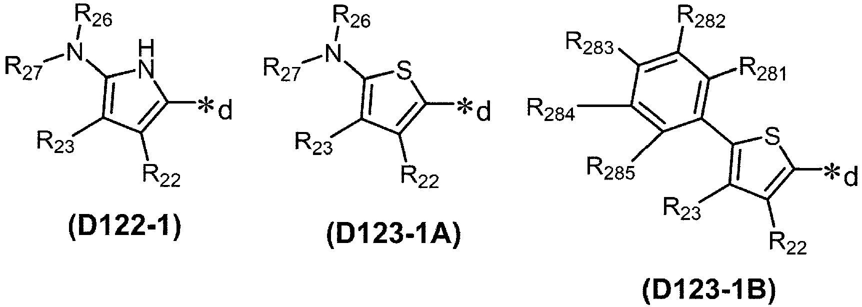 where, in the formulae (D122-1), (D123-1A), and (D123-1B):
R₂₂, R₂₃, R₂₆, and R₂₇ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃);
at least one combination of adjacent two or more of R₂₈₁, R₂₈₂, R₂₈₃, R₂₈₄, and R₂₈₅ are mutually bonded to form a substituted or unsubstituted monocyclic ring, mutually bonded to form a substituted or unsubstituted fused ring, or not mutually bonded;
R₂₈₁ to R₂₈₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by -N(R₁₀₁)(R₁₀₂), or a group represented by -O(R₁₁₃); and
*d represents a bonding position.

8. The compound according to any one of claims 1 to 7, wherein the donor moiety is represented by a formula (D123-2A), (D123-3A), or (D123-4A) below, 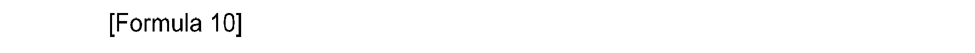 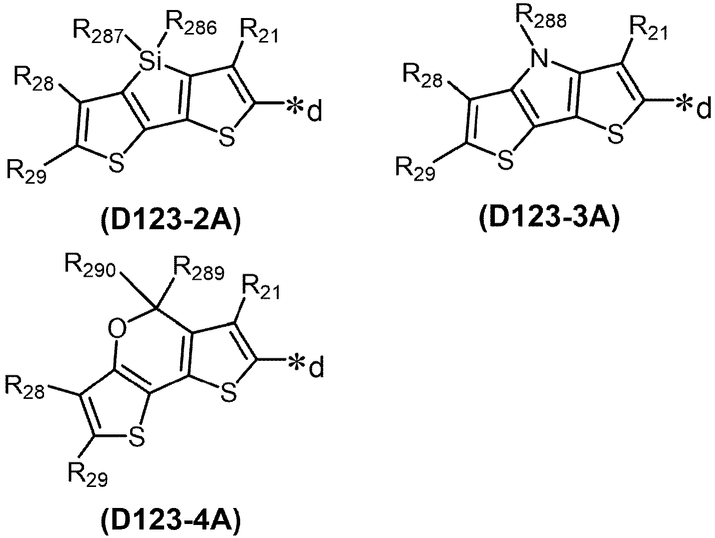 where, in the formulae (D123-2A), (D123-3A), and (D123-4A):
R₂₁, R₂₈, R₂₉, R₂₈₆, R₂₈₇, R₂₈₈, R₂₈₉, and R₂₉₀ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by -O(R₁₁₃); and
*d represents a bonding position.

9. The compound according to any one of claims 1 to 8, wherein the donor moiety is represented by a formula (D112-1) or (D121-1) below, 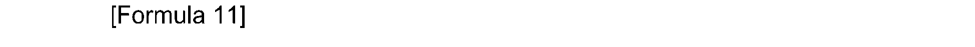 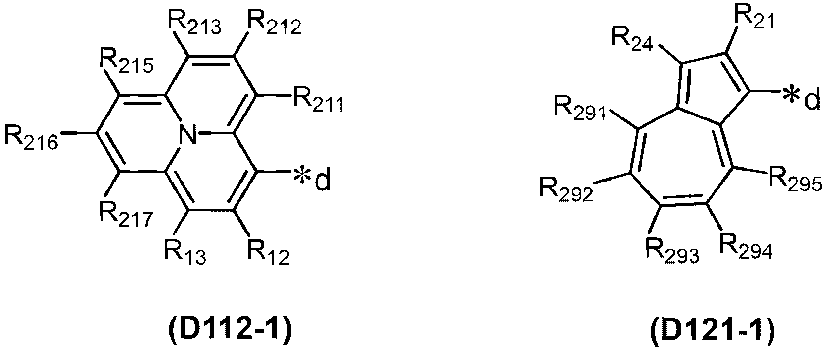 where, in the formulae (D112-1) and (D121-1):
R₁₂, R₁₃, R₂₁, R₂₄, R₂₁₁ to R_{213,}, R₂₁₅ to R₂₁₇, and R₂₉₁ to R₂₉₅ are each independently a hydrogen atom, a substituted or unsubstituted alkyl group, a substituted or unsubstituted cycloalkyl group, a substituted or unsubstituted aryl group, a substituted or unsubstituted heterocyclic group, or a group represented by - O(R₁₁₃); and
*d represents a bonding position.

10. The compound according to claim 5, wherein the donor moiety is represented by the formula (D111).

11. The compound according to claim 10, wherein R₁₁ to R₁₅ in the formula (D111) are each independently a hydrogen atom or a group represented by -O(R₁₁₃), and
at least one of R₁₁ to R₁₅ is a group represented by -O(R₁₁₃).

12. The compound according to claim 7, wherein the donor moiety is represented by the formula (D123-1B).

13. The compound according to claim 12, wherein R₂₈₁ to R₂₈₅ in the formula (D123-1B) are each independently a hydrogen atom, a group represented by - N(R₁₀₁)(R₁₀₂), or a group represented by -O(R₁₁₃).

14. The compound according to claim 12 or 13, wherein at least one of R₂₈₁ to R₂₈₅ in the formula (D123-1B) is a group represented by -O(R₁₁₃).

15. The compound according to any one of claims 12 to 14, wherein three of R₂₈₁ to R₂₈₅ in the formula (D123-1B) are groups represented by -O(R₁₁₃), and the remaining two thereof are hydrogen atoms.

16. The compound according to any one of claims 12 to 15, wherein R₂₈₃ in the formula (D123-1B) is a group represented by -O(R₁₁₃), one of R₂₈₁ and R₂₈₂ is a group represented by -O(R₁₁₃) and the other thereof is a hydrogen atom, and one of R₂₈₄ and R₂₈₅ is a group represented by -O(R₁₁₃) and the other thereof is a hydrogen atom.

17. The compound according to any one of claims 12 to 16, wherein, when at least one selected from the group consisting of R₂₈₁ to R₂₈₅ in the formula (D123-1B) is a group represented by -O(R₁₁₃), R₁₁₃ in R₂₈₁ to R₂₈₅ is a substituted or unsubstituted alkyl group having 1 to 5 carbon atoms.

18. The compound according to claim 12, wherein one of R₂₈₁ to R₂₈₅ in the formula (D123-1B) is a group represented by -N(R₁₀₁)(R₁₀₂) and the remaining four thereof are hydrogen atoms.

19. The compound according to any one of claims 1 to 18, wherein the acceptor moiety is represented by the formula (A1) or the formula (A2).

20. The compound according to any one of claims 1 to 19, wherein the acceptor moiety is represented by a formula (A11) or a formula (A12) below, 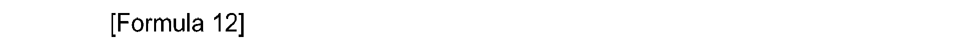 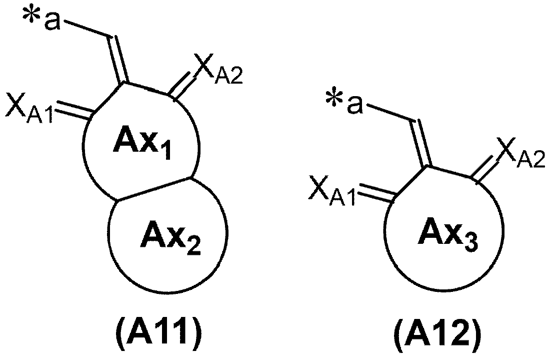 where, in the formulae (A11) and (A12):
X_{A1} and X_{A2} respectively represent the same as X_{A1} and X_{A2} in the formula (A1);
rings Ax₁, Ax₂, and Ax₃ are each independently a substituted or unsubstituted monocyclic aromatic hydrocarbon group, a substituted or unsubstituted monocyclic aliphatic hydrocarbon ring, or a substituted or unsubstituted monocyclic heterocycle; and
*a represents a bonding position.

21. The compound according to any one of claims 1 to 20, wherein the acceptor moiety is represented by a formula (A11-1), (A11-2), or (A12-1) below, 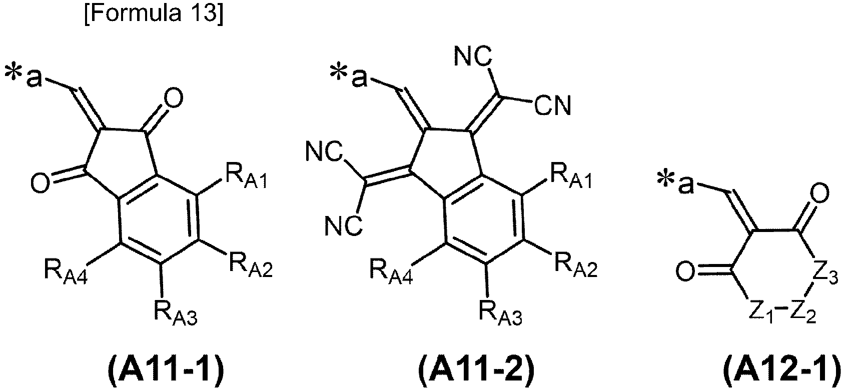 where, in the formulae (A11-1) and (A11-2):
R_{A1}, R_{A2}, R_{A3}, and R_{A4} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx;
in the formula (A12-1):
Z₁, Z₂, and Z₃ are each independently N(R_{A5}), C=O, or C(R_{A6})(R_{A7});
R_{A5}, R_{A6}, and R_{A7} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx;
Rx is a substituted or unsubstituted alkyl group, and when a plurality of Rx are present, the plurality of Rx are mutually the same or different; and
*a represents a bonding position.

22. The compound according to any one of claims 1 to 21, wherein the acceptor moiety is represented by a formula (A111-1), (A111-2), (A112-1), or (A121-1) below, 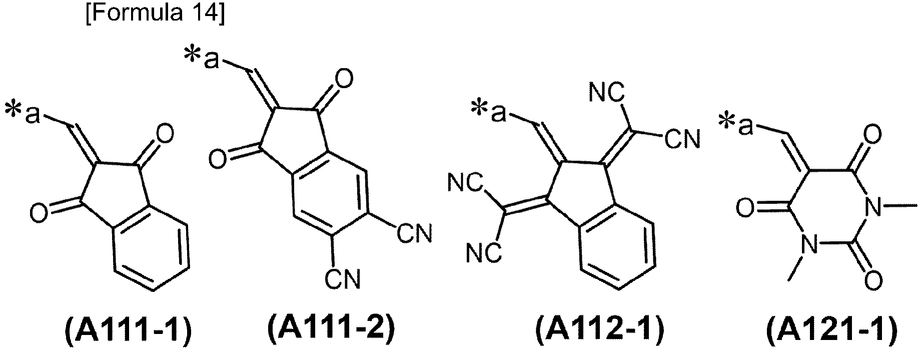 where, in the formulae (A111-1), (A111-2), (A112-1), and (A121-1), *a represents a bonding position.

23. The compound according to any one of claims 1 to 19, wherein na in the formula (A2) is 1 or 2.

24. The compound according to any one of claims 1 to 19 and 23, wherein the acceptor moiety is represented by a formula (A21) or a formula (A22) below, 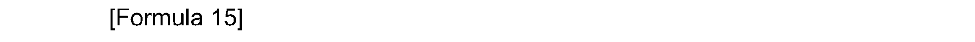 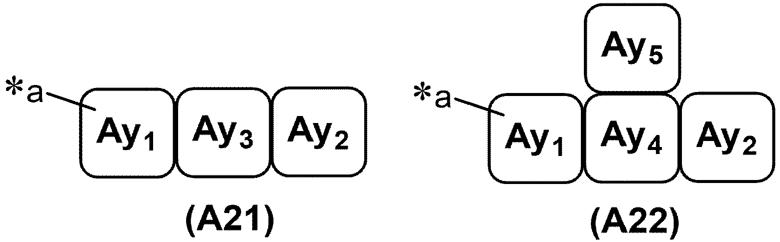 where, in the formula (A21):
the ring Ay₁ and the ring Ay₂ represent the same as the ring Ay₁ and the ring Ay₂ in the formula (A2), respectively; and
the ring Ay₃ represents the same as the ring Ay in the formula (A2),
in the formula (A22):
the ring Ay₁ and the ring Ay₂ represent the same as the ring Ay₁ and the ring Ay₂ in the formula (A2), respectively; and
the ring Ay₄ and the ring Ay₅ each independently represent the same as the ring Ay in the formula (A2), and
*a represents a bonding position.

25. The compound according to any one of claims 1 to 19, 23, and 24, wherein the rings Ay₁ and Ay₂ are each independently a nitrogen-containing ring.

26. The compound according to any one of claims 1 to 19 and 23 to 25, wherein the rings Ay₁ and Ay₂ are each independently a nitrogen-containing six-membered ring.

27. The compound according to any one of claims 1 to 19 and 23 to 26, wherein the acceptor moiety is represented by a formula (A21-1) or a formula (A22-1) below, 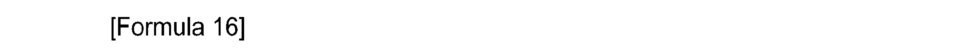 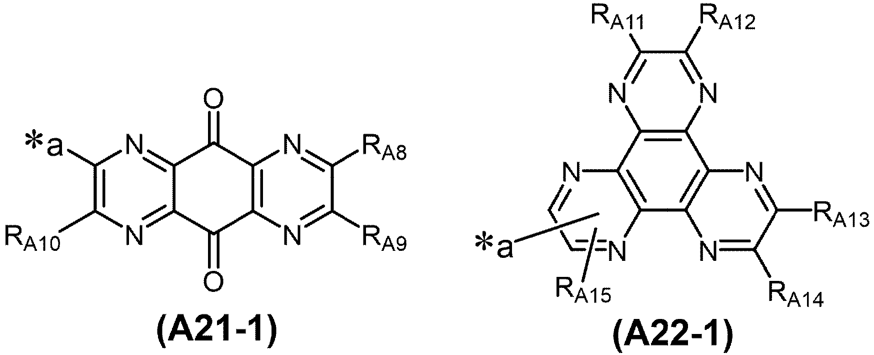 where, in the formulae (A21-1) and (A22-1):
R_{A8}, R_{A9}, R_{A10}, R_{A11}, R_{A12}, R_{A13}, R_{A14}, and R_{A15} are each independently a hydrogen atom, a halogen atom, a cyano group, a substituted or unsubstituted alkyl group, a substituted or unsubstituted aryl group, -NO₂-, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

28. The compound according to any one of claims 1 to 19 and 23 to 27, wherein the acceptor moiety is represented by a formula (A211-1) or a formula (A221-1) below, 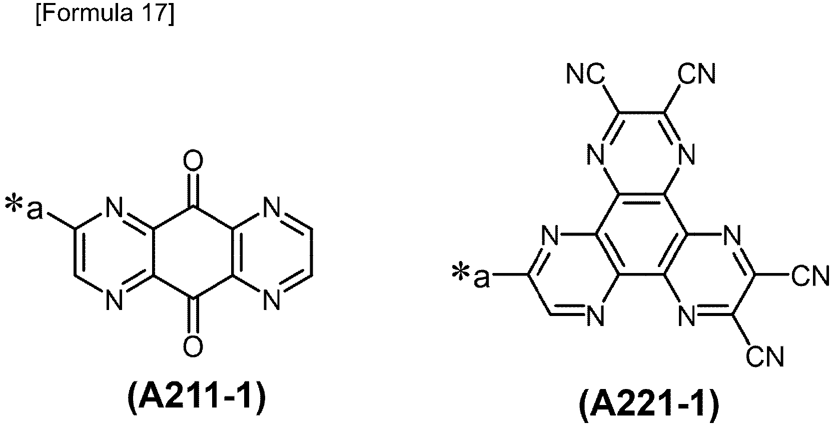 where, in the formulae (A211-1) and (A221-1), *a represents a bonding position.

29. The compound according to any one of claims 1 to 19, 23, and 24, wherein the acceptor moiety is represented by a formula (A21-2) below, 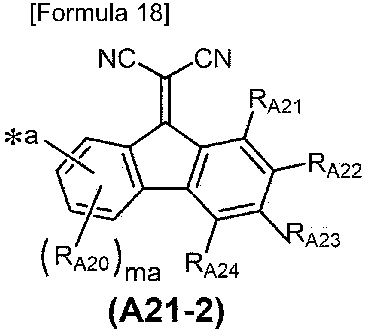 where, in the formula (A21-2):
ma represents the number of R_{A20} bonded to the ring, ma is 3, and the three R_{A20} are mutually the same or different;
R_{A20}, R_{A21}, R_{A22}, R_{A23}, and R_{A24} are each independently a hydrogen atom, a halogen atom, a cyano group, a substituted or unsubstituted alkyl group, a substituted or unsubstituted aryl group, -NO₂-, -CHO, -CORx, -COORx, or -SO₂Rx;
Rx is a substituted or unsubstituted alkyl group, and when a plurality of Rx are present, the plurality of Rx are mutually the same or different; and
*a represents a bonding position.

30. The compound according to any one of claims 1 to 19, 23, 24, and 29, wherein the acceptor moiety is represented by a formula (A212-1) below, 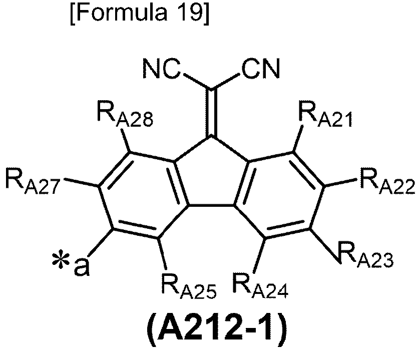 where, in the formula (A212-1):
R_{A21}, R_{A22}, R_{A23}, R_{A24}, R_{A25}, R_{A27}, and R_{A28} are each independently a hydrogen atom, a halogen atom, a cyano group, a substituted or unsubstituted alkyl group, a substituted or unsubstituted aryl group, -NO₂-, -CHO, -CORx, -COORx, or - SO₂Rx;
Rx is a substituted or unsubstituted alkyl group, and when a plurality of Rx are present, the plurality of Rx are mutually the same or different; and
*a represents a bonding position.

31. The compound according to any one of claims 1 to 30, wherein the acceptor moiety has at least one structure selected from the group consisting of a nitrogen-containing ring, CN, C=O, and C=C(CN)₂.

32. The compound according to any one of claims 1 to 31, wherein the π-conjugated moiety is represented by the formula (P1), (P2), or (P3).

33. The compound according to any one of claims 1 to 32, wherein the π-conjugated moiety is represented by a formula (P11-1), (P12-1), (P13-1), (P14-1), or (P15-1) below, 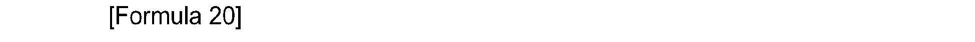 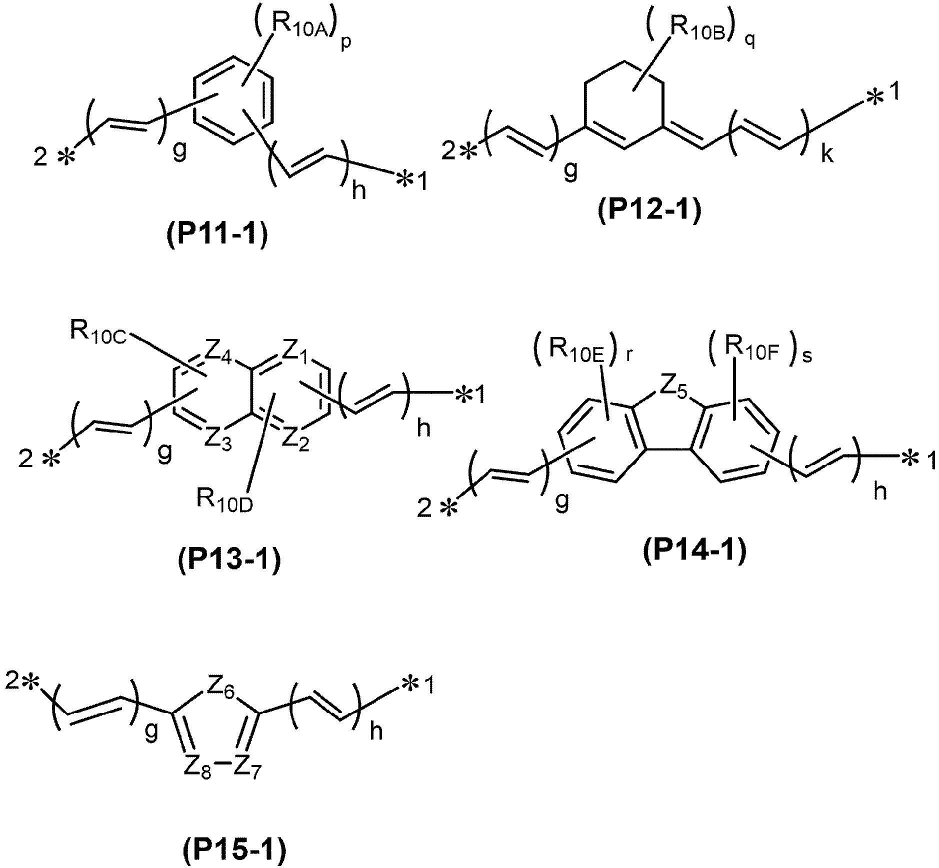 where, in the formulae (P11-1), (P12-1), (P13-1), (P14-1), and (P15-1):
p, q, r, and s are each an integer of 1 or more;
Z₁, Z₂, Z₃, Z₄, Z₇, and Z₈ are each independently C(R₁₀₀) or a nitrogen atom;
a plurality of R₁₀₀ are mutually the same or different;
Z₅ and Z₆ are each independently an oxygen atom, a sulfur atom, N(R₁₁₀), or C(R₁₁₁)(R₁₁₂);
at least one combination of adjacent two or more of R₁₀₀, R_{10A}, R_{10B}, R_{10C}, R_{10D}, R_{10E}, and R_{10F} are mutually bonded to form a substituted or unsubstituted monocyclic ring, mutually bonded to form a substituted or unsubstituted fused ring, or not mutually bonded;
R₁₀₀, R_{10A}, R_{10B}, R_{10C}, R_{10D}, R_{10E}, and R_{10F} forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
when a plurality of R_{10A} are present, the plurality of R_{10A} are mutually the same or different;
when a plurality of R_{10B} are present, the plurality of R_{10B} are mutually the same or different;
when a plurality of R_{10C} are present, the plurality of R_{10C} are mutually the same or different;
when a plurality of R_{10D} are present, the plurality of R_{10D} are mutually the same or different;
when a plurality of R_{10E} are present, the plurality of R_{10E} are mutually the same or different;
when a plurality of R_{10F} are present, the plurality of R_{10F} are mutually the same or different;
R₁₀₁, R₁₀₂, R₁₀₃, R₁₀₄, R₁₀₅, R₁₀₆, R₁₀₇, R₁₀₈, R₁₀₉, R₁₁₀, R₁₁₁, and R₁₁₂ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₀₃ are present, the plurality of R₁₀₃ are mutually the same or different;
when a plurality of R₁₀₄ are present, the plurality of R₁₀₄ are mutually the same or different;
when a plurality of R₁₀₅ are present, the plurality of R₁₀₅ are mutually the same or different;
when a plurality of R₁₀₆ are present, the plurality of R₁₀₆ are mutually the same or different;
when a plurality of R₁₀₇ are present, the plurality of R₁₀₇ are mutually the same or different;
when a plurality of R₁₀₈ are present, the plurality of R₁₀₈ are mutually the same or different;
when a plurality of R₁₀₉ are present, the plurality of R₁₀₉ are mutually the same or different;
when a plurality of R₁₁₀ are present, the plurality of R₁₁₀ are mutually the same or different;
when a plurality of R₁₁₁ are present, the plurality of R₁₁₁ are mutually the same or different;
when a plurality of R₁₁₂ are present, the plurality of R₁₁₂ are mutually the same or different; and
g, h, and k are each independently 0, 1, 2, or 3,
in the formula (P11-1): when p is 1, R_{10A} is a group represented by -O(R₁₀₉); and when p is 2 or more, at least one of R_{10A} is a group represented by -O(R₁₀₉), and R₁₀₉ is a substituted or unsubstituted alkyl group or a substituted or unsubstituted aryl group,
in the formula (P12-1): when q is 1, R_{10B} is a group represented by -O(R₁₀₉); when q is 2 or more, at least one of R_{10B} is a group represented by -O(R₁₀₉), and R₁₀₉ is a substituted or unsubstituted alkyl group or a substituted or unsubstituted aryl group,
in the formula (P13-1): when Z₁, Z₂, Z₃, and Z₄ are C(R₁₀₀), at least one of R_{10C}, R_{10D}, or the four R₁₀₀ is a group represented by -O(R₁₀₉), and R₁₀₉ is a substituted or unsubstituted alkyl group or a substituted or unsubstituted aryl group,
in the formula (P15-1): when Z₇ and Z₈ are C(R₁₀₀), at least one of the two R₁₀₀ is a group represented by -O(R₁₀₉), and R₁₀₉ is a substituted or unsubstituted alkyl group or a substituted or unsubstituted aryl group, and
*1 and *2 each represent a bonding position.

34. The compound according to any one of claims 1 to 33, wherein the π-conjugated moiety is represented by a formula (P11-1A), (P12-1A), (P13-1A), (P13-1B), (P14-1A), (P14-1B), (P15-1A), or (P15-1B) below, 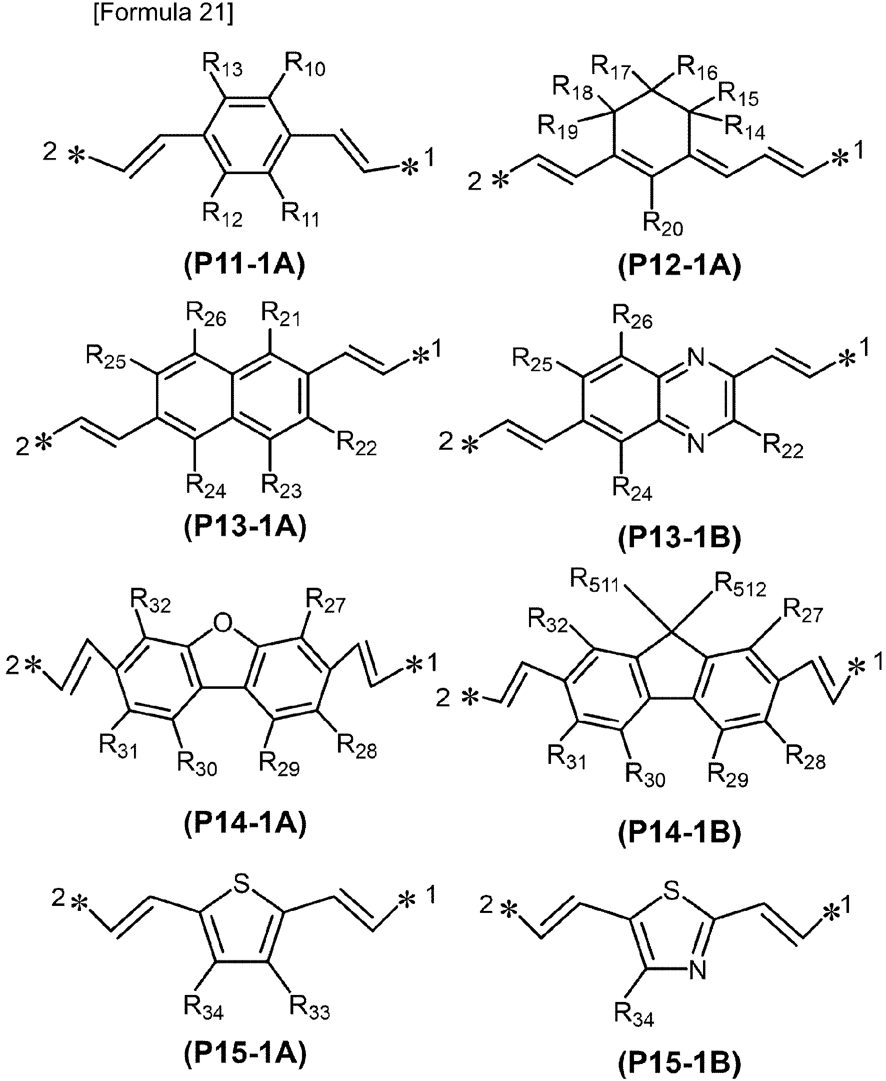 where, in the formulae (P11-1A), (P12-1A), (P13-1A), (P13-1B), (P14-1A), (P14-1B), (P15-1A), and (P15-1B):
at least one combination of adjacent two or more of R₁₀ to R₃₄, R₅₁₁, and R₅₁₂ are mutually bonded to form a substituted or unsubstituted monocyclic ring, mutually bonded to form a substituted or unsubstituted fused ring, or not mutually bonded;
R₁₀ to R₃₄, R₅₁₁, and R₅₁₂ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), a group represented by -Si(R₁₀₃)(R₁₀₄)(R₁₀₅), a group represented by - C(R₁₀₆)(R₁₀₇)(R₁₀₈), or a group represented by -O(R₁₀₉);
in the formula (P11-1A): when at least one of R₁₀ to R₁₃ is a group represented by -O(R₁₀₉), and R₁₀₉ is a substituted or unsubstituted alkyl group or a substituted or unsubstituted aryl group,
in the formula (P12-1A): when at least one of R₁₄ to R₂₀ is a group represented by -O(R₁₀₉), and R₁₀₉ is a substituted or unsubstituted alkyl group or a substituted or unsubstituted aryl group,
in the formula (P13-1A): when at least one of R₂₁ to R₂₆ is a group represented by -O(R₁₀₉), and R₁₀₉ is a substituted or unsubstituted alkyl group or a substituted or unsubstituted aryl group,
in the formula (P15-1A): when at least one of R₃₃ to R₃₄ is a group represented by -O(R₁₀₉), and R₁₀₉ is a substituted or unsubstituted alkyl group or a substituted or unsubstituted aryl group, and
*1 and *2 each represent a bonding position.

35. The compound according to any one of claims 1 to 34, represented by a formula (1) below,
[Formula 22]
D-L-A (1)
where, in the formula (1), D is the donor moiety, L is the π-conjugated moiety, and A is the acceptor moiety.

36. The compound according to any one of claims 1 to 35, wherein
the donor moiety is a group having electron-donating properties, and
the acceptor moiety is a group having electron-withdrawing properties.

37. A precursor represented by a formula (DP-XM1), (DP-XM2), (DP-XM3), (DP-XM4), (DP-YM1), (DP-YM2), (DP-YM3), or (DP-YM4) below, 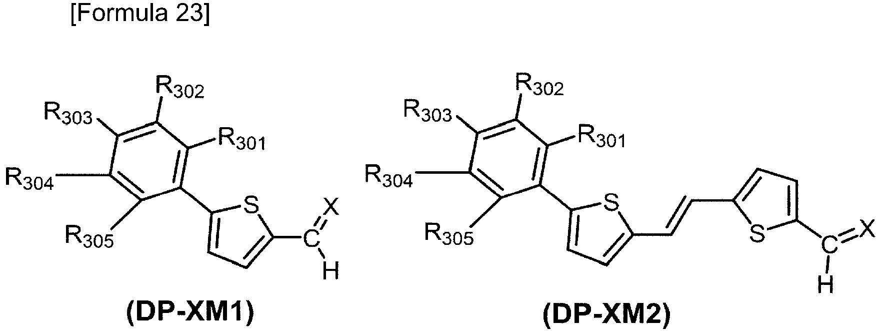 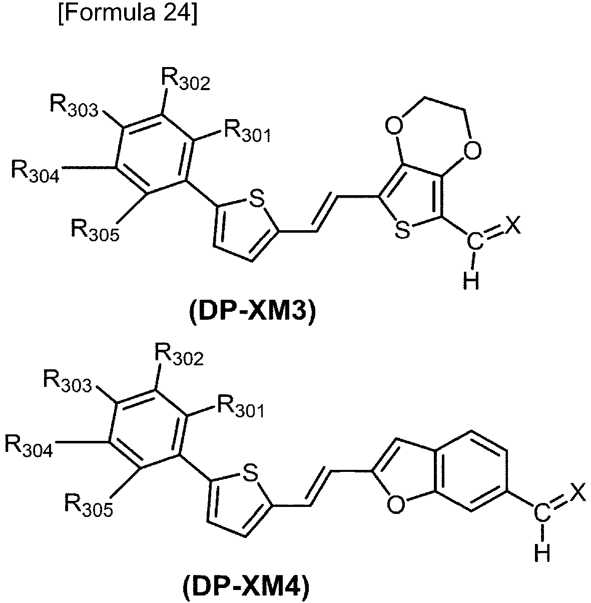 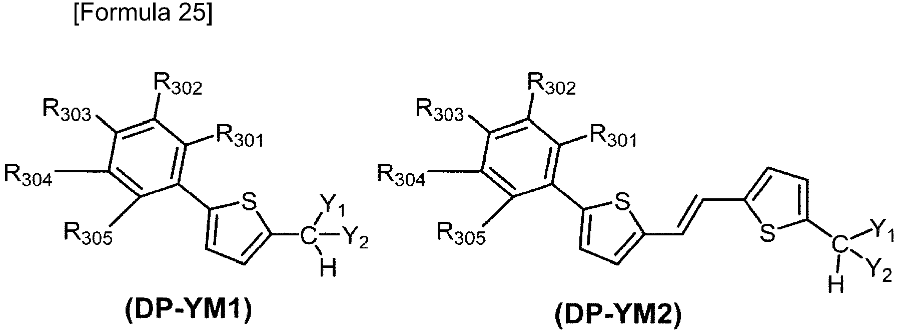 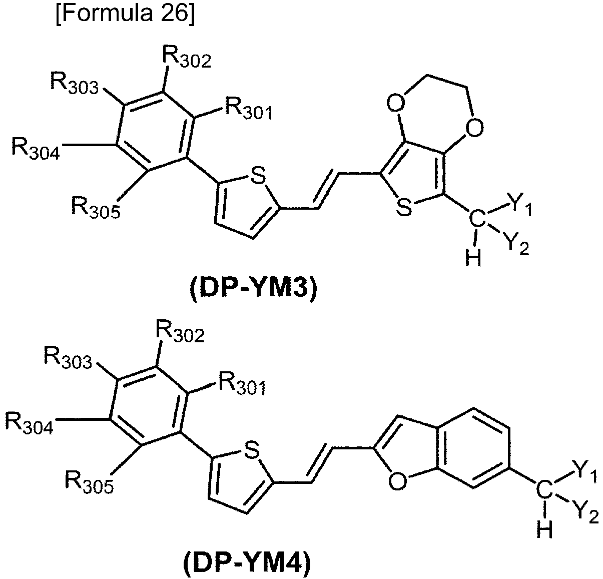 where, in the formulae (DP-XM1), (DP-XM2), (DP-XM3), (DP-XM4), (DP-YM1), (DP-YM2), (DP-YM3), and (DP-YM4):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
in the formulae (DP-XM1), (DP-XM2), (DP-XM3), and (DP-XM4): X is an oxygen atom or a sulfur atom,
in the formulae (DP-YM1), (DP-YM2), (DP-YM3), and (DP-YM4):
a combination of Y₁ and Y₂ are mutually bonded to form a substituted or unsubstituted monocyclic ring, mutually bonded to form a substituted or unsubstituted fused ring, or not mutually bonded;
Y₁ and Y₂ forming neither the substituted or unsubstituted monocyclic ring nor the substituted or unsubstituted fused ring are each independently a substituted or unsubstituted alkoxy group or a group represented by -S(R₁₁₄);
R₁₁₄ is a substituted or unsubstituted alkyl group;
when a plurality of R₁₀₁ are present, the plurality of R₁₀₁ are mutually the same or different;
when a plurality of R₁₀₂ are present, the plurality of R₁₀₂ are mutually the same or different;
when a plurality of R₁₁₃ are present, the plurality of R₁₁₃ are mutually the same or different; and
when a plurality of R₁₁₄ are present, the plurality of R₁₁₄ are mutually the same or different.

38. Use of the precursor according to claim 37, in production of an electro-optic compound.

39. A precursor used for producing the compound according to claim 1, wherein
the precursor is represented by a formula (DP-XM11), (DP-XM21), (DP-XM31), or (DP-XM41) below, 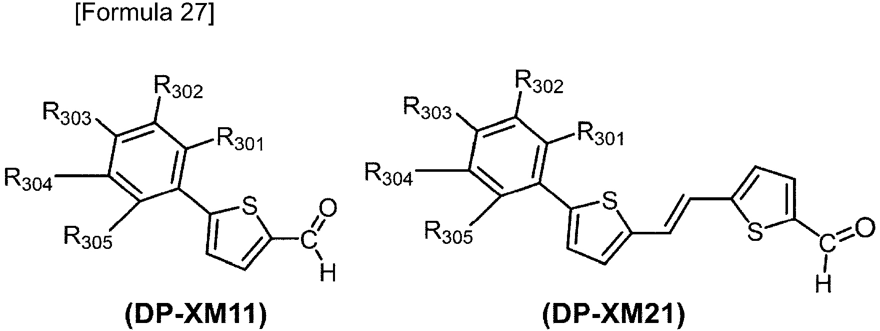 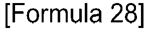 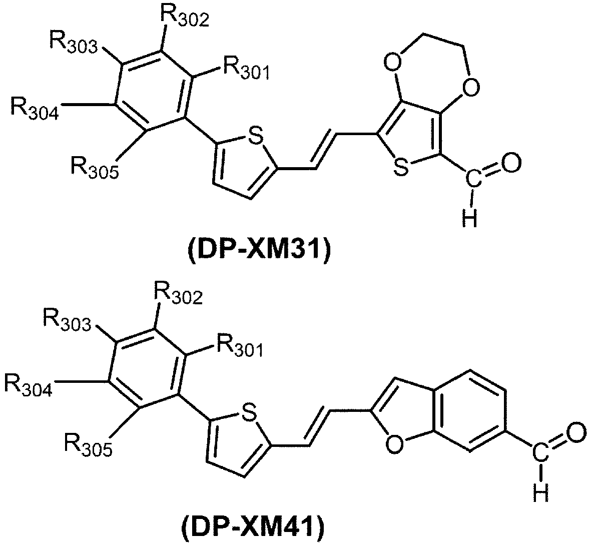 where, in the formulae (DP-XM11), (DP-XM21), (DP-XM31), and (DP-XM41):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different; and
when a plurality of R₁₁₃ are present, the plurality of R₁₁₃ are mutually the same or different.

40. A precursor used for producing an electro-optic compound, wherein
the precursor is represented by a formula (DP-XM11), (DP-XM21), (DP-XM31), or (DP-XM41) below, 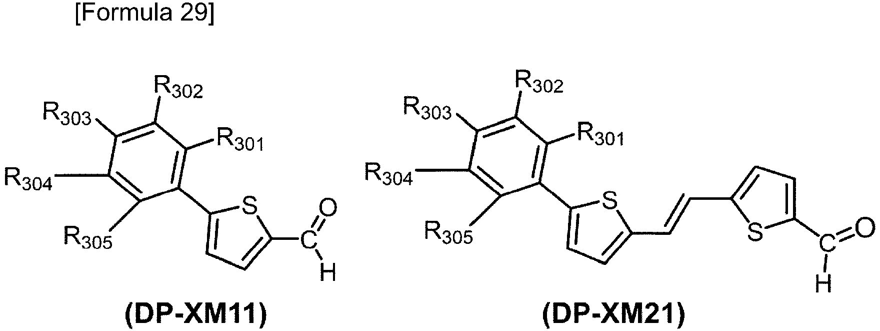 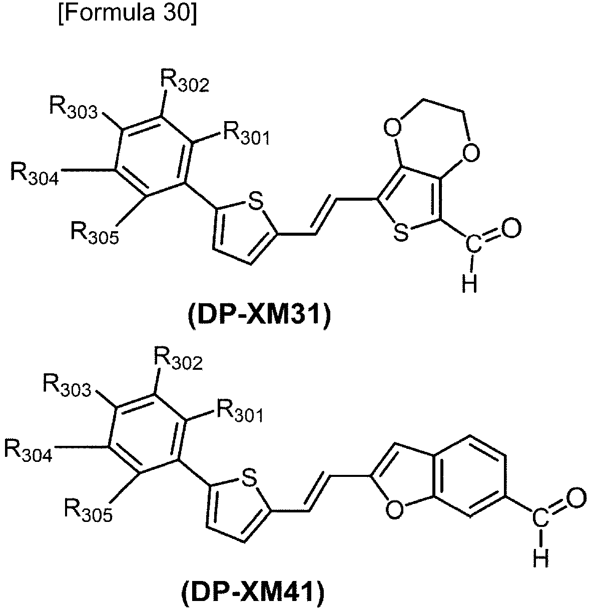 where, in the formulae (DP-XM11), (DP-XM21), (DP-XM31), and (DP-XM41):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different; and
when a plurality of R₁₁₃ are present, the plurality of R₁₁₃ are mutually the same or different.

41. The precursor according to claim 39 or 40, wherein R₃₀₁ to R₃₀₅ in the precursor represented by the formula (DP-XM11), (DP-XM21), (DP-XM31), or (DP-XM41) are each independently a hydrogen atom, a group represented by - N(R₁₀₁)(R₁₀₂), or a group represented by -O(R₁₁₃).

42. The precursor according to any one of claims 39 to 41, wherein
the precursor represented by the formula (DP-XM11), (DP-XM21), (DP-XM31), or (DP-XM41) is represented by a formula (DP-M1), (DP-M2), (DP-M3), (DP-M4), (DP-M5), (DP-M6), (DP-M7), or (DP-M8) below,

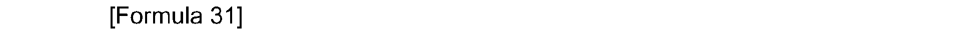

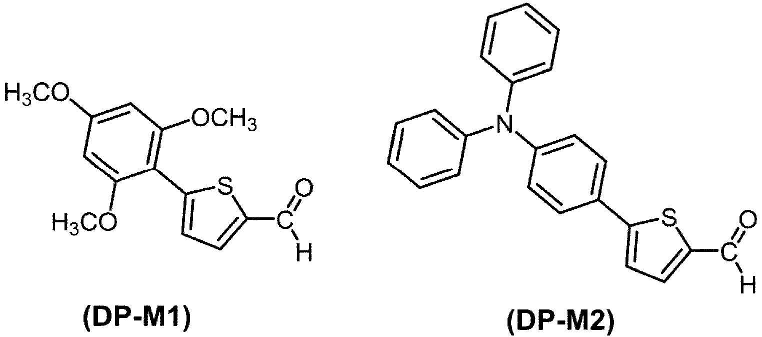

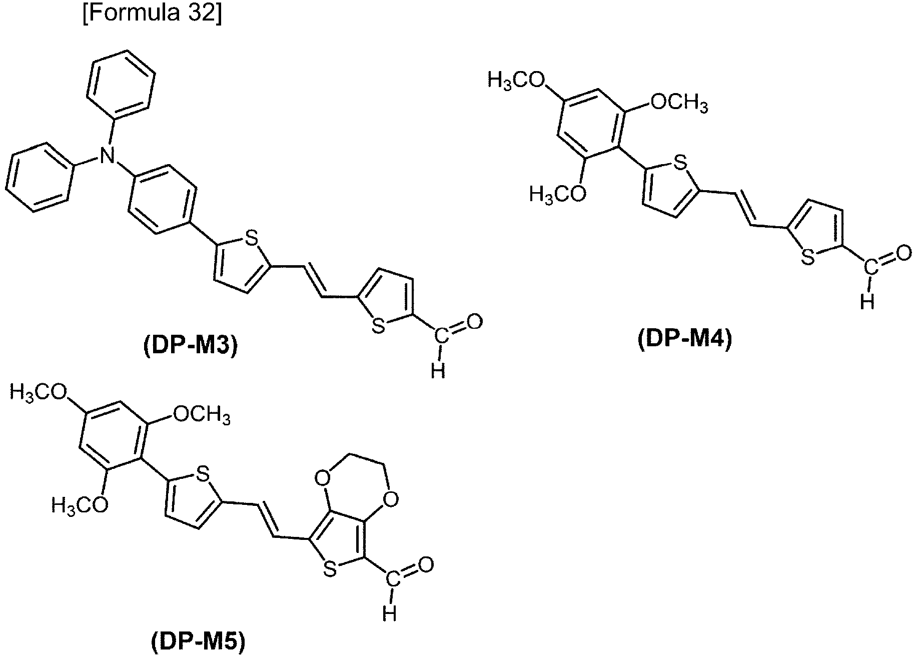

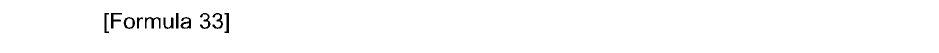

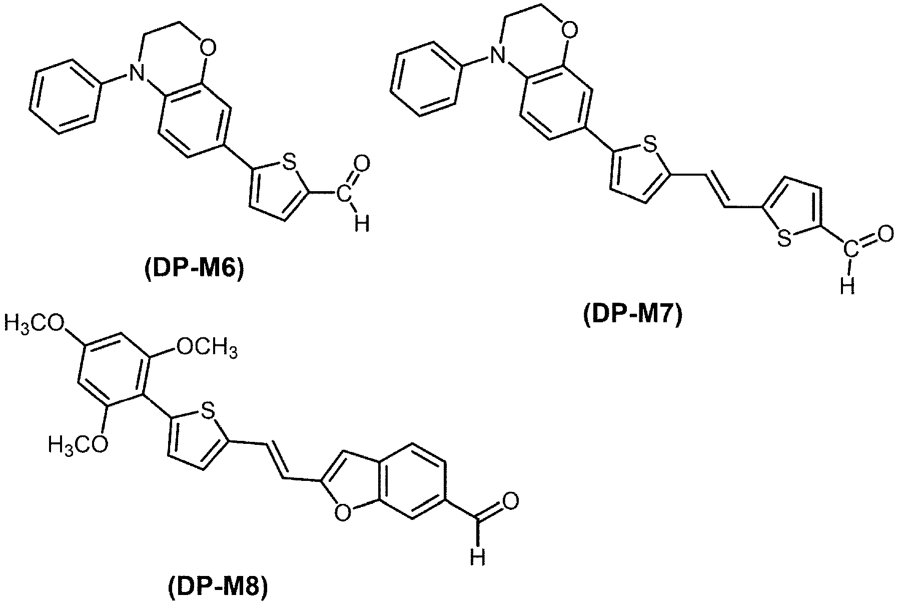

43. Use of the precursor according to any one of claims 39 to 42.

44. A method for producing the compound according to claim 1 using a precursor represented by a formula (DP-XM11), (DP-XM21), (DP-XM31), or (DP-XM41) below, 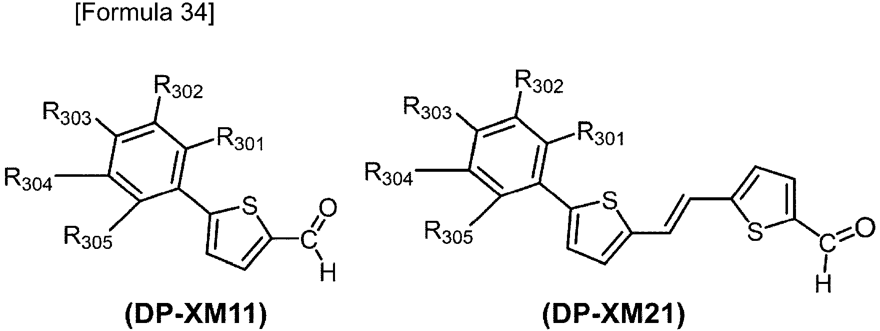 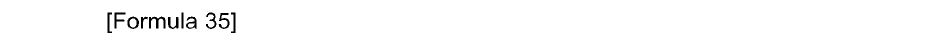 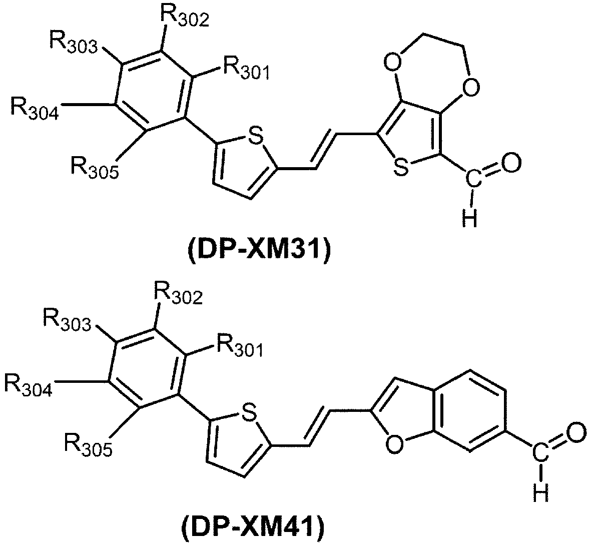 where, in the formulae (DP-XM11), (DP-XM21), (DP-XM31), and (DP-XM41):
at least one combination of adjacent two or more of R₃₀₁, R₃₀₂, R₃₀₃, R₃₀₄, and R₃₀₅ are mutually bonded to form a substituted or unsubstituted monocyclic ring, mutually bonded to form a substituted or unsubstituted fused ring, or not mutually bonded;
R₃₀₁, R₃₀₂, R₃₀₃, R₃₀₄, and R₃₀₅ forming neither the substituted or unsubstituted monocyclic ring nor the substituted or unsubstituted fused ring are each independently a hydrogen atom, a substituted or unsubstituted alkyl group, a substituted or unsubstituted cycloalkyl group, a substituted or unsubstituted aryl group, a substituted or unsubstituted heterocyclic group, a group represented by - N(R₁₀₁)(R₁₀₂), or a group represented by -O(R₁₁₃);
R₁₀₁, R₁₀₂, and R₁₁₃ are each independently a hydrogen atom, a substituted or unsubstituted alkyl group, a substituted or unsubstituted cycloalkyl group, a substituted or unsubstituted aryl group, or a substituted or unsubstituted heterocyclic group;
when a plurality of R₁₀₁ are present, the plurality of R₁₀₁ are mutually the same or different;
when a plurality of R₁₀₂ are present, the plurality of R₁₀₂ are mutually the same or different; and
when a plurality of R₁₁₃ are present, the plurality of R₁₁₃ are mutually the same or different.

45. An electro-optic material comprising: an electro-optic compound and a host material, wherein the electro-optic compound is the compound according to any one of claims 1 to 36.

46. The electro-optic material according to claim 45, wherein
the host material is a polymer, and
the electro-optic compound is bonded to the polymer as the host material.

47. The electro-optic material according to claim 45, wherein
the host material is a polymer, and
the electro-optic compound is not bonded to the polymer and is dispersed in the host material.

48. An electro-optic film comprising the compound according to any one of claims 1 to 36.

49. An electro-optic film comprising the electro-optic material according to any one of claims 45 to 47.

50. An electro-optic device comprising the compound according to any one of claims 1 to 36.

51. An electro-optic device comprising the electro-optic material according to any one of claims 45 to 47.

52. An electro-optic device comprising the electro-optic film according to claim 48 or 49.
